# EUROPEAN PATENT APPLICATION

(11) **EP 4 067 500 A1**
(43) Date of publication of application: **05.10.2022**
(21) Application number: 21166744.9
(22) Date of filing: 01.04.2021
(51) Int. Cl.: C12P 19/26, C12N 9/04, C12N 9/10, C12N 1/16, A61K 31/726

(54) **HEPAROSAN-PRODUCING RECOMBINANT CELLS**

(71) Applicant: Givaudan SA, 1214 Vernier (CH)
(72) Inventor: MERKAMM, Muriel, 91400 ORSAY (FR); LOUIS, Dominique, 91400 ORSAY (FR); JAILLARDON, Karine, 91400 Orsay (FR); THOMAS, Dominique, 95100 Argenteuil (FR)
(74) Representative: Nony

(57) **Abstract**

The present invention relates to the field of bio-production of heparosan.

There is a need in the art for heparosan production methods allowing its highly efficient synthesis and secretion.

The solution proposed in the present invention is the use of a genetically modified cell comprising many modifications as described in the present text.

The present invention further proposes methods allowing the bio-production of heparosan having a controlled molecular weight using the genetically modified cells of the invention.

SEQUENCE LISTING

<110> GIVAUDAN SA

<120> HEPAROSAN-PRODUCING RECOMBINANT CELLS

<130> PR88538/MGG

<160> 69

<170> BiSSAP 1.3.6

<210> 1
<211> 1854
<212> DNA
<213> Pasteurella multocida


<220>
<223> reencoded nucleic acid sequence of heparosan synthase originating
      from Pasteurella multocida (HSS1)

<400> 1
   atgagtttgt tcaaaagggc gactgagctg ttcaaatcgg gtaactataa ggatgcactg      60
   acattgtacg agaacatagc caaaatttat ggctcagaat ccctagtgaa gtataacata     120
   gatatctgca agaagaacat aacccaatca aaatcgaaca agatagaaga ggacaatatt     180
   tccggagaga ataagttctc cgtctctatc aaagacttat acaacgaaat tagtaattct     240
   gagctaggga taacaaagga gagattagga gcaccaccct tggtaagcat aattatgacg     300
   agtcataata cagagaaatt catcgaagct tccatcaaca gtttattatt gcaaacttat     360
   aacaatcttg aggtcattgt ggtagacgac tactccacag acaagacctt ccaaatcgcg     420
   tccaggattg caaactcgac gtcaaaagtt aagactttca gattgaattc caaccttgga     480
   acgtacttcg caaagaatac gggaatccta aaatcaaaag gggacattat attctttcaa     540
   gattctgatg atgtctgcca tcatgaacgt attgagagat gtgtaaatgc acttttgtct     600
   aacaaagata acattgccgt taggtgcgct tattctcgta ttaatctgga gacccagaac     660
   atcataaagg tcaacgacaa caagtacaag ttaggtctta ttaccctagg agtataccgt     720
   aaagtattta atgaaatagg attcttcaac tgtaccacta aggcaagtga cgacgaattt     780
   tatcacagga tcatcaaata ctatggcaag aataggataa acaatttgtt cctacctcta     840
   tactacaata ccatgcgtga agatagtttg tttagcgata tggtcgagtg ggtagacgaa     900
   aacaacatta aacagaaaac aagtgatgca cgtcagaatt atctgcatga attccaaaag     960
   atccacaacg aaaggaaatt gaatgagtta aaggagattt tcagcttccc caggattcac    1020
   gatgccctac cgataagtaa agagatgagc aagttgtcta acccaaagat ccctgtctac    1080
   attaacattt gcagtatacc atctaggata aagcaattac agtatacgat cggagtgctg    1140
   aagaatcagt gtgaccattt ccacatttac ttagatggtt atccagaggt tccagatttc    1200
   ataaagaagc taggcaacaa ggctaccgta atcaattgcc aaaacaagaa cgagtctatc 1260
   agggacaacg gcaagtttat ccttttagaa aagctgatca aagagaataa ggacggctat 1320
   tatattacct gtgatgatga cataaggtat cctgctgact acacgaatac catgataaag 1380
   aagattaaca agtacaatga caaagcggca ataggtctac acggcgtcat ctttccgtcc 1440
   agagtgaata aatacttctc atctgacagg attgtttata actttcagaa gcccttggaa 1500
   aacgacacag ccgtcaacat tttgggtact gggaccgttg catttagagt ctccatcttc 1560
   aacaagtttt ccctgagcga cttcgagcac cctggtatgg tggatattta tttctcaata 1620
   ctatgcaaga agaacaacat attgcaagtt tgtataagca ggccctcaaa ctggttaacc 1680
   gaggacaaca agaataccga aaccctgttc cacgagttcc aaaaccgtga cgagatccag 1740
   agtaagttga tcatctctaa taatccgtgg ggctacagta gcatctaccc cttacttaac 1800
   aacaatgcca actatagcga gttgatacca tgcttatcat tctataatga gtaa 1854

<210> 2
<211> 1956
<212> DNA
<213> Pasteurella multocida

<220>
<223> reencoded nucleic acid sequence of heparosan synthase originating
      from Pasteurella multocida (HSS2)

<400> 2
atgaagggca agaaagagat gacccaaata cagattgcta agaacccgcc ccaacacgag 60

aaggaaaacg agctgaatac tttccaaaac aagatcgact cccttaaaac gacattaaac 120

aaggacatta tatcacaaca gacattgttg gctaaacagg actcaaagca cccattatct 180

gcaagtcttg agaacgagaa caaacttctt cttaaacagt tgcagcttgt tttgcaggaa 240

tttgaaaaga tatacactta caaccaagca ctagaggcta agcttgagaa agacaaacag 300

accacctcaa taaccgattt atataacgaa gttgcaaagt ctgatctagg gctagttaag 360

gaaactaaca gtgctaaccc tctagtctcc attattatga cttcgcacaa taccgctcaa 420

tttatcgagg catcgattaa ctccttgttg ctacagacat ataagaacat tgagataatt 480

atagtcgatg atgacagttc cgacaacact tttgaaatcg cctcaaggat cgcaaacaca 540

acgtctaagg tccgtgtgtt tcgtttgaac tccaatctgg gcacgtactt tgctaagaat 600

acaggaatcc ttaagagtaa aggcgacata atattcttcc aggactccga cgacgtttgt 660

catcatgaga gaatcgaaag gtgcgtgaac attttgttgg ctaacaaaga gacgattgct 720

gtgaggtgcg catacagtag gctggctcct gaaacgcagc atataattaa agtcaacaac 780

atggattata ggcttggttt catcacactg ggaatgcata ggaaggtttt ccaagagatt 840

gggttcttca actgtacaac aaaaggcagt gacgacgagt tcttccaccg tatagctaaa 900

tactacggta aagaaaagat taagaacctt ttattgccgc tttattataa cacgatgcgt 960

gaaaactcac ttttcactga catggtcgag tggatcgaca accataatat catacagaag 1020

atgtctgaca cacgtcaaca ctatgctact ctgtttcagg cgatgcataa tgagactgca 1080

tcacacgact tcaagaactt gttccagttc ccacgtatat acgacgcctt gcctgttcca 1140

caggagatgt ctaagttatc taaccctaaa ataccggtgt acataaatat atgcagtata 1200

ccatctagga ttgcacagtt acgtaggatt attgggatct taaagaacca gtgcgaccac 1260

tttcacatat acctagacgg atacgtggag atccctgatt ttattaagaa tcttggtaat 1320

aaagcaacgg tcgtccactg caaagacaag gacaactcta ttagggacaa cggcaagttt 1380

attctactgg aagagttaat agaaaagaat caagacgggt actacataac gtgcgacgac 1440

gacattatct atccatcgga ctatataaac acaatgatta agaagctgaa cgagtatgat 1500

gacaaggcgg tcatagggct tcacggaata ttatttccgt caaggatgac gaaatacttt 1560

tccgccgata ggcttgttta ttcgttttac aaacccctag agaaagacaa agcagtaaat 1620

gttcttggga ccggaactgt ctcattcaga gttagtctgt tcaaccaatt ctccctttca 1680

gacttcaccc attcgggaat ggccgacatc tacttctctc tattgtgcaa gaagaacaac 1740

attttacaaa tctgtatctc cagaccggca aattggttga cggaagataa cagagattct 1800

gagaccttat accaccagta tagagataat gacgagcagc agactcagtt gatcatggaa 1860

aatgggccct ggggttactc aagtatctat ccacttgtca agaatcaccc gaagtttacg 1920

gacttaatac catgtttgcc tttctacttc ctataa 1956

<210> 3
<211> 617
<212> PRT
<213> Pasteurella multocida

<220>
<223> amino acid sequence of heparosan synthase originating from
Pasteurella multocida (HSS1)

<400> 3
Met Ser Leu Phe Lys Arg Ala Thr Glu Leu Phe Lys Ser Gly Asn Tyr
1               5                   10                  15
Lys Asp Ala Leu Thr Leu Tyr Glu Asn Ile Ala Lys Ile Tyr Gly Ser
            20                  25                  30
Glu Ser Leu Val Lys Tyr Asn Ile Asp Ile Cys Lys Lys Asn Ile Thr
        35                  40                  45
Gln Ser Lys Ser Asn Lys Ile Glu Glu Asp Asn Ile Ser Gly Glu Asn
    50                  55                  60
Lys Phe Ser Val Ser Ile Lys Asp Leu Tyr Asn Glu Ile Ser Asn Ser
65                  70                  75                  80
Glu Leu Gly Ile Thr Lys Glu Arg Leu Gly Ala Pro Pro Leu Val Ser
                85                  90                  95
Ile Ile Met Thr Ser His Asn Thr Glu Lys Phe Ile Glu Ala Ser Ile
            100                 105                 110

Asn Ser Leu Leu Leu Gln Thr Tyr Asn Asn Leu Glu Val Ile Val Val
        115                 120                 125
Asp Asp Tyr Ser Thr Asp Lys Thr Phe Gln Ile Ala Ser Arg Ile Ala
    130                 135                 140
Asn Ser Thr Ser Lys Val Lys Thr Phe Arg Leu Asn Ser Asn Leu Gly
145                 150                 155                 160
Thr Tyr Phe Ala Lys Asn Thr Gly Ile Leu Lys Ser Lys Gly Asp Ile
                165                 170                 175
Ile Phe Phe Gln Asp Ser Asp Asp Val Cys His His Glu Arg Ile Glu
            180                 185                 190
Arg Cys Val Asn Ala Leu Leu Ser Asn Lys Asp Asn Ile Ala Val Arg
        195                 200                 205
Cys Ala Tyr Ser Arg Ile Asn Leu Glu Thr Gln Asn Ile Ile Lys Val
    210                 215                 220
Asn Asp Asn Lys Tyr Lys Leu Gly Leu Ile Thr Leu Gly Val Tyr Arg
225                 230                 235                 240
Lys Val Phe Asn Glu Ile Gly Phe Phe Asn Cys Thr Thr Lys Ala Ser
                245                 250                 255
Asp Asp Glu Phe Tyr His Arg Ile Ile Lys Tyr Tyr Gly Lys Asn Arg
            260                 265                 270
Ile Asn Asn Leu Phe Leu Pro Leu Tyr Tyr Asn Thr Met Arg Glu Asp
        275                 280                 285
Ser Leu Phe Ser Asp Met Val Glu Trp Val Asp Glu Asn Asn Ile Lys
    290                 295                 300
Gln Lys Thr Ser Asp Ala Arg Gln Asn Tyr Leu His Glu Phe Gln Lys
305                 310                 315                 320
Ile His Asn Glu Arg Lys Leu Asn Glu Leu Lys Glu Ile Phe Ser Phe
                325                 330                 335
Pro Arg Ile His Asp Ala Leu Pro Ile Ser Lys Glu Met Ser Lys Leu
            340                 345                 350
Ser Asn Pro Lys Ile Pro Val Tyr Ile Asn Ile Cys Ser Ile Pro Ser
        355                 360                 365
Arg Ile Lys Gln Leu Gln Tyr Thr Ile Gly Val Leu Lys Asn Gln Cys
    370                 375                 380
Asp His Phe His Ile Tyr Leu Asp Gly Tyr Pro Glu Val Pro Asp Phe
385                 390                 395                 400
Ile Lys Lys Leu Gly Asn Lys Ala Thr Val Ile Asn Cys Gln Asn Lys
                405                 410                 415
Asn Glu Ser Ile Arg Asp Asn Gly Lys Phe Ile Leu Leu Glu Lys Leu
            420                 425                 430
Ile Lys Glu Asn Lys Asp Gly Tyr Tyr Ile Thr Cys Asp Asp Asp Ile
        435                 440                 445
Arg Tyr Pro Ala Asp Tyr Thr Asn Thr Met Ile Lys Lys Ile Asn Lys
    450                 455                 460
Tyr Asn Asp Lys Ala Ala Ile Gly Leu His Gly Val Ile Phe Pro Ser
465                 470                 475                 480
Arg Val Asn Lys Tyr Phe Ser Ser Asp Arg Ile Val Tyr Asn Phe Gln
                485                 490                 495
Lys Pro Leu Glu Asn Asp Thr Ala Val Asn Ile Leu Gly Thr Gly Thr
            500                 505                 510
Val Ala Phe Arg Val Ser Ile Phe Asn Lys Phe Ser Leu Ser Asp Phe
        515                 520                 525
Glu His Pro Gly Met Val Asp Ile Tyr Phe Ser Ile Leu Cys Lys Lys
    530                 535                 540
Asn Asn Ile Leu Gln Val Cys Ile Ser Arg Pro Ser Asn Trp Leu Thr
545                 550                 555                 560
Glu Asp Asn Lys Asn Thr Glu Thr Leu Phe His Glu Phe Gln Asn Arg
                565                 570                 575
Asp Glu Ile Gln Ser Lys Leu Ile Ile Ser Asn Asn Pro Trp Gly Tyr
            580                 585                 590
Ser Ser Ile Tyr Pro Leu Leu Asn Asn Asn Ala Asn Tyr Ser Glu Leu
        595                 600                 605
Ile Pro Cys Leu Ser Phe Tyr Asn Glu

610                 615

<210> 4
<211> 652
<212> PRT
<213> Pasteurella multocida

<220>
<223> amino acid sequence of heparosan synthase originating from
      Pasteurella multocida (HSS2)

<400> 4
Met Ala Lys Gly Lys Lys Glu Met Thr Gln Ile Gln Ile Ala Lys Asn
1               5                   10                  15
Pro Pro Gln His Glu Lys Glu Asn Glu Leu Asn Thr Phe Gln Asn Lys
            20                  25                  30
Ile Asp Ser Leu Lys Thr Thr Leu Asn Lys Asp Ile Ile Ser Gln Gln
        35                  40                  45
Thr Leu Leu Ala Lys Gln Asp Ser Lys His Pro Leu Ser Ala Ser Leu
    50                  55                  60
Glu Asn Glu Asn Lys Leu Leu Leu Lys Gln Leu Gln Leu Val Leu Gln
65                  70                  75                  80
Glu Phe Glu Lys Ile Tyr Thr Tyr Asn Gln Ala Leu Glu Ala Lys Leu
                85                  90                  95
Glu Lys Asp Lys Gln Thr Thr Ser Ile Thr Asp Leu Tyr Asn Glu Val
            100                 105                 110
Ala Lys Ser Asp Leu Gly Leu Val Lys Glu Thr Asn Ser Ala Asn Pro
        115                 120                 125
Leu Val Ser Ile Ile Met Thr Ser His Asn Thr Ala Gln Phe Ile Glu
    130                 135                 140
Ala Ser Ile Asn Ser Leu Leu Leu Gln Thr Tyr Lys Asn Ile Glu Ile
145                 150                 155                 160
Ile Ile Val Asp Asp Asp Ser Ser Asp Asn Thr Phe Glu Ile Ala Ser
                165                 170                 175
Arg Ile Ala Asn Thr Thr Ser Lys Val Arg Val Phe Arg Leu Asn Ser
            180                 185                 190
Asn Leu Gly Thr Tyr Phe Ala Lys Asn Thr Gly Ile Leu Lys Ser Lys
        195                 200                 205
Gly Asp Ile Ile Phe Phe Gln Asp Ser Asp Asp Val Cys His His Glu
    210                 215                 220
Arg Ile Glu Arg Cys Val Asn Ile Leu Leu Ala Asn Lys Glu Thr Ile
225                 230                 235                 240
Ala Val Arg Cys Ala Tyr Ser Arg Leu Ala Pro Glu Thr Gln His Ile
                245                 250                 255
Ile Lys Val Asn Asn Met Asp Tyr Arg Leu Gly Phe Ile Thr Leu Gly
            260                 265                 270
Met His Arg Lys Val Phe Gln Glu Ile Gly Phe Phe Asn Cys Thr Thr
        275                 280                 285
Lys Gly Ser Asp Asp Glu Phe Phe His Arg Ile Ala Lys Tyr Tyr Gly
    290                 295                 300
Lys Glu Lys Ile Lys Asn Leu Leu Leu Pro Leu Tyr Tyr Asn Thr Met
305                 310                 315                 320
Arg Glu Asn Ser Leu Phe Thr Asp Met Val Glu Trp Ile Asp Asn His
                325                 330                 335
Asn Ile Ile Gln Lys Met Ser Asp Thr Arg Gln His Tyr Ala Thr Leu
            340                 345                 350
Phe Gln Ala Met His Asn Glu Thr Ala Ser His Asp Phe Lys Asn Leu
        355                 360                 365
Phe Gln Phe Pro Arg Ile Tyr Asp Ala Leu Pro Val Pro Gln Glu Met
    370                 375                 380
Ser Lys Leu Ser Asn Pro Lys Ile Pro Val Tyr Ile Asn Ile Cys Ser
385                 390                 395                 400

Ile Pro Ser Arg Ile Ala Gln Leu Arg Arg Ile Ile Gly Ile Leu Lys
                405                 410                 415
Asn Gln Cys Asp His Phe His Ile Tyr Leu Asp Gly Tyr Val Glu Ile
                420                 425                 430
Pro Asp Phe Ile Lys Asn Leu Gly Asn Lys Ala Thr Val Val His Cys
        435                 440                 445
Lys Asp Lys Asp Asn Ser Ile Arg Asp Asn Gly Lys Phe Ile Leu Leu
    450                 455                 460
Glu Glu Leu Ile Glu Lys Asn Gln Asp Gly Tyr Tyr Ile Thr Cys Asp
465                 470                 475                 480
Asp Asp Ile Ile Tyr Pro Ser Asp Tyr Ile Asn Thr Met Ile Lys Lys
                485                 490                 495
Leu Asn Glu Tyr Asp Asp Lys Ala Val Ile Gly Leu His Gly Ile Leu
                500                 505                 510
Phe Pro Ser Arg Met Thr Lys Tyr Phe Ser Ala Asp Arg Leu Val Tyr
        515                 520                 525
Ser Phe Tyr Lys Pro Leu Glu Lys Asp Lys Ala Val Asn Val Leu Gly
    530                 535                 540
Thr Gly Thr Val Ser Phe Arg Val Ser Leu Phe Asn Gln Phe Ser Leu
545                 550                 555                 560
Ser Asp Phe Thr His Ser Gly Met Ala Asp Ile Tyr Phe Ser Leu Leu
                565                 570                 575
Cys Lys Lys Asn Asn Ile Leu Gln Ile Cys Ile Ser Arg Pro Ala Asn
                580                 585                 590
Trp Leu Thr Glu Asp Asn Arg Asp Ser Glu Thr Leu Tyr His Gln Tyr
        595                 600                 605
Arg Asp Asn Asp Glu Gln Gln Thr Gln Leu Ile Met Glu Asn Gly Pro
    610                 615                 620
Trp Gly Tyr Ser Ser Ile Tyr Pro Leu Val Lys Asn His Pro Lys Phe
625                 630                 635                 640
Thr Asp Leu Ile Pro Cys Leu Pro Phe Tyr Phe Leu
                645                 650

<210> 5
<211> 1446
<212> DNA
<213> Arabidopsis thaliana


<220>
<223> nucleic acid sequence of UDP-Glucose dehydrogenase (HASB)
      originating from Arabidopsis thaliana

<400> 5
atggtcaaaa tatgctgtat cggggctgga tatgtcggtg ggcctacaat ggccgttatg      60

gccctaaagt gtccagagat tgaagtcgtg gtagttgata tatcggagcc acgaattaac     120

gcatggaact cagaccgtct accaatttat gagccagggt tagaggatgt cgtcaaacag     180

tgtagaggga agaatttgtt cttctctaca gatgtagaga agcatgtatt tgagtcagat     240

atagtgtttg tttcggtaaa cactcctacg aaaacgcagg gtctgggtgc aggtaaagca     300

gcagatttga catattggga gtccgctgct cgcatgatag ctgatgtgag caaatcgtcg     360

aaaatcgtcg ttgaaaagag tacagtacca gttcgtacag ccgaggctat agaaaagatt     420

ttaacgcaca attcgaaggg tatcgaattc cagatcttat caaatccaga attcttggcc     480

gaagggacgg cgattaaaga cttatataac cctgatagag ttctaatcgg tggcagggac     540

accgctgcgg gacaaaaggc cattaaggcg ttgcgtgacg tgtatgccca ctgggttcct     600

gttgagcaaa taatctgtac taatttatgg agtgccgagc tatcaaagtt ggctgcgaac 660

gcatttctag ctcaaaggat aagttcagta aacgcaatgt cagcgctttg tgaggcaact 720

ggtgctgacg taacccaagt cgctcacgcc gttggaaccg acactagaat tggaccgaag 780

tttcttaacg catccgtagg cttcggcgga tcttgctttc agaaagacat cctgaatctt 840

atttacatct gcgaatgcaa tggtcttcca gaagcagcca attattggaa acaggtagtc 900

aaggtaaatg actaccaaaa gattaggttt gctaatcgag tcgtatcttc tatgttcaac 960

accgtctccg gtaagaaaat tgctattttg ggatttgcgt tcaagaagga caccggcgac 1020

acgcgtgaaa ctcctgccat agatgtgtgt aatcgcctcg tggctgataa agcaaagctg 1080

tcgatctatg atccgcaagt attagaagag cagatccgcc gtgatctgtc catggcccga 1140

ttcgattggg accatccagt cccactccag cagatcaaag ctgaaggtat ctccgaacag 1200

gttaacgttg tgtccgacgc ctacgaggct acgaaggatg ctcatggttt atgtgtttta 1260

accgaatggg acgaattcaa gtcacttgat tttaagaaga tctttgataa tatgcagaaa 1320

cccgctttcg ttttcgacgg aagaaacgtg gtcgacgctg tgaaattgag agaaattgga 1380

ttcatagtat attccatagg taaacctctg gatagttggc tcaaggatat gccggctgtt 1440

gcataa 1446

<210> 6
<211> 1166
<212> DNA
<213> Chlorella virus

<220>
<223> reencoded nucleic acid sequence of UDP-Glucose dehydrogenase
      (HASB) originating from Chlorella virus PBCV-1

<400> 6
atgagtagaa ttgctgtcgt tggatgcggt tacgtgggta cggcctgcgc cgtacttttg 60

gcgcagaaga acgaagttat cgttttggat atctcggagg accgggtaca actgattaag 120

aataagaagt cacctataga agataaggaa atcgaagaat tcctggagac gaaagatttg 180

aatctaacag cgacgacgga taaggtgctc gcctatgaga atgctgaatt cgttataata 240

gctacaccga ccgattacga tgtcgtcact agatatttca acactaagtc tgttgaaaat 300

gtgataggcg atgtcattaa gaacactcag acacacccta ctatagtgat caagagtact 360

attccaatcg gtttcgtgga taaagttaga gaacagttcg attatcaaaa tattatcttc 420

tcgccggaat tcttgagaga aggacgagca ttatatgata atctttaccc ctcccgtatc 480

atcgtcggtg atgactcccc aattgcctta aaattcgcga atctcttggt cgagggtagc 540

aaaactccac tagctcccgt attaactatg ggtacgcgag aggccgaagc tgtaaaacta 600

ttttcaaata catatttggc tatgagagta gcatacttca atgagctaga cacatttgca 660

atgtcccatg gtatgaacgc aaaagagatt atcgacggag tcacactaga accaaggata 720

gggcagggtt attcgaatcc ttcatttggg tatggagcct attgtttccc aaaagataca 780

aagcaattgt tggctaattt tgaaggggtt ccacaagata ttattggcgc aatcgtagag 840

tctaacgaaa ctagaaagga ggttattgtt tctgaggtcg agaacagatt tccgaccaca 900

gttggcgtat ataagttggc tgctaaagct ggttcggata atttcaggtc tagtgcgata 960

gtcgatatca tggaaaggtt ggctaataag ggctaccaca tcaaaatatt tgaacctacc 1020

gttgaacaat ttgagaattt tgaggtagat aataatctca caacttttgc aaccgagtct 1080

gatgtaataa ttgcgaacag agttccagtc gaacatcgca ttctgtttgg gaagaagtta 1140

ataacacggg atgtctatgg ggataa 1166

<210> 7
<211> 1170
<212> DNA
<213> Chlorella virus

<220>
<223> reencoded nucleic acid sequence of UDP-Glucose dehydrogenase
      (HASB-A) originating from Chlorella virus PBCV-1

<400> 7
atgtcacgaa tcgcagtagt tggctgtggg tacgtgggaa ccgcatgcgc tgtactgctg 60

gcgcaaaaga atgaggtaat cgttcttgat attagtgagg atagggtcca actaatcaag 120

aacaagaaat ccccgatcga agataaggag attgaagaat tcttggaaac aaaagaccta 180

aacttaactg caacaactga caaagtttta gcctacgaaa acgctgagtt tgtgattata 240

gcgacaccca cagattatga cgtagttacc agatacttta acacgaagtc cgtagagaac 300

gtcattggag atgttataaa gaatactcag actcatccta cgatagtaat aaagtcaacc 360

attcccatag gtttcgtaga taaggttagg gagcaattcg attaccagaa cattatattt 420

tcgccagaat ttctgagaga gggtcgcgcc ctgtatgata atctatatcc atcacggatt 480

atagtgggcg atgactctcc gattgcactt aagtttgcta atcttttagt tgagggctcc 540

aaaactccgc tcgccccagt acttacgatg ggtacacgtg aggctgaagc tgtcaagctg 600

ttttcaaaca cataccttgc tatgcgagtc gcatacttta acgaactaga tacctttgct 660

atgtcgcacg gtatgaatgc taaagaaatc atagatggcg taacgttgga gcctcggata 720

ggtcaaggat attccaatcc atcttttggc tacggtgcgt attgtttccc aaaggacacg 780

aagcaattat tagctaactt tgagggtgtt ccgcaagata taattggggc gatagtagaa 840

agcaatgaaa cacggaagga ggtaatcgtg agtgaagtgg aaaaccgatt ccccactacg 900

gtcggcgttt acaaattagc cgccaaggct ggttccgaca atttccgatc cagcgcaata 960

gtagatatta tggaaagatt agctaataag ggataccaca ttaaaatctt tgaacctact     1020

gtcgaacagt tcgagaactt cgaggttgat aataacttga cgactttcgc aacggagagc     1080

gatgtaatta ttgcaaaccg cgtacctgtg gaacatcgaa ttttgttcgg aaagaagctg     1140

attacacgcg atgtatatgg cgataactaa                                      1170


<210> 8
<211> 481
<212> PRT
<213> Arabidopsis thaliana


<220>
<223> amino acid sequence of UDP-Glucose dehydrogenase (HASB)
      originating from Arabidopsis thaliana

<400> 8
Met Val Lys Ile Cys Cys Ile Gly Ala Gly Tyr Val Gly Gly Pro Thr
1               5                   10                  15
Met Ala Val Met Ala Leu Lys Cys Pro Glu Ile Glu Val Val Val Val
            20                  25                  30
Asp Ile Ser Glu Pro Arg Ile Asn Ala Trp Asn Ser Asp Arg Leu Pro
        35                  40                  45
Ile Tyr Glu Pro Gly Leu Glu Asp Val Val Lys Gln Cys Arg Gly Lys
    50                  55                  60
Asn Leu Phe Phe Ser Thr Asp Val Glu Lys His Val Phe Glu Ser Asp
65                  70                  75                  80
Ile Val Phe Val Ser Val Asn Thr Pro Thr Lys Thr Gln Gly Leu Gly
                85                  90                  95
Ala Gly Lys Ala Ala Asp Leu Thr Tyr Trp Glu Ser Ala Ala Arg Met
            100                 105                 110
Ile Ala Asp Val Ser Lys Ser Ser Lys Ile Val Val Glu Lys Ser Thr
        115                 120                 125
Val Pro Val Arg Thr Ala Glu Ala Ile Glu Lys Ile Leu Thr His Asn
    130                 135                 140
Ser Lys Gly Ile Glu Phe Gln Ile Leu Ser Asn Pro Glu Phe Leu Ala
145                 150                 155                 160
Glu Gly Thr Ala Ile Lys Asp Leu Tyr Asn Pro Asp Arg Val Leu Ile
                165                 170                 175
Gly Gly Arg Asp Thr Ala Ala Gly Gln Lys Ala Ile Lys Ala Leu Arg
            180                 185                 190
Asp Val Tyr Ala His Trp Val Pro Val Glu Gln Ile Ile Cys Thr Asn
        195                 200                 205
Leu Trp Ser Ala Glu Leu Ser Lys Leu Ala Ala Asn Ala Phe Leu Ala
    210                 215                 220
Gln Arg Ile Ser Ser Val Asn Ala Met Ser Ala Leu Cys Glu Ala Thr
225                 230                 235                 240
Gly Ala Asp Val Thr Gln Val Ala His Ala Val Gly Thr Asp Thr Arg
                245                 250                 255
Ile Gly Pro Lys Phe Leu Asn Ala Ser Val Gly Phe Gly Gly Ser Cys
            260                 265                 270
Phe Gln Lys Asp Ile Leu Asn Leu Ile Tyr Ile Cys Glu Cys Asn Gly
        275                 280                 285
Leu Pro Glu Ala Ala Asn Tyr Trp Lys Gln Val Val Lys Val Asn Asp
    290                 295                 300
Tyr Gln Lys Ile Arg Phe Ala Asn Arg Val Val Ser Ser Met Phe Asn
305                 310                 315                 320
Thr Val Ser Gly Lys Lys Ile Ala Ile Leu Gly Phe Ala Phe Lys Lys
                325                 330                 335

Asp Thr Gly Asp Thr Arg Glu Thr Pro Ala Ile Asp Val Cys Asn Arg
            340                 345                 350
Leu Val Ala Asp Lys Ala Lys Leu Ser Ile Tyr Asp Pro Gln Val Leu
        355                 360                 365
Glu Glu Gln Ile Arg Arg Asp Leu Ser Met Ala Arg Phe Asp Trp Asp
    370                 375                 380
His Pro Val Pro Leu Gln Gln Ile Lys Ala Glu Gly Ile Ser Glu Gln
385                 390                 395                 400
Val Asn Val Val Ser Asp Ala Tyr Glu Ala Thr Lys Asp Ala His Gly
                405                 410                 415
Leu Cys Val Leu Thr Glu Trp Asp Glu Phe Lys Ser Leu Asp Phe Lys
                420                 425                 430
Lys Ile Phe Asp Asn Met Gln Lys Pro Ala Phe Val Phe Asp Gly Arg
        435                 440                 445
Asn Val Val Asp Ala Val Lys Leu Arg Glu Ile Gly Phe Ile Val Tyr
    450                 455                 460
Ser Ile Gly Lys Pro Leu Asp Ser Trp Leu Lys Asp Met Pro Ala Val
465                 470                 475                 480
Ala

<210> 9
<211> 389
<212> PRT
<213> Chlorella virus

<220>
<223> amino acid sequence of UDP-Glucose dehydrogenase (HASB)
      originating from Chlorella virus PBCV1

<400> 9
Met Ser Arg Ile Ala Val Val Gly Cys Gly Tyr Val Gly Thr Ala Cys
1               5                   10                  15
Ala Val Leu Leu Ala Gln Lys Asn Glu Val Ile Val Leu Asp Ile Ser
            20                  25                  30
Glu Asp Arg Val Gln Leu Ile Lys Asn Lys Lys Ser Pro Ile Glu Asp
        35                  40                  45
Lys Glu Ile Glu Glu Phe Leu Glu Thr Lys Asp Leu Asn Leu Thr Ala
    50                  55                  60
Thr Thr Asp Lys Val Leu Ala Tyr Glu Asn Ala Glu Phe Val Ile Ile
65                  70                  75                  80
Ala Thr Pro Thr Asp Tyr Asp Val Val Thr Arg Tyr Phe Asn Thr Lys
                85                  90                  95
Ser Val Glu Asn Val Ile Gly Asp Val Ile Lys Asn Thr Gln Thr His
            100                 105                 110
Pro Thr Ile Val Ile Lys Ser Thr Ile Pro Ile Gly Phe Val Asp Lys
        115                 120                 125
Val Arg Glu Gln Phe Asp Tyr Gln Asn Ile Ile Phe Ser Pro Glu Phe
    130                 135                 140
Leu Arg Glu Gly Arg Ala Leu Tyr Asp Asn Leu Tyr Pro Ser Arg Ile
145                 150                 155                 160
Ile Val Gly Asp Asp Ser Pro Ile Ala Leu Lys Phe Ala Asn Leu Leu
                165                 170                 175
Val Glu Gly Ser Lys Thr Pro Leu Ala Pro Val Leu Thr Met Gly Thr
            180                 185                 190
Arg Glu Ala Glu Ala Val Lys Leu Phe Ser Asn Thr Tyr Leu Ala Met
        195                 200                 205
Arg Val Ala Tyr Phe Asn Glu Leu Asp Thr Phe Ala Met Ser His Gly
    210                 215                 220
Met Asn Ala Lys Glu Ile Ile Asp Gly Val Thr Leu Glu Pro Arg Ile
225                 230                 235                 240
Gly Gln Gly Tyr Ser Asn Pro Ser Phe Gly Tyr Gly Ala Tyr Cys Phe

245                 250                 255
Pro Lys Asp Thr Lys Gln Leu Leu Ala Asn Phe Glu Gly Val Pro Gln
            260                 265                 270
Asp Ile Ile Gly Ala Ile Val Glu Ser Asn Glu Thr Arg Lys Glu Val
        275                 280                 285
Ile Val Ser Glu Val Glu Asn Arg Phe Pro Thr Thr Val Gly Val Tyr
    290                 295                 300
Lys Leu Ala Ala Lys Ala Gly Ser Asp Asn Phe Arg Ser Ser Ala Ile
305                 310                 315                 320
Val Asp Ile Met Glu Arg Leu Ala Asn Lys Gly Tyr His Ile Lys Ile
                325                 330                 335
Phe Glu Pro Thr Val Glu Gln Phe Glu Asn Phe Glu Val Asp Asn Asn
            340                 345                 350
Leu Thr Thr Phe Ala Thr Glu Ser Asp Val Ile Ile Ala Asn Arg Val
        355                 360                 365
Pro Val Glu His Arg Ile Leu Phe Gly Lys Lys Leu Ile Thr Arg Asp
    370                 375                 380
Val Tyr Gly Asp Asn
385

<210> 10
<211> 1968
<212> DNA
<213> Pedobacter heparinus

<220>
<223> reencoded nucleic acid sequence of
      glucuronosyl-disulfoglucosamine glucuronidase originating from
      Pedobacter heparinus with a N-terminal secretion signal

<400> 10
atgcaattct cgacggtcgc aagtgttgcc tttgttgctt tggctaactt cgttgccgca      60

cagtcctcgt caatcaccag gaaggacttc gatcatatca acttggaata ctccggtttg     120

gagaaggtga acaaggcggt agcagcaggc aattacgacg acgcggcaaa agccctacta     180

gcttactacc gtgaaaagag caaagcaaga gagcctgact ttagtaatgc cgagaagcct     240

gccgacatac gtcagcctat tgacaaggta acacgtgaga tggcggataa agcattagta     300

catcagtttc aacctcacaa aggatacgga tattttgatt atgggaagga tattaattgg     360

cagatgtggc cagttaaaga caacgaggtc agatggcagt tgcatagggt aaaatggtgg     420

caggcaatgg ccttggttta ccatgccacg ggcgacgaga agtacgccag ggagtgggtg     480

taccaatact cggactgggc gaggaagaac ccgttaggct tatcgcaaga caatgataag     540

tttgtctgga gacctttgga agtctcagat agagtccagt cattgccacc taccttttct     600

cttttcgtca acagtcccgc cttcacacca gcattcctaa tggagttctt aaactcgtac     660

caccaacagg cagactactt gtcaacacac tacgccgagc aggggaacca taggctgttc     720

gaagctcaga ggaacttgtt cgccggcgta tccttccccg aattcaagga ctcacccagg     780

tggagacaga ctggaatatc tgttctgaat acagagataa agaagcaggt gtacgccgat     840

ggcatgcaat tcgagttaag tccgatatac cacgttgctg ccatagatat ctttcttaaa     900

gcatacggaa gcgccaagag agttaatttg gagaaagagt tccctcaatc ctatgtgcaa     960

accgtcgaga atatgattat ggcgttaatt tcaataagtc taccggacta taatacgccc 1020

atgttcggcg actcttggat cactgataag aatttcagaa tggctcaatt tgcctcatgg 1080

gcgcgtgtct tccccgctaa ccaggcaatt aagtactttg caaccgacgg aaaacagggt 1140

aaagctccca acttcttgag taaggcattg tccaatgcag gattctacac gtttagaagt 1200

ggatgggata agaacgcgac agtgatggta ctgaaggcaa gtccgcctgg tgagtttcac 1260

gctcagcctg ataacggaac ttttgaactg ttcatcaagg gtaggaactt cacacccgac 1320

gccggtgtat tcgtctattc cggtgacgag gcgattatga aattgaggaa ctggtacagg 1380

cagacaagga ttcactctac tctaactctt gataatcaga atatggtcat aacgaaagca 1440

cgtcagaata aatgggagac aggcaataac ttggatgtct taacttacac taacccgtca 1500

taccccaact tagatcacca acgttcagtt ctattcatta acaagaagta tttcttagtc 1560

atagataggg ctatcggcga ggccacgggc aaccttggcg tgcactggca gctaaaggaa 1620

gacagtaacc ctgtcttcga taaaaccaag aatagagtct atactaccta tagggacggg 1680

aacaatctaa tgatacagag tctaaacgca gacaggacat cactgaacga ggaagaaggg 1740

aaggtatctt atgtctataa caaggagctg aagcgtccag ctttcgtatt tgagaagcct 1800

aagaagaacg cagggaccca aaactttgtg tcaattgtct atccatacga cggtcagaag 1860

gcgccggaaa tttctattag agaaaataag ggaaatgact tcgaaaaggg aaaattgaac 1920

ctaacactga ctataaacgg taagcaacag cttgtgttgg ttccttaa 1968

<210> 11
<211> 2178
<212> DNA
<213> Pedobacter heparinus

<220>
<223> reencoded nucleic acid sequence of
      glucuronosyl-disulfoglucosamine glucuronidase originating from
      Pedobacter heparinus with a N-terminal secretion signal and a
      C-terminal anchoring signal

<400> 11
atgcaattct cgacggtcgc aagtgttgcc tttgttgctt tggctaactt cgttgccgca 60

cagtcctcgt caatcaccag gaaggacttc gatcatatca acttggaata ctccggtttg 120

gagaaggtga acaaggcggt agcagcaggc aattacgacg acgcggcaaa agccctacta 180

gcttactacc gtgaaaagag caaagcaaga gagcctgact ttagtaatgc cgagaagcct 240

gccgacatac gtcagcctat tgacaaggta acacgtgaga tggcggataa agcattagta 300

catcagtttc aacctcacaa aggatacgga tattttgatt atggaaagga tattaattgg 360

cagatgtggc cagttaaaga caacgaggtc agatggcagt tgcatagggt aaaatggtgg 420

caggcaatgg ccttggttta ccatgccacg ggcgacgaga agtacgccag ggagtgggtg 480

taccaatact cggactgggc gaggaagaac ccgttaggct tatcgcaaga caatgataag 540

tttgtctgga gacctttgga agtctcagat agagtccagt cattgccacc taccttttct 600

cttttcgtca acagtcccgc cttcacacca gcattcctaa tggagttctt aaactcgtac 660

caccaacagg cagactactt gtcaacacac tacgccgagc aggggaacca taggctgttc 720

gaagctcaga ggaacttgtt cgccggcgta tccttccccg aattcaagga ctcacccagg 780

tggagacaga ctggaatatc tgttctgaat acagagataa agaagcaggt gtacgccgat 840

ggcatgcaat tcgagttaag tccgatatac cacgttgctg ccatagatat ctttcttaaa 900

gcatacggaa gcgccaagag agttaatttg gagaaagagt tccctcaatc ctatgtgcaa 960

accgtcgaga atatgattat ggcgttaatt tcaataagtc taccggacta taatacgccc 1020

atgttcggcg actcttggat cactgataag aatttcagaa tggctcaatt tgcctcatgg 1080

gcgcgtgtct tccccgctaa ccaggcaatt aagtactttg caaccgacgg aaaacagggt 1140

aaagctccca acttcttgag taaggcattg tccaatgcag gattctacac gtttagaagt 1200

ggatgggata agaacgcgac agtgatggta ctgaaggcaa gtccgcctgg tgagtttcac 1260

gctcagcctg ataacggaac ttttgaactg ttcatcaagg gtaggaactt cacacccgac 1320

gccggtgtat tcgtctattc cggtgacgag gcgattatga aattgaggaa ctggtacagg 1380

cagacaagga ttcactctac tctaactctt gataatcaga atatggtcat aacgaaagca 1440

cgtcagaata aatgggagac aggcaataac ttggatgtct taacttacac taacccgtca 1500

taccccaact tagatcacca acgttcagtt ctattcatta acaagaagta tttcttagtc 1560

atagataggg ctatcggcga ggccacgggc aaccttggcg tgcactggca gctaaaggaa 1620

gacagtaacc ctgtcttcga taaaaccaag aatagagtct atactaccta tagggacggg 1680

aacaatctaa tgatacagag tctaaacgca gacaggacat cactgaacga ggaagaaggg 1740

aaggtatctt atgtctataa caaggagctg aagcgtccag ctttcgtatt tgagaagcct 1800

aagaagaacg cagggaccca aaactttgtg tcaattgtct atccatacga cggtcagaag 1860

gcgccggaaa tttctattag agaaaataag ggaaatgact tcgaaaaggg aaaattgaac 1920

ctaacactga ctataaacgg taagcaacag cttgtgttgg ttcctggatc cgccatttct 1980

caaatcactg acggtcaaat ccaagctact accactgcta ccaccgaagc taccaccact 2040

gctgccccat cttccaccgt tgaaactgtt tctccatcca gcaccgaaac tatctctcaa 2100

caaactgaaa atggtgctgc taaggccgct gtcggtatgg gtgccggtgc tctagctgct 2160

gctgctatgt tgttataa 2178

<210> 12
<211> 1968

<212> DNA
<213> Pedobacter heparinus

<220>
<223> reencoded nucleic acid sequence of
      glucuronosyl-disulfoglucosamine glucuronidase originating from
      Pedobacter heparinus with a N-terminal secretion sigal

<400> 12
atgcagtttt caaccgtggc atctgtggca ttcgtggcat tggcaaattt tgtcgctgct      60

caatcttcca gtattacgcg taaagatttt gaccacataa atcttgagta tagtggccta     120

gagaaagtca ataaagctgt cgccgctggt aactatgatg atgcagccaa ggctttgtta     180

gcctattata gagagaaatc taaggctcgt gaaccggatt tctctaacgc tgaaaagcca     240

gcggatatca gacaaccaat cgataaagtc accagggaaa tggcagacaa ggctttggtt     300

caccaattcc agccccataa gggctatggt tacttcgact acggtaaaga cataaactgg     360

caaatgtggc ccgtaaagga taatgaagta aggtggcaac ttcacagagt gaagtggtgg     420

caagctatgg ctctggtgta tcacgcaacc ggtgatgaaa agtatgctag agaatgggta     480

tatcagtatt ctgattgggc taggaagaat ccactaggac tttctcagga taacgacaaa     540

ttcgtttggc gtccactaga ggtatctgac agggtacaat ctttaccccc gacgttcagt     600

ctgtttgtta attctccagc atttaccccg gctttcttga tggaattctt gaattcttat     660

catcagcaag cggattattt aagcacccat tatgcagaac aaggaaatca cagattattt     720

gaggcccaac gtaatttatt tgcgggtgtt agttttccag agtttaaaga ttcgccaaga     780

tggagacaaa ccggtatcag cgtgttaaac actgaaatca agaaacaagt atatgctgac     840

ggtatgcagt ttgaactatc tccaatttat catgtcgcag caattgacat attcttgaag     900

gcttatggca gtgctaaacg tgtcaactta gaaaaggaat ttccacagtc atacgtccag     960

actgtagaaa acatgatcat ggcccttatc agcatctcat tgcctgatta caacactcca    1020

atgtttggtg atagttggat aacggacaag aactttagga tggcacagtt cgcaagctgg    1080

gctagagtat ttccagcaaa tcaagctata aaatatttcg cgactgatgg taagcaaggg    1140

aaggccccta attttctatc taaagcttta agcaacgccg gcttttatac tttcaggtcc    1200

ggttgggaca agaatgcaac tgtcatggtg ttaaaagcgt ctccacccgg agaattccat    1260

gcgcaaccag acaatgggac attcgagttg tttattaaag ggagaaattt taccccagat    1320

gctggagtgt ttgtgtactc aggggatgaa gcgataatga agttaagaaa ttggtatcgt    1380

caaaccagaa tacattccac acttacactg gacaaccaaa acatggtgat tactaaggct    1440

agacaaaaca agtgggaaac tggtaacaat ctggacgttt tgacatatac gaacccttcc    1500

tatccaaatc ttgaccatca gaggtcggtc ttatttataa ataagaaata cttcttggtt    1560

atcgacagag ccattggtga agcaacaggt aatctgggtg ttcattggca attgaaagag    1620

gattctaatc cagtgtttga caagacgaag aacagggtat acacaacata ccgtgatggt 1680

aataacttaa tgatccagtc cttaaatgct gatcgtactt ctcttaatga agaggaaggt 1740

aaggtttcct acgtgtacaa taaagaacta aagagacccg cctttgtctt cgaaaagccc 1800

aagaagaatg ccggaacgca gaatttcgta tctatcgttt acccttatga tggccagaaa 1860

gctccagaga taagcatccg tgagaacaaa ggtaacgatt ttgagaaagg taagcttaat 1920

ttgacgctaa cgattaatgg gaaacagcaa ttagtcttag taccataa 1968

<210> 13
<211> 1968
<212> DNA
<213> Pedobacter heparinus

<220>
<223> reencoded nucleic acid sequence of
      glucuronosyl-disulfoglucosamine glucuronidase originating from
      Pedobacter heparinus with a N-terminal secretion signal and a
      C-terminal anchoring signal

<400> 13
atgcagtttt caaccgtggc atctgtggca ttcgtggcat tggcaaattt tgtcgctgct 60

caatcttcca gtattacgcg taaagatttt gaccacataa atcttgagta tagtggccta 120

gagaaagtca ataaagctgt cgccgctggt aactatgatg atgcagccaa ggctttgtta 180

gcctattata gagagaaatc taaggctcgt gaaccggatt tctctaacgc tgaaaagcca 240

gcggatatca gacaaccaat cgataaagtc accagggaaa tggcagacaa ggctttggtt 300

caccaattcc agccccataa gggctatggt tacttcgact acggtaaaga cataaactgg 360

caaatgtggc ccgtaaagga taatgaagta aggtggcaac ttcacagagt gaagtggtgg 420

caagctatgg ctctggtgta tcacgcaacc ggtgatgaaa agtatgctag agaatgggta 480

tatcagtatt ctgattgggc taggaagaat ccactaggac tttctcagga taacgacaaa 540

ttcgtttggc gtccactaga ggtatctgac agggtacaat ctttaccccc gacgttcagt 600

ctgtttgtta attctccagc atttaccccg gctttcttga tggaattctt gaattcttat 660

catcagcaag cggattattt aagcacccat tatgcagaac aaggaaatca cagattattt 720

gaggcccaac gtaatttatt tgcgggtgtt agttttccag agtttaaaga ttcgccaaga 780

tggagacaaa ccggtatcag cgtgttaaac actgaaatca agaaacaagt atatgctgac 840

ggtatgcagt ttgaactatc tccaatttat catgtcgcag caattgacat attcttgaag 900

gcttatggca gtgctaaacg tgtcaactta gaaaaggaat ttccacagtc atacgtccag 960

actgtagaaa acatgatcat ggcccttatc agcatctcat tgcctgatta caacactcca 1020

atgtttggtg atagttggat aacggacaag aactttagga tggcacagtt cgcaagctgg 1080

gctagagtat ttccagcaaa tcaagctata aaatatttcg cgactgatgg taagcaaggg 1140

aaggccccta attttctatc taaagcttta agcaacgccg gcttttatac tttcaggtcc 1200

ggttgggaca agaatgcaac tgtcatggtg ttaaaagcgt ctccacccgg agaattccat 1260

gcgcaaccag acaatgggac attcgagttg tttattaaag ggagaaattt taccccagat 1320

gctggagtgt ttgtgtactc aggggatgaa gcgataatga agttaagaaa ttggtatcgt 1380

caaaccagaa tacattccac acttacactg gacaaccaaa acatggtgat tactaaggct 1440

agacaaaaca agtgggaaac tggtaacaat ctggacgttt tgacatatac gaacccttcc 1500

tatccaaatc ttgaccatca gaggtcggtc ttatttataa ataagaaata cttcttggtt 1560

atcgacagag ccattggtga agcaacaggt aatctgggtg ttcattggca attgaaagag 1620

gattctaatc cagtgtttga caagacgaag aacagggtat acacaacata ccgtgatggt 1680

aataacttaa tgatccagtc cttaaatgct gatcgtactt ctcttaatga agaggaaggt 1740

aaggtttcct acgtgtacaa taaagaacta aagagacccg cctttgtctt cgaaaagccc 1800

aagaagaatg ccggaacgca gaatttcgta tctatcgttt acccttatga tggccagaaa 1860

gctccagaga taagcatccg tgagaacaaa ggtaacgatt ttgagaaagg taagcttaat 1920

ttgacgctaa cgattaatgg gaaacagcaa ttagtcttag taccataa 1968

<210> 14
<211> 655
<212> PRT
<213> Pedobacter heparinus

<220>
<223> amino acid sequence of glucuronosyl-disulfoglucosamine
      glucuronidase originating from Pedobacter heparinus with a
      N-terminal secretion signal

<400> 14
Met Gln Phe Ser Thr Val Ala Ser Val Ala Phe Val Ala Leu Ala Asn
1               5                   10                  15
Phe Val Ala Ala Gln Ser Ser Ser Ile Thr Arg Lys Asp Phe Asp His
            20                  25                  30
Ile Asn Leu Glu Tyr Ser Gly Leu Glu Lys Val Asn Lys Ala Val Ala
        35                  40                  45
Ala Gly Asn Tyr Asp Asp Ala Ala Lys Ala Leu Leu Ala Tyr Tyr Arg
    50                  55                  60
Glu Lys Ser Lys Ala Arg Glu Pro Asp Phe Ser Asn Ala Glu Lys Pro
65                  70                  75                  80
Ala Asp Ile Arg Gln Pro Ile Asp Lys Val Thr Arg Glu Met Ala Asp
                85                  90                  95
Lys Ala Leu Val His Gln Phe Gln Pro His Lys Gly Tyr Gly Tyr Phe
            100                 105                 110
Asp Tyr Gly Lys Asp Ile Asn Trp Gln Met Trp Pro Val Lys Asp Asn
        115                 120                 125
Glu Val Arg Trp Gln Leu His Arg Val Lys Trp Trp Gln Ala Met Ala
    130                 135                 140
Leu Val Tyr His Ala Thr Gly Asp Glu Lys Tyr Ala Arg Glu Trp Val
145                 150                 155                 160

Tyr Gln Tyr Ser Asp Trp Ala Arg Lys Asn Pro Leu Gly Leu Ser Gln
                165                 170                 175
Asp Asn Asp Lys Phe Val Trp Arg Pro Leu Glu Val Ser Asp Arg Val
            180                 185                 190
Gln Ser Leu Pro Pro Thr Phe Ser Leu Phe Val Asn Ser Pro Ala Phe
        195                 200                 205
Thr Pro Ala Phe Leu Met Glu Phe Leu Asn Ser Tyr His Gln Gln Ala
    210                 215                 220
Asp Tyr Leu Ser Thr His Tyr Ala Glu Gln Gly Asn His Arg Leu Phe
225                 230                 235                 240
Glu Ala Gln Arg Asn Leu Phe Ala Gly Val Ser Phe Pro Glu Phe Lys
                245                 250                 255
Asp Ser Pro Arg Trp Arg Gln Thr Gly Ile Ser Val Leu Asn Thr Glu
            260                 265                 270
Ile Lys Lys Gln Val Tyr Ala Asp Gly Met Gln Phe Glu Leu Ser Pro
        275                 280                 285
Ile Tyr His Val Ala Ala Ile Asp Ile Phe Leu Lys Ala Tyr Gly Ser
    290                 295                 300
Ala Lys Arg Val Asn Leu Glu Lys Glu Phe Pro Gln Ser Tyr Val Gln
305                 310                 315                 320
Thr Val Glu Asn Met Ile Met Ala Leu Ile Ser Ile Ser Leu Pro Asp
                325                 330                 335
Tyr Asn Thr Pro Met Phe Gly Asp Ser Trp Ile Thr Asp Lys Asn Phe
            340                 345                 350
Arg Met Ala Gln Phe Ala Ser Trp Ala Arg Val Phe Pro Ala Asn Gln
        355                 360                 365
Ala Ile Lys Tyr Phe Ala Thr Asp Gly Lys Gln Gly Lys Ala Pro Asn
    370                 375                 380
Phe Leu Ser Lys Ala Leu Ser Asn Ala Gly Phe Tyr Thr Phe Arg Ser
385                 390                 395                 400
Gly Trp Asp Lys Asn Ala Thr Val Met Val Leu Lys Ala Ser Pro Pro
                405                 410                 415
Gly Glu Phe His Ala Gln Pro Asp Asn Gly Thr Phe Glu Leu Phe Ile
            420                 425                 430
Lys Gly Arg Asn Phe Thr Pro Asp Ala Gly Val Phe Val Tyr Ser Gly
        435                 440                 445
Asp Glu Ala Ile Met Lys Leu Arg Asn Trp Tyr Arg Gln Thr Arg Ile
    450                 455                 460
His Ser Thr Leu Thr Leu Asp Asn Gln Asn Met Val Ile Thr Lys Ala
465                 470                 475                 480
Arg Gln Asn Lys Trp Glu Thr Gly Asn Asn Leu Asp Val Leu Thr Tyr
                485                 490                 495
Thr Asn Pro Ser Tyr Pro Asn Leu Asp His Gln Arg Ser Val Leu Phe
            500                 505                 510
Ile Asn Lys Lys Tyr Phe Leu Val Ile Asp Arg Ala Ile Gly Glu Ala
        515                 520                 525
Thr Gly Asn Leu Gly Val His Trp Gln Leu Lys Glu Asp Ser Asn Pro
    530                 535                 540
Val Phe Asp Lys Thr Lys Asn Arg Val Tyr Thr Thr Tyr Arg Asp Gly
545                 550                 555                 560
Asn Asn Leu Met Ile Gln Ser Leu Asn Ala Asp Arg Thr Ser Leu Asn
                565                 570                 575
Glu Glu Glu Gly Lys Val Ser Tyr Val Tyr Asn Lys Glu Leu Lys Arg
            580                 585                 590
Pro Ala Phe Val Phe Glu Lys Pro Lys Lys Asn Ala Gly Thr Gln Asn
        595                 600                 605
Phe Val Ser Ile Val Tyr Pro Tyr Asp Gly Gln Lys Ala Pro Glu Ile
    610                 615                 620
Ser Ile Arg Glu Asn Lys Gly Asn Asp Phe Glu Lys Gly Lys Leu Asn
625                 630                 635                 640
Leu Thr Leu Thr Ile Asn Gly Lys Gln Gln Leu Val Leu Val Pro
                645                 650                 655

<210> 15
<211> 725
<212> PRT
<213> Pedobacter heparinus

<220>
<223> amino acid sequence of glucuronosyl-disulfoglucosamine
      glucuronidase originating from Pedobacter heparinus with a
      N-terminal secretion signal and a C-terminal anchoring signal

<400> 15
Met Gln Phe Ser Thr Val Ala Ser Val Ala Phe Val Ala Leu Ala Asn
1               5                   10                  15
Phe Val Ala Ala Gln Ser Ser Ser Ile Thr Arg Lys Asp Phe Asp His
            20                  25                  30
Ile Asn Leu Glu Tyr Ser Gly Leu Glu Lys Val Asn Lys Ala Val Ala
        35                  40                  45
Ala Gly Asn Tyr Asp Asp Ala Ala Lys Ala Leu Leu Ala Tyr Tyr Arg
    50                  55                  60
Glu Lys Ser Lys Ala Arg Glu Pro Asp Phe Ser Asn Ala Glu Lys Pro
65                  70                  75                  80
Ala Asp Ile Arg Gln Pro Ile Asp Lys Val Thr Arg Glu Met Ala Asp
                85                  90                  95
Lys Ala Leu Val His Gln Phe Gln Pro His Lys Gly Tyr Gly Tyr Phe
            100                 105                 110
Asp Tyr Gly Lys Asp Ile Asn Trp Gln Met Trp Pro Val Lys Asp Asn
        115                 120                 125
Glu Val Arg Trp Gln Leu His Arg Val Lys Trp Trp Gln Ala Met Ala
    130                 135                 140
Leu Val Tyr His Ala Thr Gly Asp Glu Lys Tyr Ala Arg Glu Trp Val
145                 150                 155                 160
Tyr Gln Tyr Ser Asp Trp Ala Arg Lys Asn Pro Leu Gly Leu Ser Gln
                165                 170                 175
Asp Asn Asp Lys Phe Val Trp Arg Pro Leu Glu Val Ser Asp Arg Val
            180                 185                 190
Gln Ser Leu Pro Pro Thr Phe Ser Leu Phe Val Asn Ser Pro Ala Phe
        195                 200                 205
Thr Pro Ala Phe Leu Met Glu Phe Leu Asn Ser Tyr His Gln Gln Ala
    210                 215                 220
Asp Tyr Leu Ser Thr His Tyr Ala Glu Gln Gly Asn His Arg Leu Phe
225                 230                 235                 240
Glu Ala Gln Arg Asn Leu Phe Ala Gly Val Ser Phe Pro Glu Phe Lys
                245                 250                 255
Asp Ser Pro Arg Trp Arg Gln Thr Gly Ile Ser Val Leu Asn Thr Glu
            260                 265                 270
Ile Lys Lys Gln Val Tyr Ala Asp Gly Met Gln Phe Glu Leu Ser Pro
        275                 280                 285
Ile Tyr His Val Ala Ala Ile Asp Ile Phe Leu Lys Ala Tyr Gly Ser
    290                 295                 300
Ala Lys Arg Val Asn Leu Glu Lys Glu Phe Pro Gln Ser Tyr Val Gln
305                 310                 315                 320
Thr Val Glu Asn Met Ile Met Ala Leu Ile Ser Ile Ser Leu Pro Asp
                325                 330                 335
Tyr Asn Thr Pro Met Phe Gly Asp Ser Trp Ile Thr Asp Lys Asn Phe
            340                 345                 350
Arg Met Ala Gln Phe Ala Ser Trp Ala Arg Val Phe Pro Ala Asn Gln
        355                 360                 365
Ala Ile Lys Tyr Phe Ala Thr Asp Gly Lys Gln Gly Lys Ala Pro Asn
    370                 375                 380
Phe Leu Ser Lys Ala Leu Ser Asn Ala Gly Phe Tyr Thr Phe Arg Ser
385                 390                 395                 400
Gly Trp Asp Lys Asn Ala Thr Val Met Val Leu Lys Ala Ser Pro Pro

405                 410                 415
Gly Glu Phe His Ala Gln Pro Asp Asn Gly Thr Phe Glu Leu Phe Ile
            420                 425                 430
Lys Gly Arg Asn Phe Thr Pro Asp Ala Gly Val Phe Val Tyr Ser Gly
        435                 440                 445
Asp Glu Ala Ile Met Lys Leu Arg Asn Trp Tyr Arg Gln Thr Arg Ile
    450                 455                 460
His Ser Thr Leu Thr Leu Asp Asn Gln Asn Met Val Ile Thr Lys Ala
465                 470                 475                 480
Arg Gln Asn Lys Trp Glu Thr Gly Asn Asn Leu Asp Val Leu Thr Tyr
                485                 490                 495
Thr Asn Pro Ser Tyr Pro Asn Leu Asp His Gln Arg Ser Val Leu Phe
            500                 505                 510
Ile Asn Lys Lys Tyr Phe Leu Val Ile Asp Arg Ala Ile Gly Glu Ala
        515                 520                 525
Thr Gly Asn Leu Gly Val His Trp Gln Leu Lys Glu Asp Ser Asn Pro
    530                 535                 540
Val Phe Asp Lys Thr Lys Asn Arg Val Tyr Thr Thr Tyr Arg Asp Gly
545                 550                 555                 560
Asn Asn Leu Met Ile Gln Ser Leu Asn Ala Asp Arg Thr Ser Leu Asn
                565                 570                 575
Glu Glu Glu Gly Lys Val Ser Tyr Val Tyr Asn Lys Glu Leu Lys Arg
            580                 585                 590
Pro Ala Phe Val Phe Glu Lys Pro Lys Lys Asn Ala Gly Thr Gln Asn
        595                 600                 605
Phe Val Ser Ile Val Tyr Pro Tyr Asp Gly Gln Lys Ala Pro Glu Ile
    610                 615                 620
Ser Ile Arg Glu Asn Lys Gly Asn Asp Phe Glu Lys Gly Lys Leu Asn
625                 630                 635                 640
Leu Thr Leu Thr Ile Asn Gly Lys Gln Gln Leu Val Leu Val Pro Gly
                645                 650                 655
Ser Ala Ile Ser Gln Ile Thr Asp Gly Gln Ile Gln Ala Thr Thr Thr
            660                 665                 670
Ala Thr Thr Glu Ala Thr Thr Thr Ala Ala Pro Ser Ser Thr Val Glu
        675                 680                 685
Thr Val Ser Pro Ser Ser Thr Glu Thr Ile Ser Gln Gln Thr Glu Asn
    690                 695                 700
Gly Ala Ala Lys Ala Ala Val Gly Met Gly Ala Gly Ala Leu Ala Ala
705                 710                 715                 720
Ala Ala Met Leu Leu
                725

<210> 16
<211> 2154
<212> DNA
<213> Saccharomyces cerevisiae


<220>
<223> nucleic acid sequence of glutamine-fructose-6-phosphate
      amidotransferase (GFA1) originating from Saccharomyces cerevisiae

<400> 16
atgtgtggta tctttggtta ctgcaattat ctagtggaaa gatccagagg agaaattatc      60

gacaccttag tggatggttt acaaagatta gaatatagag gctatgattc caccggtatt     120

gctatcgatg gtgacgaagc tgattctact ttcatctata agcaaatcgg taaagtgagt     180

gctttgaaag aggagattac taagcaaaat ccgaacagag acgttacttt tgtctctcat     240

tgtggtattg cgcatactag atgggctact cacggtcgac cagaacaagt taactgtcac     300

cctcaaagat ctgacccaga agaccaattt gtggtcgttc ataatggtat catcacaaat 360

tttagagaac tgaagactct tttaattaac aaaggttata aattcgaaag tgataccgat 420

accgagtgta ttgctaaact atatttgcat ttatacaata caaatttaca aaatgggcat 480

gacttagatt tccacgaatt aaccaagcta gttcttttag aactagaagg ttcatacggg 540

ttattatgta aatcttgtca ctatcctaat gaggttatcg ccactagaaa agggtcccct 600

ttactgattg gtgtcaaatc tgaaaaaaaa ctaaaagtcg acttcgtgga tgtggaattt 660

cccgaagaaa acgctggtca accggaaatt ccattgaaat ctaacaacaa atcatttggc 720

ttgggcccaa agaaagctcg tgaatttgaa gctggttccc aaaatgccaa tttactacca 780

attgccgcca atgaatttaa cttgagacat tctcaatcca gggctttcct atcagaagat 840

ggatctccaa caccggtgga attttttgtt tcttcggatg cggcatctgt tgttaaacat 900

accaagaagg tgctattttt agaagatgac gatttggctc atatttacga tggtgagtta 960

catattcata gatctagaag agaagtaggc gcatcaatga caaggtccat tcaaacttta 1020

gagatggagt tagctcagat catgaagggc ccttacgacc attttatgca aaaggaaatc 1080

tatgagcaac cagaatctac tttcaatact atgagaggta gaatcgacta tgaaaataat 1140

aaagtgatat tgggtggttt aaaggcatgg ttaccagttg tcagaagagc acggagactg 1200

atcatgatcg catgcggtac ttcttatcat tcatgtttgg ctactcgtgc tatcttcgaa 1260

gaattatcag atatcccagt tagtgtggaa ttagcgtctg actttctgga cagaaaatgc 1320

cctgtcttca gagacgatgt atgcgtgttt gtttcacaaa gtggtgaaac tgcggatacc 1380

atgctggctc taaattattg tttagaaaga ggagccttaa ctgtcggaat tgttaacagt 1440

gttggttctt ctatctctcg tgtcacccac tgtggtgttc atattaacgc tggtcctgaa 1500

attggtgttg cctctacaaa agcttatact tcccagtata ttgccttagt gatgtttgct 1560

ctatcgctgt cagatgaccg tgtatcgaaa atagacagaa gaattgaaat cattcaaggc 1620

ttgaagttaa tcccgggcca aattaagcag gtattaaagc tggaaccaag aataaaaaag 1680

ctctgtgcga ctgaattaaa ggatcaaaaa tctctattgt tattgggtag aggttaccaa 1740

tttgctgctg ctctggaagg tgctttgaag atcaaagaaa tttcttatat gcattctgaa 1800

ggtgttttgg caggtgagtt gaagcacggt gtcttggcct tggtggacga aaacttgcca 1860

atcattgctt ttggtaccag agactctcta ttccctaaag tagtttcctc tattgagcaa 1920

gttactgtaa gaaaggccca tccaattatt atttgtaacg aaaatgatga agtgtgggcg 1980

caaaaatcta aatcaatcga cctgcaaacc ttagaagttc cacaaactgt tgattgttta 2040

caaggtctaa ttaatattat tccattacaa ctaatgtcat attggttggc tgttaataaa 2100

gggattgatg ttgattttcc aagaaacttg gctaaatctg ttaccgtcga ataa 2154

<210> 17
<211> 1788
<212> DNA
<213> Chlorella virus

<220>
<223> reencoded nucleic acid sequence of glutamine-fructose-6-phosphate
      amidotransferase (GFA1) originating from Chlorella virus 1
      (PBCV-1)

<400> 17
atgtgcggga tcttcggtgc tgtgtcgaac aataatagca tagaagtttc catcaagggt      60

atacagaagc tagagtaccg cgggtacgat tcgtgtggaa tagcctatac agacggaggc     120

gccattgaac ggatcaggtc aatagatggg atcgacgact taaggaagaa aacaataaca     180

gagtcttccc cggtagctat cgctcattca aggtggagta ccactgggat tccaagtgtt     240

gtgaacgccc acccgcacat ctctcggggc acgtctggat gcgagtcgcg cattgcagtc     300

gtacacaatg gcattattga aaattaccag cagatccgca agtacttgat aaatcttggg     360

tatactttcg attcacagac cgacacggaa gtcattgctc atctaataga ctcgcagtat     420

aacgggaaca tcctgcacac cgtccaaatg gcagttaagc acctcaaagg aagctacgcc     480

atagcagtca tgtgccataa ggagtccggg aagatcgtgg tggcaaaaca gaagagtccg     540

ttggtattgg gtataggaag cgacggagct tattatatcg catcggacgt attggctctt     600

cccactaaca aagtggtcta cataagtgac ggattctctg ctgagttaag tcctggatcg     660

atgactatct atgatctgga tggcaataag gtagaatacg aggtggaaga cgttgaaatg     720

gagcagacgt cgatgtcttt agataacttt gatcactaca tgatcaaaga gatcaatgaa     780

cagccgatat caattctcaa tactattaag aacaaaggat tctatgcaga gatatttggt     840

gatctagcac atgaaatatt tcagaagatc gacaatatat tgattctagc ctgcggaact     900

tcgtatcatg caggtctcgt cgggaagcaa tggatagaga ccatctcacg catcccggtg     960

gatgttcata ttgcaagcga gtatgagccg acaattccac gagccaatac acttgtgata    1020

acgattagtc aaagcggcga gactgcggat acaatcgcag cccttcaacg agcgcaaaac    1080

gcaggaatga tatacacttt gtgtatctgc aacagcccca agtcaactct agtgcgcgag    1140

tcgataatga aatacataac caaatgcggt tcagaagttt ctgttgccag tactaaagca    1200

tttacgtcgc aacttgtggt gttgtatatg cttgcgaacg ttcttgcgaa caaaactgat    1260

gatctgctag gggatcttcc tcaagctatc gagagagtaa tctgtcttac taatgacgaa    1320

atgaagcgtt gggcggatga aatttgtact gccaaaagtg ccatcttctt agggagagga    1380

ctgaacgcac ctgtagcatt tgagggcgcg ctaaagttga aggaaatctc atatatccat    1440

gccgagggtt tcctcggtgg agagttgaag catgggccgc tggctttgtt ggacgataag    1500

atccctgtaa tcgtgactgt tgctgaccac gcttatttgg atcacattaa ggctaacatc    1560

gacgaggtat tggcacgaaa cgttaccgta tacgcgatcg tcgatcagta tgttaatatt 1620

gaaccacagg agcgactgca tgtagtaaaa gtgccgtttg tgagtaaaga gttttctccc 1680

atcatccaca caattccgat gcaattgtta tcgtattatg tcgcgatcaa gctgggcaag 1740

aacgttgaca agccacgtaa cctggcgaaa agtgttacaa cattctaa 1788

<210> 18
<211> 1788
<212> DNA
<213> Chlorella virus

<220>
<223> reencoded nucleic acid sequence of glutamine-fructose-6-phosphate
      amidotransferase (GFA1) originating from Chlorella virus 1
      (PBCV-1)

<400> 18
atgtgtggca tctttggagc agtgtcaaac aacaactcta tcgaggtgtc aatcaagggt 60

attcagaagc tagaatatcg tgggtatgat tcgtgcggta ttgcgtatac agatgggggt 120

gcgattgagc gtatacgttc tattgacggc attgacgatc tgcgtaagaa aacaatcaca 180

gaatcatcac cagtggccat tgctcactcg cggtggagca ccactggaat tccatcagtg 240

gtgaacgcac atcctcatat ttctcgcgga accagtgggt gtgagtctcg tatcgcggta 300

gtccacaacg gtatcattga aaactatcag cagatccgaa aatatctcat caatcttggt 360

tatacgtttg atagtcaaac ggacacagag gtcattgcgc atttgattga ttctcagtac 420

aatgggaata tcttgcacac cgtccaaatg gctgtcaagc acctgaaggg ctcttatgcc 480

attgcagtta tgtgtcataa agagtctggt aaaatagtcg tggcgaaaca gaagtcaccc 540

ctcgtacttg gaatcggctc agatggtgct tactacatcg cttcggacgt gctggcgctg 600

ccgacaaata aagttgttta tatttcagac ggtttctccg cagaactatc tccagggagt 660

atgaccattt acgatcttga tggaaataaa gtagaatatg aagtagagga cgttgaaatg 720

gaacaaacta gtatgtctct cgataacttt gatcattaca tgattaagga aattaatgag 780

caaccaatca gtattctaaa cactataaaa aataaagggt tctatgcaga aatattcggt 840

gatttggctc atgaaatctt ccaaaaaata gacaacatcc tgatactggc ttgtggtaca 900

agttatcacg ccggtcttgt aggaaaacag tggatagaga ccatctctag aatccccgtg 960

gatgttcaca tcgcgagtga atacgaacct actattccga gagcgaacac attggtaatc 1020

actatttcac agtcgggtga aactgcggac acgatagcgg ctttgcaacg ggcccaaaac 1080

gccgggatga tttatacatt gtgtatttgc aattcaccaa agagcactct tgttcgtgag 1140

agcattatga agtacatcac gaaatgtggt tctgaggtgt cagtggcatc aacgaaggcg 1200

tttacttctc agctcgtagt actgtacatg ctggcaaacg tattggcaaa taaaaccgat 1260

gatttgctgg gagacctccc acaggcaata gaacgggtaa tttgtttgac aaatgacgaa 1320

atgaaacgat gggccgacga aatttgcact gcgaaatccg cgatcttcct gggaagagga 1380

ctaaacgcac cagttgcctt tgagggagcg ttgaagctca aagaaatctc ttacattcat 1440

gcagagggct tcctgggagg tgagttgaaa cacggccccc tcgcactcct tgatgacaag 1500

attcctgtta tcgtaaccgt agcagatcat gcttatttgg accatatcaa agcaaatatc 1560

gacgaagtgc ttgcgaggaa cgttacggta tacgccatag tagaccagta tgtgaacatc 1620

gagccccagg aacgccttca cgtcgtcaag gttccgtttg tatccaaaga attttctccg 1680

ataattcaca ctatcccgat gcaactgctt tcgtattacg tggcaattaa gcttggaaag 1740

aacgttgaca aaccaaggaa tcttgcaaaa tccgtgacta cctttttaa 1788

<210> 19
<211> 717
<212> PRT
<213> Saccharomyces cerevisiae

<220>
<223> amino acid sequence of glutamine-fructose-6-phosphate
      amidotransferase (GFA1) originating from Saccharomyces cerevisiae

<400> 19
Met Cys Gly Ile Phe Gly Tyr Cys Asn Tyr Leu Val Glu Arg Ser Arg
1               5                   10                  15
Gly Glu Ile Ile Asp Thr Leu Val Asp Gly Leu Gln Arg Leu Glu Tyr
            20                  25                  30
Arg Gly Tyr Asp Ser Thr Gly Ile Ala Ile Asp Gly Asp Glu Ala Asp
        35                  40                  45
Ser Thr Phe Ile Tyr Lys Gln Ile Gly Lys Val Ser Ala Leu Lys Glu
    50                  55                  60
Glu Ile Thr Lys Gln Asn Pro Asn Arg Asp Val Thr Phe Val Ser His
65                  70                  75                  80
Cys Gly Ile Ala His Thr Arg Trp Ala Thr His Gly Arg Pro Glu Gln
                85                  90                  95
Val Asn Cys His Pro Gln Arg Ser Asp Pro Glu Asp Gln Phe Val Val
            100                 105                 110
Val His Asn Gly Ile Ile Thr Asn Phe Arg Glu Leu Lys Thr Leu Leu
        115                 120                 125
Ile Asn Lys Gly Tyr Lys Phe Glu Ser Asp Thr Asp Thr Glu Cys Ile
    130                 135                 140
Ala Lys Leu Tyr Leu His Leu Tyr Asn Thr Asn Leu Gln Asn Gly His
145                 150                 155                 160
Asp Leu Asp Phe His Glu Leu Thr Lys Leu Val Leu Leu Glu Leu Glu
                165                 170                 175
Gly Ser Tyr Gly Leu Leu Cys Lys Ser Cys His Tyr Pro Asn Glu Val
            180                 185                 190
Ile Ala Thr Arg Lys Gly Ser Pro Leu Leu Ile Gly Val Lys Ser Glu
        195                 200                 205
Lys Lys Leu Lys Val Asp Phe Val Asp Val Glu Phe Pro Glu Glu Asn
    210                 215                 220
Ala Gly Gln Pro Glu Ile Pro Leu Lys Ser Asn Asn Lys Ser Phe Gly
225                 230                 235                 240
Leu Gly Pro Lys Lys Ala Arg Glu Phe Glu Ala Gly Ser Gln Asn Ala
                245                 250                 255

Asn Leu Leu Pro Ile Ala Ala Asn Glu Phe Asn Leu Arg His Ser Gln
            260                 265                 270
Ser Arg Ala Phe Leu Ser Glu Asp Gly Ser Pro Thr Pro Val Glu Phe
        275                 280                 285
Phe Val Ser Ser Asp Ala Ala Ser Val Val Lys His Thr Lys Lys Val
    290                 295                 300
Leu Phe Leu Glu Asp Asp Asp Leu Ala His Ile Tyr Asp Gly Glu Leu
305                 310                 315                 320
His Ile His Arg Ser Arg Arg Glu Val Gly Ala Ser Met Thr Arg Ser
                325                 330                 335
Ile Gln Thr Leu Glu Met Glu Leu Ala Gln Ile Met Lys Gly Pro Tyr
            340                 345                 350
Asp His Phe Met Gln Lys Glu Ile Tyr Glu Gln Pro Glu Ser Thr Phe
        355                 360                 365
Asn Thr Met Arg Gly Arg Ile Asp Tyr Glu Asn Asn Lys Val Ile Leu
    370                 375                 380
Gly Gly Leu Lys Ala Trp Leu Pro Val Val Arg Arg Ala Arg Arg Leu
385                 390                 395                 400
Ile Met Ile Ala Cys Gly Thr Ser Tyr His Ser Cys Leu Ala Thr Arg
                405                 410                 415
Ala Ile Phe Glu Glu Leu Ser Asp Ile Pro Val Ser Val Glu Leu Ala
            420                 425                 430
Ser Asp Phe Leu Asp Arg Lys Cys Pro Val Phe Arg Asp Asp Val Cys
        435                 440                 445
Val Phe Val Ser Gln Ser Gly Glu Thr Ala Asp Thr Met Leu Ala Leu
    450                 455                 460
Asn Tyr Cys Leu Glu Arg Gly Ala Leu Thr Val Gly Ile Val Asn Ser
465                 470                 475                 480
Val Gly Ser Ser Ile Ser Arg Val Thr His Cys Gly Val His Ile Asn
                485                 490                 495
Ala Gly Pro Glu Ile Gly Val Ala Ser Thr Lys Ala Tyr Thr Ser Gln
            500                 505                 510
Tyr Ile Ala Leu Val Met Phe Ala Leu Ser Leu Ser Asp Asp Arg Val
        515                 520                 525
Ser Lys Ile Asp Arg Arg Ile Glu Ile Ile Gln Gly Leu Lys Leu Ile
    530                 535                 540
Pro Gly Gln Ile Lys Gln Val Leu Lys Leu Glu Pro Arg Ile Lys Lys
545                 550                 555                 560
Leu Cys Ala Thr Glu Leu Lys Asp Gln Lys Ser Leu Leu Leu Leu Gly
                565                 570                 575
Arg Gly Tyr Gln Phe Ala Ala Ala Leu Glu Gly Ala Leu Lys Ile Lys
            580                 585                 590
Glu Ile Ser Tyr Met His Ser Glu Gly Val Leu Ala Gly Glu Leu Lys
        595                 600                 605
His Gly Val Leu Ala Leu Val Asp Glu Asn Leu Pro Ile Ile Ala Phe
    610                 615                 620
Gly Thr Arg Asp Ser Leu Phe Pro Lys Val Val Ser Ser Ile Glu Gln
625                 630                 635                 640
Val Thr Ala Arg Lys Gly His Pro Ile Ile Ile Cys Asn Glu Asn Asp
                645                 650                 655
Glu Val Trp Ala Gln Lys Ser Lys Ser Ile Asp Leu Gln Thr Leu Glu
            660                 665                 670
Val Pro Gln Thr Val Asp Cys Leu Gln Gly Leu Ile Asn Ile Ile Pro
        675                 680                 685
Leu Gln Leu Met Ser Tyr Trp Leu Ala Val Asn Lys Gly Ile Asp Val
    690                 695                 700
Asp Phe Pro Arg Asn Leu Ala Lys Ser Val Thr Val Glu
705                 710                 715

<210> 20
<211> 595
<212> PRT
<213> Chlorella virus

<220>
<223> amino acid sequence of glutamine-fructose-6-phosphate
      amidotransferase (GFA1) originating from Chlorella virus 1
      (PBCV-1)

<400> 20
Met Cys Gly Ile Phe Gly Ala Val Ser Asn Asn Asn Ser Ile Glu Val
1               5                   10                  15
Ser Ile Lys Gly Ile Gln Lys Leu Glu Tyr Arg Gly Tyr Asp Ser Cys
            20                  25                  30
Gly Ile Ala Tyr Thr Asp Gly Gly Ala Ile Glu Arg Ile Arg Ser Ile
        35                  40                  45
Asp Gly Ile Asp Asp Leu Arg Lys Lys Thr Ile Thr Glu Ser Ser Pro
    50                  55                  60
Val Ala Ile Ala His Ser Arg Trp Ser Thr Thr Gly Ile Pro Ser Val
65                  70                  75                  80
Val Asn Ala His Pro His Ile Ser Arg Gly Thr Ser Gly Cys Glu Ser
                85                  90                  95
Arg Ile Ala Val Val His Asn Gly Ile Ile Glu Asn Tyr Gln Gln Ile
            100                 105                 110
Arg Lys Tyr Leu Ile Asn Leu Gly Tyr Thr Phe Asp Ser Gln Thr Asp
        115                 120                 125
Thr Glu Val Ile Ala His Leu Ile Asp Ser Gln Tyr Asn Gly Asn Ile
    130                 135                 140
Leu His Thr Val Gln Met Ala Val Lys His Leu Lys Gly Ser Tyr Ala
145                 150                 155                 160
Ile Ala Val Met Cys His Lys Glu Ser Gly Lys Ile Val Val Ala Lys
                165                 170                 175
Gln Lys Ser Pro Leu Val Leu Gly Ile Gly Ser Asp Gly Ala Tyr Tyr
            180                 185                 190
Ile Ala Ser Asp Val Leu Ala Leu Pro Thr Asn Lys Val Val Tyr Ile
        195                 200                 205
Ser Asp Gly Phe Ser Ala Glu Leu Ser Pro Gly Ser Met Thr Ile Tyr
    210                 215                 220
Asp Leu Asp Gly Asn Lys Val Glu Tyr Glu Val Glu Asp Val Glu Met
225                 230                 235                 240
Glu Gln Thr Ser Met Ser Leu Asp Asn Phe Asp His Tyr Met Ile Lys
                245                 250                 255
Glu Ile Asn Glu Gln Pro Ile Ser Ile Leu Asn Thr Ile Lys Asn Lys
            260                 265                 270
Gly Phe Tyr Ala Glu Ile Phe Gly Asp Leu Ala His Glu Ile Phe Gln
        275                 280                 285
Lys Ile Asp Asn Ile Leu Ile Leu Ala Cys Gly Thr Ser Tyr His Ala
    290                 295                 300
Gly Leu Val Gly Lys Gln Trp Ile Glu Thr Ile Ser Arg Ile Pro Val
305                 310                 315                 320
Asp Val His Ile Ala Ser Glu Tyr Glu Pro Thr Ile Pro Arg Ala Asn
                325                 330                 335
Thr Leu Val Ile Thr Ile Ser Gln Ser Gly Glu Thr Ala Asp Thr Ile
            340                 345                 350
Ala Ala Leu Gln Arg Ala Gln Asn Ala Gly Met Ile Tyr Thr Leu Cys
        355                 360                 365
Ile Cys Asn Ser Pro Lys Ser Thr Leu Val Arg Glu Ser Ile Met Lys
    370                 375                 380
Tyr Ile Thr Lys Cys Gly Ser Glu Val Ser Val Ala Ser Thr Lys Ala
385                 390                 395                 400
Phe Thr Ser Gln Leu Val Val Leu Tyr Met Leu Ala Asn Val Leu Ala
                405                 410                 415
Asn Lys Thr Asp Asp Leu Leu Gly Asp Leu Pro Gln Ala Ile Glu Arg
            420                 425                 430
Val Ile Cys Leu Thr Asn Asp Glu Met Lys Arg Trp Ala Asp Glu Ile

435                 440                 445
Cys Thr Ala Lys Ser Ala Ile Phe Leu Gly Arg Gly Leu Asn Ala Pro
    450                 455                 460
Val Ala Phe Glu Gly Ala Leu Lys Leu Lys Glu Ile Ser Tyr Ile His
465                 470                 475                 480
Ala Glu Gly Phe Leu Gly Gly Glu Leu Lys His Gly Pro Leu Ala Leu
                485                 490                 495
Leu Asp Asp Lys Ile Pro Val Ile Val Thr Val Ala Asp His Ala Tyr
                500                 505                 510
Leu Asp His Ile Lys Ala Asn Ile Asp Glu Val Leu Ala Arg Asn Val
        515                 520                 525
Thr Val Tyr Ala Ile Val Asp Gln Tyr Val Asn Ile Glu Pro Gln Glu
    530                 535                 540
Arg Leu His Val Val Lys Val Pro Phe Val Ser Lys Glu Phe Ser Pro
545                 550                 555                 560
Ile Ile His Thr Ile Pro Met Gln Leu Leu Ser Tyr Tyr Val Ala Ile
                565                 570                 575
Lys Leu Gly Lys Asn Val Asp Lys Pro Arg Asn Leu Ala Lys Ser Val
                580                 585                 590
Thr Thr Phe
        595

<210> 21
<211> 1434
<212> DNA
<213> Saccharomyces cerevisiae

<220>
<223> nucleic acid sequence of UDP-N-acetylglucosamine
      pyrophosphorylase (QRI1) originating from Saccharomyces
      cerevisiae

<400> 21
atgactgaca caaaacagct attcattgaa gccggacaaa gtcaactttt ccacaattgg      60

gaaagcttgt ctcgcaaaga ccaagaagaa ttgctttcaa acctggagca aatatcttcc     120

aagaggtccc ctgcaaaact actggaagac tgtcaaaatg ctattaaatt ctcactagct     180

aactcttcta aggatactgg cgtcgaaatt tcaccattgc cccctacttc gtacgagtcg     240

cttattggca acagtaagaa agaaaatgaa tactggcgtt taggccttga agctattggc     300

aagggtgaag tcgcagtgat tttaatggct ggcggacaag gtacgcggtt aggatcctct     360

caaccaaagg gctgttacga cattggattg ccttctaaga aatctctttt tcaaattcaa     420

gctgaaaagt tgatcaggtt gcaagatatg gtaaaggaca aaaaggtaga aattccttgg     480

tatattatga catcaggccc cactagagcg gctactgagg catactttca agaacacaat     540

tattttggct tgaataaaga acaaattacg ttcttcaacc agggaaccct gcctgccttt     600

gatttaaccg ggaagcattt cctaatgaaa gacccagtaa acctatctca atcaccagat     660

ggaaatggtg gactctaccg tgccatcaag gaaaacaagt tgaacgaaga ctttgatagg     720

agaggaatca agcatgttta catgtactgt gtcgataatg tcctatctaa aatcgcagac     780

cctgtattta ttggttttgc catcaagcat ggcttcgaac tggccaccaa agccgttaga     840

aagagagatg cgcatgaatc agttgggtta attgctacta aaaacgagaa accatgtgtc     900

atagaatatt ctgaaatttc caatgaattg gctgaagcaa aggataaaga tggcttatta     960

aaactacgcg caggcaacat tgtaaatcat tattacctag tggatttact aaaacgtgat    1020

ttggatcagt ggtgtgagaa tatgccatat cacattgcga agaagaaaat tccagcttat    1080

gatagtgtta ccggcaagta cactaagcct accgaaccaa acggtataaa attagagcaa    1140

ttcatatttg atgtctttga cactgtacca ctgaacaagt ttgggtgctt agaagtagat    1200

agatgcaaag aattttcacc tttaaaaaac ggtcctggtt ctaagaacga taatcctgag    1260

accagcagac tagcatattt gaaactagga acctcgtggt tggaagatgc aggcgctatt    1320

gtaaaagatg gggtactagt cgaagtttcc agcaaattga gttatgcagg tgaaaatcta    1380

tcccagttca aaggtaaagt ctttgacaga agtggtatag tattagaaaa ataa          1434

<210> 22
<211> 477
<212> PRT
<213> Saccharomyces cerevisiae

<220>
<223> amino acid sequence of UDP-N-acetylglucosamine pyrophosphorylase
      (QRI1) originating from Saccharomyces cerevisiae

<400> 22
Met Thr Asp Thr Lys Gln Leu Phe Ile Glu Ala Gly Gln Ser Gln Leu
1               5                   10                  15
Phe His Asn Trp Glu Ser Leu Ser Arg Lys Asp Gln Glu Glu Leu Leu
            20                  25                  30
Ser Asn Leu Glu Gln Ile Ser Ser Lys Arg Ser Pro Ala Lys Leu Leu
        35                  40                  45
Glu Asp Cys Gln Asn Ala Ile Lys Phe Ser Leu Ala Asn Ser Ser Lys
    50                  55                  60
Asp Thr Gly Val Glu Ile Ser Pro Leu Pro Pro Thr Ser Tyr Glu Ser
65                  70                  75                  80
Leu Ile Gly Asn Ser Lys Lys Glu Asn Glu Tyr Trp Arg Leu Gly Leu
                85                  90                  95
Glu Ala Ile Gly Lys Gly Glu Val Ala Val Ile Leu Met Ala Gly Gly
            100                 105                 110
Gln Gly Thr Arg Leu Gly Ser Ser Gln Pro Lys Gly Cys Tyr Asp Ile
        115                 120                 125
Gly Leu Pro Ser Lys Lys Ser Leu Phe Gln Ile Gln Ala Glu Lys Leu
    130                 135                 140
Ile Arg Leu Gln Asp Met Val Lys Asp Lys Lys Val Glu Ile Pro Trp
145                 150                 155                 160
Tyr Ile Met Thr Ser Gly Pro Thr Arg Ala Ala Thr Glu Ala Tyr Phe
                165                 170                 175
Gln Glu His Asn Tyr Phe Gly Leu Asn Lys Glu Gln Ile Thr Phe Phe
            180                 185                 190
Asn Gln Gly Thr Leu Pro Ala Phe Asp Leu Thr Gly Lys His Phe Leu
        195                 200                 205
Met Lys Asp Pro Val Asn Leu Ser Gln Ser Pro Asp Gly Asn Gly Gly
    210                 215                 220
Leu Tyr Arg Ala Ile Lys Glu Asn Lys Leu Asn Glu Asp Phe Asp Arg
225                 230                 235                 240
Arg Gly Ile Lys His Val Tyr Met Tyr Cys Val Asp Asn Val Leu Ser
                245                 250                 255

Lys Ile Ala Asp Pro Val Phe Ile Gly Phe Ala Ile Lys His Gly Phe
            260                 265                 270
Glu Leu Ala Thr Lys Ala Val Arg Lys Arg Asp Ala His Glu Ser Val
        275                 280                 285
Gly Leu Ile Ala Thr Lys Asn Glu Lys Pro Cys Val Ile Glu Tyr Ser
    290                 295                 300
Glu Ile Ser Asn Glu Leu Ala Glu Ala Lys Asp Lys Asp Gly Leu Leu
305                 310                 315                 320
Lys Leu Arg Ala Gly Asn Ile Val Asn His Tyr Tyr Leu Val Asp Leu
                325                 330                 335
Leu Lys Arg Asp Leu Asp Gln Trp Cys Glu Asn Met Pro Tyr His Ile
            340                 345                 350
Ala Lys Lys Lys Ile Pro Ala Tyr Asp Ser Val Thr Gly Lys Tyr Thr
        355                 360                 365
Lys Pro Thr Glu Pro Asn Gly Ile Lys Leu Glu Gln Phe Ile Phe Asp
    370                 375                 380
Val Phe Asp Thr Val Pro Leu Asn Lys Phe Gly Cys Leu Glu Val Asp
385                 390                 395                 400
Arg Cys Lys Glu Phe Ser Pro Leu Lys Asn Gly Pro Gly Ser Lys Asn
                405                 410                 415
Asp Asn Pro Glu Thr Ser Arg Leu Ala Tyr Leu Lys Leu Gly Thr Ser
            420                 425                 430
Trp Leu Glu Asp Ala Gly Ala Ile Val Lys Asp Gly Val Leu Val Glu
        435                 440                 445
Val Ser Ser Lys Leu Ser Tyr Ala Gly Glu Asn Leu Ser Gln Phe Lys
    450                 455                 460
Gly Lys Val Phe Asp Arg Ser Gly Ile Val Leu Glu Lys
465                 470                 475

<210> 23
<211> 1713
<212> DNA
<213> Saccharomyces cerevisiae


<220>
<223> nucleic acid sequence of Phosphoglucomutase-1 (PGM1) originating
      from Saccharomyces cerevisiae

<400> 23
atgtcacttc taatagattc tgtaccaaca gttgcttata aggaccaaaa accgggtact      60

tcaggtttac gtaagaagac caaggttttc atggatgagc ctcattatac tgagaacttc     120

attcaagcaa caatgcaatc tatccctaat ggctcagagg gaaccacttt agttgttgga     180

ggagatggtc gtttctacaa cgatgttatc atgaacaaga ttgccgcagt aggtgctgca     240

aacggtgtca gaaagttagt cattggtcaa ggcggtttac tttcaacacc agctgcttct     300

catataatta gaacatacga ggaaaagtgt accggtggtg gtatcatatt aactgcctca     360

cacaacccag gcggtccaga gaatgattta ggtatcaagt ataatttacc taatggtggg     420

ccagctccag agagtgtcac taacgctatc tgggaagcgt ctaaaaaatt aactcactat     480

aaaattataa agaacttccc caagttgaat ttgaacaagc ttggtaaaaa ccaaaaatat     540

ggcccattgt tagtggacat aattgatcct gccaaagcat acgttcaatt tctgaaggaa     600

atttttgatt ttgacttaat taaaagcttc ttagcgaaac agcgcaaaga caaagggtgg      660

aagttgttgt ttgactcctt aaatggtatt acaggaccat atggtaaggc tatatttgtt     720

gatgaatttg gtttaccggc agaggaagtt cttcaaaatt ggcacccttt acctgatttc 780

ggcggtttac atcccgatcc gaatctaacc tatgcacgaa ctcttgttga cagggttgac 840

cgcgaaaaaa ttgcctttgg agcagcctcc gatggtgatg gtgataggaa tatgatttac 900

ggttatggcc ctgctttcgt ttcgccaggt gattctgttg ccattattgc cgaatatgca 960

cccgaaattc catacttcgc caaacaaggt atttatggct tggcacgttc atttcctaca 1020

tcctcagcca ttgatcgtgt tgcagcaaaa aagggattaa gatgttacga agttccaacc 1080

ggctggaaat tcttctgtgc cttatttgat gctaaaaagc tatcaatctg tggtgaagaa 1140

tccttcggta caggttccaa tcatatcaga gaaaaggacg gtctatgggc cattattgct 1200

tggttaaata tcttggctat ctaccatagg cgtaaccctg aaaaggaagc ttcgatcaaa 1260

actattcagg acgaattttg gaacgagtat ggccgtactt tcttcacaag atacgattac 1320

gaacatatcg aatgcgagca ggccgaaaaa gttgtagctc ttttgagtga atttgtatca 1380

aggccaaacg tttgtggctc ccacttccca gctgatgagt ctttaaccgt tatcgattgt 1440

ggtgattttt cgtatagaga tctagatggc tccatctctg aaaatcaagg ccttttcgta 1500

aagttttcga atgggactaa atttgttttg aggttatccg gcacaggcag ttctggtgca 1560

acaataagat tatacgtaga aaagtatact gataaaaagg agaactatgg ccaaacagct 1620

gacgtcttct tgaaacccgt catcaactcc attgtaaaat tcttaagatt taaagaaatt 1680

ttaggaacag acgaaccaac agtccgcaca tag 1713

<210> 24
<211> 570
<212> PRT
<213> Saccharomyces cerevisiae

<220>
<223> amino acid sequence Phosphoglucomutase-1 (PGM1) originating from
      Saccharomyces cerevisiae

<400> 24
Met Ser Leu Leu Ile Asp Ser Val Pro Thr Val Ala Tyr Lys Asp Gln
1               5                   10                  15
Lys Pro Gly Thr Ser Gly Leu Arg Lys Lys Thr Lys Val Phe Met Asp
            20                  25                  30
Glu Pro His Tyr Thr Glu Asn Phe Ile Gln Ala Thr Met Gln Ser Ile
        35                  40                  45
Pro Asn Gly Ser Glu Gly Thr Thr Leu Val Val Gly Gly Asp Gly Arg
    50                  55                  60
Phe Tyr Asn Asp Val Ile Met Asn Lys Ile Ala Ala Val Gly Ala Ala
65                  70                  75                  80
Asn Gly Val Arg Lys Leu Val Ile Gly Gln Gly Gly Leu Leu Ser Thr
                85                  90                  95
Pro Ala Ala Ser His Ile Ile Arg Thr Tyr Glu Glu Lys Cys Thr Gly
            100                 105                 110
Gly Gly Ile Ile Leu Thr Ala Ser His Asn Pro Gly Gly Pro Glu Asn
        115                 120                 125

Asp Leu Gly Ile Lys Tyr Asn Leu Pro Asn Gly Gly Pro Ala Pro Glu
    130                 135                 140
Ser Val Thr Asn Ala Ile Trp Glu Ala Ser Lys Lys Leu Thr His Tyr
145                 150                 155                 160
Lys Ile Ile Lys Asn Phe Pro Lys Leu Asn Leu Asn Lys Leu Gly Lys
                165                 170                 175
Asn Gln Lys Tyr Gly Pro Leu Leu Val Asp Ile Ile Asp Pro Ala Lys
            180                 185                 190
Ala Tyr Val Gln Phe Leu Lys Glu Ile Phe Asp Phe Asp Leu Ile Lys
        195                 200                 205
Ser Phe Leu Ala Lys Gln Arg Lys Asp Lys Gly Trp Lys Leu Leu Phe
    210                 215                 220
Asp Ser Leu Asn Gly Ile Thr Gly Pro Tyr Gly Lys Ala Ile Phe Val
225                 230                 235                 240
Asp Glu Phe Gly Leu Pro Ala Glu Glu Val Leu Gln Asn Trp His Pro
                245                 250                 255
Leu Pro Asp Phe Gly Gly Leu His Pro Asp Pro Asn Leu Thr Tyr Ala
            260                 265                 270
Arg Thr Leu Val Asp Arg Val Asp Arg Glu Lys Ile Ala Phe Gly Ala
        275                 280                 285
Ala Ser Asp Gly Asp Gly Asp Arg Asn Met Ile Tyr Gly Tyr Gly Pro
    290                 295                 300
Ala Phe Val Ser Pro Gly Asp Ser Val Ala Ile Ile Ala Glu Tyr Ala
305                 310                 315                 320
Pro Glu Ile Pro Tyr Phe Ala Lys Gln Gly Ile Tyr Gly Leu Ala Arg
                325                 330                 335
Ser Phe Pro Thr Ser Ser Ala Ile Asp Arg Val Ala Ala Lys Lys Gly
            340                 345                 350
Leu Arg Cys Tyr Glu Val Pro Thr Gly Trp Lys Phe Phe Cys Ala Leu
        355                 360                 365
Phe Asp Ala Lys Lys Leu Ser Ile Cys Gly Glu Glu Ser Phe Gly Thr
    370                 375                 380
Gly Ser Asn His Ile Arg Glu Lys Asp Gly Leu Trp Ala Ile Ile Ala
385                 390                 395                 400
Trp Leu Asn Ile Leu Ala Ile Tyr His Arg Arg Asn Pro Glu Lys Glu
                405                 410                 415
Ala Ser Ile Lys Thr Ile Gln Asp Glu Phe Trp Asn Glu Tyr Gly Arg
            420                 425                 430
Thr Phe Phe Thr Arg Tyr Asp Tyr Glu His Ile Glu Cys Glu Gln Ala
        435                 440                 445
Glu Lys Val Val Ala Leu Leu Ser Glu Phe Val Ser Arg Pro Asn Val
    450                 455                 460
Cys Gly Ser His Phe Pro Ala Asp Glu Ser Leu Thr Val Ile Asp Cys
465                 470                 475                 480
Gly Asp Phe Ser Tyr Arg Asp Leu Asp Gly Ser Ile Ser Glu Asn Gln
                485                 490                 495
Gly Leu Phe Val Lys Phe Ser Asn Gly Thr Lys Phe Val Leu Arg Leu
            500                 505                 510
Ser Gly Thr Gly Ser Ser Gly Ala Thr Ile Arg Leu Tyr Val Glu Lys
        515                 520                 525
Tyr Thr Asp Lys Lys Glu Asn Tyr Gly Gln Thr Ala Asp Val Phe Leu
    530                 535                 540
Lys Pro Val Ile Asn Ser Ile Val Lys Phe Leu Arg Phe Lys Glu Ile
545                 550                 555                 560
Leu Gly Thr Asp Glu Pro Thr Val Arg Thr
                565                 570

<210> 25
<211> 1500
<212> DNA
<213> Saccharomyces cerevisiae

<220>
<223> nucleic acid sequence of UTP-glucose-1-phosphate
      uridylyltransferase (UGP1) originating from Saccharomyces
      cerevisiae

<400> 25
atgtccacta agaagcacac caaaacacat tccacttatg cattcgagag caacacaaac      60

agcgttgctg cctcacaaat gagaaacgcc ttaaacaagt tggcggactc tagtaaactt     120

gacgatgctg ctcgcgctaa gtttgagaac gaactggatt cgtttttcac gcttttcagg     180

agatatttgg tagagaagtc ttctagaacc accttggaat gggacaagat caagtctccc     240

aacccggatg aagtggttaa gtatgaaatt atttctcagc agcccgagaa tgtctcaaac     300

ctttccaaat tggctgtttt gaagttgaac ggtgggctgg gtacctccat gggctgcgtt     360

ggccctaaat ctgttattga agtgagagag ggaaacacct tttggatttt gtctgttcgt     420

caaattgaat acttgaacag acagtacgat agcgacgtgc cattgttatt gatgaattct     480

ttcaacactg acaaggatac ggaacacttg attaagaagt attccgctaa cagaatcaga     540

atcagatctt tcaatcaatc caggttccca agagtctaca aggattcttt attgcctgtc     600

cccaccgaat acgattctcc actggatgct tggtatccac caggtcacgg tgatttgttt     660

gaatctttac acgtatctgg tgaactggat gccttaattg cccaaggaag agaaatatta     720

tttgtttcta acggtgacaa cttgggtgct accgtcgact taaaaatttt aaaccacatg     780

atcgagactg gtgccgaata tataatggaa ttgactgata agaccagagc cgatgttaaa     840

ggtggtactt tgatttctta cgatggtcaa gtccgtttat tggaagtcgc ccaagttcca     900

aaagaacaca ttgacgaatt caaaaatatc agaaagttta ccaacttcaa cacgaataac     960

ttatggatca atctgaaagc agtaaagagg ttgatcgaat cgagcaattt ggagatggaa    1020

atcattccaa accaaaaaac tataacaaga gacggtcatg aaattaatgt cttacaatta    1080

gaaaccgctt gtggtgctgc tatcaggcat tttgatggtg ctcacggtgt tgtcgttcca    1140

agatcaagat tcttgcctgt caagacctgt tccgatttgt tgctggttaa atcagatcta    1200

ttccgtctgg aacacggttc tttgaagtta gacccatccc gttttggtcc aaacccatta    1260

atcaagttgg gctcgcattt caaaaaggtt tctggtttta acgcaagaat ccctcacatc    1320

ccaaaaatcg tcgagctaga tcatttgacc atcactggta acgtcttttt aggtaaagat    1380

gtcactttga ggggtactgt catcatcgtt tgctccgacg gtcataaaat cgatattcca    1440

aacggctcca tattggaaaa tgttgtcgtt actggtaatt tgcaaatctt ggaacattga    1500


<210> 26
<211> 499
<212> PRT
<213> Saccharomyces cerevisiae

<220>
<223> amino acid sequence of UTP--glucose-1-phosphate
      uridylyltransferase (UGP1) originating from Saccharomyces
      cerevisiae

<400> 26
Met Ser Thr Lys Lys His Thr Lys Thr His Ser Thr Tyr Ala Phe Glu
1               5                   10                  15
Ser Asn Thr Asn Ser Val Ala Ala Ser Gln Met Arg Asn Ala Leu Asn
            20                  25                  30
Lys Leu Ala Asp Ser Ser Lys Leu Asp Asp Ala Ala Arg Ala Lys Phe
        35                  40                  45
Glu Asn Glu Leu Asp Ser Phe Phe Thr Leu Phe Arg Arg Tyr Leu Val
    50                  55                  60
Glu Lys Ser Ser Arg Thr Thr Leu Glu Trp Asp Lys Ile Lys Ser Pro
65                  70                  75                  80
Asn Pro Asp Glu Val Val Lys Tyr Glu Ile Ile Ser Gln Gln Pro Glu
                85                  90                  95
Asn Val Ser Asn Leu Ser Lys Leu Ala Val Leu Lys Leu Asn Gly Gly
            100                 105                 110
Leu Gly Thr Ser Met Gly Cys Val Gly Pro Lys Ser Val Ile Glu Val
        115                 120                 125
Arg Glu Gly Asn Thr Phe Leu Asp Leu Ser Val Arg Gln Ile Glu Tyr
    130                 135                 140
Leu Asn Arg Gln Tyr Asp Ser Asp Val Pro Leu Leu Leu Met Asn Ser
145                 150                 155                 160
Phe Asn Thr Asp Lys Asp Thr Glu His Leu Ile Lys Lys Tyr Ser Ala
                165                 170                 175
Asn Arg Ile Arg Ile Arg Ser Phe Asn Gln Ser Arg Phe Pro Arg Val
            180                 185                 190
Tyr Lys Asp Ser Leu Leu Pro Val Pro Thr Glu Tyr Asp Ser Pro Leu
        195                 200                 205
Asp Ala Trp Tyr Pro Pro Gly His Gly Asp Leu Phe Glu Ser Leu His
    210                 215                 220
Val Ser Gly Glu Leu Asp Ala Leu Ile Ala Gln Gly Arg Glu Ile Leu
225                 230                 235                 240
Phe Val Ser Asn Gly Asp Asn Leu Gly Ala Thr Val Asp Leu Lys Ile
                245                 250                 255
Leu Asn His Met Ile Glu Thr Gly Ala Glu Tyr Ile Met Glu Leu Thr
            260                 265                 270
Asp Lys Thr Arg Ala Asp Val Lys Gly Gly Thr Leu Ile Ser Tyr Asp
        275                 280                 285
Gly Gln Val Arg Leu Leu Glu Val Ala Gln Val Pro Lys Glu His Ile
    290                 295                 300
Asp Glu Phe Lys Asn Ile Arg Lys Phe Thr Asn Phe Asn Thr Asn Asn
305                 310                 315                 320
Leu Trp Ile Asn Leu Lys Ala Val Lys Arg Leu Ile Glu Ser Ser Asn
                325                 330                 335
Leu Glu Met Glu Ile Ile Pro Asn Gln Lys Thr Ile Thr Arg Asp Gly
            340                 345                 350
His Glu Ile Asn Val Leu Gln Leu Glu Thr Ala Cys Gly Ala Ala Ile
        355                 360                 365
Arg His Phe Asp Gly Ala His Gly Val Val Val Pro Arg Ser Arg Phe
    370                 375                 380
Leu Pro Val Lys Thr Cys Ser Asp Leu Leu Leu Val Lys Ser Asp Leu
385                 390                 395                 400
Phe Arg Leu Glu His Gly Ser Leu Lys Leu Asp Pro Ser Arg Phe Gly
                405                 410                 415
Pro Asn Pro Leu Ile Lys Leu Gly Ser His Phe Lys Lys Val Ser Gly
            420                 425                 430
Phe Asn Ala Arg Ile Pro His Ile Pro Lys Ile Val Glu Leu Asp His
        435                 440                 445
Leu Thr Ile Thr Gly Asn Val Phe Leu Gly Lys Asp Val Thr Leu Arg

450                 455                 460
Gly Thr Val Ile Ile Val Cys Ser Asp Gly His Lys Ile Asp Ile Pro
465                 470                 475                 480
Asn Gly Ser Ile Leu Glu Asn Val Val Val Thr Gly Asn Leu Gln Ile
                485                 490                 495
Leu Glu His

<210> 27
<211> 480
<212> DNA
<213> Saccharomyces cerevisiae

<220>
<223> nucleic acid sequence of Glucosamine 6-phosphate
      N-acetyltransferase (GNA1) originating from Saccharomyces
      cerevisiae

<400> 27
atgagcttac ccgatggatt ttatataagg cgaatggaag agggggattt ggaacaggtc      60

actgagacgc taaaggtttt gaccaccgtg ggcactatta cccccgaatc cttcagcaaa     120

ctcataaaat actggaatga agccacagta tggaatgata acgaagataa aaaaataatg     180

caatataacc ccatggtgat tgtggacaag cgcaccgaga cggttgccgc tacggggaat     240

atcatcatcg aaagaaagat cattcatgaa ctggggctat gtggccacat cgaggacatt     300

gcagtaaact ccaagtatca gggccaaggt ttgggcaagc tcttgattga tcaattggta     360

actatcggct ttgactacgg ttgttataag attattttag attgcgatga gaaaaatgtc     420

aaattctatg aaaaatgtgg gtttagcaac gcaggcgtgg aaatgcaaat tagaaaatag     480

<210> 28
<211> 159
<212> PRT
<213> Saccharomyces cerevisiae

<220>
<223> amino acid sequence of Glucosamine 6-phosphate
      N-acetyltransferase (GNA1) originating from Saccharomyces
      cerevisiae

<400> 28
Met Ser Leu Pro Asp Gly Phe Tyr Ile Arg Arg Met Glu Glu Gly Asp
1               5                   10                  15
Leu Glu Gln Val Thr Glu Thr Leu Lys Val Leu Thr Thr Val Gly Thr
            20                  25                  30
Ile Thr Pro Glu Ser Phe Ser Lys Leu Ile Lys Tyr Trp Asn Glu Ala
        35                  40                  45
Thr Val Trp Asn Asp Asn Glu Asp Lys Lys Ile Met Gln Tyr Asn Pro
    50                  55                  60
Met Val Ile Val Asp Lys Arg Thr Glu Thr Val Ala Ala Thr Gly Asn
65                  70                  75                  80
Ile Ile Ile Glu Arg Lys Ile Ile His Glu Leu Gly Leu Cys Gly His
                85                  90                  95
Ile Glu Asp Ile Ala Val Asn Ser Lys Tyr Gln Gly Gln Gly Leu Gly
            100                 105                 110

Lys Leu Leu Ile Asp Gln Leu Val Thr Ile Gly Phe Asp Tyr Gly Cys
        115                 120                 125
Tyr Lys Ile Ile Leu Asp Cys Asp Glu Lys Asn Val Lys Phe Tyr Glu
    130                 135                 140
Lys Cys Gly Phe Ser Asn Ala Gly Val Glu Met Gln Ile Arg Lys
145                 150                 155

<210> 29
<211> 1674
<212> DNA
<213> Saccharomyces cerevisiae

<220>
<223> nucleic acid sequence of phosphoacetylglucosamine mutase (PCM1)
      originating from Saccharomyces cerevisiae

<400> 29
atgaaggttg attacgagca attgtgcaaa ctctacgatg acacgtgccg cacaaagaat      60

gtgcagttca gttacggtac ggccggattc agaacgctgg ccaagaattt ggatacggtg     120

atgttcagta ctggtatact ggcggttctc aggtcgctga agcttcaggg tcagtatgtg     180

ggggtgatga tcacggcgtc gcacaaccca taccaggaca acggggtcaa gatcgtggaa     240

ccagacggat cgatgctttt ggccacatgg gagccatatg ccatgcagtt ggccaatgcg     300

gcctcttttg ccactaattt tgaagaattt cgtgttgagt tggccaagct gattgaacac     360

gaaaagattg atttgaatac aaccgtcgtg cctcacatcg tggttgggag agactctagg     420

gaaagtagtc catacttgct gcgctgcttg acttcctcca tggccagcgt cttccacgcg     480

caagttttgg acctaggctg tgtcactacg cctcaattgc attacattac tgatttgtcc     540

aacaggcgga aactggaagg agacacagcg ccagttgcca cagaacagga ctactattcg     600

ttctttatag gagccttcaa cgagctcttc gccacgtatc agctggagaa gaggctgtct     660

gtcccaaaat tgttcataga cacagccaat ggtatcggtg gtccacagtt gaaaaaacta     720

ctggcctccg aagattggga cgtgccagcg gagcaagttg aggtaatcaa cgacaggtcc     780

gatgttccag aactgttgaa ttttgaatgc ggtgcggatt atgtgaagac taaccagaga     840

ttacccaagg gtctttctcc atcctcgttt gattcgctat attgctcctt tgatggtgac     900

gcagacaggg ttgtgttcta ctatgtcgac tcaggatcaa aatttcattt gttggatggt     960

gacaaaattt ccactttgtt tgcaaagttc ttgtctaaac aactagaatt ggcacaccta    1020

gaacattctt tgaagattgg tgttgtgcaa actgcctatg caaacggcag ttccaccgct    1080

tacataaaaa atacgttgca ctgtcccgtg tcttgcacta agacaggtgt taaacacttg    1140

catcatgaag ctgccactca gtacgatatt ggcatttatt tcgaagcaaa tggacatggt    1200

acgattatat tcagcgaaaa atttcatcga actatcaaat ctgaattatc caagtccaag    1260

ttaaatggtg atacgttagc tttgagaact ttgaagtgtt tctctgaatt gattaatcag    1320

accgtgggag atgctatttc agacatgctt gctgtccttg ctactttggc gattttgaaa    1380

atgtcgccaa tggattggga tgaagagtat actgatttgc ccaacaagct ggttaagtgc 1440

atcgttcctg ataggtcaat tttccaaacc acggaccagg aaagaaaatt gctcaatcca 1500

gtggggttgc aagacaagat agatcttgtg gtagccaagt atcccatggg aagaagcttt 1560

gtcagagcca gtggtacgga ggatgcggtg agggtttatg cggaatgtaa ggactcctct 1620

aagttaggtc aattttgtga cgaagtggtg gagcacgtta aggcatctgc ttga 1674

<210> 30
<211> 557
<212> PRT
<213> Saccharomyces cerevisiae

<220>
<223> amino acid sequence of phosphoacetylglucosamine mutase (PCM1)
      originating from Saccharomyces cerevisiae

<400> 30
Met Lys Val Asp Tyr Glu Gln Leu Cys Lys Leu Tyr Asp Asp Thr Cys
1               5                   10                  15
Arg Thr Lys Asn Val Gln Phe Ser Tyr Gly Thr Ala Gly Phe Arg Thr
            20                  25                  30
Leu Ala Lys Asn Leu Asp Thr Val Met Phe Ser Thr Gly Ile Leu Ala
        35                  40                  45
Val Leu Arg Ser Leu Lys Leu Gln Gly Gln Tyr Val Gly Val Met Ile
    50                  55                  60
Thr Ala Ser His Asn Pro Tyr Gln Asp Asn Gly Val Lys Ile Val Glu
65                  70                  75                  80
Pro Asp Gly Ser Met Leu Leu Ala Thr Trp Glu Pro Tyr Ala Met Gln
                85                  90                  95
Leu Ala Asn Ala Ala Ser Phe Ala Thr Asn Phe Glu Glu Phe Arg Val
            100                 105                 110
Glu Leu Ala Lys Leu Ile Glu His Glu Lys Ile Asp Leu Asn Thr Thr
        115                 120                 125
Val Val Pro His Ile Val Val Gly Arg Asp Ser Arg Glu Ser Ser Pro
    130                 135                 140
Tyr Leu Leu Arg Cys Leu Thr Ser Ser Met Ala Ser Val Phe His Ala
145                 150                 155                 160
Gln Val Leu Asp Leu Gly Cys Val Thr Thr Pro Gln Leu His Tyr Ile
                165                 170                 175
Thr Asp Leu Ser Asn Arg Arg Lys Leu Glu Gly Asp Thr Ala Pro Val
            180                 185                 190
Ala Thr Glu Gln Asp Tyr Tyr Ser Phe Phe Ile Gly Ala Phe Asn Glu
        195                 200                 205
Leu Phe Ala Thr Tyr Gln Leu Glu Lys Arg Leu Ser Val Pro Lys Leu
    210                 215                 220
Phe Ile Asp Thr Ala Asn Gly Ile Gly Gly Pro Gln Leu Lys Lys Leu
225                 230                 235                 240
Leu Ala Ser Glu Asp Trp Asp Val Pro Ala Glu Gln Val Glu Val Ile
                245                 250                 255
Asn Asp Arg Ser Asp Val Pro Glu Leu Leu Asn Phe Glu Cys Gly Ala
            260                 265                 270
Asp Tyr Val Lys Thr Asn Gln Arg Leu Pro Lys Gly Leu Ser Pro Ser
        275                 280                 285
Ser Phe Asp Ser Leu Tyr Cys Ser Phe Asp Gly Asp Ala Asp Arg Val
    290                 295                 300
Val Phe Tyr Tyr Val Asp Ser Gly Ser Lys Phe His Leu Leu Asp Gly
305                 310                 315                 320

Asp Lys Ile Ser Thr Leu Phe Ala Lys Phe Leu Ser Lys Gln Leu Glu
                325                 330                 335
Leu Ala His Leu Glu His Ser Leu Lys Ile Gly Val Val Gln Thr Ala
            340                 345                 350
Tyr Ala Asn Gly Ser Ser Thr Ala Tyr Ile Lys Asn Thr Leu His Cys
        355                 360                 365
Pro Val Ser Cys Thr Lys Thr Gly Val Lys His Leu His His Glu Ala
    370                 375                 380
Ala Thr Gln Tyr Asp Ile Gly Ile Tyr Phe Glu Ala Asn Gly His Gly
385                 390                 395                 400
Thr Ile Ile Phe Ser Glu Lys Phe His Arg Thr Ile Lys Ser Glu Leu
                405                 410                 415
Ser Lys Ser Lys Leu Asn Gly Asp Thr Leu Ala Leu Arg Thr Leu Lys
            420                 425                 430
Cys Phe Ser Glu Leu Ile Asn Gln Thr Val Gly Asp Ala Ile Ser Asp
        435                 440                 445
Met Leu Ala Val Leu Ala Thr Leu Ala Ile Leu Lys Met Ser Pro Met
    450                 455                 460
Asp Trp Asp Glu Glu Tyr Thr Asp Leu Pro Asn Lys Leu Val Lys Cys
465                 470                 475                 480
Ile Val Pro Asp Arg Ser Ile Phe Gln Thr Thr Asp Gln Glu Arg Lys
                485                 490                 495
Leu Leu Asn Pro Val Gly Leu Gln Asp Lys Ile Asp Leu Val Val Ala
            500                 505                 510
Lys Tyr Pro Met Gly Arg Ser Phe Val Arg Ala Ser Gly Thr Glu Asp
        515                 520                 525
Ala Val Arg Val Tyr Ala Glu Cys Lys Asp Ser Ser Lys Leu Gly Gln
    530                 535                 540
Phe Cys Asp Glu Val Val Glu His Val Lys Ala Ser Ala
545                 550                 555

<210> 31
<211> 574
<212> DNA
<213> other sequences


<220>
<223> nucleic acid sequence of promotor pTDH3

<400> 31
ctgctgtaac ccgtacatgc ccaaaatagg gggcgggtta cacagaatat ataacatcgt      60

aggtgtctgg gtgaacagtt tattcctggc atccactaaa tataatggag cccgcttttt     120

aagctggcat ccagaaaaaa aaagaatccc agcaccaaaa tattgttttc ttcaccaacc     180

atcagttcat aggtccattc tcttagcgca actacagaga acaggggcac aaacaggcaa     240

aaaacgggca caacctcaat ggagtgatgc aacctgcctg gagtaaatga tgacacaagg     300

caattgaccc acgcatgtat ctatctcatt ttcttacacc ttctattacc ttctgctctc     360

tctgatttgg aaaaagctga aaaaaaaggt tgaaaccagt tccctgaaat tattccccta     420

cttgactaat aagtatataa agacggtagg tattgattgt aattctgtaa atctatttct     480

taaacttctt aaattctact tttatagtta gtcttttttt tagttttaaa acaccaagaa     540

cttagtttcg aataaacaca cataaacaaa caaa                                 574


<210> 32

<211> 585
<212> DNA
<213> other sequences

<220>
<223> nucleic acid sequence of promotor pTDH3.Sk

<400> 32
caattcatcg gcccttttag cggctacccg cgccatctaa atgatagggc gggtgacact 60

atggtaaatc ccataattag gtgtctgggt gagtggttct gatgccggca tccactaaat 120

atattggagc ccatttttta cgcgggcttc cagaaaaaaa gagaatccca gcaccaaaag 180

gtggttctct tcaccaacca tcagatcata ggtccacaac cacacataac aggggcacaa 240

aaaggcaaaa aacggacata acctcaatgg agtgatgcaa attgactgga gcaaaagctg 300

acacaaggca ttgattgacc tacgcatgta tctgtattct tttcttacac cttctattac 360

cttctaactc tttgggttgg aaaaaactga aaaaaaaggt tgggacctgg ttcccccaag 420

ttgtccccct acttggttat taaatatata aagacagcaa gtgttgatta taatcttgta 480

aatctatagt tcttaatcta tacttctatt tatattttaa attagtcttt ttatttccaa 540

gtccccaaga acttagtttc gaataaacac acacaaataa acaca 585

<210> 33
<211> 700
<212> DNA
<213> other sequences

<220>
<223> nucleic acid sequence of promotor pTDH3-1.sba

<400> 33
gcagcgcttc ttccgctcta gtttttatag ttattattac taccacctta aaaatacgta 60

aatactcaaa atagtagtga tattcccaac cttattcatc caaggcacat catcatcatc 120

agccattcat ctttcacctg ccattagtaa cccgtcttct cattgagcgg gttacggcag 180

ccacaggcca cattccgaat gtctgggtga gcggtccctt ttccagcatc cactaaatat 240

ctccgatccc gctttttaat ctggcttcct gaaaaaaaga gaatcccagc accaaaaaat 300

ggctctcttc accaaccatc agatcatagg tcccattctc ttaccgcaac cgtacagaac 360

aggggaaaac gggtacaacc tcaatggagt gatgcaaact gactggagca aaaagctgac 420

acaaggcaat cgacctacgt gtctgtctat tttctcacac cttctattac cttctaactc 480

tctgggttgg aaaaaactga aaaaaaggtt gagaccagtt tccacaaatc atccccctgt 540

ttgattaata aatatataaa gacgacaact atcgatcata aactcataaa actataactc 600

ctttacactt cttattttat agttattcta ttttaattct tattgatttt aaaaccccaa 660

gaacttagtt tcgaaaacac acacacacaa acaattaaaa 700

<210> 34
<211> 602
<212> DNA
<213> other sequences

<220>
<223> nucleic acid sequence of promotor pTDH3.Sar

<400> 34
gagctaaata tcagcccttc gggtcctgcc tgctacccgg tcctgttcga ataaaaacgc 60

gggtaacacg acccagtaac acctgtcgtt gggtgtctgg gtcagaagtt ctgataccgg 120

cttccactaa atagattggg ttccgctctt tacgctggct tcctgaaaaa agagattccg 180

ggcaccaaaa aattggtctc tttgccaacc atcagatcat aggtccattc tcttaccata 240

accacacagg ataggggcac cacaggcgaa aatgggcaca aaatctcaat ggagtgatgc 300

aaattagctg gaacaaaagc tgacacaagg caattaacct gcgcatgtat ccatctcctt 360

ttcttacacc ttctcttacg ttctaactgt ttgggttgga aaaattaaaa aaaaaaggtt 420

gagaccagtt tccccaaatc gtccccctac ttgattcata aatatataaa gacgacaact 480

attgattata atcttgtaaa tctataactc tttactttct cctatttata atttaactta 540

atctttttag atttaaaacc ccaagaactt agtttcgaac aaacacacac aaataaacaa 600

aa 602

<210> 35
<211> 550
<212> DNA
<213> other sequences

<220>
<223> nucleic acid sequence of promotor pENO2

<400> 35
cgctcagcat ctgcttcttc ccaaagatga acgcggcgtt atgtcactaa cgacgtgcac 60

caacttgcgg aaagtggaat cccgttccaa aactggcatc cactaattga tacatctaca 120

caccgcacgc cttttttctg aagcccactt tcgtggactt tgccatatgc aaaattcatg 180

aagtgtgata ccaagtcagc atacacctca ctagggtagt ttctttggtt gtattgatca 240

tttggttcat cgtggttcat taattttttt tctccattgc tttctggctt tgatcttact 300

atcatttgga tttttgtcga aggttgtaga attgtatgtg acaagtggca ccaagcatat 360

ataaaaaaaa aaagcattat cttcctacca gagttgattg ttaaaaacgt atttatagca 420

aacgcaattg taattaattc ttattttgta tcttttcttc ccttgtctca atcttttatt 480

tttattttat ttttcttttc ttagtttctt tcataacacc aagcaactaa tactataaca 540

tacaataata                                                          550

<210> 36
<211> 598
<212> DNA
<213> other sequences

<220>
<223> nucleic acid sequence of promotor pTEF3

<400> 36
ggctgataat agcgtataaa caatgcatac tttgtacgtt caaaatacaa tgcagtagat      60

atatttatgc atattacata taatacatat cacataggaa gcaacaggcg cgttggactt     120

ttaattttcg aggaccgcga atccttacat cacacccaat cccccacaag tgatccccca     180

cacaccatag cttcaaaatg tttctactcc ttttttactc ttccagattt tctcggactc      240

cgcgcatcgc cgtaccactt caaaacaccc aagcacagca tactaaattt cccctctttc     300

ttcctctagg gtgtcgttaa ttacccgtac taaaggtttg gaaaagaaaa aagagaccgc     360

ctcgtttctt tttcttcgtc gaaaaaggca ataaaaattt ttatcacgtt tctttttctt     420

gaaaattttt ttttttgatt tttttctctt tcgatgacct cccattgata tttaagttaa      480

taaacggtct tcaatttctc aagtttcagt ttcatttttc ttgttctatt acaacttttt     540

ttacttcttg ctcattagaa agaaagcata gcaatctaat ctaagtttta attacaaa       598

<210> 37
<211> 383
<212> DNA
<213> other sequences

<220>
<223> nucleic acid sequence of promotor pTEF1

<400> 37
gtttagcttg cctcgtcccc gccgggtcac ccggccagcg acatggaggc ccagaatacc      60

ctccttgaca gtcttgacgt gcgcagctca ggggcatgat gtgactgtcg cccgtacatt     120

tagcccatac atccccatgt ataatcattt gcatccatac attttgatgg ccgcacggcg     180

cgaagcaaaa attacggctc ctcgctgcag acctgcgagc agggaaacgc tcccctcaca     240

gacgcgttga attgtcccca cgccgcgccc ctgtagagaa atataaaagg ttaggatttg     300

ccactgaggt tcttctttca tatacttcct tttaaaatct tgctacgata cagttctcac     360

atcacatccg aacataaaca acc                                             383

<210> 38
<211> 383
<212> DNA
<213> other sequences

<220>
<223> nucleic acid sequence of promotor pTEF1.ago

<400> 38
gtttagcttg cctcgtcccc gccgggtcac ccggccagcg acatggaggc ccagaatacc      60

ctccttgaca gtcttgacgt gcgcagctca ggggcatgat gtgactgtcg cccgtacatt     120

tagcccatac atccccatgt ataatcattt gcatccatac attttgatgg ccgcacggcg     180

cgaagcaaaa attacggctc ctcgctgcag acctgcgagc agggaaacgc tcccctcaca     240

gacgcgttga attgtcccca cgccgcgccc ctgtagagaa atataaaagg ttaggatttg     300

ccactgaggt tcttctttca tatacttcct tttaaaatct tgctacgata cagttctcac     360

atcacatccg aacataaaca acc                                             383


<210> 39
<211> 498
<212> DNA
<213> other sequences


<220>
<223> nucleic acid sequence of promotor pTEF1.Sba

<400> 39
cgccaacaaa ccttcgaaca ctttaatttt cgaggaccgc agatcctcac atcacaccca      60

cacccaagac tgcttccccc acacaccctg catctgtaca ctttcttctg ctctgttttt     120

ctctccggcg ttctctcggg tcgcccgcat cgccgcgccg gctggaaccg cccacgcacc     180

gcatattgca aatcgcctgc cccctcttgc tcctttttga gggcgcgccg ttacccgcgc     240

ccagggtccg gaaaaagaaa caaggctcta ccgcgtttct ttttccttgt cgaaaaaggc     300

aaaaatgaaa atttttatca cgtttctttt tttttgaaaa attttttttt tggttttttt     360

tctttcgatg gcctcccatt gatatttaag ttaataaatg gttttcagtt ttcaagtttc     420

agtttgtgtt cttctttgct aactttcact tacacctcga aagaaagtat agcaatctaa     480

tcttagtttt aattacaa                                                   498


<210> 40
<211> 700
<212> DNA
<213> other sequences


<220>
<223> nucleic acid sequence of promotor pPDC1

<400> 40
ttatttacct atctctaaac ttcaacacct tatatcataa ctaatatttc ttgagataag      60

cacactgcac ccataccttc cttaaaaacg tagcttccag tttttggtgg ttccggcttc     120

cttcccgatt ccgcccgcta aacgcatatt tttgttgcct ggtggcattt gcaaaatgca 180

taacctatgc atttaaaaga ttatgtatgc tcttctgact tttcgtgtga tgaggctcgt 240

ggaaaaaatg aataatttat gaatttgaga acaattttgt gttgttacgg tattttacta 300

tggaataatc aatcaattga ggattttatg caaatatcgt ttgaatattt ttccgaccct 360

ttgagtactt ttcttcataa ttgcataata ttgtccgctg cccctttttc tgttagacgg 420

tgtcttgatc tacttgctat cgttcaacac caccttattt tctaactatt ttttttttag 480

ctcatttgaa tcagcttatg gtgatggcac atttttgcat aaacctagct gtcctcgttg 540

aacataggaa aaaaaaatat ataaacaagg ctctttcact ctccttgcaa tcagatttgg 600

gtttgttccc tttattttca tatttcttgt catattcctt tctcaattat tattttctac 660

tcataacctc acgcaaaata acacagtcaa atcaatcaaa 700

<210> 41
<211> 998
<212> DNA
<213> other sequences

<220>
<223> nucleic acid sequence of promotor pCCW12

<400> 41
aaccagggca aagcaaaata aaagaaactt aatacgttat gccgtaatga agggctacca 60

aaaacgataa tctcaactgt aaacaggtac aatgcggacc cttttgccac aaaacataca 120

tcattcattg ccggaaaaag aaagaagtga agacagcagt gcagccagcc atgttgcgcc 180

aatctaatta tagatgctgg tgccctgagg atgtatctgg agccagccat ggcatcatgc 240

gctaccgccg gatgtaaaat ccgacacgca aaagaaaacc ttcgaggttg cgcacttcgc 300

ccacccatga accacacggt tagtccaaaa ggggcagttc agattccaga tgcgggaatt 360

agcttgctgc caccctcacc tcactaacgc tgcggtgtgc ggatacttca tgctatttat 420

agacgcgcgt gtcggaatca gcacgcgcaa gaaccaaatg ggaaaatcgg aatgggtcca 480

gaactgcttt gagtgctggc tattggcgtc tgatttccgt tttgggaatc ctttgccgcg 540

cgcccctctc aaaactccgc acaagtccca gaaagcggga aagaaataaa acgccaccaa 600

aaaaaaaaat aaaagccaat cctcgaagcg tgggtggtag gccctggatt atcccgtaca 660

agtatttctc aggagtaaaa aaaccgtttg ttttggaatt ccccatttcg cggccaccta 720

cgccgctatc tttgcaacaa ctatctgcga taactcagca aattttgcat attcgtgttg 780

cagtattgcg ataatgggag tcttacttcc aacataacgg cagaaagaaa tgtgagaaaa 840

ttttgcatcc tttgcctccg ttcaagtata taaagtcggc atgcttgata atctttcttt 900

ccatcctaca ttgttctaat tattcttatt ctcctttatt ctttcctaac ataccaagaa 960

attaatcttc tgtcattcgc ttaaacacta tatcaata 998

<210> 42
<211> 976
<212> DNA
<213> other sequences

<220>
<223> nucleic acid sequence of promotor pCCW12.Sm

<400> 42
cgttgtgctg tagtgaagga agactaaaaa ggataatcac agttgtaaag aggtataatg 60

cggacccttt tgccacaaaa cacacatctt tcgtttccaa aataagaaag aagaaagcaa 120

aaagattagc agccacgttg ctgcgatcta attatagacg gtggcgtcat cattctcacc 180

caagattgtg tcttgaacct gccacgggtc ctgcgttatc gccggatgta aaactagaca 240

tgcaaaaaaa ggaccttcca ggtagcgtgc tccacaccac ccatgaccac cacagttagt 300

ccaaaagagg cagcaccact tcccgatggg ggaattagat tgctgccacc ctcacctcac 360

taatgctgcg gtgtgcggat atgccctgct atatatagct ccgcgttttt gaaccagcac 420

agcgcgagca ccaaaaagga aaatcgcata ggcccagaac tgatttcagc acgggctatt 480

ggcgttgggt ttccgttctg ggaaaccttc gccgcgtccc cctcacaaac ctccgcacaa 540

gttcgagcaa gcgggaaaaa acgaaaaacg ccattaatac taaataaagc aaatcctcga 600

agcgtgggtg gcaagcccct ggatttttcc gcacaagtac tcttctcagg agtaaaaaaa 660

cccgtttgtt ttggaattcc ccatttcgcg gccatctacg ccggtatctt cgcaatatct 720

atcagcgata actcagcaat tttaatattc gtgttgcagt gctgcgatag cgggagtctt 780

gtttgtaaca taacggcaga aaggaatgag agaaaatttt ccattctttg gcctccgttc 840

aagtatataa agccggcatg cttggtaatc tttctctctc ttctgtattg tttctataat 900

acttttatct tctaattatt ttctgaaaaa accaagaaat taatcttctg tcattcgctt 960

aaacactata tcaata 976

<210> 43
<211> 750
<212> DNA
<213> other sequences

<220>
<223> nucleic acid sequence of promotor pCCW12.Sk

<400> 43
tgtaaaatcc tacacgcaaa aaacctcttg ggttgcgcgt tttccaccac ccacgacccg 60

cacaatcaat ccaaaagggg caacgccagt tcccaatgcg ggaattagct cctcaccccc 120

ctcacccgct aacgctgcgg tgtgcggaca cgcagcagta tttatagatc ctcgtgttgg 180

aaccagcccg cgtgagcacc aaattggaaa gtcgcaatgg gcccagaacc gctttcagta 240

ctgggccatt ggcgtctagt ttccgttttg agcgtccttc gccgcgtctc tctgtgaaat 300

ccccgcacaa gtctgagcag gcaaaaaaaa aaaacgccac caaaaataaa taaagccaat 360

cctcgaagga tgagtaggaa aggaagcccc tggttttttc ccgcacgaat atttttcagg 420

agtgaaaaaa tccgtttgtt ttggaattcc ccatttcgcg ctcacctacg ccggtatctt 480

tggaacaact atcagcgata actcagcaaa atttgcatat ccgtgctgca atagtgcgat 540

agtgggattg ggagtcttgt tgcatcataa cggcagaaag gaatgaataa aaattttccg 600

ttctttgtct ccgttcaagt atataaagtc ggcatgcttg attatctttt cttctcttct 660

actacatttc tatttcactt tctactctat tcttccctga aaaacccaag aaaataatct 720

tctgtcattc gcttaaacaa tatatcaaaa 750

<210> 44
<211> 750
<212> DNA
<213> other sequences

<220>
<223> nucleic acid sequence of promotor pCCW12.Sba

<400> 44
accatgccac ggtgctggcc ccacttccac ccacgacccg cacggttggc ccgaaagggg 60

caacaccagt tcccaatacg gaaattagtc cgccaccacc ctcacctcgc tagagctgcg 120

gtgtgcgggc gtgcatcgct atttatagac atgcctgctg gcgtcaccgc gcgcgagcac 180

caaacaggaa aatcgcactg ggcccagaac cacgctatgc gctgggccga tggcgtccgg 240

tttccctttg ggagccccct gccacgttcg cctaacaaat ccccgcaccg gcttgagaaa 300

aaagcgaaaa gcgaaaaaaa aaaatcaacg ccaccaaaat taaaaaaaaa gagccatcct 360

cgaagggtga atagtagccc ctgacttttc ccgcacagac agacaccttt caggagtgaa 420

caaaaaagca gtttgttttg gaattccccc atttcgcggt ggcctgcgca ggtatctctg 480

cgtcaactat cagcgataac tcagcaaatt ttgcatattc gtgttgcgat actacgataa 540

tgggagtctg tcgcctaata acggcaacaa ggaatgagag agaaaaattt tcttcattct 600

ccagctcccg ttcaagtata taaggtcggc atggtcgatt gtctttcctt ctcttcagtt 660

acgtctctct atttacatta ttcttatttt tatttaataa aaaccccaag aaattaatct 720

tctgtcattc gcttaaacac tatatcaaaa 750

<210> 45
<211> 750
<212> DNA

<213> other sequences

<220>
<223> nucleic acid sequence of promotor pCCW12.Sar

<400> 45
aaaaaacaac cttctgccaa cctgcgtgct tctcaccacc catgacccac acaattgacc 60

cgaatggggc aactccagtt cccaatacgg gaattaactc gccaccatat ttaccgcgtt 120

gaagctgtgg tgtgcggaca ctccgtacta tttatagacc cacgcggtgg aaccagcacg 180

cgcgcgcact aaacaggaaa atcgcattga gtccagaacc gccaccagca cttggccatt 240

ggcgtctaat ttccgttttc ggcgcccctc accgcgtcct tctaacaaag cgcgcacaag 300

cttgagcaag tgaaaagaaa attaaaaata aaaaaccgcc accaaaacaa ataaagcaat 360

cttcgaagtg tgggttggtg ggaagcccct ggcttttccc gcaccagtcg ttttcaggag 420

taaaaaaata cccgtttgtt ttggaattcc ccatttcgcg gcgacctgcg ccggtatctt 480

tgcaacaact atttgcgata actcagcaaa atttgcatat tcgtgttggg atattgcgat 540

agtgggagtc ttgttgcata ataacggtaa aaagaagtga aggaaaaaaa tttgcatcct 600

ttagtctcag ttcaagtata taaagtcggg atattcaatt atctttcttt ctcttgctca 660

aaggtttcta tatttttttt atagtatttc ttttgttata aaataccaag aaattaatct 720

tctgtcattc gcttaaatac tacatcaata 750

<210> 46
<211> 500
<212> DNA
<213> other sequences

<220>
<223> nucleic acid sequence of promotor pNUP57

<400> 46
tcatctgcgc aatgactatc aagaccttct gcaagaattt caaatctcac tgaaaatctt 60

gaccgaaaag tgtcttgaaa acccatcaag cctgcaaaac ctatctttga cattagtctc 120

cattataaaa acggcatagt tgggagaaaa ctttccatac ttcaattgtg gactgatata 180

agtattttag ttttgcccgc atgatcatcc cacatggcta cagcagttct ctcataggaa 240

aatagtacaa tagctacgtg atataatcta aataattgtt gccaatgtgt aattatatca 300

ttttgaacgt acgcgaaatg gattattttc aaaaattttg tttcttgaaa tgagtaaaag 360

caaaagtcca actctccaag tcgatgtaaa caactttttg ccaaagggac tgaaagacta 420

aatcgaggat tatcccgttc aaactattcc agaaacgctc gttagtaaca aaagacatac 480

cttgttgacc aattgatcac 500

<210> 47
<211> 523
<212> DNA
<213> other sequences

<220>
<223> nucleic acid sequence of promotor pCCW10.ago

<400> 47
ggtaccacgg caacctcgtt cgctgttcat ccccttcgtc acacaggacg ttggatgccg 60

taagcagcgt tgcttttgat cctcaggatc ggccgggtaa cccgcggctg cttctatttt 120

agtattcata tctcaagcac atccattccg gccgtttggg ggcgccgccg cactcgtgtc 180

cattcctacc gtggcactta gggctatcct gtcggagcgc cccgccgacc gccttatcgg 240

caccaaaagt agaagccccg gccccgcgtg gctcagactc accatcggtg ctatttactt 300

ttcgatcaga tcgcggcgcg cggtggccgg catttccgga agcggccacg gagcagaggt 360

ggcgcattcg aatcgcatac gtcttcgcca cgccggaaaa aaaattttcg gctatataag 420

gagaggcggc cgtcttgctg caggcagttt cactttctct aaaaccaaag aacatcgatt 480

tctttagtca ctcgcttcct tacaccgaac tcgaggcggc cgc 523

<210> 48
<211> 943
<212> DNA
<213> other sequences

<220>
<223> nucleic acid sequence of promotor pCWP2

<400> 48
ctagcctccc ctttttattt tgtgcggtca ccgcaaggga caaagctttt cttagaaaac 60

cgtctgagaa gcataacgta cgccatcccc tagacatatt aataatgcta cagatactat 120

gctgctcgtc tttttttgac gaccctttta ttgcaatgtg caactaatgg caaacaacca 180

catagtatca cagtattaca ttgcctccac cgatgcggat gttagggcgc caagtctgtc 240

atgaagcatg ttcctgtcat aatcttgtat gcaaaatacc gcgttctgcg ccactgatat 300

gctaggcagc agcaacctat gcagaagatt gcttttccca cgcctgtttt acgtctccag 360

ggcacttgaa acaatgcagc gatcgccgcc acaacacgcc aaagagaagc gaaagtgggc 420

ctgggcggcc tcagtttcgg cagaggtaaa caacacgaac tgaactgcct tagctccgaa 480

gggcaattcc acaggcactc cgcggggccc ggccaaggcc caaaaggcgt ggaatatgcg 540

cgttttgggg ccataacacc cagtaccacg gccggaacgg gccatataat aagtttttca 600

ctctcaagaa tggtaaacgt aaataggaac atcccactac cctagaaatt gcggaaattt 660

cgcgcttatc attagaaaat ctggaaccgt cctttttcct ctttcttgca tttccctttc 720

cgtattattg ccattcttta actgcatttg gggaaccgta gaccaaaagc caaacagaga 780

aatgtaacgt tctaaaaaaa aaacaacgaa aaaattgaaa aataagatac aataatcgta 840

tataaatcag gcttcttgtt catcattttc aattctcttc ttgccatccc ttttcctatc 900

tttgttcttt tcttctcata atcaagaata aataacttca tca 943

<210> 49
<211> 750
<212> DNA
<213> other sequences

<220>
<223> nucleic acid sequence of promotor pCCW120.Sca

<400> 49
tatttttttt aaattaaaga tgctgtgcaa ttaattgtcc ggagggaccc ttgcatactg 60

gccagtttgt acgggcgtta catagcgccg atcgacgaac agcttattaa tagcaagatt 120

tgtttctccc atgagcaatt aaccagtaat tttcacagaa actagcacaa aaccaaccaa 180

acagttaaag gcagtttcgg agaataaaca acgcgctcac tgagcttgct gttgccaaaa 240

ataacagtcg cgtgcgatgg cgggcttggc attttcctcg aaggggaaac gccgccaggg 300

ttcgctgcgg tgtgtgggtg gggcagcccc cgggcgccca gacggtgccg cagggccttg 360

agacagccca aaatgccgcg aaaacgcaag ggcgcggtga cctctttcct aatgcggaaa 420

gccccaccgc cagcgacgtg acgcgttgtg cgggctcttc tcgggacccg gaaaaagaaa 480

tacgtacaca aacaccagca aattttttcg cttgagaggc tgcccgtgtt tctgcgcaac 540

gtgaaacacg gtggacaaga caagagagat tgacaagcgt cgttatcgtc ccagtgaggt 600

ttcccaatta tataaataca tccctgttgg tggattctta attacaactt ccatctctct 660

ctctctcact tcttttgttc ccttacatat ccccaagaaa tcaatcttct gccattcgct 720

ttaacaacaa acaaactttt aacttttaaa 750

<210> 50
<211> 535
<212> DNA
<213> other sequences

<220>
<223> nucleic acid sequence of promotor pRPLA1

<400> 50
tcaagttgga tactgatctg atctctccgc cctactacca gggaccctca tgattaccgc 60

tcgaatgcga cgtttcctgc ctcataaaac tggcttgaaa atatttattc gctgaacagt 120

agcctagctt ataaaaattt catttaatta atgtaatatg aaaactcaca tgccttctgt 180

ttctaaaatt gtcacagcaa gaaataacat taccatacgt gatcttatta aactctagta 240

tcttgtctaa tacttcattt aaaagaagcc ttaaccctgt agcctcatct atgtctgcta 300

catatcgtga ggtacgaata tcgtaagatg ataccacgca actttgtaat gatttttttt 360

ttttcatttt ttaaagaatg cctttacatg gtatttgaaa aaaatatctt tataaagttt 420

gcgatctctt ctgttctgaa taatttttag taaaagaaat caaaagaata aagaaatagt 480

ccgctttgtc caatacaaca gcttaaaccg attatctcta aaataacaag aagaa 535

<210> 51
<211> 505
<212> DNA
<213> other sequences

<220>
<223> nucleic acid sequence of promotor pCUP1

<400> 51
cggcaaactt caacgatttc tatgatgcat tttataatta gtaagccgat cccattaccg 60

acatttgggc gctatacgtg catatgttca tgtatgtatc tgtatttaaa acacttttgt 120

attatttttc ctcatatatg tgtataggtt tatacggatg atttaattat tacttcacca 180

ccctttattt caggctgata tcttagcctt gttactagtt agaaaaagac atttttgctg 240

tcagtcactg tcaagagatt cttttgctgg catttcttct agaagcaaaa agagcgatgc 300

gtcttttccg ctgaaccgtt ccagcaaaaa agactaccaa cgcaatatgg attgtcagaa 360

tcatataaaa gagaagcaaa taactccttg tcttgtatca attgcattat aatatcttct 420

tgttagtgca atatcatata gaagtcatcg aaatagatat taagaaaaac aaactgtaca 480

atcaatcaat caatcatcac ataaa 505

<210> 52
<211> 510
<212> DNA
<213> other sequences

<220>
<223> nucleic acid sequence of promotor pMET6

<400> 52
ccacaggaaa tatttcacgt gacttacaaa cagagtcgta cgtcaggacc ggagtcaggt 60

gaaaaaatgt gggccggtaa agggaaaaaa ccagaaacgg gactactatc gaactcgttt 120

agtcgcgaac gtgcaaaagg ccaatatttt tcgctagagt catcgcagtc atggcagctc 180

tttcgctcta tctcccggtc gcaaaactgt ggtagtcata gctcgttctg ctcaattgag 240

aactgtgaat gtgaatatgg aacaaatgcg atagatgcac taatttaagg gaagctagct 300

agttttccca actgcgaaag aaaaaaagga aagaaaaaaa aattctatat aagtgataga 360

tatttccatc tttactagca ttagtttctc ttttacgtat tcaatatttt tgttaaactc 420

ttcctttatc ataaaaaagc aagcatctaa gagcattgac aacactctaa gaaacaaaat 480

accaatataa tttcaaagta catatcaaaa 510

<210> 53
<211> 686
<212> DNA
<213> other sequences

<220>
<223> nucleic acid sequence of promotor pMET25

<400> 53
ttacattatc aatccttgcg tttcagcttc cactaattta gatgactatt tctcatcatt 60

tgcgtcatct tctaacaccg tatatgataa tatactagta acgtaaatac tagttagtag 120

atgatagttg atttttattc caacactaag aaataatttc gccatttctt gaatgtattt 180

aaagatattt aatgctataa tagacattta aatccaattc ttccaacata caatgggagt 240

ttggccgagt ggtttaaggc gtcagattta ggtggattta acctctaaaa tctctgatat 300

cttcggatgc aagggttcga atcccttagc tctcattatt ttttgctttt tctcttgagg 360

tcacatgatc gcaaaatggc aaatggcacg tgaagctgtc gatattgggg aactgtggtg 420

gttggcaaat gactaattaa gttagtcaag gcgccatcct catgaaaact gtgtaacata 480

ataaccgaag tgtcgaaaag gtggcacctt gtccaattga acacgctcga tgaaaaaaat 540

aagatatata taaggttaag taaagcgtct gttagaaagg aagtttttcc tttttcttgc 600

tctcttgtct tttcatctac tatttccttc gtgtaataca gggtcgtcag atacatagat 660

acaattctat tacccccatc cataca 686

<210> 54
<211> 363
<212> DNA
<213> other sequences

<220>
<223> nucleic acid sequence of promotor pSAM1

<400> 54
gaaacggacg taagacggaa atagaatttg aagataaagt tatatatcac tacacacgaa 60

tactttcttt tttttttttc acaggaaaac tgtggtggcg cccttgccta ctagtgcatt 120

tcttttttcg ggttcttgtc tcgacgaaat tttagcctca tcgtagtttt tcactctggt 180

atcgatgaaa aagggaagag taaaaagttt tccgtttagt acttaatggg attggtttgg 240

gacgtatata tcgactggtg ttgtctgtta ttcatcgttg tttttcggtt agcttcgaaa 300

aaaaaataga gtaaaaacca ggaatttacc ctaaaaacaa gaaaaaataa gataaacgaa 360

aat                                                                 363

<210> 55
<211> 300
<212> DNA
<213> other sequences

<220>
<223> nucleic acid sequence of terminator tTPI1

<400> 55
gattaatata attatataaa aatattatct tcttttcttt atatctagtg ttatgtaaaa      60

taaattgatg actacggaaa gcttttttat attgtttctt tttcattctg agccacttaa     120

atttcgtgaa tgttcttgta agggacggta gatttacaag tgatacaaca aaaagcaagg     180

cgctttttct aataaaaaga agaaaagcat ttaacaattg aacacctcta tatcaacgaa     240

gaatattact ttgtctctaa atccttgtaa aatgtgtacg atctctatat gggttactca     300

<210> 56
<211> 299
<212> DNA
<213> other sequences

<220>
<223> nucleic acid sequence of terminator tMET25

<400> 56
gtgtgcgtaa tgagttgtaa aattatgtat aaacctactt tctctcacaa gtactatact      60

tttataaaac gaactttatt gaaatgaata tccttttttt cccttgttac atgtcgtgac     120

tcgtactttg aacctaaatt gttctaacat caaagaacag tgttaattcg cagtcgagaa     180

gaaaaatatg gtgaacaaga ctcatctact tcatgagact actttacgcc tcctataaag     240

ctgtcacact ggataaattt attgtaggac caagttacaa aagaggatga tggaggttt      299

<210> 57
<211> 300
<212> DNA
<213> other sequences

<220>
<223> nucleic acid sequence of terminator tDIT1

<400> 57
taaagtaaga gcgctacatt ggtctacctt tttgttcttt tacttaaaca ttagttagtt      60

cgttttcttt ttctcatttt tttatgtttc ccccccaaag ttctgatttt ataatatttt     120

atttcacaca attccattta acagaggggg aatagattct ttagcttaga aaattagtga     180

tcaatatata tttgcctttc ttttcatctt ttcagtgata ttaatggttt cgagacactg     240

caatggccct agttgtctaa gaggatagat gttactgtca aagatgatat tttgaatttc 300

<210> 58
<211> 300
<212> DNA
<213> other sequences

<220>
<223> nucleic acid sequence of terminator tRPL3

<400> 58
gaagttttgt tagaaaataa atcatttttt aattgagcat tcttattcct attttattta 60

aatagtttta tgtattgtta gctacataca acagtttaaa tcaaattttc tttttcccaa 120

gtccaaaatg gaggtttatt ttgatgaccc gcatgcgatt atgttttgaa agtataagac 180

tacatacatg tacatatatt taaacatgta aacccgtcca ttatattgct tactttcttc 240

ttttttgccg ttttgacttg gacctctggt ttgctatttc cttacaatct ttgctacaat 300

<210> 59
<211> 304
<212> DNA
<213> other sequences

<220>
<223> nucleic acid sequence of terminator tRPL3.sm

<400> 59
gaagttttta aagcattttt tagacacttc tcatttttct aagttttttt aaaatagttt 60

tatgtattta ctacgtatca caatttgaaa taattcatct tcccaaaaaa ctaagatttt 120

tatccttgtc acgatccgta accagtttat aatattttag agcttataca cgtacgtata 180

cacacgtgtc ggtacatgag aattacgttc aaaattattc actttttttt tctctgccgt 240

tttacttttg aactctgtct cgctatttcc ttacaatctt cgctacaata ccacttgccc 300

ttgg 304

<210> 60
<211> 299
<212> DNA
<213> other sequences

<220>
<223> nucleic acid sequence of terminator tRPL3.sba

<400> 60
gaagttttct gacaaaaaca taacgttttt tccaatcatt tcttattttt ccggtttatt 60

taaatagttt ttatgtacta ttatacgtat gactatttaa cttaaattct tcctcccaag 120

aaatctccca agtttttcat tatcatggca tacaccacta tcagttacaa aatggtagct 180

caaccatata tatatctcta tatacacata taaatgcaaa caggtccaag tcaccgctca 240

ctgcagtttc ttttgccgtt ttgacttcga tctctgcttg gctattttct cacaatcct 299

<210> 61
<211> 300
<212> DNA
<213> other sequences

<220>
<223> nucleic acid sequence of terminator tRPL41B

<400> 61
gcggattgag agcaaatcgt taagttcagg tcaagtaaaa attgatttcg aaaactaatt 60

tctcttatac aatcctttga ttggaccgtc atcctttcga atataagatt ttgttaagaa 120

tattttagac agagatctac tttatattta atatctagat attacataat ttcctctcta 180

ataaaatatc attaataaaa taaaaatgaa gcgatttgat tttgtgttgt caacttagtt 240

tgccgctatg cctcttgggt aatgctatta ttgaatcgaa gggctttatt atattaccct 300

<210> 62
<211> 300
<212> DNA
<213> other sequences

<220>
<223> nucleic acid sequence of terminator tRPL15A

<400> 62
gctggttgat ggaaaatata attttattgg gcaaactttt gtttatctga tgtgttttat 60

actattatct ttttaattaa tgattctata tacaaacctg tatatttttt ctttaaccaa 120

tttttttttt tatagaccta gagctgtact tttattctgc tatcaagcaa acccctaccc 180

cctcttctca atcctcccct caggcagaac ttatctacct gtatcaagga gcggacgagg 240

gagtcctaat tgttctacgt ataccaatgc tagcagctta cataggtggt ggcactacca 300

<210> 63
<211> 300
<212> DNA
<213> other sequences

<220>
<223> nucleic acid sequence of terminator tRPL15A.sba

<400> 63
gctggctgat gaaaaatata gttctgttgg gcaagcattt gtttacctag catctctttt 60

atactattat tatctttata tttgatgatt ttatatacaa gttgtatacc ttttctttaa 120

ccaatttttt tttttctaat ggtgcaccta gaagtacatt ttttctcacc aatagatagt 180

caagatactc ccagcctcta tggcgttacc acggagccga caagggaaag ctgcttatct    240

tacatatgca gatgccaagg cccgttacag gccgccttat tgatgtttag aaatagcttc    300

<210> 64
<211> 300
<212> DNA
<213> other sequences

<220>
<223> nucleic acid sequence of terminator tIDP1

<400> 64
tcgaatttac gtagcccaat ctaccacttt tttttttcat tttttaaagt gttatactta     60

gttatgctct aggataatga actacttttt tttttttttt tttactgtta tcataaatat    120

atatacctta ttgttgtttg caaccgtcgg ttaattcctt atcaaggttc cccaagttcg    180

gatcattacc atcaatttcc aacattttca tgagttcttc ttcttcatta ccgtgtttta    240

gggggctgtt cgcacttcta atagggctat caccaagctg ttctaattcg tccaaaagtt    300

<210> 65
<211> 300
<212> DNA
<213> other sequences

<220>
<223> nucleic acid sequence of terminator tTEF1.sba

<400> 65
ggagattgat aggacttttc tagttgcata tcttttattt ttaaatctta tctattagtt     60

aattttttgt aatttatcct tatatatata gtttggttat tctaaaacat catttcagta    120

tctaaaacct ctcttattca ttaccttttt atttaatggt ttttgctaca ggcaaaaatt    180

taatggtttt tgctacaggc aaaaatcccg ccgtggactt attccacgtt aactcggtta    240

cagggtcatg aaccattttg tcaattatcg aaataacttc ttcaaaagtc cctcttactt    300

<210> 66
<211> 60
<212> DNA
<213> other sequences

<220>
<223> nucleic acid sequence of the secretion sequence added in 5â\200\231

<400> 66
atgcaattta gcacagtcgc atcagtagcc ttcgttgcct tggccaactt cgtggcagca     60

<210> 67
<211> 20

<212> PRT
<213> other sequences

<220>
<223> amino acid sequence of the secretion sequence added in N-ter

<400> 67
Met Gln Phe Ser Thr Val Ala Ser Val Ala Phe Val Ala Leu Ala Asn
1               5                   10                  15
Phe Val Ala Ala
            20

<210> 68
<211> 213
<212> DNA
<213> other sequences

<220>
<223> nucleic acid sequence of the anchoring sequence added in 3â\200\231

<400> 68
ggatccgcca tttctcaaat cactgacggt caaatccaag ctactaccac tgctaccacc      60

gaagctacca ccactgctgc cccatcttcc accgttgaaa ctgtttctcc atccagcacc     120

gaaactatct ctcaacaaac tgaaaatggt gctgctaagg ccgctgtcgg tatgggtgcc     180

ggtgctctag ctgctgctgc tatgttgtta taa                                  213

<210> 69
<211> 68
<212> PRT
<213> other sequences

<220>
<223> amino acid sequence of the anchoring sequence added in C-ter

<400> 69
Ala Ile Ser Gln Ile Thr Asp Gly Gln Ile Gln Ala Thr Thr Thr Ala
1               5                   10                  15
Thr Thr Glu Ala Thr Thr Thr Ala Ala Pro Ser Ser Thr Val Glu Thr
            20                  25                  30
Val Ser Pro Ser Ser Thr Glu Thr Ile Ser Gln Gln Thr Glu Asn Gly
        35                  40                  45
Ala Ala Lys Ala Ala Val Gly Met Gly Ala Gly Ala Leu Ala Ala Ala
    50                  55                  60
Ala Met Leu Leu
65

## Description

### FIELD OF THE INVENTION

The present invention relates to the field of bio-production of heparosan.

### BACKGROUND OF THE INVENTION

Heparosan, also known as N-acetylheparosan, is the natural eukaryotic biosynthesis precursor of heparin and heparan sulfate found in animals from hydra to vertebrates. It is more particularly a polysaccharide with the repeating diassachride unit [→4) β-D-glucuronic acid (GlcA) (1→4) N-acetyl- a-D- glucosamine (GlcNAc) (1→]_{n.}. In nature, the *Escherichia coli K5* and the *Pasteurella multocida* type D strains produce heparosan as a capsular polysaccharide (Lindahl U. et al. (1998) J. Biol. Chem., 273(39):24979-24982).

Derivates from heparosan bind to a variety of extremely important polypeptides including hemostasis factors (e.g., antithrombin III, thrombin), growth factors (e.g., EGF, VEGF), and chemokines (e.g., IL-8, platelet factor 4) as well as the adhesive proteins for viral pathogens (e.g., herpes, Dengue fever). Among these derivatives, heparin, one of the most widely used anticoagulant or antithrombotic drug, is for example useful in therapeutic treatments of thromboembolism and disseminated intravascular coagulation (DIC), extracorporeal circulation and prevention of blood coagulation during artificial dialysis. In addition, recent studies suggest that heparin may have significant antineoplastic activity, separate and distinct from its anticoagulant activity, while other studies indicate a role for heparin in treating inflammation, infertility, and infectious disease.

Today's main known sources of heparin are extractions from animal tissue, such as porcine intestinal mucosa, with the attendant risks of adulteration and contamination.

Fermentation methods for preparing heparosan from microorganisms are particularly of interest since they facilitate the production of a large quantity of heparosan through possible scale-up, at a reduced cost. Moreover, contrary to isolating heparosan and its derivatives from animal sources which provide heparosan of very high molecular weights, microbial fermentation allows to control, to a certain extent, the size of the starting molecular weight. This avoids the need for further fractionation steps by mechanical, physical or chemical means.

In this aim, recombinant *Bacillus megaterium* host cells have shown the ability to produce heparosan in the range of 10 to 300 KDa (A. Williams et al. Microb. Cell. Fact. 2009 Aug 12; 18(1): 132). Similarly, *Escherichia coli* K5 strains shown the ability to produce heparosan (Wang Z. et al. (2010) Biotechnol. Bioeng., 107(6):964-973), but there are still many issues that remain to be resolved, such as the improvement of the substrate consumption rate and the increase of the oxygen supply in fermentation tank.

Contrary to other microorganisms that are commonly used for the production of biological molecules, yeasts are generally recognised as safe. They can grow rapidly and be cultivated at higher density, compared to bacteria, and do not require an aseptic environment. Furthermore, yeast cells can be more easily separated from the culture medium than bacteria, greatly simplifying the process for product extraction and purification. Finally, yeasts present the advantage of being more resistant to pH changes in the culture medium, and as such represent stronger fermentation systems.

However, among yeasts, distinguishing features between species may also present certain challenges. This is mainly due to the difference in metabolisms between the species. For example, genome integrations in *P. pastoris* are stable but often a high variability of clones from one transformation is encoutenred, displaying various productivity characteristics or changes in their physiology. This requires a time-consuming screening process so as to find the clone with the optimal features for the desired application. This clonal variability is an important drawback for the further development of *P. pastoris* as a platform for producing value-added chemicals. Another drawback of using *P. pastoris* is that it is a methanotrophic organism.

On the contrary, *Saccharomyces cerevisiae* is particularly useful as a tool for the production of molecules of interest since it has a long safe history when it comes to being used by humans (such as in wine, beer, or bread) and as such is a well-established model whose genetic information is well known in the art. S. *cerevisiae* further presents the advantage of being generally recognised as safe for humans and animals. What's more, the acidification of the medium which occurs when cultivating this yeast reduces the possibility of contamination of the bio fermenters, and therefore eliminates the need to add antibiotics to the medium. Finally, many genetic tools have been developed allowing for stable modifications of its genome (integration within the chromosome).

Accordingly, there is still a need in the art for further heparosan production methods allowing its highly efficient synthesis and secretion. In particular, there is still a need to provide production methods that are cost-efficient and supply heparosan which is safe for human application.

In a particular case, there is still a need in the art for heparosan production methods allowing to obtain large amounts of heparosan of a particular and controlled size.

### SUMMARY OF THE INVENTION

The present invention accordingly relates to the following items:
**Item 1:** A recombinant yeast cell producing heparosan wherein the recombinant cell comprises:
(a) one or more recombinant nucleic acids encoding a polypeptide having a heparosan synthase (HSS) activity; and
(b) one or more recombinant nucleic acids encoding a polypeptide having a UDP-Glucose dehydrogenase (UDP-GlcDH or HASB) activity.

As illustrated in the examples, the recombinant yeasts of the invention allow for the production of heparosan in a yeast cell which is not naturally able to produce heparosan.

Said advantageous properties can be further increased by recombining the yeast with additional modifications described here-after.

**Item 2:** The recombinant cell according to item 1 wherein the recombinant cell comprises a nucleic acid encoding a polypeptide having a glucuronosyl-disulfoglucosamine glucuronidase activity, wherein the polypeptide having glucuronosyl-disulfoglucosamine glucuronidase activity comprises a secretion signal and optionaly an anchoring signal.

**Item 3:** A recombinant host cell producing heparosan wherein the recombinant cell comprises:
(a) one or more recombinant nucleic acids encoding a polypeptide having a heparosan synthase (HSS) activity;
(b) one or more recombinant nucleic acids encoding a polypeptide having a UDP-Glucose dehydrogenase (UDP-GlcDH or HASB) activity; and
(c) one or more recombinant nucleic acids encoding a polypeptide having a glucuronosyl-disulfoglucosamine glucuronidase activity,
wherein the polypeptide having a glucuronosyl-disulfoglucosamine glucuronidase activity comprises a secretion signal, and optionaly an anchoring signal, so that heparosan, in particular of a desired molecular weight is produced by the host cell.

**Item 4:** The recombinant cell according to item 2 or 3, wherein the nucleic acid encoding a polypeptide having glucuronosyl-disulfoglucosamine glucuronidase activity is obtained or derived from *Pedobacter heparinus.*

**Item 5:** The recombinant cell according to any one of items 2 to 4, wherein the molecular weight of the heparosan is in the range of less than 50kDa, preferably is in the range of about 20kDa to about 50kDa.

**Item 6:** The recombinant cell according to any one of items 2 to 4, wherein the molecular weight of the heparosan is in the range of greater than 50kDa, preferably in the range of about 50kDa to about 250kD.

**Item 7:** The recombinant cell according to any one of items 2 to 4 wherein the molecular weight of the heparosan is in the range of greater than 100kDa, preferably is in the range of about 100kDa to about 1500kDa.

**Item 8:** The recombinant cell according to any one of items 1 to 7, wherein the nucleic acid encoding a polypeptide having a UDP-Glucose dehydrogenase (UDP-GlcDH or HASB) activity is obtained or derived from at least one of *Arabidopsis thaliana, Chlorella virus PBCV1* or *Streptococcus zooepidemicus,* and is in particular from *Arabidopsis thaliana* or *Chlorella virus PBCV1.*

**Item 9:** The recombinant cell according to any one of items 1 to 8, wherein the nucleic acid encoding a polypeptide having a heparosan synthase (HSS) activity is obtained or derived from *Pasteurella multocida.*

**Item 10:** The recombinant cell according to any one of items 1 to 9, wherein the recombinant cell further comprises at least one recombinant nucleic acid encoding one or more of:
(i) a polypeptide having a glutamine-fructose-6-phosphate amidotransferase (GFA1) activity; and/or
(ii) a polypeptide having a UDP-N-acetylglucosamine pyrophosphorylase (QRI1) activity.

**Item 11:** The recombinant cell according to any one of items 1 to 10, wherein the recombinant cell further comprises at least one recombinant nucleic acid encoding one or more of:
(i) a polypeptide having a Phosphoglucomutase-1 (PGM1) activity; and/or
(ii) a polypeptide having a UTP-glucose-1-phosphate uridylyltransferase (UGP1) activity; and/or
(iii) a polypeptide having a Glucosamine-6-phosphate N-acetyltransferase (GNA1) activity; and/or
(iv) a polypeptide having a phosphoacetylglucosamine mutase (PCM1) activity.

**Item 12:** The recombinant cell according to item 10 or 11, wherein:
- the nucleic acid encoding the polypeptide having glutamine-fructose-6-phosphate amidotransferase (GFA1) activity; and/or
- the nucleic acid encoding the polypeptide having UDP-N-acetylglucosamine pyrophosphorylase (QRI1) activity; and/or
- the nucleic acid encoding the polypeptide having Phosphoglucomutase-1 (PGM1) activity; and/or
- the nucleic acid encoding the polypeptide having UTP-glucose-1-phosphate uridylyltransferase (UGP1) activity; and/or
- the nucleic acid encoding the polypeptide having Glucosamine-6-phosphate N-acetyltransferase (GNA1) activity; and/or
- the nucleic acid encoding the polypeptide having phosphoacetylglucosamine mutase (PCM1);
are obtained or derived from *Saccharomyces cerevisiae.*

**Item 13:** The recombinant host cell according to any one of items 3 to 12, wherein the recombinant host cell is a yeast.

**Item 14:** The recombinant cell according to any one of items 1 to 13, wherein the recombinant cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus, and in particular belongs to the *Saccharomyces* genus.

**Item 15:** The recombinant cell according to item 14, wherein the recombinant cell is selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

**Item 16:** A method of producing heparosan comprising:
(a) cultivating a recombinant cell as defined in any one of items 1 to 15 in a cultivation medium for a time sufficient to produce heparosan; and
(b) optionally isolating or recovering the heparosan from the recombinant cell and/or from the cultivation medium.

**Item 17:** The method according to item 16, wherein the heparosan has a molecular weight of from about 20kDa to about 50kDa.

**Item 18:** The method according to item 16, wherein the heparosan has a molecular weight of from about 50kDa to about 150kDa.

**Item 19:** The method according to item 14, wherein the heparosan has a molecular weight of from about 150kDa to about 1500kDa.

**Item 20:** The method according to any one of items 16 to 19, wherein the recombinant cell is a yeast belonging to the *Saccharomyces* genus, and in particular is *Saccharomyces cerevisiae.*

**Item 21:** The method according to any one of items 16 to 20, wherein the time sufficient to produce heparosan is a period of from about 35 to about 50 hours, preferably from about 40 to about 50 hours, preferably of about 48 hours.

**Item 22:** The method according to any one of items 16 to 21, wherein the molecular weight of the produced heparosan is controlled by regulating the pH of the cultivation medium during step (a).

**Item 23:** The method according to any one of items 16 to 22, wherein the method is carried out on an industrial scale, preferably where the cultivation medium is at least about 100L, more preferably is in the range of about 1000L to about 3000L, even more preferably is about 10,000L or even more preferably is about 100,000L, or even is about 250,000L.

**Item 24:** Heparosan obtainable from a recombinant cell according to any one of items 1 to 15 or from the method according to any one of items 16 to 23.

**Item 25:** A cultivation medium comprising the heparosan according to item 24.

**Item 26:** A composition comprising the heparosan according to item 24.

**Item 27:** An industrial product or a consumer product or a consumable comprising (i) the heparosan according to item 24 having a molecular weight as defined in any one of items 5 to 7, (ii) the cultivation medium of item 25 or (iii) the composition of item 26.

**Item 28:** The industrial product or the consumer product or the consumable of item 27, wherein the industrial product or the consumer product or the consumable is a cosmetic product, a flavour product, a fragrance product, a food product, a food, a beverage, a texturant, a pharmaceutical composition, a dietary supplement, a nutraceutical, a cleaning product, a dental and/or an oral hygiene composition.

**Item 29:** Use of a recombinant cell as defined in any one of items 1 to 15 for the production of heparosan having a molecular weight in the range of from about 20kDa to about 50kDa or from about 50kDa to about 1000kDa.

Certain embodiments of the present invention may provide one or more of the following advantages:
- a process for producing heparosan of controlled molecular weight;
- a process for producing heparosan of controlled molecular weight in a *Saccharomyces* yeast cell; and
- a process for producing heparosan of controlled molecular weight by varying genetic parameters (eg regulatory sequences) and/or process parameters (pH or fermentation time).

### BRIEF DESCRIPTION OF THE FIGURES

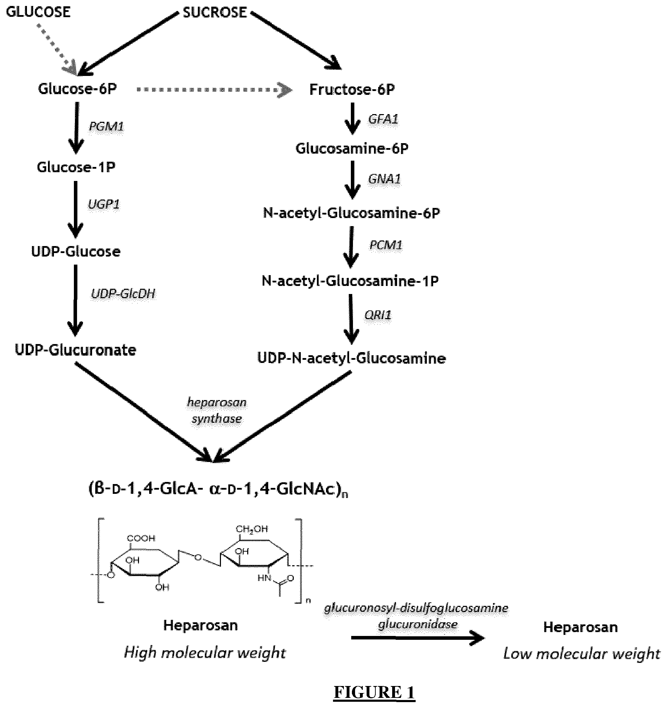

Figure 1 shows a schematic pathway for the production of heparosan.

### SUMMARY OF THE SEQUENCES

**SEQ ID NO: 1** is a reencoded nucleic acid sequence of heparosan synthase originating from *Pasteurella multocida* (HSS1)
**SEQ ID NO: 2** is a reencoded nucleic acid sequence of heparosan synthase originating from *Pasteurella multocida* (HSS2)
**SEQ ID NO: 3** is an amino acid sequence of heparosan synthase originating from *Pasteurella multocida* (HSS1)
**SEQ ID NO: 4** is an amino acid sequence of heparosan synthase originating from *Pasteurella multocida* (HSS2)
**SEQ ID NO: 5** is a nucleic acid sequence of UDP-Glucose dehydrogenase (HASB) originating from *Arabidopsis thaliana*
**SEQ ID NO: 6** is a reencoded nucleic acid sequence of UDP-Glucose dehydrogenase (HASB) originating from *Chlorella* virus PBCV-1
**SEQ ID NO: 7** is a reencoded nucleic acid sequence of UDP-Glucose dehydrogenase (HASB-A) originating from *Chlorella* virus PBCV-1
**SEQ ID NO: 8** is an amino acid sequence of UDP-Glucose dehydrogenase (HASB) originating from *Arabidopsis thaliana*
**SEQ ID NO: 9** is an amino acid sequence of UDP-Glucose dehydrogenase (HASB) originating from *Chlorella* virus PBCV1
**SEQ ID NO: 10** is a reencoded nucleic acid sequence of glucuronosyl-disulfoglucosamine glucuronidase originating from *Pedobacter heparinus* with a N-terminal secretion signal
**SEQ ID NO: 11** is a reencoded nucleic acid sequence of glucuronosyl-disulfoglucosamine glucuronidase originating from *Pedobacter heparinus* with a N-terminal secretion signal and a C-terminal anchoring signal
**SEQ ID NO: 12** is a reencoded nucleic acid sequence of glucuronosyl-disulfoglucosamine glucuronidase originating from *Pedobacter heparinus* with a N-terminal secretion signal
**SEQ ID NO: 13** is a reencoded nucleic acid sequence of glucuronosyl-disulfoglucosamine glucuronidase originating from *Pedobacter heparinus* with a N-terminal secretion signal and a C-terminal anchoring signal
**SEQ ID NO: 14** is an amino acid sequence of glucuronosyl-disulfoglucosamine glucuronidase originating from *Pedobacter heparinus* with a N-terminal secretion signal
**SEQ ID NO: 15** is an amino acid sequence of glucuronosyl-disulfoglucosamine glucuronidase originating from *Pedobacter heparinus* with a N-terminal secretion signal and a C-terminal anchoring signal
**SEQ ID NO: 16** is a nucleic acid sequence of glutamine-fructose-6-phosphate amidotransferase (GFA1) originating from *Saccharomyces cerevisiae*
**SEQ ID NO: 17** is a reencoded nucleic acid sequence of glutamine-fructose-6-phosphate amidotransferase (GFA1) originating from *Chlorella* virus 1 (PBCV-1)
**SEQ ID NO: 18** is a reencoded nucleic acid sequence of glutamine-fructose-6-phosphate amidotransferase (GFA1) originating from *Chlorella* virus 1 (PBCV-1)
**SEQ ID NO: 19** is an amino acid sequence of glutamine-fructose-6-phosphate amidotransferase (GFA1) originating from *Saccharomyces cerevisiae*
**SEQ ID NO: 20** is an amino acid sequence of glutamine-fructose-6-phosphate amidotransferase (GFA1) originating from *Chlorella* virus 1 (PBCV-1)
**SEQ ID NO: 21** is a nucleic acid sequence of UDP-N-acetylglucosamine pyrophosphorylase (QRI1) originating from *Saccharomyces cerevisiae*
**SEQ ID NO: 22** is an amino acid sequence of UDP-N-acetylglucosamine pyrophosphorylase (QRI1) originating from *Saccharomyces cerevisiae*
**SEQ ID NO: 23** is a nucleic acid sequence of Phosphoglucomutase-1 (PGM1) originating from *Saccharomyces cerevisiae*
**SEQ ID NO: 24** is an amino acid sequence Phosphoglucomutase-1 (PGM1) originating from *Saccharomyces cerevisiae*
**SEQ ID NO: 25** is a nucleic acid sequence of UTP--glucose-1-phosphate uridylyltransferase (UGP1) originating from *Saccharomyces cerevisiae*
**SEQ ID NO: 26** is an amino acid sequence of UTP--glucose-1-phosphate uridylyltransferase (UGP1) originating from *Saccharomyces cerevisiae*
**SEQ ID NO: 27** is a nucleic acid sequence of Glucosamine 6-phosphate N-acetyltransferase (GNA1) originating from *Saccharomyces cerevisiae*
**SEQ ID NO: 28** is an amino acid sequence of Glucosamine 6-phosphate N-acetyltransferase (GNA1) originating from *Saccharomyces cerevisiae*
**SEQ ID NO: 29** is a nucleic acid sequence of phosphoacetylglucosamine mutase (PCM1) originating from *Saccharomyces cerevisiae*
**SEQ ID NO: 30** is an amino acid sequence of phosphoacetylglucosamine mutase (PCM1) originating from *Saccharomyces cerevisiae*
**SEQ ID NO: 31** is a nucleic acid sequence of promotor pTDH3
**SEQ ID NO: 32** is a nucleic acid sequence of promotor pTDH3.Sk
**SEQ ID NO: 33** is a nucleic acid sequence of promotor pTDH3-1.sba
**SEQ ID NO: 34** is a nucleic acid sequence of promotor pTDH3.Sar
**SEQ ID NO: 35** is a nucleic acid sequence of promotor pENO2
**SEQ ID NO: 36** is a nucleic acid sequence of promotor pTEF3
**SEQ ID NO: 37** is a nucleic acid sequence of promotor pTEF1
**SEQ ID NO: 38** is a nucleic acid sequence of promotor pTEF1.ago
**SEQ ID NO: 39** is a nucleic acid sequence of promotor pTEF1.Sba
**SEQ ID NO: 40** is a nucleic acid sequence of promotor pPDC1
**SEQ ID NO: 41** is a nucleic acid sequence of promotor pCCW12
**SEQ ID NO: 42** is a nucleic acid sequence of promotor pCCW12.Sm
**SEQ ID NO: 43** is a nucleic acid sequence of promotor pCCW12.Sk
**SEQ ID NO: 44** is a nucleic acid sequence of promotor pCCW12.Sba
**SEQ ID NO: 45** is a nucleic acid sequence of promotor pCCW12.Sar
**SEQ ID NO: 46** is a nucleic acid sequence of promotor pNUP57
**SEQ ID NO: 47** is a nucleic acid sequence of promotor pCCW10.ago
**SEQ ID NO: 48** is a nucleic acid sequence of promotor pCWP2
**SEQ ID NO: 49** is a nucleic acid sequence of promotor pCCW120.Sca
**SEQ ID NO: 50** is a nucleic acid sequence of promotor pRPLA1
**SEQ ID NO: 51** is a nucleic acid sequence of promotor pCUP1
**SEQ ID NO: 52** is a nucleic acid sequence of promotor pMET6
**SEQ ID NO: 53** is a nucleic acid sequence of promotor pMET25
**SEQ ID NO: 54** is a nucleic acid sequence of promotor pSAM1
**SEQ ID NO: 55** is a nucleic acid sequence of terminator tTPI1
**SEQ ID NO: 56** is a nucleic acid sequence of terminator tMET25
**SEQ ID NO: 57** is a nucleic acid sequence of terminator tDIT1
**SEQ ID NO: 58** is a nucleic acid sequence of terminator tRPL3
**SEQ ID NO: 59** is a nucleic acid sequence of terminator tRPL3.sm
**SEQ ID NO: 60** is a nucleic acid sequence of terminator tRPL3.sba
**SEQ ID NO: 61** is a nucleic acid sequence of terminator tRPL41B
**SEQ ID NO: 62** is a nucleic acid sequence of terminator tRPL15A
**SEQ ID NO: 63** is a nucleic acid sequence of terminator tRPL15A.sba
**SEQ ID NO: 64** is a nucleic acid sequence of terminator tIDP1
**SEQ ID NO: 65** is a nucleic acid sequence of terminator tTEF1.sba
**SEQ ID NO: 66** is a nucleic acid sequence of the secretion sequence added in 5'
**SEQ ID NO: 67** is an amino acid sequence of the secretion sequence added in N-ter
**SEQ ID NO: 68** is a nucleic acid sequence of the anchoring sequence added in 3'
**SEQ ID NO: 69** is an amino acid sequence of the anchoring sequence added in C-ter

### DETAILED DESCRIPTION OF THE INVENTION

The inventors have conceived genetically modified cells, and especially genetically modified yeasts, having the ability to produce heparosan, as compared to the parent cells, and especially as compared to the parent yeasts which are not naturally capable of doing so.

These genetically modified cells are described throughout the present specification.

### Definitions

The term "heparosan" is a polysaccharide a polymer composed of [-4-N-acetylglucosamine-α1,4-glucuronic acid-β1-] repeats ([→4) β-D-glucuronic acid (GlcA) (1→4) N-acetyl-a-D-glucosamine (GlcNAc) (1→]ₙ).

Heparosan may be produced in a recombinant cell.

As used herein, the term "recombinant", when used in reference to a cell, indicates that the cell has been modified by the introduction of an endogenous and/or heterologous nucleic acid or protein into the cell or the alteration of a native cell or that the cell is derived from a cell so modified. Thus, for example, recombinant cells express genes or nucleic acid that are not found within the native (non-recombinant) form of the cell or express native (eg endogenous) genes at a different level than their native level or express additional or supplementary copies of native (eg endogenous) at a different level than their native level.

As used herein, the term "recombinant", when used in reference to a nucleic acid or vector, are sequences formed/obtained by technics of genetic engineering well known to the man skilled in the art. The guidelines of the US National Institute for Health (NIH) accordingly indicate that:
"*recombinant [...] nucleic acids are defined as:*
*i. molecules that (a) are constructed by joining nucleic acid molecules and (b) that can replicate in a living cell, i.e., recombinant nucleic acids;*",
   which reflects the conventional use of the word "recombinant" attached to nucleic acid sequences to mean recombined following the insertion of, or joining together with, another nucleic acid.

Proteins that result from the expression of recombinant DNA or recombinant vector within living cells are also termed recombinant proteins.

The term "recombinant" is accordingly synonymous with the term "genetically modified". The term "gene" is synonymous with the term "nucleic acid" or "nucleotide sequence".
A recombinant nucleic acid sequence for use in a recombinant cell, and in particular a recombinant yeast, of the invention may be provided in the form of a nucleic acid construct. The term "nucleic acid construct" refers to as a nucleic acid molecule, either single-or doublestranded, which is isolated or derived from a native (eg endogenous) naturally occurring gene or is a heterologous nucleic acid or which has been modified to contain segments of nucleic acid which are combined and juxtaposed in a manner which would not otherwise exist in nature. The term "nucleic acid construct" is synonymous with the term "expression cassette" or "heterologous nucleic acid expression cassette" when the nucleic acid construct contains one or more regulatory elements required for expression of a coding sequence, wherein said control sequences are operably linked to said coding sequence. Non-limiting examples of regulatory elements include promoters, enhancers, silencers, terminators, and poly-A signals.

A recombinant nucleic acid sequence for use in a recombinant cell of the invention may be provided in the form of an expression vector, wherein the polynucleotide sequence is operably linked to at least one control sequence for the expression of the polynucleotide sequence in a recombinant cell.

The terms *"obtained from"* or *"originate from"* or *"originating from"* a microorganism or animal generally means that a substance (e.g., a nucleic acid molecule or polypeptide) originating from the microorganism or animal is native to that microorganism or animal.

The terms *"derived from"* a microorganism or animal generally means that a substance (e.g., a nucleic acid molecule or polypeptide) derived from the microorganism or animal is the result of modifications brought to the substance native to (i.e. present as such) in that microorganism or animal. For example, regarding a nucleic acid sequence derived from a microorganism or animal, said nucleic acid sequence can correspond to a reencoded and/or truncated version of the native nucleic acid sequence from this microorganism or animal. Other modifications well known to the man skilled in the art can be brought to the native substance of a microorganism or animal leading to the substance used being "derived from" said microorganism or animal.

As used herein, the term *"polypeptide"* refers to a molecule comprising amino acid residues linked by peptide bonds and containing more than five amino acid residues. The amino acids are identified by either the single-letter or three-letter designations. The term "protein" as used herein is synonymous with the term "polypeptide" and may also refer to two or more polypeptides. Thus, the terms "protein", "peptide" and "polypeptide" can be used interchangeably. Polypeptides may optionally be modified (e.g., glycosylated, phosphorylated, acylated, farnesylated, prenylated, sulfonated, and the like) to add functionality. Polypeptides exhibiting activity may be referred to as enzymes. It will be understood that, as a result of the degeneracy of the genetic code, a multitude of nucleotide sequences encoding a given polypeptide may be produced.

A polypeptide encoded by a recombinant nucleic acid for use in a recombinant cell, and in particular in a recombinant yeast, of the invention may comprise a signal peptide and/or a propeptide sequence. In the event that a polypeptide expressed by a recombinant cell, and in particular a recombinant yeast, of the invention comprises a signal peptide and/or a propeptide, sequence identity may be calculated over the mature polypeptide sequence.

The term *"operably linked"* as used herein refers to two or more nucleic acid sequence elements that are physically linked and are in a functional relationship with each other. For instance, a promoter is operably linked to a coding sequence if the promoter is able to initiate or regulate the transcription or expression of the coding sequence, in which case the coding sequence should be understood as being "under the control of" the promoter. Generally, when two nucleic acid sequences are operably linked, they will be in the same orientation and usually also in the same reading frame. They usually will be essentially contiguous, although this may not be required.

The term *"native"* or *"endogenous"* as used herein with reference to molecules, and in particular enzymes and nucleic acids, indicates molecules that are expressed in the organism in which they originated or are found in nature.

The term *"endogenous gene"* means that the gene was present in the cell before any genetic modification, in the wild-type strain. Endogenous genes may be overexpressed by introducing heterologous sequences in addition to, or to replace endogenous regulatory elements, or by introducing one or more additional or supplementary copies of the gene into the chromosome or a plasmid (said additional or supplementary copies being designated "exogenous or heterologous genes" or "heterologous nucleotide sequences" or "heterologous nucleic acids" as defined herein). Endogenous genes may also be modified to modulate their expression and/or activity. For example, mutations may be introduced into the coding sequence to modify the gene product or heterologous sequences may be introduced in addition to or to replace endogenous regulatory elements. Modulation of an endogenous gene may result in the up-regulation and/or enhancement of the activity of the gene product, or alternatively, in the down-regulation and/or attenuation of the activity of the endogenous gene product. Another way to enhance expression of endogenous genes is to introduce one or more additional or supplementary copies of the gene onto the chromosome or a plasmid (said supplementary copies being designated "exogenous or heterologous genes" or "heterologous nucleotide sequences" or heterologous nucleic acids" as defined herein).

By "*one or more additional or supplementary copies of a gene*" according to the invention, it is for example understood in the present invention from 1 to 50 copies, in particular from 1 to 30 copies, more particularly from 1 to 20 copies, and preferably from 1 to 10 copies. Said copies may be inserted into the same locus or into different loci of a recombinant cell of the invention.

The term *"exogenous gene"* means that the gene was introduced into a cell, by means well known to the man skilled in the art, whereas this gene is or is not naturally occurring in the wild-type cell. Cells can express exogenous genes if these genes are introduced into the cell with all the elements allowing their expression in the cell. Transforming cells with exogenous DNA is a routine task for the man skilled in the art. Exogenous genes may be integrated into the host chromosome, or be expressed extra-chromosomally from plasmids or vectors. A variety of plasmids, which differ with respect to their origin of replication and their copy number in the cell, are all known in the art. The sequence of exogenous genes may be adapted for its expression in the cell. Indeed, the man skilled in the art knows the notion of codon usage bias and how to adapt nucleic sequences for a particular codon usage bias without modifying the deduced protein. In particular embodiments, codon optimized genes express native enzymes.

The term *"heterologous gene"* or heterologous nucleic acid sequence" refers to a gene or nucleic acid sequence not normally found in a given cell in nature. As such, a heterologous nucleic acid sequence may be: (a) foreign to its host cell (i.e. is "exogenous" to the cell); (b) naturally found in the host cell (i.e. "endogenous") but present at an unnatural quantity in the cell (i.e., greater or lesser quantity than naturally found in the host cell); or (c) be naturally found in the host cell but positioned outside of its natural locus.

In the present application, all genes are referenced with their common names and with references to their nucleotide sequences and, the case arising, to their amino acid sequences. Using the references given in accession number for known genes, those skilled in the art are able to determine the equivalent genes in other organisms, bacterial strains, yeast, fungi, mammals, plants, etc. This routine work is advantageously done using consensus sequences that can be determined by carrying out sequence alignments with genes derived from other cells and designing degenerated probes to clone the corresponding gene in another organism.

The man skilled in the art knows different means to modulate, and in particular up-regulate or down-regulate, the expression of endogenous genes. For example, a way to enhance expression of, or over express, endogenous genes is to introduce one or more additional or supplementary copies of the gene onto the chromosome or a plasmid.

Another way is to replace the endogenous promoter of a gene with a stronger promoter. These promoters may be homologous or heterologous. Promoters particularly interesting in the present invention are described in more detail elsewhere in the present specification.

The nucleic acid expression construct may further comprise 5' and/or 3' recognition sequences and/or selection markers.

The term *"inducible promoter"* is used to qualify a promoter whose activity is induced, i.e. increased:
- in the presence of one or more particular metabolite(s). The higher the metabolite concentration in the medium, the stronger the promoter activity; or
- in the presence of a low concentration, or in the absence, of one or more metabolite(s). These metabolites are different from those whose increasing presence induces the activity of the promoter. The lower the metabolite concentration in the medium, the stronger the promoter activity.

The term *"repressible promoter"* is used to qualify a promoter whose activity is repressed, i.e. reduced:
- in the presence of one or more particular metabolite(s). The higher the metabolite concentration in the medium, the weaker the promoter activity; or
- in the presence of a low concentration, or in the absence, of one or more metabolite(s). These metabolites are different from those whose increasing presence represses the activity of the promoter. The lower the metabolite concentration in the medium, the weaker the promoter activity.

As used herein, the term *"anchoring signal"* when used in conjunction with a protein or polypeptide such as an enzyme (such as, for example a polypeptide having a glucuronosyl-disulfoglucosamine glucuronidase activity) means for example a first nucleic acid encoding a protein that is operably linked to a second nucleic acid encoding a protein or a polypeptide, or a first protein or polypeptide that is operably linked to a second protein or polypeptide, such as an enzyme (such as, for example a polypeptide having a glucuronosyl-disulfoglucosamine glucuronidase activity to form, for example a fusion protein), and that enables the cellular transport machinery of a cell, in particular of a S. *cerevisiae* cell, to correctly anchor and/or position in the membrane of the cell the second protein operably linked to the first protein.

As used herein, the term *"secretion signal"* when used in conjunction with a protein or polypeptide such as an enzyme (such as, for example a polypeptide having a glucuronosyl-disulfoglucosamine glucuronidase activity) means for example a first nucleic acid encoding a peptide or protein that is operably linked to a second nucleic acid encoding a protein, or a first protein that is linked to a second protein, such as an enzyme (such as, for example a polypeptide having a glucuronosyl-disulfoglucosamine glucuronidase activity to form, for example a fusion protein), and that enables the cellular transport machinery of a cell, in particular of a S. *cerevisiae* cell, to locate at least the second protein to the membrane of the cell and to secrete the second protein outside of the cell, after the first protein has, for example, been cleaved from the second protein.

As used herein the terms *"secretion signal"* and *"anchoring signal"* when used in conjunction with a protein or polypeptide such as an enzyme (such as, for example, a polypeptide having a glucuronosyl-disulfoglucosamine glucuronidase activity), mean for example a first nucleic acid encoding a peptide or protein that is operably linked to a second nucleic acid encoding a protein, or a first protein that is operably linked to a second protein, such as an enzyme (such as, for example a polypeptide having a glucuronosyl-disulfoglucosamine glucuronidase activity), and that enables the cellular transport machinery of a cell, in particular of a *S*. *cerevisiae* cell, to locate at least the second protein to the membrane of the cell wherein if the second protein is also operably linked to an "anchoring signal" the second protein is not secreted but remains attached to the membrane of the cell. In some cases, a secretion-anchoring signal may provide a dual secretion signal and anchoring signal function.

Sequences of secretion and anchoring signals, methods for the expression, anchoring and/or secretion of heterologous proteins, such as enzymes (such as, for example, glucuronosyl-disulfoglucosamine glucuronidase) on the surface of a cell (such, as for example, a yeast cell) are well known in the art (see for example, Ast et al (2013) Cell 152: 1134-1145, Ast and Schuldiner (2013) Crit Rev Biochem Mol Biol 48(3) 273-288, Van der Vaart et al (1997) Applied Environmental Microbiology 63(2) 615-620 and the entire contents of each of these publications are incorporated herein by reference).

The *"activity"* of an enzyme is used interchangeably with the term "*function*" and designates, in the context of the invention, the capacity of an enzyme to catalyze a desired reaction. The amount of an enzyme in a host cell may be altered by modifying the transcription of the gene that encodes the enzyme. This can be achieved for example by modifying the copy number of the nucleotide sequence encoding the enzyme (e.g., by using a higher or lower copy number expression vector comprising the nucleotide sequence, or by introducing additional copies of the nucleotide sequence into the genome of the host cell or by deleting or disrupting the nucleotide sequence in the genome of the host cell), by changing the order of coding sequences on a polycistronic mRNA of an operon or breaking up an operon into individual genes each with its own control elements, or by increasing the strength of the promoter or operator to which the nucleotide sequence is operably linked.

Alternatively or in addition, the copy number of an enzyme in a host cell may be altered by modifying the level of translation of an mRNA that encodes the enzyme. This can be achieved for example by modifying the stability of the mRNA, modifying the sequence of the ribosome binding site, modifying the distance or sequence between the ribosome binding site and the start codon of the enzyme coding sequence, modifying the entire intercistronic region located "upstream of' or adjacent to the 5' side of the start codon of the enzyme coding region, stabilizing the 3'-end of the mRNA transcript using hairpins and specialized sequences, modifying the codon usage of enzyme, altering expression of rare codon tRNAs used in the biosynthesis of the enzyme, and/or increasing the stability of the enzyme, as, for example, via mutation of its coding sequence.

The activity of an enzyme in a host cell can be altered in a number of ways, including, but not limited to, expressing a modified form of the enzyme that exhibits increased or decreased solubility in the host cell, expressing an altered form of the enzyme that lacks a domain through which the activity of the enzyme is inhibited, expressing a modified form of the enzyme that has a higher or lower Kcat or a lower or higher Km for the substrate, or expressing an altered form of the enzyme that is more or less affected by feed back or feed-forward regulation by another molecule in the pathway.

The terms *"encoding"* or *"coding for"* refer to the process by which a polynucleotide, through the mechanisms of transcription and translation, produces an amino-acid sequence.

The gene(s) encoding the enzyme(s) considered in the present invention can be exogenous or endogenous.

The methods implemented in the present invention preferably require the use of one or more chromosomal integration constructs for the stable introduction of a heterologous nucleotide sequence into a specific location on a chromosome or for the functional disruption of one or more target genes in a genetically modified cell. In some embodiments, disruption of a target gene prevents the expression of the related functional protein. In some embodiments, disruption of a target gene results in the expression of a non-functional protein from the disrupted gene.

Parameters of chromosomal integration constructs that may be varied in the practice of the present invention include, but are not limited to, the lengths of the homologous sequences; the nucleotide sequence of the homologous sequences; the length of the integrating sequence; the nucleotide sequence of the integrating sequence; and the nucleotide sequence of the target locus. In some embodiments, an effective range for the length of each homologous sequence is 20 to 5,000 base pairs, preferentially 50 to 100 base pairs. In particular embodiments, the length of each homologous sequence is about 50 base pairs. For more information on the length of homology required for gene targeting, see D. Burke et al., Methods in yeast Genetics - A cold spring harbor laboratory course Manual (2000).

In some embodiments, (a) disrupted gene(s) in which the above-mentioned DNA construct(s) is/are intended to be inserted may advantageously comprises one or more selectable markers useful for the selection of transformed cells. Preferably, said selectable marker(s) are comprised in the DNA construct(s) according to the present invention.

In some embodiments, the selectable marker is an antibiotic resistance marker. Illustrative examples of antibiotic resistance markers include, but are not limited to the, NAT1, AUR1-C, HPH, DSDA, KAN<R>, and SH BLE gene products. The NAT 1 gene product from *S*. *noursei* confers resistance to nourseothricin; the AUR1-C gene product from *Saccharomyces cerevisiae* confers resistance to Auerobasidin A (AbA); the HPH gene product of *Klebsiella pneumoniae* confers resistance to Hygromycin B; the DSDA gene product of *E. coli* allows cells to grow on plates with D-serine as the sole nitrogen source; the KAN<R> gene of the Tn903 transposon confers resistance to G418; and the SH BLE gene product from *Streptoalloteichus hindustanus* confers resistance to Zeocin (bleomycin).

In some embodiments, the antibiotic resistance marker is deleted after the genetically modified cell of the invention is isolated. The man skilled in the art is able to choose suitable marker in specific genetic context.

In a particular embodiment, a recombinant cell according to the invention is devoid of any antibiotic resistance marker. This advantageously prevents the necessity to add antibiotics in the selection medium.

In some embodiments, the selectable marker rescues an auxotrophy (e.g., a nutritional auxotrophy) in the genetically modified cell. In such embodiments, a parent cell, and in particular a parent yeast, comprises a functional disruption in one or more gene products that function in an amino acid or nucleotide biosynthetic pathway, such as, for example, the HIS3, LEU2, LYS1, LYS2, MET15, TRP1, ADE2, and URA3 gene products in yeast, which renders the parent cell incapable of growing in media without supplementation with one or more nutrients (auxotrophic phenotype). The auxotrophic phenotype can then be rescued by transforming the parent cell with a chromosomal integration encoding a functional copy of the disrupted gene product (in some embodiments the functional copy of the gene may originate from close species, such as *Kluveromyces*, *Candida* etc.), and the genetically modified cell generated can be selected based on the loss of the auxotrophic phenotype of the parent microbial cell.

For each of the nucleic acid sequences comprising a promoter sequence, a coding sequence (e.g. an enzyme coding sequence), or a terminator sequence, reference sequences are described herein. The present description also encompasses nucleic acid sequences having specific percentages of nucleic acid identity with a reference nucleic acid sequence.

For each or the amino acid sequences of interest, reference sequences are described herein. The present description also encompasses amino acid sequences (e.g. enzyme amino acid sequences), having specific percentages of amino acid identity with a reference amino acid sequence.

For obvious reasons, in all the present description, a specific nucleic acid sequence or a specific amino acid sequence which complies with, respectively, the considered nucleotide or amino acid identity, should further lead to obtaining a protein (or enzyme) which displays the desired biological activity. As used herein, the "percentage of identity" between two nucleic acid sequences or between two amino acid sequences is determined by comparing both optimally aligned sequences through a comparison window.

The portion of the nucleotide or amino-acid sequence in the comparison window may thus include additions or deletions (for example "gaps") as compared to the reference sequence (which does not include these additions or these deletions) so as to obtain an optimal alignment between both sequences.

The terms "sequence homology" or "sequence identity" or "homology" or "identity" are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region.

A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. and Bleasby,A. Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al (1990) J. Mol. Biol. 215, 403-410. BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al (1997) Nucleic Acids Res. 25, 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

The *"fermentation"* or *"culture"* is generally conducted in fermenters with an appropriate culture medium adapted to the cell being cultivated, containing at least one simple carbon source, and if necessary co-substrates.

The term *"fermentation composition"* refers to a composition which comprises genetically modified host cells and products or metabolites produced by the genetically modified host cells. An example of a fermentation composition is a whole cell broth, which can be the entire contents of a vessel (e.g., a flasks, plate, or fermentor), including cells, aqueous phase, and compounds produced from the genetically modified host cells.

The term "medium" refers to a culture medium or cultivation medium or fermentation medium.

For maximal production of heparosan, the recombinant cells used as production hosts preferably have a high rate of carbohydrate utilization. These characteristics may be conferred by mutagenesis and selection, genetic engineering, or may be natural. Fermentation media, or *"culture medium"* or *"cultivation medium",* for the present cells may contain at least about 10 g/L of glucose and/or sucrose. Additional carbon substrates may include but are not limited to monosaccharides such as fructose, mannose, xylose and arabinose; oligosaccharides such as lactose, maltose, galactose, or sucrose; polysaccharides such as starch or cellulose or mixtures thereof and unpurified mixtures from renewable feedstocks such as cheese whey permeate cornsteep liquor, sugar beet molasses, and barley malt. Other carbon substrates may include glycerol, acetate and/or ethanol.

Hence, it is contemplated that the source of carbon utilized in the present invention may encompass a wide variety of carbon containing substrates and will only be limited by the choice of cell, and in particular of yeast.

Although it is contemplated that all of the above-mentioned carbon substrates and mixtures thereof are suitable in the present invention, preferred carbon substrates are glucose, fructose, and sucrose, or mixtures of these with C5 sugars such as xylose and/or arabinose for cells, and in particular yeasts, modified to use C5 sugars, and more particularly glucose.

Preferred carbon substrates are glucose or sucrose.

In addition to an appropriate carbon source, fermentation media may contain suitable minerals, salts, cofactors, buffers and other components, known to those skilled in the art, suitable for the growth of the cultures and promotion of the enzymatic pathway necessary for the production of the desired product.

Besides, additional genetic modifications suitable for the growth of recombinant cells according to the invention may be considered.

The terms *"Aerobic conditions"* refers to concentrations of oxygen in the culture medium that are sufficient for an aerobic or facultative anaerobic cell, and in particular yeast, to use di-oxygene as a terminal electron acceptor.

"*Microaerobic condition"* refers to a culture medium in which the concentration of oxygen is less than that in air, i.e. oxygen concentration up to 6% O₂.

An *"appropriate culture medium"* designates a medium (e.g. a sterile, liquid medium) comprising nutrients essential or beneficial to the maintenance and/or growth of the cell such as carbon sources or carbon substrate, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids, vitamins, growth promoters, and the like. The term "carbon source" or "carbon substrate" or "source of carbon" according to the present invention denotes any source of carbon that can be used by those skilled in the art to support the normal growth of a cell, including hexoses (such as glucose, galactose or lactose), pentoses, monosaccharides, oligosaccharides, disaccharides (such as sucrose, cellobiose or maltose), molasses, starch or its derivatives, cellulose, hemicelluloses and combinations thereof.

Culture mediums that are particularly appropriate to produce recombinant cells of the invention, and in particular recombinant yeasts of the invention, are described in greater details further below in the text.

By "*controlled*" molecular weight of a heparosan of the invention is intended to mean that at least 80%, in particular at least 85%, of the heparosan produced by a method of the invention has a molecular weight comprised within a pre-determined molecular weight's range through the regulation of at least one parameter of the method and/or of the recombinant cell of the invention, such as for example:
- the nature and origin of the nucleic acid encoding a polypeptide having glucuronosyl-disulfoglucosamine glucuronidase activity of the recombinant cell,
- the nature and origin of the promoter controlling the expression of the nucleic acid encoding the polypeptide having glucuronosyl-disulfoglucosamine glucuronidase activity of the recombinant cell,
- the presence of an anchoring and/or of a secretion signal associated to the encoded polypeptide having glucuronosyl-disulfoglucosamine glucuronidase activity of the recombinant cell,
- the pH of the culture medium during the step of culturing the recombinant cell, and/or
- the duration of the culturing of the recombinant cell.

As used herein, the term "*about*" refers to a reasonable range about a value as determined by the practitioner of skill. In certain embodiments, the term about refers to ± one, two, or three standard deviations. In certain embodiments, the term about refers to ± 5%, 10%, 20%, or 25%. In certain embodiments, the term about refers to ± 0.1, 0.2, or 0.3 logarithmic units, e.g. pH units.

### General features of genetic modifications introduced according to the invention

- All the genome modifications are inserted in recombinant cells, and in particular recombinant yeasts, according to known genetic engineering techniques:
- The successive nucleic acid sequences included in a gene construct that is introduced in the recombinant cell genome according to the invention are of the following structure:
   Prom₁-ORF₁-term₁-ORF₂-gene₂-term₂ - .../... -Promₙ-ORFₙ-termₙ, wherein:
   - Prom1 is a sequence regulating the expression of the coding sequence ORF1,
   - ORF1 is a nucleic acid sequence encoding a desired protein PROT1, and especially a desired enzyme PROT1,
   - Term1 is a transcription terminator sequence that mediates transcriptional termination by providing signals in the newly synthesized mRNA that trigger processes which release the mRNA from the transcriptional complex, and
   - "1", "2", .../... "n" may or may not describe the same ORF (Open Reading Frame), promoter or terminator. The order of the nucleic acid sequences does not matter. "n" is an integer usually ranging from 5 and 20. These constructs are inserted in one of the recombinant cell chromosome at a controlled location. In some embodiments, the insertion site is neither essential for the functionality of the inserted construct, nor for the viability of the resulting genetically modified cell.

As will be understood by those of skill in the art, it can be advantageous to modify a coding sequence to enhance its expression in a particular host. The genetic code is redundant with 64 possible codons, but most organisms typically use a subset of these codons. The codons that are utilized most often in a species are called optimal codons, and those not utilized very often are classified as rare or low-usage codons. Codons can be substituted to reflect the preferred codon usage of the host, in a process sometimes called "codon optimization" or"controlling for species codon bias." Codon optimization for other host cells can be readily determined using codon usage tables or can be performed using commercially available software, such as CodonOp (www.idtdna.com/CodonOptfrom) from Integrated DNA Technologies. Optimized coding sequences containing codons preferred by a particular prokaryotic or eukaryotic host (Murray et al, 1989, Nucl Acids Res. 17: 477-508) can be prepared, for example, to increase the rate of translation or to produce recombinant RNA transcripts having desirable properties, such as a longer half-life, as compared with transcripts produced from a non-optimized sequence. Translation stop codons can also be modified to reflect host preference. For example, typical stop codons for *S*. *cerevisiae* and mammals are UAA and UGA, respectively. The typical stop codon for monocotyledonous plants is UGA, whereas insects and E. coli commonly use UAA as the stop codon (Dalphin et al., 1996, Nucl Acids Res. 24: 216-8).
- When the recombinant cell is a yeast cell, and in particular is *Saccharomyces cerevisiae* yeast cell, nucleic acid sequences introduced in the yeast genome and originating from other organisms than *Saccharomyces cerevisiae* are generally "transcoded" (generally "codon-optimized"), meaning that these nucleic acid sequences are synthesized with an optimal codon usage for expression in *S*. *cerevisiae.* The nucleotide sequence (and not the protein sequence) of some nucleic acid sequences from *S*. *cerevisiae* has also been modified ("transcoded") to minimize recombination with an endogenous copy of the said gene.
- Genes may be deleted through standard procedures used in cell genetic engineering. In some embodiments, the genes targeted for deletion may be interrupted by insertion of one of the above described gene constructs, or alternatively the genes targeted for deletion are replaced by a short stretch of nucleotide.
- A nucleic acid sequences may be rendered *"inducible or repressible"* by deleting an endogenous copy of the nucleic acid sequences (if necessary) and placing a new copy of the ORF under the control of an inducible or repressible promoter. An inducible or repressible promoter is a promoter which activity is modulated or controlled, i.e. either increased or decreased, upon a change in the environmental conditions or external stimuli. Induction or repression may be artificially controlled, which encompasses induction or repression by abiotic factors such as chemical compounds not found naturally in the cell, and in particular yeast, of interest, light, oxygen levels, heat or cold. A list and sequences of inducible or repressible promoters are described elsewhere in the present specification.

### Recombinant cells according to the invention

The inventors have conceived recombinant cells, and especially recombinant yeasts, having an ability of producing heparosan.

The present invention relates to recombinant cells, and in particular recombinant yeasts, having the ability to produce heparosan, and wherein this ability to produce heparosan is obtained through a plurality of alterations that have been introduced in the genome thereof, by genetic engineering methods.

This present invention pertains to a recombinant yeast cell producing heparosan, wherein the recombinant cell comprises:
(a) one or more recombinant nucleic acids encoding a polypeptide having a heparosan synthase (HSS) activity; and
(b) one or more recombinant nucleic acids encoding a polypeptide having a UDP-Glucose dehydrogenase (UDP-GlcDH or HASB) activity.

The present invention further relates to a recombinant host cell producing heparosan, wherein the recombinant cell comprises:
(a) one or more recombinant nucleic acids encoding a polypeptide having a heparosan synthase (HSS) activity;
(b) one or more recombinant nucleic acids encoding a polypeptide having a UDP-Glucose dehydrogenase (UDP-GlcDH or HASB) activity; and
(c) one or more recombinant nucleic acids encoding a polypeptide having a glucuronosyl-disulfoglucosamine glucuronidase activity,
wherein the polypeptide having a glucuronosyl-disulfoglucosamine glucuronidase activity comprises a secretion signal, and optionaly an anchoring signal, so that heparosan, in particular of a desired molecular weight, is produced by the host cell.

The inventors have found that an ability to produce heparosan by cells, and in particular yeast cells, may be reached by introducing in the genome of these cells a plurality of genetic alterations.

The production of heparosan by cells of the invention, and in particular by yeast cells of the invention, achieved by optimizing the endogenous metabolism of UDP-Glucose, and optionally UDP-N-Acetyl-glucosamine, and direct the subsequent artificially modified metabolic pathway mainly towards heparosan production while in the same time maintaining an optimal viability of the resulting genetically modified cells.

It has been determined that a heparosan production by recombinant cells according to the invention may be increased by increasing the conversion of glucose-6-phosphate into the successive intermediate metabolites (i) glucose-1-phosphate, UDP-glucose, UDP-glucuronate and heparosan and (ii) fructose-6-phosphate, glucosamine-6-phosphate, N-acetyl-glucosamine-6-phosphate, N-acetyl-glucosamine-1-phosphate, UDP-N-acetyl-glucosamine and heparosan, while maintaining a balance allowing a good viability of the resulting recombinant cells.

Indeed, in order to obtain a viable recombinant cell of the invention, many different constructs were tested in order to obtain a viable and efficient recombinant cell, and in particular a viable recombinant yeast. In particular such recombinant yeast were difficult to obtain because the temporary accumulation of some intermediates appeared to be toxic for yeasts.

Unexpected technical difficulties were encountered in establishing the conditions suitable for the preparation of a recombinant cell able to produce heparosan, and in particular to produce heparosan with a controlled molecular weight.

By "controlled" molecular weight of a heparosan of the invention is intended to mean that at least 80%, in particular at least 85%, of the heparosan produced by a method of the invention has a molecular weight comprised within a certain molecular weight's range through the regulation of at least one parameter of the method and/or of the recombinant cell of the invention, such as for example:
- the nature and origin of the nucleic acid encoding polypeptide having a glucuronosyl-disulfoglucosamine glucuronidase activity of the recombinant cell,
- the nature and origin of the promoter controlling the expression of the nucleic acid encoding the polypeptide having a glucuronosyl-disulfoglucosamine glucuronidase activity of the recombinant cell,
- the presence of an anchoring and/or of a secretion signal associated to the polypeptide having a glucuronosyl-disulfoglucosamine glucuronidase activity of the recombinant cell,
- the pH of the culture medium during the step of culturing the recombinant cell, and/or
- the duration of the culturing of the recombinant cell.

Indeed, after much research and experimental trial, the inventors discovered that it was possible to cultivate a recombinant cell, and in particular a recombinant yeast cell, more particularly a recombinant *Saccharomyces cerevisiae* yeast cell, able to produce heparosan having a controlled molecular weight controlled using the following parameters:
- the selection of the nature and origin of the nucleic acid sequences encoding the polypeptide having glucuronosyl-disulfoglucosamine glucuronidase activity of the recombinant cell, in particular the recombinant yeast, of the invention; and/or
- the nature and origin of the promoter controlling the expression of the nucleic acid sequences encoding the polypeptide having glucuronosyl-disulfoglucosamine glucuronidase activity of the recombinant cell, in particular the recombinant yeast, of the invention; and/or
- the optional presence of an anchoring signal, in addition to a secretion signal, associated to the encoded polypeptide having glucuronosyl-disulfoglucosamine glucuronidase activity of the recombinant cell, in particular the recombinant yeast, according to the invention; and/or
- the pH of the culture medium during the step of culturing the recombinant cell, in particular the recombinant yeast cell, according to the invention; and/or
- the duration of the culturing of the recombinant cell, in particular the recombinant yeast cell, according to the invention.

To the inventors' knowledge, this has never been achieved before.

The molecular weight of the heparosan can be in the range of less than 50kDa, preferably in the range of about 20kDa to about 50kDa.

Alternatively, the molecular weight of the heparosan can be in the range of greater than 50kDa, preferably in the range of about 50kDa to about 250kD.

In another alternative, the molecular weight of the heparosan can be in the range of greater than 100kDa, preferably in the range of about 100kDa to about 1500kDa.

The nucleic acid encoding a polypeptide having a UDP-Glucose dehydrogenase (UDP-GlcDH or HASB) activity may be obtained or derived from at least one of *Arabidopsis thaliana*, *Chlorella virus PBCV1* or *Streptococcus zooepidemicus,* and may in particular be from *Arabidopsis thaliana* or *Chlorella virus PBCV1.*

The nucleic acid encoding a polypeptide having a heparosan synthase (HSS) activity may be obtained or derived from *Pasteurella multocida.*

The recombinant cell according to the invention can further comprise at least one recombinant nucleic acid encoding one or more of:
(i) a polypeptide having a glutamine-fructose-6-phosphate amidotransferase (GFA1) activity; and/or
(ii) a polypeptide having a UDP-N-acetylglucosamine pyrophosphorylase (QRI1) activity.

The recombinant cell according to the invention can further comprise at least one recombinant nucleic acid encoding one or more of:
(i) a polypeptide having Phosphoglucomutase-1 (PGM1) activity; and/or
(ii) a polypeptide having UTP-glucose-1-phosphate uridylyltransferase (UGP1) activity; and/or
(iii) a polypeptide having Glucosamine-6-phosphate N-acetyltransferase (GNA1) activity; and/or
(iv) a polypeptide having phosphoacetylglucosamine mutase (PCM1) activity.

The recombinant cell according to the invention can further be such that:
- the nucleic acid encoding the polypeptide having glutamine-fructose-6-phosphate amidotransferase (GFA1) activity; and/or
- the nucleic acid encoding the polypeptide having UDP-N-acetylglucosamine pyrophosphorylase (QRI1) activity; and/or
- the nucleic acid encoding the polypeptide having Phosphoglucomutase-1 (PGM1) activity; and/or
- the nucleic acid encoding the polypeptide having UTP-glucose-1-phosphate uridylyltransferase (UGP1) activity; and/or
- the nucleic acid encoding the polypeptide having Glucosamine-6-phosphate N-acetyltransferase (GNA1) activity; and/or
- the nucleic acid encoding the polypeptide having phosphoacetylglucosamine mutase (PCM1);
are obtained or derived from *Saccharomyces cerevisiae.*

A recombinant host cell of the invention may in particular be a yeast.

The recombinant cell of the invention may be a yeast cell belonging to the Saccharomyces genus, or to the Candida genus, or to the Kluyveromyces genus, or to the Ogataea genus, or to the Yarrowia genus, or to the Debaryomyces genus, or to the Ashbya genus, and in particular may belong to the *Saccharomyces* genus, and may more particularly be selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and may preferably be *Saccharomyces cerevisiae.*

Another object of the present invention relates to a method of producing heparosan of a desired molecular weight comprising:
(a) cultivating a recombinant cell of the invention, i.e. a recombinant yeast cell or a recombinant host cell of the invention, in a cultivation medium for a time sufficient to produce heparosan of the desired molecular weight; and
(b) optionally isolating or recovering the heparosan from the recombinant cell and/or from the cultivation medium.

In the method of the invention, the heparosan may have a molecular weight of from about 20 kDa to about 50 kDa, and preferably from 20 kDa to 30 kDa.

In the method of the invention, the heparosan may alternatively have a molecular weight of from 30 kDa to 50 kDa.

In the method of the invention, the heparosan may alternatively have a molecular weight of from about 50 kDa to about 150 kDa.

In the method of the invention, the heparosan may alternatively have a molecular weight of from about 150 kDa to about 1500 kDa.

In a method of the invention, the recombinant cell may be a yeast, in particular a member of the genus *Saccharomyces* genus, and may in particular be *Saccharomyces cerevisiae.*

In the method of the invention, the time sufficient to produce heparosan, in particular of a desired molecular weight, may be a period of from about 35 hours to about 50 hours, preferably from about 40 hours to about 50 hours, preferably of about 48 hours.

In the method of the invention, the molecular weight of the produced heparosan may be controlled by the pH of the cultivation medium.

In the method of the invention, the molecular weight of the heparosan may be controlled by regulating the pH of the cultivation medium during the cultivation step (a) of a method of the invention.

In the method of the invention, the molecular weight of the heparosan may be controlled by removing the biomass from the cultivation medium.

The method of the invention can be carried out on an industrial scale, preferably where the cultivation medium is at least about 100L, more preferably in the range of about 1000L to about 3000L, even more preferably about 10,000L or even more preferably about 100,000L, or even about 250,000L.

Another object of the invention relates to heparosan obtained or obtainable from a recombinant cell of the invention or from the method of the invention.

The present invention further relates to a cultivation medium comprising the heparosan according to the invention.

The present invention further relates to a composition comprising the heparosan according to the invention.

The present invention moreover relates to an industrial product or a consumer product or a consumable comprising (i) the heparosan having a molecular weight of less than 50kDa, preferably in the range of about 20kDa to about 50kDa; or greater than 50kDa, preferably in the range of about 50kDa to about 250kD; or greater than 100kDa, preferably in the range of about 100kDa to about 1500kDa; (ii) the cultivation medium of the invention or (iii) the composition of the invention.

The industrial product or the consumer product or the consumable of the invention can be a cosmetic product, a flavour product, a fragrance product, a food product, a food, a beverage, a texturant, a pharmaceutical composition, a dietary supplement, a nutraceutical, a cleaning product, a dental and/or an oral hygiene composition.

Another object of the invention relates to the use of a recombinant cell of the invention for the production of heparosan having a molecular weight in the range of from about 20kDa to about 50kDa or from about 50kDa to about 1000kDa.

### Recombinant nucleic acids encoding a polypeptide having Heparosan synthase activity

A recombinant cell according to the invention, and in particular a recombinant yeast of the invention, comprises one or more recombinant nucleic acids encoding a polypeptide having heparosan synthase activity.

A polypeptide having heparosan synthase activity according to the invention means a polypeptide that converts the intermediate metabolites UDP-Glucoronate and UDP-N-acetylglucosamine (UDP-GlcNAc) into heparosan [→4) β-D-glucuronic acid (GlcA) (1→4) N-acetyl-a-D-glucosamine (GlcNAc) (1→]ₙ.

In an embodiment, one or more of the recombinant nucleic acids encoding a polypeptide having heparosan synthase activity are under the control of an inducible or repressible promoter that is functional in the recombinant cells of the invention.

One or more of the recombinant nucleic acids encoding a polypeptide having heparosan synthase activity may be under the control of a promoter selected from the group consisting of pCCW12.Sar, pCCW120.sca, pTDH3-1.Sba and pTDH3.Sar.

The said one or more recombinant nucleic acids encoding a polypeptide having heparosan synthase activity may originate or be derived from *Streptococcus zooepidemicus (sz), Chlorella virus PBCV1 (Vir), Xenopus laevis (xl)* or *Pasteurella multocida (pm),* and in particular from *Pasteurella multocida,* as shown in the examples herein.

A recombinant cell according to the invention, and in particular a recombinant yeast according to the invention, may comprise between 1 and 5, in particular between 1 and 3 recombinant nucleic acids encoding a polypeptide having heparosan synthase activity. A recombinant cell according to the invention, and in particular a recombinant yeast according to the invention, may for example comprise 1, 2 or 3 recombinant nucleic acids encoding a polypeptide having heparosan synthase activity.

Illustratively, the one or more recombinant nucleic acids encoding a polypeptide having heparosan synthase activity may be inserted within the JLP1 gene and/or within the LEU2 gene and/or within the SAM3 gene of the recombinant cell, and in particular of the recombinant yeast, as it is shown in the examples herein.

In an embodiment of the invention, a recombinant cell, and in particular a recombinant yeast, of the invention comprises:
- between 1 and 5, in particular between 1 and 3 recombinant nucleic acids encoding a polypeptide having heparosan synthase activity;
- said one or more recombinant nucleic acids encoding a polypeptide having heparosan synthase activity originating or being derived from *Streptococcus zooepidemicus (sz), Chlorella virus PBCV1 (Vir), Xenopus laevis (xl)* or *Pasteurella multocida (pm),* and originating or being derived in particular from *Pasteurella multocida; and*
- said one or more recombinant nucleic acids encoding a polypeptide having heparosan synthase activity being under the control of an inducible or repressible promoter that is functional in the recombinant cells of the invention and/or under the control of a promoter selected from the group consisting of pCCW12.Sar, pCCW120.sca, pTDH3-1.Sba and pTDH3.Sar.

### Recombinant nucleic acids encoding a polypeptide having UDP-Glucose dehydrogenase activity

A recombinant cell according to the invention, and in particular a recombinant yeast according to the invention, comprises one or more recombinant nucleic acids encoding a polypeptide having UDP-Glucose dehydrogenase (UDP-GlcDH or HASB) activity.

A polypeptide having UDP-Glucose dehydrogenase (UDP-GlcDH or HASB) activity according to the invention means a polypeptide that converts the intermediate metabolite Uridine-Diphosphate-Glucose (UDP-Glucose) into UDP-Glucuronate.

In an embodiment, one or more of the recombinant nucleic acids encoding a polypeptide having UDP-Glucose dehydrogenase (UDP-GlcDH or HASB) activity are under the control of an inducible or repressible promoter that is functional in recombiant cells of the invention.

One or more of the recombinant nucleic acids encoding a polypeptide having UDP-Glucose dehydrogenase activity may be under the control of a pCCW12 promoter, and in particular may be under the control of a promoter selected from the group consisting of pCCW12.sk and pCCW12.sba.

The one or more recombinant nucleic acids encoding a polypeptide having UDP-Glucose dehydrogenase activity may originate or be derived from *Arabidopsis thaliana*, *Chlorella virus PBCV1* or *Streptococcus zooepidemicus,* and in particular originates or is derived from *Arabidopsis thaliana* or *Chlorella virus PBCV1.*

A recombinant cell according to the invention, and in particular a recombinant yeast according to the invention, may comprise between 1 and 7, in particular between 2 and 7 recombinant nucleic acids encoding a polypeptide having UDP-Glucose dehydrogenase activity. A recombinant cell according to the invention, and in particular a recombinant yeast according to the invention, may for example comprise 2 or 5 recombinant nucleic acids encoding a polypeptide having UDP-Glucose dehydrogenase activity.

Illustratively, the one or more recombinant nucleic acids encoding a polypeptide having UDP-Glucose dehydrogenase activity may be inserted within the JLP1 gene and/or within the LEU2 gene, and/or within the SAM3 gene of the recombinant cell, and in particular of the recombinant yeast, as it is shown in the examples herein.

In an embodiment of the invention, a recombinant cell, and in particular a recombinant yeast, of the invention comprises:
- between 1 and 7, and in particular between 2 and 7 recombinant nucleic acids encoding a polypeptide having UDP-Glucose dehydrogenase activity;
- said one or more recombinant nucleic acids encoding a polypeptide having UDP-Glucose dehydrogenase activity originating or being derived from *Arabidopsis thaliana*, *Chlorella virus PBCV1* or *Streptococcus zooepidemicus,* and originating or being derived in particular from *Arabidopsis thaliana* or *Chlorella virus PBCV1*; and
- said one or more recombinant nucleic acids encoding a polypeptide having UDP-Glucose dehydrogenase activity being under the control of an inducible or repressible promoter that is functional in the recombinant cells of the invention and/or under the control of a pCCW12 promoter, and in particular may be under the control of a promoter selected from the group consisting of pCCW12.sk and pCCW12.sba.

### Recombinant nucleic acids encoding a polypeptide having a glucuronosyl-disulfoglucosamine glucuronidase activity

A recombinant cell according to the invention, and in particular a recombinant yeast according to the invention, comprises one or more recombinant nucleic acids encoding a polypeptide having glucuronosyl-disulfoglucosamine glucuronidase activity.

A polypeptide having glucuronosyl-disulfoglucosamine glucuronidase activity according to the invention means a polypeptide that degrades heparosan, i.e. that converts a heparosan of a given molecular weight into a heparosan of a lower molecular weight.

As previously indicated, polypeptides having glucuronosyl-disulfoglucosamine glucuronidase activity of the present invention comprise a secretion signal.

In an embodiment, polypeptide having glucuronosyl-disulfoglucosamine glucuronidase activity comprise a secretion signal but no anchoring signal.

In an embodiment, polypeptide having glucuronosyl-disulfoglucosamine glucuronidase activity comprise both a secretion signal and an anchoring signal.

In an embodiment, the said one or more recombinant nucleic acids encoding a polypeptide having glucuronosyl-disulfoglucosamine glucuronidase activity are under the control of an inducible or repressible promoter that is functional in the recombinant cells of the invention.

One or more of the recombinant nucleic acids encoding a polypeptide having glucuronosyl-disulfoglucosamine glucuronidase activity may be under the control of a promoter selected from the group consisting of pTEF1, pCCW12, pCCW12.sba, pCCW12.Sar, pPDC1, pTEF3, pTDH3, pNUP57 and pCCW10.ago.

The said one or more recombinant nucleic acids encoding a polypeptide having glucuronosyl-disulfoglucosamine glucuronidase activity may originate or be derived from *Pedobacter heparinus (Ph), Cupiennius salei (Csa)*, *Loxosceles intermedia (Li)*, *Hirudo nipponia (Hn), Bothrops atrox (Ba)* or *Tityus serrulatus (Ts)* as shown in the examples herein. In a particular embodiment, the said one or more recombinant nucleic acids encoding a polypeptide having glucuronosyl-disulfoglucosamine glucuronidase activity originates or is derived from *Pedobacter heparinus (Ph).*

A recombinant cell according to the invention, and in particular a recombinant yeast according to the invention, may comprise only one recombinant nucleic acid encoding a polypeptide having glucuronosyl-disulfoglucosamine glucuronidase activity. A recombinant cell according to the invention, and in particular a recombinant yeast according to the invention, may alternatively comprise between 1 and 8, in particular between 1 to 5 recombinant nucleic acid encoding a polypeptide having glucuronosyl-disulfoglucosamine glucuronidase activity.

Illustratively, the one or more recombinant nucleic acids encoding a polypeptide having glucuronosyl-disulfoglucosamine glucuronidase activity may be inserted within the JLP1 gene and/or within the LYP1 gene and/or within the SAM3 gene and/or within the HIS3 gene, as it is shown in the examples herein.

### Recombinant nucleic acids encoding a polypeptide having Glutamine-fructose-6-phosphate amidotransferase activity

A recombinant cell according to the invention, and in particular a recombinant yeast cell according to the invention, may comprise one or more recombinant nucleic acids encoding a polypeptide having glutamine-fructose-6-phosphate amidotransferase (GFA1) activity.

A polypeptide having glutamine-fructose-6-phosphate amidotransferase activity according to the invention means a polypeptide that converts fructose-6-phosphate into glucosamine-6-phosphate.

In an embodiment, one or more of the recombinant nucleic acids encoding a polypeptide having glutamine-fructose-6-phosphate amidotransferase activity are under the control of an inducible or repressible promoter that is functional in the recombinant cells of the invention.

One or more of the recombinant nucleic acids encoding a polypeptide having glutamine-fructose-6-phosphate amidotransferase synthase activity may be under the control of a pTEF1 promoter, and in particular pTEF1.Ago.

The said one or more recombinant nucleic acids encoding a polypeptide having glutamine-fructose-6-phosphate amidotransferase activity may originate or be derived from the group consisting of *Saccharomyces cerevisiae* and *Chlorella virus PBCV1.*

A recombinant cell according to the invention, and in particular a recombinant yeast according to the invention, may comprise between 1 and 5, and in particular between 1 and 3 recombinant nucleic acid(s) encoding a polypeptide having glutamine-fructose-6-phosphate amidotransferase activity.

Illustratively, the one or more recombinant nucleic acids encoding a polypeptide having glutamine-fructose-6-phosphate amidotransferase activity may be inserted within the SAM3 gene and/or within the JLP1 gene of the recombinant cell, and in particular of the recombinant yeast, as it is shown in the examples herein.

In an embodiment of the invention, a recombinant cell, and in particular a recombinant yeast, of the invention comprises:
- between 1 and 5, and in particular between 1 and 3 recombinant nucleic acid(s) encoding a polypeptide having glutamine-fructose-6-phosphate amidotransferase activity;
- said recombinant nucleic acid encoding a polypeptide having glutamine-fructose-6-phosphate amidotransferase activity originating or being derived from the group consisting of *Saccharomyces cerevisiae and Chlorella virus PBCV1*; and
- said recombinant nucleic acid encoding a polypeptide having glutamine-fructose-6-phosphate amidotransferase activity being under the control of a pTEF1 promoter, and in particular pTEF1.Ago.

### Recombinant nucleic acids encoding a polypeptide having UDP-N-acetylglucosamine pyrophosphorylase activity

A recombinant cell according to the invention, and in particular a recombinant yeast cell according to the invention, may comprise one or more recombinant nucleic acids encoding a polypeptide having UDP-N-acetylglucosamine pyrophosphorylase activity.

A polypeptide having UDP-N-acetylglucosamine pyrophosphorylase activity according to the invention means a polypeptide that converts N-acetyl-glucosamine into UDP-N-acetyl-glucosamine.

In an embodiment, one or more of the recombinant nucleic acids encoding a polypeptide having UDP-N-acetylglucosamine pyrophosphorylase activity are under the control of an inducible or repressible promoter that is functional in the recombinant cells of the invention, such as for example the inducible or repressible promoters pMET6 or pCUP1.

One or more of the recombinant nucleic acids encoding a polypeptide having UDP-N-acetylglucosamine pyrophosphorylase activity may be under the control of the promoter pTDH3.

The said one or more recombinant nucleic acids encoding a polypeptide having UDP-N-acetylglucosamine pyrophosphorylase activity may originate or be derived from *Saccharomyces cerevisiae,* as shown in the examples herein.

A recombinant cell according to the invention, and in particular a recombinant yeast according to the invention, may comprise between 5 and 15, in particular between 5 and 12 recombinant nucleic acids encoding a polypeptide having UDP-N-acetylglucosamine pyrophosphorylase activity. A recombinant cell according to the invention, and in particular a recombinant yeast according to the invention, may for example comprise 8 or 10 recombinant nucleic acids encoding a polypeptide having UDP-N-acetylglucosamine pyrophosphorylase activity.

Illustratively, the one or more recombinant nucleic acids encoding a polypeptide having UDP-N-acetylglucosamine pyrophosphorylase activity may be inserted within the HIS3 gene and/or within the JLP1 gene and/or within the SAM3 gene of the recombinant cell, and in particular of the recombinant yeast, as it is shown in the examples herein.

In an embodiment of the invention, a recombinant cell, and in particular a recombinant yeast, of the invention comprises:
- between 5 and 15, in particular between 5 and 12 recombinant nucleic acids encoding a polypeptide having UDP-N-acetylglucosamine pyrophosphorylase activity;
- said one or more recombinant nucleic acids encoding a polypeptide having UDP-N-acetylglucosamine pyrophosphorylase activity originating or being derived from *Saccharomyces cerevisiae;* and
- said one or more recombinant nucleic acids encoding a polypeptide having UDP-N-acetylglucosamine pyrophosphorylase activity being under the control of an inducible or repressible promoter that is functional in the recombinant cells of the invention, such as for example the inducible or repressible promoters pMET6 or pCUP1 and/or under the control of the promoter pTDH3.

### Recombinant nucleic acids encoding a polypeptide having Phosphoglucomutase-1 activity

A recombinant cell according to the invention, and in particular a recombinant yeast according to the invention, may comprise one or more recombinant nucleic acids encoding a polypeptide having Phosphoglucomutase-1 (PGM1) activity.

A polypeptide having Phosphoglucomutase-1 (PGM1) activity according to the invention means a polypeptide that converts Glucose-6-phosphate into the intermediate metabolite Glucose-1-phosphate.

In an embodiment, one or more of the recombinant nucleic acids encoding a polypeptide having Phosphoglucomutase-1 (PGM1) activity are under the control of an inducible or repressible promoter that is functional in recombiant cells of the invention, such as for example the inducible or repressible promoters.

One or more of the recombinant nucleic acids encoding a polypeptide having Phosphoglucomutase-1 (PGM1) activity may be under the control of the promoter pPDC1.

The one or more recombinant nucleic acids encoding a polypeptide having Phosphoglucomutase-1 (PGM1) activity may originate or be derived from *Saccharomyces cerevisiae,* as shown in the examples herein.

A recombinant cell according to the invention, and in particular a recombinant yeast according to the invention, may comprise only one recombinant nucleic acid encoding a polypeptide having Phosphoglucomutase-1 (PGM1) activity.

Illustratively, the one or more recombinant nucleic acids encoding a polypeptide having Phosphoglucomutase-1 activity may be inserted within the SAM3 gene of the recombinant cell, and in particular of the recombinant yeast, as it is shown in the examples herein.

In an embodiment of the invention, a recombinant cell, and in particular a recombinant yeast, of the invention comprises:
- only one recombinant nucleic acid encoding a polypeptide having Phosphoglucomutase-1 activity;
- said recombinant nucleic acid encoding a polypeptide having Phosphoglucomutase-1 activity originating or being derived from *Saccharomyces cerevisiae;* and
- said recombinant nucleic acid encoding a polypeptide having Phosphoglucomutase-1 activity being under the control of the promoter pPDC1.

### Recombinant nucleic acids encoding a polypeptide having UTP-glucose-1-phosphate uridylyltransferase activity

A recombinant cell according to the invention, and in particular a recombinant yeast according to the invention, may comprise one or more recombinant nucleic acids encoding a polypeptide having UTP-glucose-1-phosphate uridylyltransferase (UGP1) activity.

A polypeptide having UTP-glucose-1-phosphate uridylyltransferase (UGP1) activity according to the invention means a polypeptide that converts the intermediate metabolite Glucose-1-Phosphate into the intermediate metabolite UDP-glucose.

In an embodiment, one or more of the recombinant nucleic acids encoding a polypeptide having UTP-glucose-1-phosphate uridylyltransferase activity are under the control of an inducible or repressible promoter that is functional in recombiant cells of the invention, such as for example the inducible or repressible promoters pSAM1 or pCUP1.

One or more of the recombinant nucleic acids encoding a polypeptide having UTP-glucose-1-phosphate uridylyltransferase activity may be under the control of a promoter selected from the group consisting of pPDC1 and pENO2.

The one or more recombinant nucleic acids encoding a polypeptide having UTP-glucose-1-phosphate uridylyltransferase activity may originate or be derived from *Saccharomyces cerevisiae,* as shown in the examples herein.

A recombinant cell according to the invention, and in particular a recombinant yeast according to the invention, may comprise between 5 and 15, in particular between 5 and 12 recombinant nucleic acids encoding a polypeptide having UTP-glucose-1-phosphate uridylyltransferase activity. A recombinant cell according to the invention, and in particular a recombinant yeast according to the invention, may for example comprise 8 or 11 recombinant nucleic acids encoding a polypeptide having UTP-glucose-1-phosphate uridylyltransferase activity.

Illustratively, the one or more recombinant nucleic acids encoding a polypeptide having UTP-glucose-1-phosphate uridylyltransferase activity gene may be inserted within the HIS3 gene and/or within the JLP1 gene and/or within the SAM3 gene of the recombinant cell, and in particular of the recombinant yeast, as it is shown in the examples herein.

In an embodiment of the invention, a recombinant cell, and in particular a recombinant yeast, of the invention comprises:
- between 5 and 15, in particular between 5 and 12 recombinant nucleic acids encoding a polypeptide having UTP-glucose-1-phosphate uridylyltransferase activity;
- said one or more recombinant nucleic acids encoding a polypeptide having UTP-glucose-1-phosphate uridylyltransferase activity originating or being derived from *Saccharomyces cerevisiae;* and
- said one or more recombinant nucleic acids encoding a polypeptide having UTP-glucose-1-phosphate uridylyltransferase activity being under the control of an inducible or repressible promoter that is functional in the recombinant cells of the invention, such as for example the inducible or repressible promoters pSAM1 or pCUP1 and/or under the control of a promoter selected from the group consisting of pPDC1 and pENO2.

### Recombinant nucleic acids encoding a polypeptide having Glucosamine-6-phosphate N-acetyltransferase activity

A recombinant cell according to the invention, and in particular a recombinant yeast cell according to the invention, may comprise one or more recombinant nucleic acids encoding a polypeptide having Glucosamine-6-phosphate N-acetyltransferase (GNA1) activity.

A polypeptide having Glucosamine-6-phosphate N-acetyltransferase (GNA1) activity according to the invention means a polypeptide that converts Glucosamine-6-phosphate into N-acetyl-glucosamine-6-phosphate.

In an embodiment, one or more of the recombinant nucleic acids encoding a polypeptide having Glucosamine-6-phosphate N-acetyltransferase activity are under the control of an inducible or repressible promoter that is functional in the recombinant cells of the invention.

One or more of the recombinant nucleic acids encoding a polypeptide having Glucosamine-6-phosphate N-acetyltransferase activity may be under the control of the promoter pCWP2.

The said one or more recombinant nucleic acids encoding a polypeptide having Glucosamine-6-phosphate N-acetyltransferase activity may originate or be derived from *Saccharomyces cerevisiae,* as shown in the examples herein.

A recombinant cell according to the invention, and in particular a recombinant yeast according to the invention, may comprise only one recombinant nucleic acid encoding a polypeptide having Glucosamine-6-phosphate N-acetyltransferase activity.

Illustratively, the one or more recombinant nucleic acids encoding a polypeptide having Glucosamine-6-phosphate N-acetyltransferase activity may be inserted within the SAM3 gene of the recombinant cell, and in particular of the recombinant yeast, as it is shown in the examples herein.

In an embodiment of the invention, a recombinant cell, and in particular a recombinant yeast, of the invention comprises:
- only one recombinant nucleic acid encoding a polypeptide having Glucosamine-6-phosphate N-acetyltransferase activity;
- said recombinant nucleic acid encoding a polypeptide having Glucosamine-6-phosphate N-acetyltransferase activity originating or being derived from *Saccharomyces cerevisiae;* and
- said recombinant nucleic acid encoding a polypeptide having Glucosamine-6-phosphate N-acetyltransferase activity being under the control of the promoter pCWP2.

### Recombinant nucleic acids encoding a polypeptide having Phosphoacetylglucosamine mutase activity

A recombinant cell according to the invention, and in particular a recombinant yeast cell according to the invention, may comprise one or more recombinant nucleic acids encoding a polypeptide having phosphoacetylglucosamine mutase (PCM1) activity.

A polypeptide having phosphoacetylglucosamine mutase (PCM1) activity according to the invention means a polypeptide that converts N-acetyl-glucosamine-6-phosphate N-acetyl-glucosamine-1-phosphate.

In an embodiment, one or more of the recombinant nucleic acids encoding a polypeptide having phosphoacetylglucosamine mutase activity are under the control of an inducible or repressible promoter that is functional in the recombinant cells of the invention.

One or more of the recombinant nucleic acids encoding a polypeptide having phosphoacetylglucosamine mutase activity may be under the control of a pTEF1 promoter.

The said one or more recombinant nucleic acids encoding a polypeptide having phosphoacetylglucosamine mutase activity can originate or be derived from *Saccharomyces cerevisiae,* as shown in the examples herein.

A recombinant cell according to the invention, and in particular a recombinant yeast according to the invention, can comprise only one recombinant nucleic acid encoding a polypeptide having phosphoacetylglucosamine mutase activity.

Illustratively, the one or more recombinant nucleic acids encoding a polypeptide having phosphoacetylglucosamine mutase activity may be inserted within the SAM3 gene of the recombinant cell, and in particular of the recombinant yeast, as it is shown in the examples herein.

In an embodiment of the invention, a recombinant cell, and in particular a recombinant yeast, of the invention comprises:
- only one recombinant nucleic acid encoding a polypeptide having phosphoacetylglucosamine mutase activity;
- said recombinant nucleic acid encoding a polypeptide having phosphoacetylglucosamine mutase activity originating or being derived from *Saccharomyces cerevisiae;* and
- said recombinant nucleic acid encoding a polypeptide having phosphoacetylglucosamine mutase activity being under the control of a pTEF1 promoter.

### HEPAROSAN SYNTHASE (HSS)

The heparosan synthase enzyme is a protein which is described in the art for catalyzing the conversion of UDP-glucuronate or UDP-N-acetyl-glucose into heparosan. The heparosan synthase originating from *Streptococcus zooepidemicus, Chlorella virus PBCV1*, *Xenopus laevis* or *Pasteurella multocida* may be termed HSS.

A method implemented to measure the activity level of a polypeptide having heparosan synthase activity belongs to the general knowledge of the one skilled in the art.

In this regard, the one skilled in the art may advantageously refer to the method of colorimetric determination after treatment with concentrated sulfuric acid and carbazole described by Bitter and Muir (Analytical Biochemistry, 4, 330-334, 1962).

Preferred polypeptide having heparosan synthase activity in the present specification are enzymes having a nucleic acid sequence selected from the group consisting of SEQ ID NO: 1 (HSS1) and SEQ ID NO: 2 (HSS2).

According to a preferred embodiment, the one or more recombinant nucleic acids encoding a polypeptide having heparosan synthase activity may originate or be derived from organisms preferably selected in a group consisting of prokaryotic organisms and eukaryotic organisms. In some embodiments, the one or more recombinant nucleic acids encoding a polypeptide having heparosan synthase activity may originate or be derived from archaebacteria. In some embodiments, the one or more recombinant nucleic acids encoding a polypeptide having heparosan synthase activity may originate or be derived from bacteria, and especially from *Streptococcus zooepidemicus (Sz), Chlorella virus PBCV1 (Vir), Xenopus laevis* (X1) or *Pasteurella multocida (Pm),* and in particular from *Pasteurella multocida.*

According to a yet preferred embodiment, the one or more recombinant nucleic acids encoding a polypeptide having heparosan synthase activity may be selected from the group consisting of nucleic acid sequences having (i) at least 65%, advantageously at least 70%, preferably at least 80%, nucleic acid identity with a nucleic acid sequence as set forth as sequence SEQ ID NO: 1 (HSS1-Pm) or SEQ ID NO: 2 (HSS2-Pm), and (ii) a biological activity of the same nature as the nucleic acid sequence having, respectively, the nucleic acid sequence as set forth as sequence SEQ ID NO: 1 (HSS1-Pm) or SEQ ID NO: 2 (HSS2-Pm).

A biological activity of the same nature regarding this sequence is, as previously explained, the capacity to code for an enzyme that converts UDP-glucuronate or UDP-N-acetyl-glucose into heparosan.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having heparosan synthase activity originating from *Pasteurella multocida,* the one skilled in the art may refer, respectively, to the accession numbers Q8RP78 and Q5SGE1 in the UniProt database, or to SEQ ID NO: 3 (HSS1-Pm) or SEQ ID NO: 4 (HSS2-Pm) described herein.

According to another particular embodiment, the one or more recombinant nucleic acids encoding a polypeptide having heparosan synthase activity may be nucleic acid(s) encoding polypeptide having an amino acid sequence selected from the group consisting of sequences having at least 50%, advantageously at least 65%, preferably at least 80%, amino acid identity with the amino acid sequence set forth as SEQ ID NO: 3 (Pm HSS1) or SEQ ID NO: 4 (Pm HSS2), and also a biological activity of the same nature as the amino acid sequence set forth as SEQ ID NO: 3 (Pm HSS1) or SEQ ID NO: 4 (Pm HSS2).

A biological activity of the same nature regarding this sequence is as described previously, i.e. the capacity to catalyze the conversion of UDP-glucuronate or UDP-N-acetyl-glucose into heparosan.

As described herein, an amino acid sequence having at least 50% amino acid identity with a reference amino acid sequence encompasses amino acid sequences having at least 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having heparosan synthase activity in the present invention is regulated by at least one promoter and at least one terminator, such as herein after defined more in details, which are present in 5' and 3' position respectively of the one or more recombinant nucleic acids encoding a polypeptide having heparosan synthase activity.

### UDP-GLUCOSE DEHYDROGENASE (UDP-GlcDH OR HASB)

The UDP-Glucose dehydrogenase is a protein which is known in the art to catalyze the conversion of UDP-glucose into UDP-glucuronate. The UDP-Glucose dehydrogenase originating from the genome of *Arabidopsis thaliana, Chlorella virus PBCV1* or *Streptococcus zooepidemicus* may be termed HASB.

A method implemented to measure the activity level of a polypeptide having UDP-Glucose dehydrogenase activity belongs to the general knowledge of the one skilled in the art.

In this regard, the one skilled in the art may advantageously refer to the method described by Oka and Jigami (FEBS Journal 273, 2645-2657, 2006).

Preferred polypeptide having UDP-Glucose dehydrogenase activity in the present specification is an enzyme having an EC number 1.1.1.22.

According to a preferred embodiment, the one or more recombinant nucleic acids encoding a polypeptide having UDP-Glucose dehydrogenase activity may originate or be derived from organisms preferably selected in a group comprising prokaryotic organisms and eukaryotic organisms. In some embodiments, the one or more recombinant nucleic acids encoding a polypeptide having UDP-Glucose dehydrogenase activity may originate or be derived from archaebacteria. In some preferred embodiments, the one or more recombinant nucleic acids encoding a polypeptide having UDP-Glucose dehydrogenase activity may originate or be derived from yeast, and especially from *Arabidopsis thaliana*, *Chlorella virus PBCV1* or *Streptococcus zooepidemicus,* and more particularly from *Arabidopsis thaliana or Chlorella virus PBCV1.*

According to a yet preferred embodiment, the one or more recombinant nucleic acids encoding a polypeptide having UDP-Glucose dehydrogenase activity may be selected from the group consisting of nucleic acid sequences having (i) at least 65%, advantageously at least 70%, preferably at least 80%, nucleic acid identity with a nucleic acid as set forth as sequence SEQ ID NO: 5 (At), SEQ ID NO. 6 (Vir) or SEQ ID NO. 7 (Vir), and (ii) a biological activity of the same nature as the nucleic acid as set forth as sequence SEQ ID NO: 5 (At), SEQ ID NO. 6 (Vir) or SEQ ID NO. 7 (Vir). The nucleic acids set forth as sequences SEQ ID NO: 5 (At), SEQ ID NO: 6 (Vir) and SEQ ID NO. 7 (Vir) encode a polypeptide having UDP-Glucose dehydrogenase activity originating, respectively, from *Arabidopsis thaliana (At)* or *Chlorella virus PBCV1 (Vir)* that may herein also be collectively termed HASB.

A biological activity of the same nature regarding this sequence is, as previously explained, the capacity to code for a polypeptide that converts UDP-glucose into UDP-glucuronate.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequences, and also a biological activity of the same nature as the said reference nucleic acid sequences.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequences.

For the amino acid sequence of the polypeptide having UDP-Glucose dehydrogenase activity from *Arabidopsis thaliana*, *Chlorella virus PBCV1* or *Streptococcus zooepidemicus,* the one skilled in the art may refer to the accession numbers NP_173979.1, NP_048965 or KIS19289, respectively, in the UniProt database, or to the sequences as set forth in SEQ ID NO: 8 (At) and SEQ ID NO. 9 (Vir) described herein.

According to another particular embodiment, the one or more recombinant nucleic acids encoding a polypeptide having UDP-Glucose dehydrogenase activity may be nucleic acid(s) encoding a polypeptide having an amino acid sequence selected from the group consisting of sequences having at least 55%, advantageously at least 65%, preferably at least 80%, amino acid identity with the amino acid sequence of SEQ ID NO: 8 (At) and SEQ ID NO. 9 (Vir), and also a biological activity of the same nature as the amino acid sequence of SEQ ID NO: 8 (At) and SEQ ID NO. 9 (Vir).

A biological activity of the same nature regarding this sequence is as described previously, i.e. the capacity to catalyze the conversion of UDP-glucose into UDP-glucuronate.

As described herein, an amino acid sequence having at least 55% amino acid identity with a reference amino acid sequence encompasses amino acid sequences having at least 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having UDP-Glucose dehydrogenase activity in the present invention is regulated by at least one promoter and at least one terminator, such as herein after defined more in details, which are present in 5' and 3' position respectively of the one or more recombinant nucleic acids encoding a polypeptide having UDP-Glucose dehydrogenase activity.

### GLUCURONOSYL-DISULFOGLUCOSAMINE GLUCURONIDASE

The glucuronosyl-disulfoglucosamine glucuronidase enzyme is a protein which is described in the art for catalyzing the degradation of heparosan molecules into smaller heparosan molecules. The glucuronosyl-disulfoglucosamine glucuronidase encoded by the genome of *Pedobacter heparinus* may be termed HEPC.

The polypeptide having glucuronosyl-disulfoglucosamine glucuronidase activity of the invention may possess both a secretion signal and an anchoring signal or a secretion signal and no anchoring signal or a secretion-anchor signal with a dual secretion and anchoring function.

A method implemented to measure the activity level of a polypeptide having glucuronosyl-disulfoglucosamine glucuronidase activity belongs to the general knowledge of the one skilled in the art.

In this regard, the one skilled in the art may advantageously monitor the molecular weight of the obtained heparosan on an agarose gel.

Preferred polypeptide having glucuronosyl-disulfoglucosamine glucuronidase activity in the present specification is an enzyme having an EC number of 3.2.1.56.

According to a preferred embodiment, the one or more recombinant nucleic acids encoding a polypeptide having glucuronosyl-disulfoglucosamine glucuronidase activity may originate or be derived from organisms preferably selected in a group comprising prokaryotic organisms and eukaryotic organisms. In some embodiments, the one or more recombinant nucleic acids encoding a polypeptide having glucuronosyl-disulfoglucosamine glucuronidase activity may originate or be derived from archaebacteria. In some embodiments, the one or more recombinant nucleic acids encoding a polypeptide having glucuronosyl-disulfoglucosamine glucuronidase activity may originate or be derived from organisms preferably selected from yeasts. In some other preferred embodiments, the one or more recombinant nucleic acids encoding a polypeptide having glucuronosyl-disulfoglucosamine glucuronidase activity may originate or be derived from *Pedobacter heparinus.*

According to a yet preferred embodiment, the one or more recombinant nucleic acids encoding a polypeptide having glucuronosyl-disulfoglucosamine glucuronidase activity may be selected from the group consisting of nucleic acid sequences having at least 65%, advantageously at least 70%, preferably at least 80%, nucleic acid identity with a nucleic acid of SEQ ID NO: 10 (Ph) or of SEQ ID NO: 12 (Ph) and also a biological activity of the same nature as the nucleic acid of SEQ ID NO: 10 (Ph) or of SEQ ID NO: 12 (Ph). The nucleic acids of SEQ ID NO: 10 (Ph) and of SEQ ID NO: 12 (Ph) encode a polypeptide having glucuronosyl-disulfoglucosamine glucuronidase activity, comprises a secretion signal and no anchoring signal, and originate or derive from *Pedobacter heparinus.*

According to another preferred embodiment, the one or more recombinant nucleic acids encoding a polypeptide having glucuronosyl-disulfoglucosamine glucuronidase activity may be selected from the group consisting of nucleic acid sequences having at least 65%, advantageously at least 70%, preferably at least 80%, nucleic acid identity with a nucleic acid of SEQ ID NO: 11 (Ph) or of SEQ ID NO: 13 (Ph) and also a biological activity of the same nature as the nucleic acid of SEQ ID NO: 11 (Ph) or of SEQ ID NO: 13 (Ph). The nucleic acids of SEQ ID NO: 11 (Ph) and of SEQ ID NO: 13 (Ph) encode a polypeptide having glucuronosyl-disulfoglucosamine glucuronidase activity, comprises a secretion signal and an anchoring signal, and originate or derive from *Pedobacter heparinus.*

A biological activity of the same nature regarding this sequence is, as previously explained, the capacity to code for a polypeptide that catalyzes the degradation of heparosan molecules into smaller heparosan molecules.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the one or more recombinant nucleic acids encoding a polypeptide having glucuronosyl-disulfoglucosamine glucuronidase activity from *Pedobacter heparinus*, the one skilled in the art may refer to the accession numbers Q59289 in the UniProt database, corresponding to both SEQ ID NO: 10 (Ph) and SEQ ID NO: 12 (Ph) described herein.

According to another particular embodiment, the one or more recombinant nucleic acids encoding a polypeptide having glucuronosyl-disulfoglucosamine glucuronidase activity may be nucleic acid(s) encoding polypeptide having an amino acid sequence selected from the group consisting of sequences having at least 50%, advantageously at least 65%, preferably at least 80%, amino acid identity with the amino acid sequence of SEQ ID NO: 14 (Ph) which comprise a secretion signal and no anchoring signal, and also a biological activity of the same nature as the amino acid sequence SEQ ID NO: 14 (Ph).

According to another particular embodiment, the one or more recombinant nucleic acids encoding a polypeptide having glucuronosyl-disulfoglucosamine glucuronidase activity may be nucleic acid(s) encoding polypeptide having an amino acid sequence selected from the group consisting of sequences having at least 55%, advantageously at least 65%, preferably at least 80%, amino acid identity with the amino acid sequence of SEQ ID NO: 15 (Ph) which comprise a secretion signal and an anchoring signal, and also a biological activity of the same nature as the amino acid sequence of SEQ ID NO: 15 (Ph).

A biological activity of the same nature regarding this sequence is as described previously, i.e. the capacity to catalyze the degradation of heparosan molecules into smaller heparosan molecules.

As described herein, an amino acid sequence having at least 55% amino acid identity with a reference amino acid sequence encompasses amino acid sequences having at least 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having glucuronosyl-disulfoglucosamine glucuronidase activity in the present invention is regulated by at least one promoter and at least one terminator, such as herein after defined more in details, which are present in 5' and 3' position respectively of the one or more recombinant nucleic acids encoding a polypeptide having glucuronosyl-disulfoglucosamine glucuronidase activity.

### GLUTAMINE-FRUCTOSE-6-PHOSPHATE AMIDOTRANSFERASE (GFA1)

The glutamine-fructose-6-phosphate amidotransferase enzyme is a protein which is described in the art for catalyzing the conversion of fructose-6-phosphate into glucosamine-6-phosphate. The glutamine-fructose-6-phosphate amidotransferase originating from *Saccharomyces cerevisiae* and *Chlorella virus PBCV1* may be termed GFA1.

A method implemented to measure the activity level of a polypeptide having glutamine-fructose-6-phosphate amidotransferase activity belongs to the general knowledge of the one skilled in the art.

In this regard, the one skilled in the art may advantageously refer to the method described by Shiga Shibatan and Hiroaki Kitazawa (Plant Biotechnology 26, 149-152, 2009).

Preferred polypeptide having glutamine-fructose-6-phosphate amidotransferase activity in the present invention is an enzyme having an EC number of n° EC 2.6.1.16.

According to a preferred embodiment, the one or more recombinant nucleic acids encoding a polypeptide having glutamine-fructose-6-phosphate amidotransferase activity may originate or be derived from organisms preferably selected in a group comprising prokaryotic organisms and eukaryotic organisms. In some embodiments, the one or more recombinant nucleic acids encoding a polypeptide having glutamine-fructose-6-phosphate amidotransferase activity may originate or be derived from archaebacteria. In some embodiments, the one or more recombinant nucleic acids encoding a polypeptide having glutamine-fructose-6-phosphate amidotransferase activity may originate or be derived from organisms preferably selected from *Bacillus subtilis,* and yeasts. In some other preferred embodiments, the one or more recombinant nucleic acids encoding a polypeptide having glutamine-fructose-6-phosphate amidotransferase activity may originate or be derived from *Saccharomyces cerevisiae* or from *Chlorella virus PBCV1.*

According to a yet preferred embodiment, the one or more recombinant nucleic acids encoding a polypeptide having glutamine-fructose-6-phosphate amidotransferase activity may be selected from the group consisting of nucleic acid sequences having (i) at least 65%, advantageously at least 70%, preferably at least 80%, nucleic acid identity with the nucleic acid sequence as set forth as sequence SEQ ID NO: 16 (Sc) or EQ, and (ii) a biological activity of the same nature as the nucleic acid sequence as set forth as sequence SEQ ID NO: 16 (Sc). The nucleic acid as set forth as sequence SEQ ID NO: 16 encodes a polypeptide having glutamine-fructose-6-phosphate amidotransferase activity originating from *Saccharomyces cerevisiae,* that may also be termed GFA1.

According to yet another embodiment, the one or more recombinant nucleic acids encoding a polypeptide having glutamine-fructose-6-phosphate amidotransferase activity may be selected from the group consisting of nucleic acid sequences having (i) at least 65%, advantageously at least 70%, preferably at least 80%, nucleic acid identity with the nucleic acid sequence as set forth as sequence SEQ ID NO: 15 or SEQ ID NO: 16, and (ii) a biological activity of the same nature as the nucleic acid sequence as set forth as sequence SEQ ID NO: 17 or SEQ ID NO: 18. The nucleic acid sequence as set forth as sequence SEQ ID NO: 17 or SEQ ID NO: 18 encodes a polypeptide having glutamine-fructose-6-phosphate amidotransferase activity originating from *Chlorella virus PBCV1.*

A biological activity of the same nature regarding this sequence is, as previously explained, the capacity to code for a polypeptide that converts fructose-6-phosphate into glucosamine-6-phosphate.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having glutamine-fructose-6-phosphate amidotransferase activity originating from *Saccharomyces cerevisiae,* the one skilled in the art may refer to the accession number NP8012818 in the UniProt database, or to the sequence SEQ ID NO: 19 described herein.

For the amino acid sequence of the polypeptide having glutamine-fructose-6-phosphate amidotransferase activity originating from *Chlorella virus PBCV1*, the one skilled in the art may also refer to the accession number NP_048448 in the UniProt database, or to the sequence SEQ ID NO: 20 described herein.

According to another particular embodiment, the one or more recombinant nucleic acids encoding a polypeptide having glutamine-fructose-6-phosphate amidotransferase activity may be nucleic acid(s) encoding polypeptide having an amino acid sequence selected from the group consisting of sequences having at least 35%, advantageously at least 65%, preferably at least 80%, amino acid identity with the amino acid sequence of SEQ ID NO: 19 or with SEQ ID NO: 20, and also a biological activity of the same nature as the amino acid sequence of SEQ ID NO: 19 or with SEQ ID NO: 20.

A biological activity of the same nature regarding this sequence is as described previously, i.e. the capacity to catalyze the conversion of of fructose-6-phosphate into glucosamine-6-phosphate.

As described herein, an amino acid sequence having at least 35% amino acid identity with a reference amino acid sequence encompasses amino acid sequences having at least 36%, 37%, 38%, 39%, 40% 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the polypeptide having glutamine-fructose-6-phosphate amidotransferase activity in the present invention is regulated by at least one promoter and at least one terminator, such as herein after defined more in details, which are present in 5' and 3' position respectively of one or more recombinant nucleic acids encoding a polypeptide having glutamine-fructose-6-phosphate amidotransferase activity.

### UDP-N-ACETYLGLUCOSAMINE PYROPHOSPHORYLASE (QRI1)

The UDP-N-acetylglucosamine pyrophosphorylase enzyme is a protein which is described in the art for catalyzing the conversion of N-acetyl-glucosmine-6-phosphate into UDP-N-acetyl-glucose. The UDP-N-acetylglucosamine pyrophosphorylase originating from *Saccharomyces cerevisiae* may be termed QRI1.

A method implemented to measure the activity level of a polypeptide having UDP-N-acetylglucosamine pyrophosphorylase activity belongs to the general knowledge of the one skilled in the art.

In this regard, the one skilled in the art may advantageously refer to the method described by Mio et al. (The Journal of Biological Chemistry, Col. 273, No 23, June 5, 1998, 14392-14397) except that UDP-N-acetyl-glucosamine is detected by LC MS/MS using a Synergi RP Fusion column.

Preferred polypeptide having UDP-N-acetylglucosamine pyrophosphorylase activity in the present invention is an enzyme having an EC number of n° EC 2.7.7.23

According to a preferred embodiment, the one or more recombinant nucleic acids encoding a polypeptide having UDP-N-acetylglucosamine pyrophosphorylase activity may originate or be derived from organisms preferably selected in a group consisting of prokaryotic organisms and eukaryotic organisms. In some embodiments, the one or more recombinant nucleic acids encoding a polypeptide having UDP-N-acetylglucosamine pyrophosphorylase activity may originate or be derived from archaebacteria. In some embodiments, the one or more recombinant nucleic acids encoding a polypeptide having UDP-N-acetylglucosamine pyrophosphorylase activity may originate or be derived from organisms preferably selected from *Bacillus subtilis,* and yeasts. In some other preferred embodiments, the one or more recombinant nucleic acids encoding a polypeptide having UDP-N-acetylglucosamine pyrophosphorylase activity may originate or be derived from yeasts, and especially from *Saccharomyces cerevisiae.*

According to a yet preferred embodiment, the one or more recombinant nucleic acids encoding a polypeptide having UDP-N-acetylglucosamine pyrophosphorylase activity may be selected from the group consisting of nucleic acid sequences having (i) at least 65%, advantageously at least 70%, preferably at least 80%, nucleic acid identity with a nucleic acid sequence as set forth as SEQ ID NO: 21, and (ii) a biological activity of the same nature as the nucleic acid sequence as set forth as SEQ ID NO: 21. The nucleic acid sequence set forth as SEQ ID NO: 21 encodes a polypeptide having UDP-N-acetylglucosamine pyrophosphorylase activity originating from *Saccharomyces cerevisiae,* that may also be termed QRI1.

A biological activity of the same nature regarding this sequence is, as previously explained, the capacity to code for a polypeptide that converts N-acetyl-glucosmine-6-phosphate into UDP-N-acetyl-glucose.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having UDP-N-acetylglucosamine pyrophosphorylase activity originating from *Saccharomyces cerevisiae,* the one skilled in the art may refer to the accession number NP_010180 in the UniProt database, or to SEQ ID NO. 22 described herein.

According to another particular embodiment, the one or more recombinant nucleic acids encoding a polypeptide having UDP-N-acetylglucosamine pyrophosphorylase activity may be nucleic acid(s) encoding polypeptide having an amino acid sequence selected from the group consisting of sequences having at least 35%, advantageously at least 45%, preferably at least 80%, amino acid identity with the amino acid sequence set forth as sequence SEQ ID NO. 22, and also a biological activity of the same nature as the amino acid sequence set forth as sequence SEQ ID NO. 22.

A biological activity of the same nature regarding this sequence is as described previously, i.e. the capacity to catalyze the conversion of of N-acetyl-glucosmine-6-phosphate into UDP-N-acetyl-glucose.

As described herein, an amino acid sequence having at least 35% amino acid identity with a reference amino acid sequence encompasses amino acid sequences having at least 36%, 37%, 38%, 39%, 40% 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 45% amino acid identity with a reference amino acid sequence encompasses amino acid sequences having at least 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having UDP-N-acetylglucosamine pyrophosphorylase activity in the present invention is regulated by at least one promoter and at least one terminator, such as herein after defined more in details, which are present in 5' and 3' position respectively of the one or more recombinant nucleic acids encoding a polypeptide having UDP-N-acetylglucosamine pyrophosphorylase activity.

### PHOSPHOGLUCOMUTASE-1 (PGM1)

The Phosphoglucomutase-1 enzyme is a protein which is described in the art for catalyzing the conversion of Glucose-6-phosphate into Glucose-1-phosphate. The Phosphoglucomutase-1 originating from *Saccharomyces cerevisiae* may be termed PGM1.

A method implemented to measure the activity level of a polypeptide facing Phosphoglucomutase-1 activity belongs to the general knowledge of the one skilled in the art.

In this regard, the one skilled in the art may advantageously refer to the method described by Tiwari and Bhat (Biochemical and Biophysical Research Communications 366, 340-345, 2008).

Preferred polypeptide having Phosphoglucomutase-1 activity in the present invention is an enzyme having an EC number of n° 5.4.2.2.

According to a preferred embodiment, the one or more recombinant nucleic acids encoding a polypeptide having Phosphoglucomutase-1 activity may originate from organisms preferably selected in a group consisting of prokaryotic organisms and eukaryotic organisms. In some embodiments, the one or more recombinant nucleic acids encoding a polypeptide having Phosphoglucomutase-1 activity may originate or be derived from archaebacteria. In some embodiments, the one or more recombinant nucleic acids encoding a polypeptide having Phosphoglucomutase-1 activity may originate or be derived from organisms preferably selected from bacteria. In a preferred embodiment, the one or more recombinant nucleic acids encoding a polypeptide having Phosphoglucomutase-1 activity may orginate or be derived from *Saccharomyces cerevisiae.*

According to an embodiment, the one or more recombinant nucleic acids encoding a polypeptide having Phosphoglucomutase-1 activity may be selected from the group consisting of nucleic acid sequences having (i) at least 80% nucleic acid identity with the nucleic acid sequence set forth as sequence SEQ ID NO: 23, which originates from *Saccharomyces cerevisiae,* and (ii) a biological activity of the same nature as the nucleic acid sequence set forth as sequence SEQ ID NO: 23.

A biological activity of the same nature regarding this sequence is, as previously explained, the capacity to code for a polypeptide that converts Glucose-6-phosphate into Glucose-1-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the peptide having Phosphoglucomutase-1 activity from *Saccharomyces cerevisiae,* the one skilled in the art may refer to the accession number NP33401 in the UniProt database, or to SEQ ID NO: 24 described herein.

According to another particular embodiment, the one or more recombinant nucleic acids encoding a polypeptide having Phosphoglucomutase-1 activity may be nucleic acid(s) encoding polypeptide having an amino acid sequence selected from the group consisting of sequences having at least least 80% amino acid identity with the amino acid sequence set forth as sequence SEQ ID NO: 24, and also a biological activity of the same nature as the amino acid sequence set forth as sequence SEQ ID NO: 24.

A biological activity of the same nature regarding this sequence is as described previously, i.e. the capacity to catalyze the conversion of Glucose-6-phosphate into Glucose-1-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having Phosphoglucomutase-1 activity in the present invention is regulated by at least one promoter and at least one terminator, such as herein after defined more in details, which are present in 5' and 3' position respectively of the one or more recombinant nucleic acids encoding a polypeptide having Phosphoglucomutase-1 activity.

### UTP-GLUCOSE-1-PHOSPHATE URIDYLYLTRANSFERASE (UGP1)

The UTP-glucose-1-phosphate uridylyltransferase enzyme is a protein which is described in the art for catalyzing the conversion of Glucose-1-Phosphate into UDP-glucose. The UTP-glucose-1-phosphate uridylyltransferase originating from *Saccharomyces cerevisiae* may be termed UGP1.

A method implemented to measure the activity level of a polypeptide having UTP-glucose-1-phosphate uridylyltransferase activity belongs to the general knowledge of the one skilled in the art.

In this regard, the one skilled in the art may advantageously refer to the method described by Roeben (J. Mol. Biol 364, 551-560, 2006).

Preferred polypeptide having UTP-glucose-1-phosphate uridylyltransferase activity in the present invention is an enzyme having an EC number of n° 2.7.7.9.

According to a preferred embodiment, the one or more recombinant nucleic acids encoding a polypeptide having UTP-glucose-1-phosphate uridylyltransferase activity mayoriginate or be derived from organisms preferably selected in a group consisting of prokaryotic organisms and eukaryotic organisms. In some embodiments, the one or more recombinant nucleic acids encoding a polypeptide having UTP-glucose-1-phosphate uridylyltransferase activity may originate or be derived from archaebacteria. In some embodiments, the one or more recombinant nucleic acids encoding a polypeptide having UTP-glucose-1-phosphate uridylyltransferase activity may originate or be derived from organisms preferably selected from bacteria. In a preferred embodiment, the one or more recombinant nucleic acids encoding a polypeptide having UTP-glucose-1-phosphate uridylyltransferase activity may originate or be derived from *Saccharomyces cerevisiae.*

According to a particular embodiment, the one or more recombinant nucleic acids encoding a polypeptide having UTP-glucose-1-phosphate uridylyltransferase activity may be selected from the group consisting of nucleic acid sequences having (i) at least 80% nucleic acid identity with a nucleic acid as set forth as sequence SEQ ID NO: 25, originating from *Saccharomyces cerevisiae,* and (ii) a biological activity of the same nature as the nucleic acid as set forth as sequence SEQ ID NO: 25.

A biological activity of the same nature regarding this sequence is, as previously explained, the capacity to code for a polypeptide that converts Glucose-1-phosphate into UDP-glucose.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having UTP-glucose-1-phosphate uridylyltransferase activity originating from *Saccharomyces cerevisiae,* the one skilled in the art may refer to the accession number NP_32861 in the UniProt database, or to the sequence set forth as sequence SEQ ID NO: 26 described herein.

According to another particular embodiment, the one or more recombinant nucleic acids encoding a polypeptide having UTP-glucose-1-phosphate uridylyltransferase activity may be nucleic acid(s) encoding polypeptide having an amino acid sequence selected from the group consisting of sequences having at least 80% amino acid identity with the amino acid sequence set forth as SEQ ID NO: 26, and also a biological activity of the same nature as the amino acid sequence set forth as SEQ ID NO: 26.

A biological activity of the same nature regarding this sequence is as described previously, i.e. the capacity to catalyze the conversion of Glucose-1-phosphate into UDP-glucose.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having UTP-glucose-1-phosphate uridylyltransferase activity in the present invention is regulated by at least one promoter and at least one terminator, such as herein after defined more in details, which are present in 5' and 3' position respectively of the one or more recombinant nucleic acids encoding a polypeptide having UTP-glucose-1-phosphate uridylyltransferase activity.

### GLUCOSAMINE-6-PHOSPHATE N-ACETYLTRANSFERASE (GNA1)

The Glucosamine-6-phosphate N-acetyltransferase enzyme is a protein which is described in the art for catalyzing the conversion of Glucosamine-6-phosphate into N-acetyl-glucosamine-6-phosphate. The Glucosamine-6-phosphate N-acetyltransferase originating from *Saccharomyces cerevisiae* may be termed GNA1.

A method implemented to measure the activity level of a polypeptide having Glucosamine-6-phosphate N-acetyltransferase activity belongs to the general knowledge of the one skilled in the art.

In this regard, the one skilled in the art may advantageously refer to the method described by Li et al. (Anal. Biochem. 370, 142-146, 2007).

Preferred polypeptide having Glucosamine-6-phosphate N-acetyltransferase activity in the present invention is an enzyme having an EC number of n° 2.3.1.4.

According to a preferred embodiment, the one or more recombinant nucleic acids encoding a polypeptide having Glucosamine-6-phosphate N-acetyltransferase activity may originate or be derived from organisms preferably selected in a group consisting of prokaryotic organisms and eukaryotic organisms. In some preferred embodiments, the one or more recombinant nucleic acids encoding a polypeptide having Glucosamine-6-phosphate N-acetyltransferase activity may originate or be derived from a yeast, and especially from *Saccharomyces cerevisiae.*

According to a particular embodiment, the one or more recombinant nucleic acids encoding a polypeptide having Glucosamine-6-phosphate N-acetyltransferase activity may be selected from the group consisting of nucleic acid sequences having (i) at least 80% nucleic acid identity with a nucleic acid sequence as set forth as sequence SEQ ID NO: 27, and (ii) a biological activity of the same nature as the nucleic acid sequence as set forth as sequence SEQ ID NO: 27. The nucleic acid sequence as set forth as sequence SEQ ID NO: 27 encodes a polypeptide having Glucosamine-6-phosphate N-acetyltransferase activity originating from *Saccharomyces cerevisiae,* that may also be termed GNA1.

A biological activity of the same nature regarding this sequence is, as previously explained, the capacity to code for a polypeptide that converts Glucosamine-6-phosphate into N-acetyl-glucosamine-6-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having Glucosamine-6-phosphate N-acetyltransferase activity originating from *Saccharomyces cerevisiae,* the one skilled in the art may refer to the accession number NP_116637 in the UniProt database, or to SEQ ID NO. 28 described herein.

According to another particular embodiment, the one or more recombinant nucleic acids encoding a polypeptide having Glucosamine-6-phosphate N-acetyltransferase activity may be nucleic acid(s) encoding polypeptide having an amino acid sequence selected from the group consisting of sequences having at least 80% amino acid identity with the amino acid sequence set forth as SEQ ID NO. 28, and also a biological activity of the same nature as the amino acid sequence set forth as SEQ ID NO. 28.

A biological activity of the same nature regarding this sequence is as described previously, i.e. the capacity to catalyze the conversion of Glucosamine-6-phosphate into N-acetyl-glucosamine-6-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having Glucosamine-6-phosphate N-acetyltransferase activity in the present invention is regulated by at least one promoter and at least one terminator, such as herein after defined more in details, which are present in 5' and 3' position respectively of the one or more recombinant nucleic acids encoding a polypeptide having Glucosamine-6-phosphate N-acetyltransferase activity.

### PHOSPHOACETYLGLUCOSAMINE MUTASE (PCM1)

The phosphoacetylglucosamine mutase enzyme is a protein which is described in the art for catalyzing the conversion of N-acetyl-glucosamine-6-phosphate into N-acetyl-glucosamine-1-phosphate. The phosphoacetylglucosamine mutase originating from *Saccharomyces cerevisiae* may be termed PCM1.

A method implemented to measure the activity level of a polypeptide having phosphoacetylglucosamine mutase activity belongs to the general knowledge of the one skilled in the art.

In this regard, the one skilled in the art may advantageously refer to the method described by Bandini et al. (Molecular Microbiology 85(3), 513-534, 2012).

Preferred polypeptide having phosphoacetylglucosamine mutase activity in the present invention is an enzyme having an EC number of n° 5.4.2.3.

According to a preferred embodiment, the one or more recombinant nucleic acids encoding a polypeptide having phosphoacetylglucosamine mutase activity mayoriginate or be derived from organisms preferably selected in a group consisting of prokaryotic organisms and eukaryotic organisms. In some preferred embodiments, the one or more recombinant nucleic acids encoding a polypeptide having phosphoacetylglucosamine mutase activity may originate or be derived from a yeast, and especially from *Saccharomyces cerevisiae.*

According to a particular embodiment, the one or more recombinant nucleic acids encoding a polypeptide having phosphoacetylglucosamine mutase activity may be selected from the group consisting of nucleic acid sequences having (i) at least 80% nucleic acid identity with a nucleic acid sequence set forth as SEQ ID NO: 29, and (ii) a biological activity of the same nature as the nucleic acid sequence set forth as SEQ ID NO: 29. The nucleic acid set forth as SEQ ID NO: 29 encodes a polypeptide having phosphoacetylglucosamine mutase activity originating from *Saccharomyces,* that may also be termed PCM1.

A biological activity of the same nature regarding this sequence is, as previously explained, the capacity to code for a polypeptide that converts N-acetyl-glucosamine-6-phosphate into N-acetyl-glucosamine-1-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having phosphoacetylglucosamine mutase activity originating from *Saccharomyces cerevisiae,* the one skilled in the art may refer to the accession number NP_010856 in the UniProt database, or to SEQ ID NO. 30 described herein.

According to another particular embodiment, the one or more recombinant nucleic acids encoding a polypeptide having phosphoacetylglucosamine mutase activity may be nucleic acid(s) encoding polypeptide having an amino acid sequence selected from the group consisting of sequences having at least 80% amino acid identity with the amino acid sequence set forth as SEQ ID NO. 30, and also a biological activity of the same nature as the amino acid sequence set forth as SEQ ID NO. 30.

A biological activity of the same nature regarding this sequence is as described previously, i.e. the capacity to catalyze the conversion of N-acetyl-glucosamine-6-phosphate into N-acetyl-glucosamine-1-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having phosphoacetylglucosamine mutase activity in the present invention is regulated by at least one promoter and at least one terminator, such as herein after defined more in detail, which are present in 5' and 3' position respectively of the one or more recombinant nucleic acids encoding a polypeptide having phosphoacetylglucosamine mutase activity.

### PROMOTERS

As disclosed herein, the expression of the genes of interest that have been genetically engineered for obtaining a recombinant cell according to the invention comprise appropriate regulatory sequences that are functional in the recombinant cells of the invention, and in particular in recombinant yeast cells of the invention, including in particular *Saccharomyces cerevisiae.*

Various promoters may be used for the desired expression of the coding sequences of interest.

Promoters according to the invention may be selected from the group consisting of the following promoters:
- pTDH3 (SEQ ID NO: 31),
- pTDH3.sk (SEQ ID NO: 32),
- pTDH3-1.Sba (SEQ ID NO: 33),
- pTDH3.Sar (SEQ ID NO: 34),
- pENO2 (SEQ ID NO: 35),
- pTEF3 (SEQ ID NO: 36),
- pTEF1 (SEQ ID NO: 37),
- pTEF1.ago (SEQ ID NO: 38),
- pTEF1.sba (SEQ ID NO: 39),
- pPDC1 (SEQ ID NO: 40),
- pCCW12 (SEQ ID NO: 41),
- pCCW12.Sm (SEQ ID NO: 42),
- pCCW12.sk (SEQ ID NO: 43),
- pCCW12.sba (SEQ ID NO: 44),
- pCCW12.sar (SEQ ID NO: 45),
- pNUP57 (SEQ ID NO: 46),
- pCCW10.ago (SEQ ID NO: 47),
- pCWP2 (SEQ ID NO: 48),
- pCCW120.Sca (SEQ ID NO: 49), and
- pRPLA1 (SEQ ID NO: 50).

Promoters more particularly interesting in the present invention may be selected from the group consisting of:
- pTDH3 (SEQ ID NO: 31),
- pTDH3.sk (SEQ ID NO: 32),
- pTDH3-1.Sba (SEQ ID NO: 33),
- pTDH3.Sar (SEQ ID NO: 34),
- pENO2 (SEQ ID NO: 35),
- pTEF3 (SEQ ID NO: 36),
- pTEF1 (SEQ ID NO: 37),
- pTEF1.ago (SEQ ID NO: 38),
- pTEF1.sba (SEQ ID NO: 39),
- pPDC1 (SEQ ID NO: 40),
- pCCW12 (SEQ ID NO: 41),
- pCCW12.Sm (SEQ ID NO: 42),
- pCCW12.sk (SEQ ID NO: 43),
- pCCW12.sba (SEQ ID NO: 44), and
- pCCW12.sar (SEQ ID NO: 45).

Said promoters can in particular be selected from the group consisting of pTDH3, pTDH3-1.Sba, pTDH3.Sar, pENO2, pTEF3, pTEF1, pPDC1, pCCW12, pCCW12.Sm, pCCW12.sk, pCCW12.sba, and pCCW12.sar.

Alternatively, promoters interesting in the present invention may be selected from the group consisting of:
- pNUP57 (SEQ ID NO: 46), and
- pCCW10.ago (SEQ ID NO: 47).

Other promoters of interest in the present invention may be:
- pCWP2 (SEQ ID NO: 48);
- pCCW120.Sca (SEQ ID NO: 49); and
- pRPLA1 (SEQ ID NO: 50).

As previously mentioned, inducible or repressible promoters are promoters whose activity is controlled by the presence or absence of biotic or abiotic factors and also by the quantity of said factor. Accordingly, for some promoters, their activity will in particular be induced and thus increased when the quantity of a given factor increases or is increased, and, accordingly, the activity of these same promoters can be repressed and thus reduced when the quantity of said factor diminishes or is reduced. The quantity of said factor(s) in the culture medium of a recombinant yeast cell of the invention comprising inducible or repressible promoters can be decided and thus controlled by the man skilled in the art.

For example, increasing the quantity of copper in a culture medium of a recombinant yeast cell according to the invention comprising a pCUP-1 promoter will induce and thus increase transcription of the gene under the control of this promoter. On the contrary, reducing the quantity of copper in said culture medium will lead to a repression, and thus a reduced, transcription of the gene under the control of this promoter.

In another example, increasing the quantity of methionine in a culture medium of a recombinant yeast cell according to the invention comprising a pMET6 promoter will repress and thus decrease transcription of the gene under the control of this promoter. On the contrary, reducing the quantity of methionine in said culture medium will lead to an induced, and thus an increased, transcription of the gene under the control of this promoter.

For this reason, the following promoters are referred to in the present text as being "inducible or repressible promoters".

According to a first embodiment, inducible or repressible promoters according to the invention may be selected from the group comprising promoters inducible or repressible with copper, promoters inducible or repressible with methionine and promoters inducible or repressible with threonine, and are in particular is pCUP1 - copper inducible or repressible.

According to this embodiment, the inducible or repressible promoter according to the invention can in particular be pCUP1 (SEQ ID NO: 51).

The activity of these promoters is thus induced by the increasing presence of methionine, copper or threonine as indicated above, and their activity diminishes, i.e. is repressed, when the quantity of methionine, copper or threonine is reduced.

According to a second embodiment, inducible or repressible promoters according to the invention may be selected from the group comprising promoters inducible or repressible with copper, promoters inducible or repressible with lysine and promoters inducible or repressible with methionine, and in particular selected from the group consisting of:
- pMET6 - methionine inducible or repressible (SEQ ID NO: 52),
- pMET25 - methionine inducible or repressible (SEQ ID NO: 53), and
- pSAM1 - methionine inducible or repressible (SEQ ID NO: 54),

According to this particular embodiment, the inducible or repressible promoter according ot the invention may be selected from the group consisting of pMET6, pMET25 and pSAM1.

The activity of these promoters is thus repressed by the increasing presence of methionine, copper, lysine or glucose as indicated above, and their activity increases, i.e. is induced, when the quantity of methionine, copper, lysine or glucose is reduced.

In a particular embodiment, inducible or repressible promoters according to the invention may be selected from the group comprising promoters inducible or repressible with copper, promoters inducible or repressible with glucose, promoters inducible or repressible with lysine, promoters inducible or repressible with methionine and promoters inducible or repressible with threonine.

In a more particular embodiment, the inducible or repressible promoter according to the invention may be selected from the group consisting of pCUP1, pMET6, pSAM1, and pMET25.

In a particular embodiment of the invention, promoters interesting in the present invention may be selected from the group consisting of:
- pTDH3 (SEQ ID NO: 31),
- pTEF1 (SEQ ID NO: 37),
- pTEF1.ago (SEQ ID NO: 38),
- pPDC1 (SEQ ID NO: 40),
- pCCW12 (SEQ ID NO: 41),
- pCCW12.sk (SEQ ID NO: 43),
- pCCW12.sba (SEQ ID NO: 44),
- pCCW12.sar (SEQ ID NO: 45),
- pCWP2 (SEQ ID NO: 48),
- pCCW120.Sca (SEQ ID NO: 49),
- pCUP1 (SEQ ID NO: 51),
- pMET6 (SEQ ID NO: 52), and
- pSAM1 (SEQ ID NO: 54).

Synthetic promoters as described in Blazeck & Alper (2013) Biotechnol. J. 8 46-58 can also be used.

The promoters of the invention may originate from any organism from the Saccharomycetes class and may in particular originate from an organism selected from the group consisting of at least one of *Saccharomyces cerevisiae, Saccharomyces boulardii, Saccharomyces castelii, Saccharomyces bayanus, Saccharomyces arboricola, Saccharomyces kudriavzevii, Saccharomyces mikatae, Ashbya gossypii, Kluveromyces lactis, Pichia pastoris, Candida glabrata, Candida tropicalis, Debaryomyces castelii, Yarrowia lipolitica and Cyberlindnera jadinii.*

The promoters of the invention can preferably originate from an organism selected from the group consisting of *Saccharomyces cerevisiae (sc), Saccharomyces mikatae (Sm), Saccharomyces kudriavzevii (sk), Saccharomyces bayanus (sba), and Saccharomyces arboricola (Sar).*

### TERMINATORS

As it is disclosed herein, the expression of the genes of interest that have been genetically engineered for obtaining a recombinant cell according to the invention, and in particular a recombinant yeast according to the invention, comprise appropriate transcription terminator sequences that are functional in recombinant cells of the invention, and in particular in recombinant yeast cells of the invention, in particular in *Saccharomyces cerevisiae.*

Said transcription terminators, identical or different, may be found in literature Yamanishi et al., (2013) ACS synthetic biology 2, 337-347.

Terminators more particularly interesting in the present invention may be selected from the group comprising:
- tTPI1 from the gene encoding for the Triose Phosphate Isomerase (SEQ ID NO: 55),
- tMET25 from the gene encoding for the O-acetyl homoserine-O-acetyl serine sulfhydrylase (SEQ ID NO: 56),
- tDIT1 (SEQ ID NO: 57),
- tRPL3 (SEQ ID NO: 58),
- tRPL3.sm (SEQ ID NO: 59),
- tRPL3.sba (SEQ ID NO: 60),
- tRPL41B (SEQ ID NO: 61),
- tRPL15A (SEQ ID NO: 62),
- tRPL15A.sba (SEQ ID NO: 63),
- tIDP1 (SEQ ID NO: 64), and
- tTEF1.sba (SEQ ID NO: 65).

In particular, said terminator may be selected from the group consisting of tTPI1, tMET25, tDIT1, tRPL3, tRPL3.sm, tRPL3.sba, tRPL41B, tRPL15A, tRPL15A.sba, tIDP1 and tTEF1.sba.

The terminators of the invention can originate from any organism from the Saccharomycetes class and may in particular originate from an organism selected from the group consisting of *Saccharomyces cerevisiae* and *Saccharomyces Bayanus.*

### RECOMBINANT YEAST

Recombinant cells of the invention can be selected from the group consisting of yeasts and bacteria.

Recombinant cells of the invention, such as a recombinant host cell of the invention, are preferably recombinant yeast cells.

Generally, yeast can grow rapidly and can be cultivated at higher density as compared with bacteria, and does not require an aseptic environment in the industrial setting. Furthermore, yeast cells can be more easily separated from the culture medium compared to bacterial cells, greatly simplifying the process for product extraction and purification.

A recombinant cell of the invention, and in particular a recombinant yeast cell of the invention is preferably a *Saccharomycetales* cell.

A recombinant cell of the invention, and in particular a recombinant yeast of the invention can in particular belong to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus.

A recombinant cell of the invention belonging to the *Saccharomyces* genus can be selected from the group consisting of *Saccharomyces cerevisiae, Saccharomyces boulardii, Saccharomyces bayanus, Saccharomyces paradoxus, Saccharomyces mikatae, Saccharomyces castelli, Saccharomyces cariocanus, Saccharomyces kudriavzevii, Saccharomyces arboricolus, Saccharomyces pastorianus, Saccharomyces uvarum* and *Saccharomyces delbrueckii.*

A recombinant cell of the invention belonging to the *Candida* genus can be selected from the group consisting of *Candida albicans, Candida glabrata, Candida tropicalis, Candida dubliniensis, Candida parapsilosis, Candida lusitaniae* and *Candida guilliermondii.*

A recombinant cell of the invention belonging to the *Kluyveromyces* genus can be selected from the group consisting of *Kluyveromyces lactis, Kluyveromyces marxianus, Kluyveromyces polysporus, Kluyveromyces thermotoleren, Kluyveromyces dobzhanskii* and *Kluyveromyces wickerhamii.*

A recombinant cell of the invention belonging to the *Ogataea* genus can be selected from the group consisting of *Ogataea polymorpha, Ogataea histrianica, Ogataea deakii, Ogataea kolombanensis, Ogataea philodendra, Ogataea siamensis, Ogataea angusta, Ogataea parapolymorpha, Ogataea minuta, Ogataea nonfermentans* and *Ogataea kodamae.*

A recombinant cell of the invention belonging to the *Yarrowia* genus can be selected from the group consisting of *Yarrowia lypolytica, Yarrowia parophonii, Yarrowia galli, Yarrowia oslonensis, Yarrowia alimentaria, Yarrowia hollandica* and *Yarrowia yakushimensis.*

A recombinant cell of the invention belonging to the *Debaryomyces* genus can be selected from the group consisting of *Debaryomyces hansenii, Debaryomyces carsonii, Debaryomyces castellii, Debaryomyces marama, Debaryomyces occidentalis, Debaryomyces oviformis, Debaryomyces nepalensis, Debaryomyces coudertii, Debaryomyces udenii, Debaryomyces psychrosporus* and *Debaryomyces yamadae.*

A recombinant cell of the invention belonging to the *Ashbya* genus can be selected from the group consisting of *Ashbya gossypii* and *Ashbya aceri.*

A recombinant cell of the invention, and in particular a recombinant yeast cell of the invention, can in particular be selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

In a particular embodiment, the recombinant yeast cell according to the invention is of the genus *Saccharomyces, Kluyveromyces,* or *Eremothecium,* more particularly is of the species selected from the group consisting of *Saccharomyces cerevisiae, Kluyveromyces Marxianus, Ogataea polymorpha* and *Ashbya gossypii.*

In an embodiment, a recombinant host cell of the invention is a yeast selected belonging to the *Saccharomycetales* order, in particular to the *Saccharomycetaceae* family, and is in particular selected from the group consisting of *Yarrowia lipolitica, Kluyveromyces Marxianus, Ogataea polymorpha, Ashbya gossypii and Saccharomyces cerevisiae.*

A recombinant cell of the invention can most preferably be a *Saccharomyces cerevisiae* cell.

As above-mentioned, a recombinant cell according to the invention has the ability to produce heparosan by insertion of the one or more recombinant nucleic acids according to the present invention. In certain embodiments, a recombinant yeast according to the invention has the ability to produce heparosan having a controlled size by insertion of the one or more recombinant nucleic acids according to the present invention.

Methods implemented to insert a specific DNA construct within a gene belong to the general knowledge of a man skilled in the art. A related method is described in more details in the herein after examples.

However, unexpected technical difficulties were encountered because the consequences of the insertion of DNA constructs within the genome of a cell, and in particular within the genome of a yeast, such as for example in the genome of *Saccharomyces cerevisiae,* are unpredictable. In particular, the survival rate of the cells, and in particular of the yeasts, and their ability to grow and produce the desired heparosan is also unpredictable.

In order to obtain a recombinant cell, and in particular a recombinant yeast, of the invention, many different constructs were tested by the inventors in order to obtain a viable and efficient recombinant cell, and in particular yeast cell.

### CULTURE CONDITIONS

The present invention also relates to the use of a recombinant cell of the invention for the production of heparosan, in particular of heparosan of controlled molecular weight.

The present invention further relates to a method of producing heparosan, in particular heparosan of a desired molecular weight, comprising:
(a) culturing a recombinant cell according to the invention in a cultivation medium for a time sufficient to produce heparosan; and
(b) optionally isolating or recovering the heparosan from the recombinant cell and/or from the cultivation medium.

Typically, cells of the invention, and in particular yeasts of the invention, are grown at a temperature in the range of about 20°C to about 37°C, preferably at a temperature ranging from 27 to 34°C, in an appropriate culture medium.

Suitable growth media for cells of the invention, and in particular for yeasts of the invention, are common commercially prepared media such as broth that includes yeast nitrogen base, ammonium sulfate, and dextrose as the carbon/energy source or YPD Medium, a blend of peptone, yeast extract, and dextrose in optimal proportions for growing most. Other defined or synthetic growth media may also be used and the appropriate medium for growth of the particular cell, and in particular yeast, will be known by one skilled in the art of microbiology or fermentation science.

A particular medium that is suitable herein is the SY medium which comprises the following elements:
KH2PO4: 100 mM; MgSO4 7H2O: 2,8 mM; K2SO4: 11,5 mM; Na2SO4: 1,1 mM; NaCl: 2,6 mM; CaCl2 2H2O: 0,7 mM; CuSO4 5H2O: 15 µM; KI: 6 µM; FeCl3: 30 µM; ZnSO4 7H2O: 61 µM; MnSO4 H2O: 25 µM; H2SO4: 110 µM; Panthotenic Acids hemicalcium salt: 42 µM; Thiamin hydrochloride: 59 µM; Pyridoxin hydrochloride: 49 µM; Myo-Inositol (C6H12O6): 555 µM; Nicotinic acid (C6H5NO2): 29 µM; D-Biotine: 0,82 µM; Ammonium citrate tribasic: 33 mM; and glucose or sucrose 2-30%.

Carbon sources that may be used in the culture medium include fructose, mannose, xylose and arabinose; oligosaccharides such as lactose, maltose, galactose, or sucrose; polysaccharides such as starch or cellulose or mixtures thereof; and unpurified mixtures from renewable feedstocks such as cheese whey permeate cornsteep liquor, sugar beet molasses, and barley malt.

Nitrogen sources that may be included in the culture medium include peptone, yeast extracts, meat extracts, malt extracts, urea, ammonium sulfate, ammonium chloride, ammonium nitrate, ammonium phosphate, ammonium citrate and combinations thereof.

The culture medium may further comprise trace elements (e.g., metal salts), for example magnesium salts, cobalt salts and/or manganese salts; as well as growth factors such as amino acids, vitamins, growth promoters, and the like.

Examples of vitamins that may be included are Panthotenic Acids hemicalcium, Thiamin hydrochloride; Pyridoxin hydrochloride; Myo-Inositol; Nicotinic acid; D-Biotine, folic acid, p-aminobenzoïque acid, riboflavin.

A culture medium of the invention may further include rare elements, such as CuSO₄· 5H₂O, KI, FeCl₃, ZnSO₄ ·7H₂O, MnSO₄ ·H₂O, or H₂SO₄, MgCl2, CaCl2, NaCl, K2HPO4, KH2PO4, ZnCl, H3BO3, MnSO4, Na2MoO4.

The term "appropriate culture medium" is above-defined.

Examples of known culture media for a recombinant cell according to the present invention are known to the person skilled in the art, and are presented in the following publication D. Burke et al., Methods in yeast Genetics - A cold spring harbor laboratory course Manual (2000).

Suitable pH ranges for the fermentation may be between pH 3.0 to pH 7.5, where pH 4 to pH 6 is preferred as the initial condition.

As mentioned elsewhere in this specification, the pH value of the culture medium can be regulated during the cultivation step of a method of the invention in order to modulate the activity of the polypeptide having glucuronosyl-disulfoglucosamine glucuronidase activity, which will impact the molecular weight of the heparosan molecules produced by the recombinant cell of the invention, and in particular by the recombinant yeast of the invention.

In particular, the pH of the culture medium can be modified according to the heparosan that is meant to be produced by the recombinant yeast. For example, the pH of the culture medium may remain at a pH of 4, of 5.5 or of 6 during the culture time.

In a particular embodiment, the pH of the culture medium may change or be changed during the time length of the culture of the recombinant cell of the invention, in particular of the recombinant yeast of the invention. As previously mentioned, *Saccharomyces cerevisiae* acidifies the medium in which it is cultivated and thus reduces the pH of its culture medium. For example, the pH of the culture medium may begin at 6, may be taken down to 4 and then brought back up to 6. In another example, the pH of the culture medium may begin at 6, remain or be maintained at 6, and then may be lowered to 4.

In a particular embodiment, the pH of the culture medium may be modulated during the cultivating step (a) of a method of the invention so that at the end of the cultivating step of a method of the invention, the pH of the cultivation medium is the same as the pH at the beginning of said cultivation step (a).

In another embodiment, the pH of the culture medium may remain the same during the time length of the culture of the recombinant cell of the invention, and in particular the recombinant yeast cell of the invention.

Said time length of the culture of a recombinant cell according to the invention, and in particular of the recombinant yeast cell of the invention, can vary depending on the molecular weight of the heparosan of interest. The longer said time length, the lower the molecular weight of the heparosan in a given culture medium of a recombinant cell of the invention, and in particular a recombinant yeast cell of the invention.

The time length of the culture time of a recombinant cell of the invention, and in particular a recombinant yeast in the present invention can be a period of from about 35 hours to about 50 hours, preferably from about 40 hours to about 50 hours, and is in particular about 48 hours.

Fermentations may be performed under aerobic conditions or micro-aerobic conditions.

The amount of heparosan product in the fermentation medium can be determined using a number of methods known in the art, for example, high performance liquid chromatography (HPLC) or gas chromatography (GC).

The present process may employ a batch method of fermentation. A classical batch fermentation is a closed system where the composition of the medium is set at the beginning of the fermentation and not subject to artificial alterations during the fermentation. Thus, at the beginning of the fermentation, the medium is inoculated with the desired organism or organisms, and fermentation is permitted to occur without adding anything to the system. Typically, however, a "batch" fermentation method or system is batch with respect to the addition of carbon source and attempts are often made at controlling factors such as temperature, pH and oxygen concentration. In batch systems, the metabolite and biomass compositions of the system change constantly up to the time when the fermentation is stopped. Within batch cultures cells progress through a static lag phase to a high growth log phase and finally to a stationary phase where growth rate is diminished or halted. If untreated, cells in the stationary phase will eventually die. Cells in log phase generally are responsible for the bulk of production of end product or intermediate.

A Fed-Batch system may also be used in the present invention. A Fed-Batch system is similar to a typical batch system with the exception that the carbon source substrate is added in increments as the fermentation progresses. Fed-Batch systems are useful when catabolite repression (e.g. glucose repression) is apt to inhibit the metabolism of the cells and where it is desirable to have limited amounts of substrate in the media. Measurement of the actual substrate concentration in Fed-Batch systems is difficult and is therefore estimated on the basis of the changes of measurable factors such as pH, dissolved oxygen and the partial pressure of waste gases such as CO₂.

Batch and Fed-Batch culturing methods are common and well known in the art and examples may be found in Biotechnology: A Textbook of Industrial Microbiology, Crueger, Crueger, and Brock, Second Edition (1989) Sinauer Associates, Inc., Sunderland, MA , or Deshpande, Mukund V., Appl. Biochem. Biotechnol., 36, 227, (1992). Although the present invention is performed in batch mode it is contemplated that the method would be adaptable to continuous fermentation.

Continuous fermentation is an open system where a defined fermentation medium is added continuously to a bioreactor and an equal amount of conditioned media is removed simultaneously for processing. Continuous fermentation generally maintains the cultures at a constant high density where cells are primarily in log phase growth.

Continuous fermentation allows for the modulation of one factor or any number of factors that affect cell growth or end product concentration. For example, one method will maintain a limiting nutrient such as the carbon source or nitrogen level at a fixed rate and allow all other parameters to vary. In other systems a number of factors affecting growth can be altered continuously while the cell concentration, measured by media turbidity, is kept constant. Continuous systems strive to maintain steady state growth conditions and thus the cell loss due to the medium being drawn off must be balanced against the cell growth rate in the fermentation. Methods of modulating nutrients and growth factors for continuous fermentation processes as well as techniques for maximizing the rate of product formation are well known in the art of industrial microbiology.

It is contemplated that the present invention may be practiced using either batch, fed- batch or continuous processes and that any known mode of fermentation would be suitable. Additionally, it is contemplated that cells may be immobilized on a substrate as whole cell catalysts and subjected to fermentation conditions for production.

In order to still improve the heparosan production, a particular embodiment may consist of culturing the recombinant cells of the invention, in particular the recombinant yeast cells of the invention, in an appropriate culture medium, such as above-mentioned, wherein the said culture medium comprises an optimal amount of carbon source, especially glucose or sucrose.

In preferred embodiments, the carbon source comprised in said optimal culture medium consists of glucose and/or sucrose. In preferred embodiments, the said optimal culture medium comprises 1% w/w or more glucose and/or sucrose, in particular comprises 5% w/w or more glucose and/or sucrose, in particular comprises 10% w/w or more glucose and/or sucrose, in particular comprises 15% w/w or more glucose and/or sucrose. In preferred embodiments, the said optimal culture medium comprises at most 40% w/w glucose, which includes at most 35% w/w glucose.

In a preferred embodiment, the method of the invention is carried out on an industrial scale.

More particularly, the cultivation medium of the method according to the invention can be at least about 100L, more preferably in the range of about 1000L to about 3000L, even more preferably about 10,000L, even more preferably 100,000L, or even about 250,000L.

The invention further relates to a method for producing heparosan as previously described, and comprising the steps of:
(a) culturing a recombinant cell of the invention in a culture medium; and
(b) recovering the heparosan from said culture medium,
wherein the heparosan recovered in step (b) has a molecular weight controlled through the selection of:
- the nature and origin of the one or more recombinant nucleic acids encoding a polypeptide having glucuronosyl-disulfoglucosamine glucuronidase activity of the recombinant cell of the invention, and in particular of the recombinant yeast of the invention,
- the nature and origin of the promoter controlling the expression of the one or more recombinant nucleic acids encoding a polypeptide having glucuronosyl-disulfoglucosamine glucuronidase activity of the recombinant cell of the invention, and in particular of the recombinant yeast of the invention,
- the presence or absence of an anchoring signal associated to the one or more recombinant nucleic acids encoding a polypeptide having glucuronosyl-disulfoglucosamine glucuronidase activity of the recombinant cell of the invention, and in particular of the recombinant yeast of the invention,
- the pH of the culture medium during the step of culturing the recombinant cell of the invention, and in particular of the recombinant yeast of the invention, and/or
- the duration of the culturing of the recombinant cell of the invention, and in particular of the recombinant yeast of the invention.

The molecular weight of the heparosan can be a particular molecular weight or more preferably a specific range of molecular weights such as, for example, less than 50 kDa, in the range of about 20 kDa to about 50 kDa, greater than or equal to 50 kDa, in the range of about 50 kDa to about 150kDa, in the range of about 50 kDa to about 250kDa, greater than or equal to 100 kDa, in the range of about 100kDa to about 1500 kDa, in the range of about 150kDa to about 1500 kDa, greater than 1000 kDa or greater than 1500kDa.

The present invention also relates to the use of a recombinant cell according to the invention, and in particular a recombinant yeast cell of the invention, for the production of heparosan having a molecular weight in the range of from about 20kDa to about 50kDa or from about 50kDa to about 1000 kDa.

The secretion signal of the present invention may for example have:
- the nucleic acid sequence set forth as SEQ ID NO: 66; and/or
- the amino acid sequence set forth as SEQ ID NO: 67.

The anchoring signal of the present invention may for example have:
- the nucleic acid sequence set forth as SEQ ID NO: 68; and/or
- the amino acid sequence set forth SEQ ID NO: 69.

The secretion signal may be fused to the polypeptide having glucuronosyl-disulfoglucosamine glucuronidase activity by creating a chimeric nucleic acid starting by a nucleic acid sequence encoding the signal peptide followed by a recombinant nucleic acid encoding a polypeptide having glucuronosyl-disulfoglucosamine glucuronidase activity as previously defined.

The secretion signal and anchoring signal may be fused to the polypeptide having glucuronosyl-disulfoglucosamine glucuronidase activity by creating a chimeric nucleic acid starting by a nucleic acid sequence encoding the signal peptide followed by a recombinant nucleic acid encoding a polypeptide having glucuronosyl-disulfoglucosamine glucuronidase activity as previously defined, followed by a nucleic acid sequence encoding for the anchoring signal.

Such chimeric nucleic acid sequences may be obtained by techniques known by the man skilled in the art such as chemical synthesis of nucleic acids or any recombination techniques such as cloning or PCR.

### PURIFICATION OF HEPAROSAN

According to a specific aspect of the invention, the fermentative production of heparosan comprises a step of isolation of the heparosan from the culture medium. Recovering the heparosan from the culture medium is a routine task for a man skilled in the art. It may be achieved by a number of techniques well known in the art including but not limiting to pervaporation, selective precipitation, filtration, centrifugation, spray drying, lyophylisation or liquid extraction. The expert in the field knows how to adapt parameters of each technique dependant on the characteristics of the material to be separated.

The yeast as model of a cell in the present invention is preferred in that the synthesized heparosan is/are entirely exported outside the cells, thus simplifying the purification process.

Gas stripping is achieved with a stripping gas chosen among helium, argon, carbon dioxide, hydrogen, nitrogen or mixture thereof.

Liquid extraction is achieved with organic solvent as the hydrophobic phase such as pentane, hexane, heptane or dodecane. Renewal solvents may also be used.

### DERIVATIVES

The term "heparosan", as used herein, is also intended to cover derivatives of heparosan, such as but not limited to heparin (sulfated heparosan) useful for applications in a cosmetic product, a flavor product, a fragrance product, a food product, a food, a beverage, a texturant, a pharmaceutical composition, a dietary supplement, a nutraceutical, a cleaning product, and/or a dental and/or an oral hygiene composition or combinations thereof.

Heparin is useful as an anticoagulant (blood thinner) and in alleviating the risk of reducing, preventing and/or treating heart attacks and unstable angina.

In particular, Heparin is generally used for anticoagulation purposes in order to alleviate the risk of, reduce, prevent and/or treat conditions such as, but not limited to:
- Acute coronary syndrome, e.g., NSTEMI;
- Atrial fibrillation;
- Deep-vein thrombosis and pulmonary embolism;
- Cardiopulmonary bypass for heart surgery;
- ECMO circuit for extracorporeal life support;
- Hemofiltration; and/or
- Indwelling central or peripheral venous catheters.

Heparin and its low-molecular-weight derivatives (e.g., enoxaparin, dalteparin, tinzaparin) are effective in preventing deep vein thromboses and pulmonary emboli in people at risk.

Heparin is given by injection into a vein or under the skin.

Other uses include applications inside test tubes and kidney dialysis machines.

Heparosan, in particular Heparin, may be prepared as a composition.

### FORMULATIONS AND PRODUCTS

A composition of the invention may be incorporated into a formulation/product, such as a nutraceutical, pharmaceutical, veterinary, oenological or cosmetic formulation/product.

Thus, the present invention provides a formulation comprising a composition of the invention. For example, the present invention may provide a cosmetic formulation comprising the composition of the invention.

The present invention also provides a product comprising a composition of the invention. For example, the present invention may provide a cosmetic product comprising the composition of the invention.

A "cosmetic product" is intended to mean any substance or mixture intended to be placed in contact with the external parts of the human body (epidermis, hair system, nails, lips and external genital organs) or with the teeth and the mucous membranes of the oral cavity with a view exclusively or mainly to cleaning them, perfuming them, changing their appearance, protecting them, keeping them in good condition, correcting body odours and/or combinations thereof.

A "substance" is intended to mean a chemical element and its compounds in the natural state or obtained by any manufacturing process, including any additive necessary to preserve its stability and any impurity deriving from the process used but excluding any solvent which may be separated without affecting the stability of the substance or changing its composition.

A "mixture" is intended to mean a mixture or solution composed of two or more substances.

The present invention also provides the use of a composition of the invention in a nutraceutical, pharmaceutical, veterinary, oenological or cosmetic formulation/product.

Such formulations or products are hereinafter referred to as the "formulations or products of the invention".

The nutraceutical, pharmaceutical, veterinary, oenological or cosmetic formulation/product may optionally further comprise pharmaceutically/veterinary/cosmetic (including cosmetic active) ingredients, such as excipients, carriers and mixtures thereof as appropriate.

"Cosmetic" or "Cosmetic Active Ingredients" means any and all natural, naturally occurring, nature identical, synthetic synthetically produced, biosynthetically produced, sustainable, renewable and/or biodegradable compounds, ingredients, intermediates, molecules, substances, raw materials or products individually or as part of a mixture of compounds, ingredients, intermediates, molecules, substances, raw materials or products, blends, compositions, formulations (including but not limited to skin moisturizers, creams, balms, serums, oils, eye, facial makeup, wash off hair products, leaveon hair products, hair colorants (including but not limited to natural hair colorants) and/or combinations thereof), finished products and related technologies including, but not limited to components, incorporated, for example, into a cosmetic formulation (such as but not limited to natural colorants, preservatives, emulsifiers, anti-oxidants and the like which do not, for example, have an activity on the skin, hair, scalp and the like but play a role in the formulation of the finished product), delivery systems, marketing aids (such as, for example, coloured unispheres applied to translucent formulations) and methods of making anything related thereto useful in/used for/intended for:
- application by rubbing, pouring, sprinkling, spraying or otherwise directly on or to a human or animal body and/or by placing in contact with the various external and/or on surface parts of a human or animal body (including but not limited to the skin, hair, body hair, the hair system, scalp, nails, lips, external genitalia, teeth, oral and/or nasal mucosa and the like); and/or application indirectly on or to a human or animal body such as for example, application as part of a textile or application to a textile as part of a delivery device (such as a capsule) or a delivery system (such as blends or formulations) applied to a textile; and/or
- cleansing, caring, cooling, beautifying, conditioning, treating, soothing, texturizing, promoting attractiveness, protecting, maintaining, improving, enhancing, altering and/or changing an external part and/or surface of a human or animal body (such as, but not limited to the scalp) or the aesthetic appearance of a human or animal body; and/or with a view to mainly cleaning or perfuming, or protecting or maintaining in good condition or combating body odour or changing the appearance of or correcting or repairing a state of imbalance in skin, oral mucosa, scalp or hair by providing a calming, healing, repairing, or revitalization, hydration of the skin or in order to provide relief to, lubricate, moisten, tone, heal, sterilize, relieve, correct and/or remedy states of dryness, irritation, injury or fatigue, and/or with a view to correcting pigmentation disorders or providing a non-pharmaceutical prevention and/or treatment of dandruff, acne, irritation and/or inflammation and the like and/or rebalancing the bacterial flora (such as, for example, the microbiome) on the surface of the skin (such as, for example, by promoting the level of beneficial bacterial flora on the skin surface) and/or for the purposes of keeping a human or animal body in good condition for health and/or wellbeing purposes and/or for improving the appearance of a human or animal body by, for example, improving the appearance of a product applied to a human or body; and/or
- providing a cosmetic and/or dermatological function and/or benefit with a biological activity benefit (but without affecting a body's structure or function. For the avoidance of doubt, a Cosmetic or Cosmetic Active Ingredient or any part thereof may also qualify as a Functional Ingredient and/or Nutraceutical.

"Functional Ingredient" means a food ingredient or part of a food that provides medicinal or health benefits, including any of the following: a carotenoid, dietary fiber, fatty acid, saponin, antioxidant, flavonoid, isothiocyanate, phenol, polyphenol (such as resveratrol), plant sterol or stanol (phytosterols and phytostanols), a polyol, a prebiotic, a phytoestrogen, soy protein, sulfides/thiol, a vitamin, glucosamine, preservatives, hydration agents, edible gelling ingredients, edible gel mixes and gel compositions, long chain primary aliphatic saturated alcohols, colour agents, texturizing agents, emulsifiers and combinations thereof.

"Nutraceutical" means any and all natural, naturally occurring, sustainable, synthetically-produced and biosynthetically-produced compounds, mixtures of compounds, Functional Ingredients, molecules, compositions, raw materials, and intermediates (including components and delivery devices (such as capsules) related thereto, delivery systems thereof (such as blends or formulations) and methods of making the foregoing) that are associated with health and/or cosmetic benefits, as well as improving or maintaining the appearance of the human body. For the avoidance of doubt, Nutraceuticals includes compounds that can be used as supplements to food or beverage, whether a solid formulation, capsule, tablet, liquid formulation, solution or suspension.

Alternatively, the nutraceutical, pharmaceutical, veterinary, oenological or cosmetic formulation/product may consist or consist essentially of the composition of the invention.

The cosmetic formulation/product may be an anti-aging formulation.

As used herein, references to pharmaceutically, veterinary or cosmetically acceptable excipients may refer to pharmaceutically, veterinary or cosmetically acceptable adjuvants, diluents and/or carriers as known to those skilled in the art.

By "pharmaceutically/veterinary/cosmetically acceptable" we mean that the additional components of the composition are generally safe, non-toxic, and neither biologically nor otherwise undesirable. For example, the additional components may be generally sterile and pyrogen free. Such components must be "acceptable" in the sense of being compatible with the composition of the invention and not deleterious to the recipients thereof. Thus, "pharmaceutically acceptable excipients" includes any compound(s) used in forming a part of the formulation that is intended to act merely as an excipient, i.e. not intended to have biological activity itself.

The nutraceutical, pharmaceutical, veterinary, oenological or cosmetic formulation/product may be in the form of a liquid or a solid.

Liquid dosage formulations/products for oral administration include solutions, emulsions, aqueous or oily suspensions, syrups and elixirs.

Formulations and products (e.g. pharmaceutical, veterinary or cosmetic formulations/products) described herein, such as those intended for oral administration, may be prepared according to methods known to those skilled in the art, such as by mixing the components of the formulation/product together.

The formulation or product (e.g. pharmaceutical, veterinary or cosmetic formulation/product) may contain one or more additional ingredients, such as pharmaceutical ingredients and excipients, such as sweetening agents, flavouring agents, colouring agents and preserving agents.

The formulation or product (e.g. pharmaceutical, veterinary or cosmetic formulation/product) may also contain one or more additional active ingredients, such as cosmetic or pharmaceutical active ingredients, such as hyaluronic acid, centella asiatica extract, peptides such as Matrixyl^{®} and Argireline^{®}, and mixtures thereof.

The formulation or product of the invention may contain the active ingredient(s) in admixture with non-toxic pharmaceutically acceptable excipients (or ingredients). These excipients (or ingredients) may, for example, be: inert diluents, such as calcium carbonate, sodium carbonate, lactose, calcium phosphate or sodium phosphate; granulating and disintegrating agents, for example, corn starch, maltodextrin or alginic acid; binding agents, for example, starch, gelatine or acacia; or lubricating agents, for example magnesium stearate, stearic acid, talc and mixtures thereof.

Liquid formulations or products (e.g. pharmaceutical, veterinary or cosmetic formulations/products) may be contained within a capsule, which may be uncoated or coated as defined above.

Suitable pharmaceutical or veterinary carriers include inert solid diluents or fillers, sterile aqueous solutions and various organic solvents. Examples of liquid carriers are syrup, peanut oil, olive oil, phospholipids, fatty acids, fatty acid amines, polyoxyethylene and water.

Moreover, the carrier or diluent may include any sustained release material known in the art, such as glyceryl monostearate or glyceryl distearate, alone or mixed with a wax.

Suitable pharmaceutical carriers include inert sterile aqueous solutions and various organic solvents. Examples of liquid carriers are syrup, vegetables oils, phospholipids, fatty acids, fatty acid amines, polyoxyethylene and water. Moreover, the carrier or diluent may include any sustained release material known in the art, such as glyceryl monostearate or glyceryl distearate, alone or mixed with a wax.

Suitable cosmetic carriers are typically those that are suitable for topical administration to the outer surface of the human body, such as the skin and/or hair and/or scalp.

Typically, such carriers are dermatologically acceptable.

The phrase "dermatologically acceptable carrier" means that the carrier is suitable for topical application to the keratinous tissue, has good aesthetic properties, is compatible with the actives in the composition, and will not cause any unreasonable safety or toxicity concerns.

The carrier can be in a wide variety of forms. In some instances, the solubility or dispersibility of the components (e.g. extracts, sunscreen active, additional components) may dictate the form and character of the carrier. Non-limiting examples include simple solutions (e.g. aqueous or anhydrous), dispersions, emulsions, and solid forms (e.g. gels, sticks, flowable solids, or amorphous materials).

The dermatologically acceptable carrier may be in the form of an emulsion. An emulsion may be generally classified as having a continuous aqueous phase (e.g. oil-in-water and water-in-oil-in-water) or a continuous oil phase (e.g. water-in-oil or oil-in-water). The oil phase of the present invention may comprise silicone oils, non-silicone oils such as hydrocarbon oils, esters, ethers, and the like, and mixtures thereof. The aqueous phase typically comprises water and water-soluble ingredients (e.g. water-soluble moisturizing agents, conditioning agents, anti-microbials, humectants and/or other skin care actives). However, in some instances, the aqueous phase may comprise components other than water, including but not limited to watersoluble moisturizing agents, conditioning agents, antimicrobials, humectants and/or other watersoluble skin care actives. In some instances, the non-water component of the composition comprises a humectant, such as glycerin and/or other polyol(s). Emulsions may also contain an emulsifier. Emulsifiers may be non-ionic, anionic or cationic.

The carrier may contain one or more dermatologically acceptable, hydrophilic diluents. As used herein, "diluent" includes materials in which the composition of the invention can be dispersed, dissolved, or otherwise incorporated. Hydrophilic diluents include water, organic hydrophilic diluents, such as lower monovalent alcohols (e.g., C1-C4), and low molecular weight glycols and polyols, including propylene glycol, polyethylene glycol, polypropylene glycol, glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof.

The cosmetic formulation/product may optionally include one or more additional ingredients commonly used in cosmetic compositions (e.g., colorants, skin tone agents, skin anti-aging agents, anti-inflammatory agents, sunscreen agents, combinations of these and the like), provided that the additional ingredients do not undesirably alter the anti-glycation benefits provided by the composition.

In some instances, it may be desirable to select skin tone agents that function via different biological pathways so that the actives do not interfere with one another, which could reduce the efficacy of both agents. The additional ingredients, when incorporated into the composition, should be suitable for use in contact with human skin tissue without undue toxicity, incompatibility, instability, allergic response, and the like.

The term "carrier" as used herein, may also refer to a natural product or a product originating from nature that has been transformed or modified so that it is distinct from the natural product from which it originated, such as maltodextrin.

The amount of the composition of the invention present in nutraceutical, pharmaceutical, veterinary, oenological or cosmetic formulations or products will vary depending on the application.

Typically, the amount of composition of the invention that may be present in nutraceutical, pharmaceutical, veterinary, oenological or cosmetic formulations or products will be from about 0.001 to about 50% by weight, such as from about 0.01% to about 30% or from about 1% to about 20% of the nutraceutical, pharmaceutical, veterinary, oenological or cosmetic formulations or products, such as from about 0.01 to about 20%, or from about 0.1 to 10% or from about 1 to about 5% by weight of the formulation or product.

Those of skill in the art will recognize that, due to the degenerate nature of the genetic code, a variety of DNA molecules differing in their nucleotide sequences can be used to encode a given enzyme of the disclosure. The native DNA sequence encoding the biosynthetic enzymes described above are referenced herein merely to illustrate an embodiment of the disclosure, and the disclosure includes DNA molecules of any sequence that encode the amino acid sequences of the polypeptides and proteins of the enzymes utilized in the methods of the disclosure. In similar fashion, a polypeptide can typically tolerate one or more amino acid substitutions, deletions, and insertions in its amino acid sequence without loss or significant loss of a desired activity. The disclosure includes such polypeptides with different amino acid sequences than the specific proteins described herein so long as the modified or variant polypeptides have the enzymatic anabolic or catabolic activity of the reference polypeptide. Furthermore, the amino acid sequences encoded by the DNA sequences shown herein merely illustrate embodiments of the disclosure.

Described herein are specific genes and proteins useful in the methods, compositions and organisms of the disclosure; however it will be recognized that absolute identity to such genes is not necessary. For example, changes in a particular gene or polynucleotide comprising a sequence encoding a polypeptide or enzyme can be performed and screened for activity. Typically such changes comprise conservative mutations and silent mutations. Such modified or mutated polynucleotides and polypeptides can be screened for expression of a functional enzyme using methods known in the art.

Due to the inherent degeneracy of the genetic code, other polynucleotides which encode substantially the same or functionally equivalent polypeptides can also be used to clone and express the polynucleotides encoding such enzymes.

Techniques known to those skilled in the art may be suitable to identify additional homologous genes and homologous enzymes. Generally, analogous genes and/or analogous enzymes can be identified by functional analysis and will have functional similarities.

Techniques known to those skilled in the art may be suitable to identify analogous genes and analogous enzymes or any biosynthetic pathway genes, proteins, or enzymes, techniques may include, but are not limited to, cloning a gene by PCR using primers based on a published sequence of a gene/enzyme of interest, or by degenerate PCR using degenerate primers designed to amplify a conserved region among a gene of interest. Further, one skilled in the art can use techniques to identify homologous or analogous genes, proteins, or enzymes with functional homology or similarity. Techniques include examining a cell or cell culture for the catalytic activity of an enzyme through *in vitro* enzyme assays for said activity (e.g. as described herein or in Kiritani, K., Branched-Chain Amino Acids Methods Enzymology, 1970), then isolating the enzyme with said activity through purification, determining the protein sequence of the enzyme through techniques such as Edman degradation, design of PCR primers to the likely nucleic acid sequence, amplification of said DNA sequence through PCR, and cloning of said nucleic acid sequence. To identify homologous or similar genes and/or homologous or similar enzymes, analogous genes and/or analogous enzymes or proteins, techniques also include comparison of data concerning a candidate gene or enzyme with databases such as BRENDA, KEGG, or MetaCYC. The candidate gene or enzyme may be identified within the above-mentioned databases in accordance with the teachings herein.

The terms "between... and..." and "ranging from... to..." should be understood as being inclusive of the limits, unless otherwise specified. Throughout this specification and the claims which follow, unless the context requires otherwise, the word "comprise", and variations such as "comprises", "comprising" and "possesses", will be understood to imply the inclusion of a stated integer or step or group of integers or steps but not the exclusion of any other integer or step or group of integer or step. The term "comprising" also means "including" as well as "consisting" e.g. a composition "comprising" X may consist exclusively of X or may include something additional e.g. X + Y. It must be noted also that, as used in this specification and the appended claims, the singular forms "a", "an" and "the" include plural referents unless the content clearly dictates otherwise. By way of example, a reference to "a gene" or "an enzyme" is a reference to "one or more genes" or "one or more enzymes".

It is to be understood that this disclosure is not limited to the particular methodology, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by the person skilled in the art. In accordance with the present disclosure there may be conventional molecular biology, microbiology, and recombinant DNA techniques employed which are within the skill of the art.

This disclosure is not limited in its application to the details of construction and the arrangement of components set forth in the following description or illustrated in the drawings. The disclosure is capable of other embodiments and of being practiced or of being carried out in various ways. Also, the phraseology and terminology used herein is for the purpose of description and should not be regarded as limiting. Preferably, the terms used herein are defined as described in "A multilingual glossary of biotechnological terms: (IUPAC Recommendations)", Leuenberger, H.G.W, Nagel, B. and Kolbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety.

The examples and figures which follow are presented by way of illustration and without implied limitation of the invention.

### EXAMPLES

### Exemple 1: Protocol for making a recombinant Saccharomyces cerevisiae strain according to the invention

All the hereinafter implemented recombinant *Saccharomyces cerevisiae* strains were constructed from standard strains using standard yeast molecular genetics procedure (Methods in yeast Genetics - A cold spring harbor laboratory course Manual (2000) by D. Burke, D. Dawson, T. Stearns CSHL Press).

Cluster of the following-mentioned genes were integrated in recombinant yeast at once using the ability of yeast to efficiently recombine free DNA ends which have sequence homology.

In addition, for a better comprehension of following genotypes:
- jlp1, lyp1, sam3, his3, leu2, trp1 and ura3 are insertion sites.
- Lowercase letters mean that the considered gene is inactive, uppercase letters reflect an active gene.
- "::": following a gene name means that the gene is interrupted by what follows (if more than one gene are inserted, they are noted in brackets []). The interruption of the gene is concomitant with an entire deletion of the coding sequence but preserves the promoter. In consequence the gene followed by "::" is inactive and is noted in lowercase. If not specified, the transcription of the gene inserted is controlled by the promoter of the disrupted gene.
- "gene.Kl" means that the gene originates from *Kluyveromyces lactis.* When nothing is indicated after a gene in the present examples, this means that the gene is from *Saccharomyces cerevisiae.*

More particularly, the coding sequences to be cloned were artificially synthetized. For heterologous sequences (non-yeast), the nucleic sequences were modified in order to obtain a synonymous coding sequence using the yeast codon usage. Using restriction enzyme and classical cloning technology, each synthetic sequence was cloned in between a transcription promoter and a transcription terminator. Each promoter sequence is preceded by a 50 to 200 nucleotide sequence homologous to the sequence of the terminator of the upstream gene. Similarly, the terminator of each gene (a gene comprising the promoter-coding sequence-terminator) is followed by sequences homologous to the gene immediately following. So that each of the unit to be integrated have a 50-200 nucleotide overlap with both the unit upstream and the unit downstream. For the first unit, the promoter is preceded by 50-200 nucleotides homologous to the yeast chromosome nucleotide for the locus in which it will be integrated. Similarly, for the last unit, the terminator is followed by 50-200 nucleotides homologous to the yeast chromosome nucleotide for the locus in which it will be integrated.

Each unit is then PCR amplified from the plasmids constructs, yielding X unit of linear DNA having overlapping sequences. At least one of this gene is an auxotrophic marker, in order to select for recombination event. All the linear fragments are transformed in the yeast at once, and a recombinant yeast cell is selected for the auxotrophy related to the marker used. The integrity of the sequence is then verified by PCR and sequencing.

### Example 2: Comparative examples for the production of Heparosan

Seven recombinant strains are derived from the Wild Type strain CC788-2D (Cherest et al. (2000) J Biol. Chem. 275: 14056-14063) which does not produce any heparosan: YA5398-3, YA5402-1, YA5400-1, YA5401-1, YA5437-18, YA5435-2 and DA2531-12.

These seven strains are as follows:
**YA5398-3** : MAT-a, canl-100, his3::[tRPL3-UGP1-pSAM1, pMET6-QRI1-tIDP1, HIS3]x8, leu2::[URA3.Sba-loxP, pCCW12.Sba-HASB.Vir-tRPL3.Sm, pCCW12.Sk-HASB-A.Vir-tTEF1.Sba, pCCW12.Sar-HSS2.Pm-tRPL3.Sba, pCCW120.Sca-HSS2.Pm-tRPL15A.Sm], leu2, trp1, ura3
**YA5402-1** : MAT-a, canl-100, his3::[tRPL3-UGP1-pSAM1, pMET6-QRI1-tIDP1, HIS3]x8, jlp1::[LEU2.Sba-loxP, pCCW12.Sba-HASB.Vir-tRPL3.Sm, pCCW12.Sk-HASB-A.Vir-tTEF1.Sba, pCCW12.Sar-HSS2.Pm-tRPL3.Sba, pCCW120.Sca-HSS2.Pm-tRPL15A.Sm], leu2, trp1, ura3
**YA5400-1** : MAT-a, canl-100, his3::[tRPL3-UGP1-pSAM1, pMET6-QRI1-tIDP1, HIS3]x8, jlp1::[LEU2.Sba-loxP, pCCW12.Sba-HASB.Vir-tRPL3.Sm, pCCW12.Sk-HASB-A.Vir-tTEF1.Sba, pCCW12.Sar-HSS2.Pm-tRPL3.Sba, pCCW120.Sca-HSS2.Pm-tRPL15A.Sm, pTEF1.Ago-GFA1-tRPL15A], leu2, trp1, ura3
**YA5401-1** : MAT-a, canl-100, his3::[tRPL3-UGP1-pSAM1, pMET6-QRI1-tIDP1, HIS3]x8, jlp1::[LEU2.Sba-loxP, pCCW12.Sba-HASB.Vir-tRPL3.Sm, pCCW12.Sk-HASB-A.Vir-tTEF1.Sba, pCCW12.Sar-HSS2.Pm-tRPL3.Sba, pCCW120.Sca-HSS2.Pm-tRPL15A.Sm, pTEF1.Ago-GFA1.Vir-tRPL15A], leu2, trp1, ura3
**YA5437-18** : MAT-a, canl-100, his3::[tRPL3-UGP1-pSAM1, pMET6-QRI1-tIDP1, HIS3]x8, jlp1::[LEU2.Sba-loxP, pCCW12.Sba-HASB.Vir-tRPL3.Sm, pCCW12.Sk-HASB-A.Vir-tTEF1.Sba, pCCW12.Sar-HSS1.Pm-tRPL15A.Sm], leu2, trp1, ura3
**YA5435-2:** MAT-α, canl-100, his3::[tRPL3-UGP1-pSAM1, pMET6-QRI1-tIDP1, HIS3]x8, jlp1::[LEU2.Sba-loxP, pCCW12.Sba-HASB.Vir-tRPL3.Sm, pCCW12.Sk-HASB-A.Vir-tTEF1.Sba, pCCW12.Sar-HSS1.Pm-tRPL15A.Sm, pTEF1.Ago-GFA1.Vir-tRPL15A], leu2, trp1, ura3
**DA2531-12** : MAT-a/MAT-α, CAN1/can1-100, his3/his3::[tRPL3-UGP1-pSAM1, pMET6-QRI1-tIDP1, HIS3]x8, jlp1::[LEU2.K1-RS, pCUP1-UGP1-tRPL3, pCUP1-QRI1-tIDP1, pPDC1-UGP1-tTPI1, pTDH3-QRI1-tMET25, pCCW12-HASB.At-tRPL15A]/jlp1::[LEU2.Sba-loxP, pCCW12.Sba-HASB.Vir-tRPL3.Sm, pCCW12.Sk-HASB-A.Vir-tTEF1.Sba, pCCW12.Sar-HSS1.Pm-tRPL15A.Sm, pTEF1.Ago-GFA1.Vir-tRPL15A], leu2/leu2, SAM3/sam3::[LEU2.K1-RS, pPDC1-PGM1-t1DP1, pTEF1.Ago-GFA1-tRPL15A, pENO2-UGP1-tRPL3, pCWP2-GNA1-tTPI1, pTEF1-PCM1-tRPL41B, pCCW12.Sba-HASB.vir-tRPL3.Sm, pCCW12.Sk-HASB-A.Vir-tTEF1.Sba, pCCW12.Sar-HSS2.Pm-tRPL3.Sba, pCCW120.Sca-HSS2.Pm-tRPL15A.Sm, pTDH3-1.Sba-QRI1-tIDP1.Sba, HIS3.Sba-loxP], trp1/trp1, ura3
HSS1.Pm represents a sequence encoding an heparosan synthase A from *Pasteurella multocida* (UniProt reference Q8RP78). HSS2.Pm represents a sequence encoding an heparosan synthase B from *Pasteurella multocida* (UniProt reference Q5SGE1).

All these strains were incubated in 25 ml of SY medium buffered with MES 0,1 M ph 5.5 baffled erlenmeyer for 48 hours at 28°C under vigourous agitation in Erlenmeyers flasks.

SY medium comprises the following elements:
KH2PO4: 100 mM; MgSO4 7H2O: 2,8 mM; K2SO4: 11,5 mM; Na2SO4: 1,1 mM; NaCl: 2,6 mM; CaCl2 2H2O: 0,7 mM; CuSO4 5H2O: 15 µM; KI: 6 µM; FeC13: 30 µM; ZnSO4 7H2O: 61 µM; MnSO4 H2O: 25 µM; H2SO4: 110 µM; Panthotenic Acids hemicalcium salt: 42 µM; Thiamin hydrochloride: 59 µM; Pyridoxin hydrochloride: 49 µM ; Myo-Inositol (C6H12O6): 555 µM ; Nicotinic acid (C6H5NO2): 29 µM; D-Biotine: 0,82 µM; Ammonium citrate tribasic: 33 mM; and glucose 2%

Growth medium was recovered at 48h hours and assayed for heparosan content and quality. An aliquot of the medium was loaded and ran on a 0,5% agarose gel subsequently colored with bromophenol blue. The amount of heparosan present in the medium was evaluated by a colorimetric determination after treatment with concentrated sulfuric acid and carbazole (Bitter and Muir (1962) analytical biochemistry 4, 330-334).

The amounts of heparosan obtained with these different strains are respectively:
- YA5398-3: 170 mg.L⁻¹.
- YA5402-1: 170 mg.L⁻¹.
- YA5400-1: 110 mg.L⁻¹.
- YA5401-1: 180 mg.L⁻¹.
- YA5437-18: 200 mg.L⁻¹.
- YA5435-2: 190 mg.L⁻¹.
- DA2531-12: 235 mg.L⁻¹.

In comparision, as indicated above, a native strain does not produce heparosan.

It results from this experiment that a recombinant strain comprising the modifications according to the invention produces a greater amount of heparosan when cultured in the same conditions as compared with other recombinant strains not comprising all the genetic modifications according to the invention.

### SEQUENCES

SEQ ID NO: 1 is a reencoded nucleic acid sequence of heparosan synthase originating from Pasteurella multocida (HSS1)
ATGAGTTTGTTCAAAAGGGCGACTGAGCTGTTCAAATCGGGTAACTATAAGGATGCACTGACATTGTACGAGAAC
ATAGCCAAAATTTATGGCTCAGAATCCCTAGTGAAGTATAACATAGATATCTGCAAGAAGAACATAACCCAATCA
AAATCGAACAAGATAGAAGAGGACAATATTTCCGGAGAGAATAAGTTCTCCGTCTCTATCAAAGACTTATACAAC
GAAATTAGTAATTCTGAGCTAGGGATAACAAAGGAGAGATTAGGAGCACCACCCTTGGTAAGCATAATTATGACG
AGTCATAATACAGAGAAATTCATCGAAGCTTCCATCAACAGTTTATTATTGCAAACTTATAACAATCTTGAGGTC
ATTGTGGTAGACGACTACTCCACAGACAAGACCTTCCAAATCGCGTCCAGGATTGCAAACTCGACGTCAAAAGTT
AAGACTTTCAGATTGAATTCCAACCTTGGAACGTACTTCGCAAAGAATACGGGAATCCTAAAATCAAAAGGGGAC
ATTATATTCTTTCAAGATTCTGATGATGTCTGCCATCATGAACGTATTGAGAGATGTGTAAATGCACTTTTGTCT
AACAAAGATAACATTGCCGTTAGGTGCGCTTATTCTCGTATTAATCTGGAGACCCAGAACATCATAAAGGTCAAC
GACAACAAGTACAAGTTAGGTCTTATTACCCTAGGAGTATACCGTAAAGTATTTAATGAAATAGGATTCTTCAAC
TGTACCACTAAGGCAAGTGACGACGAATTTTATCACAGGATCATCAAATACTATGGCAAGAATAGGATAAACAAT
TTGTTCCTACCTCTATACTACAATACCATGCGTGAAGATAGTTTGTTTAGCGATATGGTCGAGTGGGTAGACGAA
AACAACATTAAACAGAAAACAAGTGATGCACGTCAGAATTATCTGCATGAATTCCAAAAGATCCACAACGAAAGG
AAATTGAATGAGTTAAAGGAGATTTTCAGCTTCCCCAGGATTCACGATGCCCTACCGATAAGTAAAGAGATGAGC
AAGTTGTCTAACCCAAAGATCCCTGTCTACATTAACATTTGCAGTATACCATCTAGGATAAAGCAATTACAGTAT
ACGATCGGAGTGCTGAAGAATCAGTGTGACCATTTCCACATTTACTTAGATGGTTATCCAGAGGTTCCAGATTTC
ATAAAGAAGCTAGGCAACAAGGCTACCGTAATCAATTGCCAAAACAAGAACGAGTCTATCAGGGACAACGGCAAG
TTTATCCTTTTAGAAAAGCTGATCAAAGAGAATAAGGACGGCTATTATATTACCTGTGATGATGACATAAGGTAT
CCTGCTGACTACACGAATACCATGATAAAGAAGATTAACAAGTACAATGACAAAGCGGCAATAGGTCTACACGGC
GTCATCTTTCCGTCCAGAGTGAATAAATACTTCTCATCTGACAGGATTGTTTATAACTTTCAGAAGCCCTTGGAA
AACGACACAGCCGTCAACATTTTGGGTACTGGACCGTTGCATTTAGAGTCTCCATCTTCAACAAGTTTTCCCTG
AGCGACTTCGAGCACCCTGGTATGGTGGATATTTATTTCTCAATACTATGCAAGAAGAACAACATATTGCAAGTT
TGTATAAGCAGGCCCTCAAACTGGTTAACCGAGGACAACAAGAATACCGAAACCCTGTTCCACGAGTTCCAAAAC
CGTGACGAGATCCAGAGTAAGTTGATCATCTCTAATAATCCGTGGGGCTACAGTAGCATCTACCCCTTACTTAAC
AACAATGCCAACTATAGCGAGTTGATACCATGCTTATCATTCTATAATGAGTAA

SEQ ID NO: 2 is a reencoded nucleic acid sequence of heparosan synthase originating from Pasteurella multocida (HSS2)
ATGAAGGGCAAGAAAGAGATGACCCAAATACAGATTGCTAAGAACCCGCCCCAACACGAGAAGGAAAACGAGCTG
AATACTTTCCAAAACAAGATCGACTCCCTTAAAACGACATTAAACAAGGACATTATATCACAACAGACATTGTTG
GCTAAACAGGACTCAAAGCACCCATTATCTGCAAGTCTTGAGAACGAGAACAAACTTCTTCTTAAACAGTTGCAG
CTTGTTTTGCAGGAATTTGAAAAGATATACACTTACAACCAAGCACTAGAGGCTAAGCTTGAGAAAGACAAACAG
ACCACCTCAATAACCGATTTATATAACGAAGTTGCAAAGTCTGATCTAGGGCTAGTTAAGGAAACTAACAGTGCT
AACCCTCTAGTCTCCATTATTATGACTTCGCACAATACCGCTCAATTTATCGAGGCATCGATTAACTCCTTGTTG

CTACAGACATATAAGAACATTGAGATAATTATAGTCGATGATGACAGTTCCGACAACACTTTTGAAATCGCCTCA
AGGATCGCAAACACAACGTCTAAGGTCCGTGTGTTTCGTTTGAACTCCAATCTGGGCACGTACTTTGCTAAGAAT
ACAGGAATCCTTAAGAGTAAAGGCGACATAATATTCTTCCAGGACTCCGACGACGTTTGTCATCATGAGAGAATC
GAAAGGTGCGTGAACATTTTGTTGGCTAACAAAGAGACGATTGCTGTGAGGTGCGCATACAGTAGGCTGGCTCCT
GAAACGCAGCATATAATTAAAGTCAACAACATGGATTATAGGCTTGGTTTCATCACACTGGGAATGCATAGGAAG
GTTTTCCAAGAGATTGGGTTCTTCAACTGTACAACAAAAGGCAGTGACGACGAGTTCTTCCACCGTATAGCTAAA
TACTACGGTAAAGAAAAGATTAAGAACCTTTTATTGCCGCTTTATTATAACACGATGCGTGAAAACTCACTTTTC
ACTGACATGGTCGAGTGGATCGACAACCATAATATCATACAGAAGATGTCTGACACACGTCAACACTATGCTACT
CTGTTTCAGGCGATGCATAATGAGACTGCATCACACGACTTCAAGAACTTGTTCCAGTTCCCACGTATATACGAC
GCCTTGCCTGTTCCACAGGAGATGTCTAAGTTATCTAACCCTAAAATACCGGTGTACATAAATATATGCAGTATA
CCATCTAGGATTGCACAGTTACGTAGGATTATTGGGATCTTAAAGAACCAGTGCGACCACTTTCACATATACCTA
GACGGATACGTGGAGATCCCTGATTTTATTAAGAATCTTGGTAATAAAGCAACGGTCGTCCACTGCAAAGACAAG
GACAACTCTATTAGGGACAACGGCAAGTTTATTCTACTGGAAGAGTTAATAGAAAAGAATCAAGACGGGTACTAC
ATAACGTGCGACGACGACATTATCTATCCATCGGACTATATAAACACAATGATTAAGAAGCTGAACGAGTATGAT
GACAAGGCGGTCATAGGGCTTCACGGAATATTATTTCCGTCAAGGATGACGAAATACTTTTCCGCCGATAGGCTT
GTTTATTCGTTTTACAAACCCCTAGAGAAAGACAAAGCAGTAAATGTTCTTGGGACCGGAACTGTCTCATTCAGA
GTTAGTCTGTTCAACCAATTCTCCCTTTCAGACTTCACCCATTCGGGAATGGCCGACATCTACTTCTCTCTATTG
TGCAAGAAGAACAACATTTTACAAATCTGTATCTCCAGACCGGCAAATTGGTTGACGGAAGATAACAGAGATTCT
GAGACCTTATACCACCAGTATAGAGATAATGACGAGCAGCAGACTCAGTTGATCATGGAAAATGGGCCCTGGGGT
TACTCAAGTATCTATCCACTTGTCAAGAATCACCCGAAGTTTACGGACTTAATACCATGTTTGCCTTTCTACTTC
CTATAA

SEQ ID NO: 3 is an amino acid sequence of heparosan synthase originating
from Pasteurella multocida (HSS1)
MSLFKRATELFKSGNYKDALTLYENIAKIYGSESLVKYNIDICKKNITQSKSNKIEEDNISGENKFSVSIKDLYN
EISNSELGITKERLGAPPLVSIIMTSHNTEKFIEASINSLLLQTYNNLEVIVVDDYSTDKTFQIASRIANSTSKV
KTFRLNSNLGTYFAKNTGILKSKGDIIFFQDSDDVCHHERIERCVNALLSNKDNIAVRCAYSRINLETQNIIKVN
DNKYKLGLITLGVYRKVFNEIGFFNCTTKASDDEFYHRIIKYYGKNRINNLFLPLYYNTMREDSLFSDMVEWVDE
NNIKQKTSDARQNYLHEFQKIHNERKLNELKEIFSFPRIHDALPISKEMSKLSNPKIPVYINICSIPSRIKQLQY
TIGVLKNQCDHFHIYLDGYPEVPDFIKKLGNKATVINCQNKNESIRDNGKFILLEKLIKENKDGYYITCDDDIRY
PADYTNTMIKKINKYNDKAAIGLHGVIFPSRVNKYFSSDRIVYNFQKPLENDTAVNILGTGTVAFRVSIFNKFSL
SDFEHPGMVDIYFSILCKKNNILQVCISRPSNWLTEDNKNTETLFHEFQNRDEIQSKLIISNNPWGYSSIYPLLN
NNANYSELIPCLSFYNE

SEQ ID NO: 4 is an amino acid sequence of heparosan synthase originating
from Pasteurella multocida (HSS2)
MAKGKKEMTQIQIAKNPPQHEKENELNTFQNKIDSLKTTLNKDIISQQTLLAKQDSKHPLSASL
ENENKLLLKQLQLVLQEFEKIYTYNQALEAKLEKDKQTTSITDLYNEVAKSDLGLVKETNSANPLVSIIMTSHNT

AQFIEASINSLLLQTYKNIEIIIVDDDSSDNTFEIASRIANTTSKVRVFRLNSNLGTYFAKNTGILKSKGDIIFF
QDSDDVCHHERIERCVNILLANKETIAVRCAYSRLAPETQHIIKVNNMDYRLGFITLGMHRKVFQEIGFFNCTTK
GSDDEFFHRIAKYYGKEKIKNLLLPLYYNTMRENSLFTDMVEWIDNHNIIQKMSDTRQHYATLFQAMHNETASHD
FKNLFQFPRIYDALPVPQEMSKLSNPKIPVYINICSIPSRIAQLRRIIGILKNQCDHFHIYLDGYVEIPDFIKNL
GNKATVVHCKDKDNSIRDNGKFILLEELIEKNQDGYYITCDDDIIYPSDYINTMIKKLNEYDDKAVIGLHGILFP
SRMTKYFSADRLVYSFYKPLEKDKAVNVLGTGTVSFRVSLFNQFSLSDFTHSGMADIYFSLLCKKNNILQICISR
PANWLTEDNRDSETLYHQYRDNDEQQTQLIMENGPWGYSSIYPLVKNHPKFTDLIPCLPFYFL

SEQ ID NO: 5 is a nucleic acid sequence of UDP-Glucose dehydrogenase (HASB) originating from Arabidopsis thaliana
ATGGTCAAAATATGCTGTATCGGGGCTGGATATGTCGGTGGGCCTACAATGGCCGTTATGGCCCTAAAGTGTCCA
GAGATTGAAGTCGTGGTAGTTGATATATCGGAGCCACGAATTAACGCATGGAACTCAGACCGTCTACCAATTTAT
GAGCCAGGGTTAGAGGATGTCGTCAAACAGTGTAGAGGGAAGAATTTGTTCTTCTCTACAGATGTAGAGAAGCAT
GTATTTGAGTCAGATATAGTGTTTGTTTCGGTAAACACTCCTACGAAAACGCAGGGTCTGGGTGCAGGTAAAGCA
GCAGATTTGACATATTGGGAGTCCGCTGCTCGCATGATAGCTGATGTGAGCAAATCGTCGAAAATCGTCGTTGAA
AAGAGTACAGTACCAGTTCGTACAGCCGAGGCTATAGAAAAGATTTTAACGCACAATTCGAAGGGTATCGAATTC
CAGATCTTATCAAATCCAGAATTCTTGGCCGAAGGGACGGCGATTAAAGACTTATATAACCCTGATAGAGTTCTA
ATCGGTGGCAGGGACACCGCTGCGGGACAAAGGCCATTAAGGCGTTGCGTGACGTGTATGCCCACTGGGTTCCT
GTTGAGCAAATAATCTGTACTAATTTATGGAGTGCCGAGCTATCAAAGTTGGCTGCGAACGCATTTCTAGCTCAA
AGGATAAGTTCAGTAAACGCAATGTCAGCGCTTTGTGAGGCAACTGGTGCTGACGTAACCCAAGTCGCTCACGCC
GTTGGAACCGACACTAGAATTGGACCGAAGTTTCTTAACGCATCCGTAGGCTTCGGCGGATCTTGCTTTCAGAAA
GACATCCTGAATCTTATTTACATCTGCGAATGCAATGGTCTTCCAGAAGCAGCCAATTATTGGAAACAGGTAGTC
AAGGTAAATGACTACCAAAAGATTAGGTTTGCTAATCGAGTCGTATCTTCTATGTTCAACACCGTCTCCGGTAAG
AAAATTGCTATTTTGGGATTTGCGTTCAAGAAGGACACCGGCGACACGCGTGAAACTCCTGCCATAGATGTGTGT
AATCGCCTCGTGGCTGATAAAGCAAAGCTGTCGATCTATGATCCGCAAGTATTAGAAGAGCAGATCCGCCGTGAT
CTGTCCATGGCCCGATTCGATTGGGACCATCCAGTCCCACTCCAGCAGATCAAAGCTGAAGGTATCTCCGAACAG
GTTAACGTTGTGTCCGACGCCTACGAGGCTACGAAGGATGCTCATGGTTTATGTGTTTTAACCGAATGGGACGAA
TTCAAGTCACTTGATTTTAAGAAGATCTTTGATAATATGCAGAAACCCGCTTTCGTTTTCGACGGAAGAAACGTG
GTCGACGCTGTGAAATTGAGAGAAATTGGATTCATAGTATATTCCATAGGTAAACCTCTGGATAGTTGGCTCAAG
GATATGCCGGCTGTTGCATAA

SEQ ID NO: 6 is a reencoded nucleic acid sequence of UDP-Glucose
dehydrogenase (HASB) originating from Chlorella virus PBCV-1
ATGAGTAGAATTGCTGTCGTTGGATGCGGTTACGTGGGTACGGCCTGCGCCGTACTTTTGGCGCAGAAGAACGAA
GTTATCGTTTTGGATATCTCGGAGGACCGGGTACAACTGATTAAGAATAAGAAGTCACCTATAGAAGATAAGGAA
ATCGAAGAATTCCTGGAGACGAAAGATTTGAATCTAACAGCGACGACGGATAAGGTGCTCGCCTATGAGAATGCT
GAATTCGTTATAATAGCTACACCGACCGATTACGATGTCGTCACTAGATATTTCAACACTAAGTCTGTTGAAAAT
GTGATAGGCGATGTCATTAAGAACACTCAGACACACCCTACTATAGTGATCAAGAGTACTATTCCAATCGGTTTC

GTGGATAAAGTTAGAGAACAGTTCGATTATCAAAATATTATCTTCTCGCCGGAATTCTTGAGAGAAGGACGAGCA
TTATATGATAATCTTTACCCCTCCCGTATCATCGTCGGTGATGACTCCCCAATTGCCTTAAAATTCGCGAATCTC
TTGGTCGAGGGTAGCAAAACTCCACTAGCTCCCGTATTAACTATGGGTACGCGAGAGGCCGAAGCTGTAAAACTA
TTTTCAAATACATATTTGGCTATGAGAGTAGCATACTTCAATGAGCTAGACACATTTGCAATGTCCCATGGTATG
AACGCAAAAGAGATTATCGACGGAGTCACACTAGAACCAAGGATAGGGCAGGGTTATTCGAATCCTTCATTTGGG
TATGGAGCCTATTGTTTCCCAAAAGATACAAAGCAATTGTTGGCTAATTTTGAAGGGGTTCCACAAGATATTATT
GGCGCAATCGTAGAGTCTAACGAAACTAGAAAGGAGGTTATTGTTTCTGAGGTCGAGAACAGATTTCCGACCACA
GTTGGCGTATATAAGTTGGCTGCTAAAGCTGGTTCGGATAATTTCAGGTCTAGTGCGATAGTCGATATCATGGAA
AGGTTGGCTAATAAGGGCTACCACATCAAAATATTTGAACCTACCGTTGAACAATTTGAGAATTTTGAGGTAGAT
AATAATCTCACAACTTTTGCAACCGAGTCTGATGTAATAATTGCGAACAGAGTTCCAGTCGAACATCGCATTCTG
TTTGGGAAGAAGTTAATAACACGGGATGTCTATGGGGATAA

SEQ ID NO: 7 is a reencoded nucleic acid sequence of UDP-Glucose
dehydrogenase (HASB-A) originating from Chlorella virus PBCV-1
ATGTCACGAATCGCAGTAGTTGGCTGTGGGTACGTGGGAACCGCATGCGCTGTACTGCTGGCGCAAAAGAATGAG
GTAATCGTTCTTGATATTAGTGAGGATAGGGTCCAACTAATCAAGAACAAGAAATCCCCGATCGAAGATAAGGAG
ATTGAAGAATTCTTGGAAACAAAAGACCTAAACTTAACTGCAACAACTGACAAAGTTTTAGCCTACGAAAACGCT
GAGTTTGTGATTATAGCGACACCCACAGATTATGACGTAGTTACCAGATACTTTAACACGAAGTCCGTAGAGAAC
GTCATTGGAGATGTTATAAAGAATACTCAGACTCATCCTACGATAGTAATAAAGTCAACCATTCCCATAGGTTTC
GTAGATAAGGTTAGGGAGCAATTCGATTACCAGAACATTATATTTTCGCCAGAATTTCTGAGAGAGGGTCGCGCC
CTGTATGATAATCTATATCCATCACGGATTATAGTGGGCGATGACTCTCCGATTGCACTTAAGTTTGCTAATCTT
TTAGTTGAGGGCTCCAAAACTCCGCTCGCCCCAGTACTTACGATGGGTACACGTGAGGCTGAAGCTGTCAAGCTG
TTTTCAAACACATACCTTGCTATGCGAGTCGCATACTTTAACGAACTAGATACCTTTGCTATGTCGCACGGTATG
AATGCTAAAGAAATCATAGATGGCGTAACGTTGGAGCCTCGGATAGGTCAAGGATATTCCAATCCATCTTTTGGC
TACGGTGCGTATTGTTTCCCAAAGGACACGAAGCAATTATTAGCTAACTTTGAGGGTGTTCCGCAAGATATAATT
GGGGCGATAGTAGAAAGCAATGAAACACGGAAGGAGGTAATCGTGAGTGAAGTGGAAAACCGATTCCCCACTACG
GTCGGCGTTTACAAATTAGCCGCCAAGGCTGGTTCCGACAATTTCCGATCCAGCGCAATAGTAGATATTATGGAA
AGATTAGCTAATAAGGGATACCACATTAAAATCTTTGAACCTACTGTCGAACAGTTCGAGAACTTCGAGGTTGAT
AATAACTTGACGACTTTCGCAACGGAGAGCGATGTAATTATTGCAAACCGCGTACCTGTGGAACATCGAATTTTG
TTCGGAAAGAAGCTGATTACACGCGATGTATATGGCGATAACTAA

SEQ ID NO: 8 is an amino acid sequence of UDP-Glucose dehydrogenase
(HASB) originating from Arabidopsis thaliana
MVKICCIGAGYVGGPTMAVMALKCPEIEVVVVDISEPRINAWNSDRLPIYEPGLEDVVKQCRGKNLFFSTDVEKH
VFESDIVFVSVNTPTKTQGLGAGKAADLTYWESAARMIADVSKSSKIVVEKSTVPVRTAEAIEKILTHNSKGIEF
QILSNPEFLAEGTAIKDLYNPDRVLIGGRDTAAGQKAIKALRDVYAHWVPVEQIICTNLWSAELSKLAANAFLAQ
RISSVNAMSALCEATGADVTQVAHAVGTDTRIGPKFLNASVGFGGSCFQKDILNLIYICECNGLPEAANYWKQVV
KVNDYQKIRFANRVVSSMFNTVSGKKIAILGFAFKKDTGDTRETPAIDVCNRLVADKAKLSIYDPQVLEEQIRRD

LSMARFDWDHPVPLQQIKAEGISEQVNVVSDAYEATKDAHGLCVLTEWDEFKSLDFKKIFDNMQKPAFVFDGRNV
VDAVKLREIGFIVYSIGKPLDSWLKDMPAVA

SEQ ID NO: 9 is an amino acid sequence of UDP-Glucose dehydrogenase (HASB) originating from Chlorella virus PBCV1
MSRIAVVGCGYVGTACAVLLAQKNEVIVLDISEDRVQLIKNKKSPIEDKEIEEFLETKDLNLTATTDKVLAYENA
EFVIIATPTDYDVVTRYFNTKSVENVIGDVIKNTQTHPTIVIKSTIPIGFVDKVREQFDYQNIIFSPEFLREGRA
LYDNLYPSRIIVGDDSPIALKFANLLVEGSKTPLAPVLTMGTREAEAVKLFSNTYLAMRVAYFNELDTFAMSHGM
NAKEIIDGVTLEPRIGQGYSNPSFGYGAYCFPKDTKQLLANFEGVPQDIIGAIVESNETRKEVIVSEVENRFPTT
VGVYKLAAKAGSDNFRSSAIVDIMERLANKGYHIKIFEPTVEQFENFEVDNNLTTFATESDVIIANRVPVEHRIL
FGKKLITRDVYGDN

SEQ ID NO: 10 is a reencoded nucleic acid sequence of glucuronosyl-
disulfoglucosamine glucuronidase originating from Pedobacter heparinus with a N-terminal
secretion signal
ATGCAATTCTCGACGGTCGCAAGTGTTGCCTTTGTTGCTTTGGCTAACTTCGTTGCCGCACAGTCCTCGTCAATC
ACCAGGAAGGACTTCGATCATATCAACTTGGAATACTCCGGTTTGGAGAAGGTGAACAAGGCGGTAGCAGCAGGC
AATTACGACGACGCGGCAAAAGCCCTACTAGCTTACTACCGTGAAAAGAGCAAAGCAAGAGAGCCTGACTTTAGT
AATGCCGAGAAGCCTGCCGACATACGTCAGCCTATTGACAAGGTAACACGTGAGATGGCGGATAAAGCATTAGTA
CATCAGTTTCAACCTCACAAAGGATACGGATATTTTGATTATGGAAGGATATTAATTGGCAGATGTGGCCAGTT
AAAGACAACGAGGTCAGATGGCAGTTGCATAGGGTAAAATGGTGGCAGGCAATGGCCTTGGTTTACCATGCCACG
GGCGACGAGAAGTACGCCAGGGAGTGGGTGTACCAATACTCGGACTGGGCGAGGAAGAACCCGTTAGGCTTATCG
CAAGACAATGATAAGTTTGTCTGGAGACCTTTGGAAGTCTCAGATAGAGTCCAGTCATTGCCACCTACCTTTTCT
CTTTTCGTCAACAGTCCCGCCTTCACACCAGCATTCCTAATGGAGTTCTTAAACTCGTACCACCAACAGGCAGAC
TACTTGTCAACACACTACGCCGAGCAGGGGAACCATAGGCTGTTCGAAGCTCAGAGGAACTTGTTCGCCGGCGTA
TCCTTCCCCGAATTCAAGGACTCACCCAGGTGGAGACAGACTGGAATATCTGTTCTGAATACAGAGATAAAGAAG
CAGGTGTACGCCGATGGCATGCAATTCGAGTTAAGTCCGATATACCACGTTGCTGCCATAGATATCTTTCTTAAA
GCATACGGAAGCGCCAAGAGAGTTAATTTGGAGAAAGAGTTCCCTCAATCCTATGTGCAAACCGTCGAGAATATG
ATTATGGCGTTAATTTCAATAAGTCTACCGGACTATAATACGCCCATGTTCGGCGACTCTTGGATCACTGATAAG
AATTTCAGAATGGCTCAATTTGCCTCATGGGCGCGTGTCTTCCCCGCTAACCAGGCAATTAAGTACTTTGCAACC
GACGGAAAACAGGGTAAAGCTCCCAACTTCTTGAGTAAGGCATTGTCCAATGCAGGATTCTACACGTTTAGAAGT
GGATGGGATAAGAACGCGACAGTGATGGTACTGAAGGCAAGTCCGCCTGGTGAGTTTCACGCTCAGCCTGATAAC
GGAACTTTTGAACTGTTCATCAAGGGTAGGAACTTCACACCCGACGCCGGTGTATTCGTCTATTCCGGTGACGAG
GCGATTATGAAATTGAGGAACTGGTACAGGCAGACAAGGATTCACTCTACTCTAACTCTTGATAATCAGAATATG
GTCATAACGAAAGCACGTCAGAATAAATGGGAGACAGGCAATAACTTGGATGTCTTAACTTACACTAACCCGTCA
TACCCCAACTTAGATCACCAACGTTCAGTTCTATTCATTAACAAGAAGTATTTCTTAGTCATAGATAGGGCTATC
GGCGAGGCCACGGGCAACCTTGGCGTGCACTGGCAGCTAAAGGAAGACAGTAACCCTGTCTTCGATAAAACCAAG
AATAGAGTCTATACTACCTATAGGGACGGGAACAATCTAATGATACAGAGTCTAAACGCAGACAGGACATCACTG

AACGAGGAAGAAGGGAAGGTATCTTATGTCTATAACAAGGAGCTGAAGCGTCCAGCTTTCGTATTTGAGAAGCCT
AAGAAGAACGCAGGGACCCAAAACTTTGTGTCAATTGTCTATCCATACGACGGTCAGAAGGCGCCGGAAATTTCT
ATTAGAGAAAATAAGGGAAATGACTTCGAAAAGGGAAAATTGAACCTAACACTGACTATAAACGGTAAGCAACAG
CTTGTGTTGGTTCCTTAA

SEQ ID NO: 11 is a reencoded nucleic acid sequence of glucuronosyl-
disulfoglucosamine glucuronidase originating from Pedobacter heparinus with a N-terminal
secretion signal and a C-terminal anchoring signal
ATGCAATTCTCGACGGTCGCAAGTGTTGCCTTTGTTGCTTTGGCTAACTTCGTTGCCGCACAGTCCTCGTCAATC
ACCAGGAAGGACTTCGATCATATCAACTTGGAATACTCCGGTTTGGAGAAGGTGAACAAGGCGGTAGCAGCAGGC
AATTACGACGACGCGGCAAAAGCCCTACTAGCTTACTACCGTGAAAAGAGCAAAGCAAGAGAGCCTGACTTTAGT
AATGCCGAGAAGCCTGCCGACATACGTCAGCCTATTGACAAGGTAACACGTGAGATGGCGGATAAAGCATTAGTA
CATCAGTTTCAACCTCACAAAGGATACGGATATTTTGATTATGGAAGGATATTAATTGGCAGATGTGGCCAGTT
AAAGACAACGAGGTCAGATGGCAGTTGCATAGGGTAAAATGGTGGCAGGCAATGGCCTTGGTTTACCATGCCACG
GGCGACGAGAAGTACGCCAGGGAGTGGGTGTACCAATACTCGGACTGGGCGAGGAAGAACCCGTTAGGCTTATCG
CAAGACAATGATAAGTTTGTCTGGAGACCTTTGGAAGTCTCAGATAGAGTCCAGTCATTGCCACCTACCTTTTCT
CTTTTCGTCAACAGTCCCGCCTTCACACCAGCATTCCTAATGGAGTTCTTAAACTCGTACCACCAACAGGCAGAC
TACTTGTCAACACACTACGCCGAGCAGGGGAACCATAGGCTGTTCGAAGCTCAGAGGAACTTGTTCGCCGGCGTA
TCCTTCCCCGAATTCAAGGACTCACCCAGGTGGAGACAGACTGGAATATCTGTTCTGAATACAGAGATAAAGAAG
CAGGTGTACGCCGATGGCATGCAATTCGAGTTAAGTCCGATATACCACGTTGCTGCCATAGATATCTTTCTTAAA
GCATACGGAAGCGCCAAGAGAGTTAATTTGGAGAAAGAGTTCCCTCAATCCTATGTGCAAACCGTCGAGAATATG
ATTATGGCGTTAATTTCAATAAGTCTACCGGACTATAATACGCCCATGTTCGGCGACTCTTGGATCACTGATAAG
AATTTCAGAATGGCTCAATTTGCCTCATGGGCGCGTGTCTTCCCCGCTAACCAGGCAATTAAGTACTTTGCAACC
GACGGAAAACAGGGTAAAGCTCCCAACTTCTTGAGTAAGGCATTGTCCAATGCAGGATTCTACACGTTTAGAAGT
GGATGGGATAAGAACGCGACAGTGATGGTACTGAAGGCAAGTCCGCCTGGTGAGTTTCACGCTCAGCCTGATAAC
GGAACTTTTGAACTGTTCATCAAGGGTAGGAACTTCACACCCGACGCCGGTGTATTCGTCTATTCCGGTGACGAG
GCGATTATGAAATTGAGGAACTGGTACAGGCAGACAAGGATTCACTCTACTCTAACTCTTGATAATCAGAATATG
GTCATAACGAAAGCACGTCAGAATAAATGGGAGACAGGCAATAACTTGGATGTCTTAACTTACACTAACCCGTCA
TACCCCAACTTAGATCACCAACGTTCAGTTCTATTCATTAACAAGAAGTATTTCTTAGTCATAGATAGGGCTATC
GGCGAGGCCACGGGCAACCTTGGCGTGCACTGGCAGCTAAAGGAAGACAGTAACCCTGTCTTCGATAAAACCAAG
AATAGAGTCTATACTACCTATAGGGACGGGAACAATCTAATGATACAGAGTCTAAACGCAGACAGGACATCACTG
AACGAGGAAGAAGGGAAGGTATCTTATGTCTATAACAAGGAGCTGAAGCGTCCAGCTTTCGTATTTGAGAAGCCT
AAGAAGAACGCAGGGACCCAAAACTTTGTGTCAATTGTCTATCCATACGACGGTCAGAAGGCGCCGGAAATTTCT
ATTAGAGAAAATAAGGGAAATGACTTCGAAAAGGGAAAATTGAACCTAACACTGACTATAAACGGTAAGCAACAG
CTTGTGTTGGTTCCTGGATCCGCCATTTCTCAAATCACTGACGGTCAAATCCAAGCTACTACCACTGCTACCACC
GAAGCTACCACCACTGCTGCCCCATCTTCCACCGTTGAAACTGTTTCTCCATCCAGCACCGAAACTATCTCTCAA
CAAACTGAAAATGGTGCTGCTAAGGCCGCTGTCGGTATGGGTGCCGGTGCTCTAGCTGCTGCTGCTATGTTGTTA
TAA

SEQ ID NO: 12 is a reencoded nucleic acid sequence of glucuronosyl-
disulfoglucosamine glucuronidase originating from Pedobacter heparinus with a N-terminal
secretion sigal
ATGCAGTTTTCAACCGTGGCATCTGTGGCATTCGTGGCATTGGCAAATTTTGTCGCTGCTCAATCTTCCAGTATT
ACGCGTAAAGATTTTGACCACATAAATCTTGAGTATAGTGGCCTAGAGAAAGTCAATAAAGCTGTCGCCGCTGGT
AACTATGATGATGCAGCCAAGGCTTTGTTAGCCTATTATAGAGAGAAATCTAAGGCTCGTGAACCGGATTTCTCT
AACGCTGAAAAGCCAGCGGATATCAGACAACCAATCGATAAAGTCACCAGGAAATGGCAGACAAGGCTTTGGTT
CACCAATTCCAGCCCCATAAGGGCTATGGTTACTTCGACTACGGTAAAGACATAAACTGGCAAATGTGGCCCGTA
AAGGATAATGAAGTAAGGTGGCAACTTCACAGAGTGAAGTGGTGGCAAGCTATGGCTCTGGTGTATCACGCAACC
GGTGATGAAAAGTATGCTAGAGAATGGGTATATCAGTATTCTGATTGGGCTAGGAAGAATCCACTAGGACTTTCT
CAGGATAACGACAAATTCGTTTGGCGTCCACTAGAGGTATCTGACAGGGTACAATCTTTACCCCCGACGTTCAGT
CTGTTTGTTAATTCTCCAGCATTTACCCCGGCTTTCTTGATGGAATTCTTGAATTCTTATCATCAGCAAGCGGAT
TATTTAAGCACCCATTATGCAGAACAAGGAAATCACAGATTATTTGAGGCCCAACGTAATTTATTTGCGGGTGTT
AGTTTTCCAGAGTTTAAAGATTCGCCAAGATGGAGACAAACCGGTATCAGCGTGTTAAACACTGAAATCAAGAAA
CAAGTATATGCTGACGGTATGCAGTTTGAACTATCTCCAATTTATCATGTCGCAGCAATTGACATATTCTTGAAG
GCTTATGGCAGTGCTAAACGTGTCAACTTAGAAAAGGAATTTCCACAGTCATACGTCCAGACTGTAGAAAACATG
ATCATGGCCCTTATCAGCATCTCATTGCCTGATTACAACACTCCAATGTTTGGTGATAGTTGGATAACGGACAAG
AACTTTAGGATGGCACAGTTCGCAAGCTGGGCTAGAGTATTTCCAGCAAATCAAGCTATAAAATATTTCGCGACT
GATGGTAAGCAAGGGAAGGCCCCTAATTTTCTATCTAAAGCTTTAAGCAACGCCGGCTTTTATACTTTCAGGTCC
GGTTGGGACAAGAATGCAACTGTCATGGTGTTAAAAGCGTCTCCACCCGGAGAATTCCATGCGCAACCAGACAAT
GGGACATTCGAGTTGTTTATTAAAGGGAGAAATTTTACCCCAGATGCTGGAGTGTTTGTGTACTCAGGGGATGAA
GCGATAATGAAGTTAAGAAATTGGTATCGTCAAACCAGAATACATTCCACACTTACACTGGACAACCAAAACATG
GTGATTACTAAGGCTAGACAAAACAAGTGGGAAACTGGTAACAATCTGGACGTTTTGACATATACGAACCCTTCC
TATCCAAATCTTGACCATCAGAGGTCGGTCTTATTTATAAATAAGAAATACTTCTTGGTTATCGACAGAGCCATT
GGTGAAGCAACAGGTAATCTGGGTGTTCATTGGCAATTGAAAGAGGATTCTAATCCAGTGTTTGACAAGACGAAG
AACAGGGTATACACAACATACCGTGATGGTAATAACTTAATGATCCAGTCCTTAAATGCTGATCGTACTTCTCTT
AATGAAGAGGAAGGTAAGGTTTCCTACGTGTACAATAAAGAACTAAAGAGACCCGCCTTTGTCTTCGAAAAGCCC
AAGAAGAATGCCGGAACGCAGAATTTCGTATCTATCGTTTACCCTTATGATGGCCAGAAAGCTCCAGAGATAAGC
ATCCGTGAGAACAAAGGTAACGATTTTGAGAAAGGTAAGCTTAATTTGACGCTAACGATTAATGGAAACAGCAA
TTAGTCTTAGTACCATAA

SEQ ID NO: 13 is a reencoded nucleic acid sequence of glucuronosyl-
disulfoglucosamine glucuronidase originating from Pedobacter heparinus with a N-terminal
secretion signal and a C-terminal anchoring signal
ATGCAGTTTTCAACCGTGGCATCTGTGGCATTCGTGGCATTGGCAAATTTTGTCGCTGCTCAATCTTCCAGTATT
ACGCGTAAAGATTTTGACCACATAAATCTTGAGTATAGTGGCCTAGAGAAAGTCAATAAAGCTGTCGCCGCTGGT
AACTATGATGATGCAGCCAAGGCTTTGTTAGCCTATTATAGAGAGAAATCTAAGGCTCGTGAACCGGATTTCTCT
AACGCTGAAAAGCCAGCGGATATCAGACAACCAATCGATAAAGTCACCAGGGAAATGGCAGACAAGGCTTTGGTT

CACCAATTCCAGCCCCATAAGGGCTATGGTTACTTCGACTACGGTAAAGACATAAACTGGCAAATGTGGCCCGTA
AAGGATAATGAAGTAAGGTGGCAACTTCACAGAGTGAAGTGGTGGCAAGCTATGGCTCTGGTGTATCACGCAACC
GGTGATGAAAAGTATGCTAGAGAATGGGTATATCAGTATTCTGATTGGGCTAGGAAGAATCCACTAGGACTTTCT
CAGGATAACGACAAATTCGTTTGGCGTCCACTAGAGGTATCTGACAGGGTACAATCTTTACCCCCGACGTTCAGT
CTGTTTGTTAATTCTCCAGCATTTACCCCGGCTTTCTTGATGGAATTCTTGAATTCTTATCATCAGCAAGCGGAT
TATTTAAGCACCCATTATGCAGAACAAGGAAATCACAGATTATTTGAGGCCCAACGTAATTTATTTGCGGGTGTT
AGTTTTCCAGAGTTTAAAGATTCGCCAAGATGGAGACAAACCGGTATCAGCGTGTTAAACACTGAAATCAAGAAA
CAAGTATATGCTGACGGTATGCAGTTTGAACTATCTCCAATTTATCATGTCGCAGCAATTGACATATTCTTGAAG
GCTTATGGCAGTGCTAAACGTGTCAACTTAGAAAAGGAATTTCCACAGTCATACGTCCAGACTGTAGAAAACATG
ATCATGGCCCTTATCAGCATCTCATTGCCTGATTACAACACTCCAATGTTTGGTGATAGTTGGATAACGGACAAG
AACTTTAGGATGGCACAGTTCGCAAGCTGGGCTAGAGTATTTCCAGCAAATCAAGCTATAAAATATTTCGCGACT
GATGGTAAGCAAGGGAAGGCCCCTAATTTTCTATCTAAAGCTTTAAGCAACGCCGGCTTTTATACTTTCAGGTCC
GGTTGGGACAAGAATGCAACTGTCATGGTGTTAAAAGCGTCTCCACCCGGAGAATTCCATGCGCAACCAGACAAT
GGGACATTCGAGTTGTTTATTAAAGGGAGAAATTTTACCCCAGATGCTGGAGTGTTTGTGTACTCAGGGGATGAA
GCGATAATGAAGTTAAGAAATTGGTATCGTCAAACCAGAATACATTCCACACTTACACTGGACAACCAAAACATG
GTGATTACTAAGGCTAGACAAAACAAGTGGGAAACTGGTAACAATCTGGACGTTTTGACATATACGAACCCTTCC
TATCCAAATCTTGACCATCAGAGGTCGGTCTTATTTATAAATAAGAAATACTTCTTGGTTATCGACAGAGCCATT
GGTGAAGCAACAGGTAATCTGGGTGTTCATTGGCAATTGAAAGAGGATTCTAATCCAGTGTTTGACAAGACGAAG
AACAGGGTATACACAACATACCGTGATGGTAATAACTTAATGATCCAGTCCTTAAATGCTGATCGTACTTCTCTT
AATGAAGAGGAAGGTAAGGTTTCCTACGTGTACAATAAAGAACTAAAGAGACCCGCCTTTGTCTTCGAAAAGCCC
AAGAAGAATGCCGGAACGCAGAATTTCGTATCTATCGTTTACCCTTATGATGGCCAGAAAGCTCCAGAGATAAGC
ATCCGTGAGAACAAAGGTAACGATTTTGAGAAAGGTAAGCTTAATTTGACGCTAACGATTAATGGGAAACAGCAA
TTAGTCTTAGTACCATAA

SEQ ID NO: 14 is an amino acid sequence of glucuronosyl-disulfoglucosamine
glucuronidase originating from Pedobacter heparinus with a N-terminal secretion signal
MQFSTVASVAFVALANFVAAQSSSITRKDFDHINLEYSGLEKVNKAVAAGNYDDAAKALLAYYREKSKAREPDFS
NAEKPADIRQPIDKVTREMADKALVHQFQPHKGYGYFDYGKDINWQMWPVKDNEVRWQLHRVKWWQAMALVYHAT
GDEKYAREWVYQYSDWARKNPLGLSQDNDKFVWRPLEVSDRVQSLPPTFSLFVNSPAFTPAFLMEFLNSYHQQAD
YLSTHYAEQGNHRLFEAQRNLFAGVSFPEFKDSPRWRQTGISVLNTEIKKQVYADGMQFELSPIYHVAAIDIFLK
AYGSAKRVNLEKEFPQSYVQTVENMIMALISISLPDYNTPMFGDSWITDKNFRMAQFASWARVFPANQAIKYFAT
DGKQGKAPNFLSKALSNAGFYTFRSGWDKNATVMVLKASPPGEFHAQPDNGTFELFIKGRNFTPDAGVFVYSGDE
AIMKLRNWYRQTRIHSTLTLDNQNMVITKARQNKWETGNNLDVLTYTNPSYPNLDHQRSVLFINKKYFLVIDRAI
GEATGNLGVHWQLKEDSNPVFDKTKNRVYTTYRDGNNLMIQSLNADRTSLNEEEGKVSYVYNKELKRPAFVFEKP
KKNAGTQNFVSIVYPYDGQKAPEISIRENKGNDFEKGKLNLTLTINGKQQLVLVP

SEQ ID NO: 15 is an amino acid sequence of glucuronosyl-disulfoglucosamine glucuronidase originating from Pedobacter heparinus with a N-terminal secretion signal and a C-terminal anchoring signal
MQFSTVASVAFVALANFVAAQSSSITRKDFDHINLEYSGLEKVNKAVAAGNYDDAAKALLAYYREKSKAREPDFS
NAEKPADIRQPIDKVTREMADKALVHQFQPHKGYGYFDYGKDINWQMWPVKDNEVRWQLHRVKWWQAMALVYHAT
GDEKYAREWVYQYSDWARKNPLGLSQDNDKFVWRPLEVSDRVQSLPPTFSLFVNSPAFTPAFLMEFLNSYHQQAD
YLSTHYAEQGNHRLFEAQRNLFAGVSFPEFKDSPRWRQTGISVLNTEIKKQVYADGMQFELSPIYHVAAIDIFLK
AYGSAKRVNLEKEFPQSYVQTVENMIMALISISLPDYNTPMFGDSWITDKNFRMAQFASWARVFPANQAIKYFAT
DGKQGKAPNFLSKALSNAGFYTFRSGWDKNATVMVLKASPPGEFHAQPDNGTFELFIKGRNFTPDAGVFVYSGDE
AIMKLRNWYRQTRIHSTLTLDNQNMVITKARQNKWETGNNLDVLTYTNPSYPNLDHQRSVLFINKKYFLVIDRAI
GEATGNLGVHWQLKEDSNPVFDKTKNRVYTTYRDGNNLMIQSLNADRTSLNEEEGKVSYVYNKELKRPAFVFEKP
KKNAGTQNFVSIVYPYDGQKAPEISIRENKGNDFEKGKLNLTLTINGKQQLVLVPGSAISQITDGQIQATTTATT
EATTTAAPSSTVETVSPSSTETISQQTENGAAKAAVGMGAGALAAAAMLL

SEQ ID NO: 16 is a nucleic acid sequence of glutamine-fructose-6-phosphate
amidotransferase (GFA1) originating from Saccharomyces cerevisiae
ATGTGTGGTATCTTTGGTTACTGCAATTATCTAGTGGAAAGATCCAGAGGAGAAATTATCGACACCTTAGTGGAT
GGTTTACAAAGATTAGAATATAGAGGCTATGATTCCACCGGTATTGCTATCGATGGTGACGAAGCTGATTCTACT
TTCATCTATAAGCAAATCGGTAAAGTGAGTGCTTTGAAAGAGGAGATTACTAAGCAAAATCCGAACAGAGACGTT
ACTTTTGTCTCTCATTGTGGTATTGCGCATACTAGATGGGCTACTCACGGTCGACCAGAACAAGTTAACTGTCAC
CCTCAAAGATCTGACCCAGAAGACCAATTTGTGGTCGTTCATAATGGTATCATCACAAATTTTAGAGAACTGAAG
ACTCTTTTAATTAACAAAGGTTATAAATTCGAAAGTGATACCGATACCGAGTGTATTGCTAAACTATATTTGCAT
TTATACAATACAAATTTACAAAATGGGCATGACTTAGATTTCCACGAATTAACCAAGCTAGTTCTTTTAGAACTA
GAAGGTTCATACGGGTTATTATGTAAATCTTGTCACTATCCTAATGAGGTTATCGCCACTAGAAAAGGGTCCCCT
TTACTGATTGGTGTCAAATCTGAAAAAAAACTAAAAGTCGACTTCGTGGATGTGGAATTTCCCGAAGAAAACGCT
GGTCAACCGGAAATTCCATTGAAATCTAACAACAAATCATTTGGCTTGGGCCCAAAGAAAGCTCGTGAATTTGAA
GCTGGTTCCCAAAATGCCAATTTACTACCAATTGCCGCCAATGAATTTAACTTGAGACATTCTCAATCCAGGGCT
TTCCTATCAGAAGATGGATCTCCAACACCGGTGGAATTTTTTGTTTCTTCGGATGCGGCATCTGTTGTTAAACAT
ACCAAGAAGGTGCTATTTTTAGAAGATGACGATTTGGCTCATATTTACGATGGTGAGTTACATATTCATAGATCT
AGAAGAGAAGTAGGCGCATCAATGACAAGGTCCATTCAAACTTTAGAGATGGAGTTAGCTCAGATCATGAAGGGC
CCTTACGACCATTTTATGCAAAAGGAAATCTATGAGCAACCAGAATCTACTTTCAATACTATGAGAGGTAGAATC
GACTATGAAAATAATAAAGTGATATTGGGTGGTTTAAAGGCATGGTTACCAGTTGTCAGAAGAGCACGGAGACTG
ATCATGATCGCATGCGGTACTTCTTATCATTCATGTTTGGCTACTCGTGCTATCTTCGAAGAATTATCAGATATC
CCAGTTAGTGTGGAATTAGCGTCTGACTTTCTGGACAGAAAATGCCCTGTCTTCAGAGACGATGTATGCGTGTTT
GTTTCACAAAGTGGTGAAACTGCGGATACCATGCTGGCTCTAAATTATTGTTTAGAAAGAGGAGCCTTAACTGTC
GGAATTGTTAACAGTGTTGGTTCTTCTATCTCTCGTGTCACCCACTGTGGTGTTCATATTAACGCTGGTCCTGAA
ATTGGTGTTGCCTCTACAAAAGCTTATACTTCCCAGTATATTGCCTTAGTGATGTTTGCTCTATCGCTGTCAGAT
GACCGTGTATCGAAAATAGACAGAAGAATTGAAATCATTCAAGGCTTGAAGTTAATCCCGGGCCAAATTAAGCAG

GTATTAAAGCTGGAACCAAGAATAAAAAAGCTCTGTGCGACTGAATTAAAGGATCAAAAATCTCTATTGTTATTG
GGTAGAGGTTACCAATTTGCTGCTGCTCTGGAAGGTGCTTTGAAGATCAAAGAAATTTCTTATATGCATTCTGAA
GGTGTTTTGGCAGGTGAGTTGAAGCACGGTGTCTTGGCCTTGGTGGACGAAAACTTGCCAATCATTGCTTTTGGT
ACCAGAGACTCTCTATTCCCTAAAGTAGTTTCCTCTATTGAGCAAGTTACTGTAAGAAAGGGCCATCCAATTATT
ATTTGTAACGAAAATGATGAAGTGTGGGCGCAAAAATCTAAATCAATCGACCTGCAAACCTTAGAAGTTCCACAA
ACTGTTGATTGTTTACAAGGTCTAATTAATATTATTCCATTACAACTAATGTCATATTGGTTGGCTGTTAATAAA
GGGATTGATGTTGATTTTCCAAGAAACTTGGCTAAATCTGTTACCGTCGAATAA

SEQ ID NO: 17 is a reencoded nucleic acid sequence of glutamine-fructose-6-
phosphate amidotransferase (GFA1) originating from Chlorella virus 1 (PBCV-1)
ATGTGCGGGATCTTCGGTGCTGTGTCGAACAATAATAGCATAGAAGTTTCCATCAAGGGTATACAGAAGCTAGAG
TACCGCGGGTACGATTCGTGTGGAATAGCCTATACAGACGGAGGCGCCATTGAACGGATCAGGTCAATAGATGGG
ATCGACGACTTAAGGAAGAAAACAATAACAGAGTCTTCCCCGGTAGCTATCGCTCATTCAAGGTGGAGTACCACT
GGGATTCCAAGTGTTGTGAACGCCCACCCGCACATCTCTCGGGGCACGTCTGGATGCGAGTCGCGCATTGCAGTC
GTACACAATGGCATTATTGAAAATTACCAGCAGATCCGCAAGTACTTGATAAATCTTGGGTATACTTTCGATTCA
CAGACCGACACGGAAGTCATTGCTCATCTAATAGACTCGCAGTATAACGGGAACATCCTGCACACCGTCCAAATG
GCAGTTAAGCACCTCAAAGGAAGCTACGCCATAGCAGTCATGTGCCATAAGGAGTCCGGGAAGATCGTGGTGGCA
AAACAGAAGAGTCCGTTGGTATTGGGTATAGGAAGCGACGGAGCTTATTATATCGCATCGGACGTATTGGCTCTT
CCCACTAACAAAGTGGTCTACATAAGTGACGGATTCTCTGCTGAGTTAAGTCCTGGATCGATGACTATCTATGAT
CTGGATGGCAATAAGGTAGAATACGAGGTGGAAGACGTTGAAATGGAGCAGACGTCGATGTCTTTAGATAACTTT
GATCACTACATGATCAAAGAGATCAATGAACAGCCGATATCAATTCTCAATACTATTAAGAACAAAGGATTCTAT
GCAGAGATATTTGGTGATCTAGCACATGAAATATTTCAGAAGATCGACAATATATTGATTCTAGCCTGCGGAACT
TCGTATCATGCAGGTCTCGTCGGGAAGCAATGGATAGAGACCATCTCACGCATCCCGGTGGATGTTCATATTGCA
AGCGAGTATGAGCCGACAATTCCACGAGCCAATACACTTGTGATAACGATTAGTCAAAGCGGCGAGACTGCGGAT
ACAATCGCAGCCCTTCAACGAGCGCAAAACGCAGGAATGATATACACTTTGTGTATCTGCAACAGCCCCAAGTCA
ACTCTAGTGCGCGAGTCGATAATGAAATACATAACCAAATGCGGTTCAGAAGTTTCTGTTGCCAGTACTAAAGCA
TTTACGTCGCAACTTGTGGTGTTGTATATGCTTGCGAACGTTCTTGCGAACAAAACTGATGATCTGCTAGGGGAT
CTTCCTCAAGCTATCGAGAGAGTAATCTGTCTTACTAATGACGAAATGAAGCGTTGGGCGGATGAAATTTGTACT
GCCAAAAGTGCCATCTTCTTAGGGAGAGGACTGAACGCACCTGTAGCATTTGAGGGCGCGCTAAAGTTGAAGGAA
ATCTCATATATCCATGCCGAGGGTTTCCTCGGTGGAGAGTTGAAGCATGGGCCGCTGGCTTTGTTGGACGATAAG
ATCCCTGTAATCGTGACTGTTGCTGACCACGCTTATTTGGATCACATTAAGGCTAACATCGACGAGGTATTGGCA
CGAAACGTTACCGTATACGCGATCGTCGATCAGTATGTTAATATTGAACCACAGGAGCGACTGCATGTAGTAAAA
GTGCCGTTTGTGAGTAAAGAGTTTTCTCCCATCATCCACACAATTCCGATGCAATTGTTATCGTATTATGTCGCG
ATCAAGCTGGGCAAGAACGTTGACAAGCCACGTAACCTGGCGAAAAGTGTTACAACATTCtaa

SEQ ID NO: 18 is a reencoded nucleic acid sequence of glutamine-fructose-6-
phosphate amidotransferase (GFA1) originating from Chlorella virus 1 (PBCV-1)
ATGTGTGGCATCTTTGGAGCAGTGTCAAACAACTCTATCGAGGTGTCAATCAAGGGTATTCAGAAGCTAGAA
TATCGTGGGTATGATTCGTGCGGTATTGCGTATACAGATGGGGGTGCGATTGAGCGTATACGTTCTATTGACGGC
ATTGACGATCTGCGTAAGAAAACAATCACAGAATCATCACCAGTGGCCATTGCTCACTCGCGGTGGAGCACCACT
GGAATTCCATCAGTGGTGAACGCACATCCTCATATTTCTCGCGGAACCAGTGGGTGTGAGTCTCGTATCGCGGTA
GTCCACAACGGTATCATTGAAAACTATCAGCAGATCCGAAAATATCTCATCAATCTTGGTTATACGTTTGATAGT
CAAACGGACACAGAGGTCATTGCGCATTTGATTGATTCTCAGTACAATGGGAATATCTTGCACACCGTCCAAATG
GCTGTCAAGCACCTGAAGGGCTCTTATGCCATTGCAGTTATGTGTCATAAAGAGTCTGGTAAAATAGTCGTGGCG
AAACAGAAGTCACCCCTCGTACTTGGAATCGGCTCAGATGGTGCTTACTACATCGCTTCGGACGTGCTGGCGCTG
CCGACAAATAAAGTTGTTTATATTTCAGACGGTTTCTCCGCAGAACTATCTCCAGGGAGTATGACCATTTACGAT
CTTGATGGAAATAAAGTAGAATATGAAGTAGAGGACGTTGAAATGGAACAAACTAGTATGTCTCTCGATAACTTT
GATCATTACATGATTAAGGAAATTAATGAGCAACCAATCAGTATTCTAAACACTATAAAAAATAAAGGGTTCTAT
GCAGAAATATTCGGTGATTTGGCTCATGAAATCTTCCAAAAAATAGACAACATCCTGATACTGGCTTGTGGTACA
AGTTATCACGCCGGTCTTGTAGGAAAACAGTGGATAGAGACCATCTCtAGAATCCCCGTGGATGTTCACATCGCG
AGTGAATACGAACCTACTATTCCGAGAGCGAACACATTGGTAATCACTATTTCACAGTCGGGTGAAACTGCGGAC
ACGATAGCGGCTTTGCAACGGGCCCAAAACGCCGGGATGATTTATACATTGTGTATTTGCAATTCACCAAAGAGC
ACTCTTGTTCGTGAGAGCATTATGAAGTACATCACGAAATGTGGTTCTGAGGTGTCAGTGGCATCAACGAAGGCG
TTTACTTCTCAGCTCGTAGTACTGTACATGCTGGCAAACGTATTGGCAAATAAAACCGATGATTTGCTGGGAGAC
CTCCCACAGGCAATAGAACGGGTAATTTGTTTGACAAATGACGAAATGAAACGATGGGCCGACGAAATTTGCACT
GCGAAATCCGCGATCTTCCTGGGAAGAGGACTAAACGCACCAGTTGCCTTTGAGGGAGCGTTGAAGCTCAAAGAA
ATCTCTTACATTCATGCAGAGGGCTTCCTGGGAGGTGAGTTGAAACACGGCCCCCTCGCACTCCTTGATGACAAG
ATTCCTGTTATCGTAACCGTAGCAGATCATGCTTATTTGGACCATATCAAAGCAAATATCGACGAAGTGCTTGCG
AGGAACGTTACGGTATACGCCATAGTAGACCAGTATGTGAACATCGAGCCCCAGGAACGCCTTCACGTCGTCAAG
GTTCCGTTTGTATCCAAAGAATTTTCTCCGATAATTCACACTATCCCGATGCAACTGCTTTCGTATTACGTGGCA
ATTAAGCTTGGAAAGAACGTTGACAAACCAAGGAATCTTGCAAAATCCGTGACTACCTTTTAA

SEQ ID NO: 19 is an amino acid sequence of glutamine-fructose-6-phosphate amidotransferase (GFA1) originating from Saccharomyces cerevisiae
MCGIFGYCNYLVERSRGEIIDTLVDGLQRLEYRGYDSTGIAIDGDEADSTFIYKQIGKVSALKEEITKQNPNRDV
TFVSHCGIAHTRWATHGRPEQVNCHPQRSDPEDQFVVVHNGIITNFRELKTLLINKGYKFESDTDTECIAKLYLH
LYNTNLQNGHDLDFHELTKLVLLELEGSYGLLCKSCHYPNEVIATRKGSPLLIGVKSEKKLKVDFVDVEFPEENA
GQPEIPLKSNNKSFGLGPKKAREFEAGSQNANLLPIAANEFNLRHSQSRAFLSEDGSPTPVEFFVSSDAASVVKH
TKKVLFLEDDDLAHIYDGELHIHRSRREVGASMTRSIQTLEMELAQIMKGPYDHFMQKEIYEQPESTFNTMRGRI
DYENNKVILGGLKAWLPVVRRARRLIMIACGTSYHSCLATRAIFEELSDIPVSVELASDFLDRKCPVFRDDVCVF
VSQSGETADTMLALNYCLERGALTVGIVNSVGSSISRVTHCGVHINAGPEIGVASTKAYTSQYIALVMFALSLSD
DRVSKIDRRIEIIQGLKLIPGQIKQVLKLEPRIKKLCATELKDQKSLLLLGRGYQFAAALEGALKIKEISYMHSE
GVLAGELKHGVLALVDENLPIIAFGTRDSLFPKVVSSIEQVTARKGHPIIICNENDEVWAQKSKSIDLQTLEVPQ
TVDCLQGLINIIPLQLMSYWLAVNKGIDVDFPRNLAKSVTVE

SEQ ID NO: 20 is an amino acid sequence of glutamine-fructose-6-phosphate
amidotransferase (GFA1) originating from Chlorella virus 1 (PBCV-1)
MCGIFGAVSNNNSIEVSIKGIQKLEYRGYDSCGIAYTDGGAIERIRSIDGIDDLRKKTITESSPVAIAHSRWSTT
GIPSVVNAHPHISRGTSGCESRIAVVHNGIIENYQQIRKYLINLGYTFDSQTDTEVIAHLIDSQYNGNILHTVQM
AVKHLKGSYAIAVMCHKESGKIVVAKQKSPLVLGIGSDGAYYIASDVLALPTNKVVYISDGFSAELSPGSMTIYD
LDGNKVEYEVEDVEMEQTSMSLDNFDHYMIKEINEQPISILNTIKNKGFYAEIFGDLAHEIFQKIDNILILACGT
SYHAGLVGKQWIETISRIPVDVHIASEYEPTIPRANTLVITISQSGETADTIAALQRAQNAGMIYTLCICNSPKS
TLVRESIMKYITKCGSEVSVASTKAFTSQLVVLYMLANVLANKTDDLLGDLPQAIERVICLTNDEMKRWADEICT
AKSAIFLGRGLNAPVAFEGALKLKEISYIHAEGFLGGELKHGPLALLDDKIPVIVTVADHAYLDHIKANIDEVLA
RNVTVYAIVDQYVNIEPQERLHVVKVPFVSKEFSPIIHTIPMQLLSYYVAIKLGKNVDKPRNLAKSVTTF

SEQ ID NO: 21 is a nucleic acid sequence of UDP-N-acetylglucosamine
pyrophosphorylase (QRI1) originating from Saccharomyces cerevisiae
ATGACTGACACAAAACAGCTATTCATTGAAGCCGGACAAAGTCAACTTTTCCACAATTGGGAAAGCTTGTCTCGC
AAAGACCAAGAAGAATTGCTTTCAAACCTGGAGCAAATATCTTCCAAGAGGTCCCCTGCAAAACTACTGGAAGAC
TGTCAAAATGCTATTAAATTCTCACTAGCTAACTCTTCTAAGGATACTGGCGTCGAAATTTCACCATTGCCCCCT
ACTTCGTACGAGTCGCTTATTGGCAACAGTAAGAAAGAAAATGAATACTGGCGTTTAGGCCTTGAAGCTATTGGC
AAGGGTGAAGTCGCAGTGATTTTAATGGCTGGCGGACAAGGTACGCGGTTAGGATCCTCTCAACCAAAGGGCTGT
TACGACATTGGATTGCCTTCTAAGAAATCTCTTTTTCAAATTCAAGCTGAAAAGTTGATCAGGTTGCAAGATATG
GTAAAGGACAAAAAGGTAGAAATTCCTTGGTATATTATGACATCAGGCCCCACTAGAGCGGCTACTGAGGCATAC
TTTCAAGAACACAATTATTTTGGCTTGAATAAAGAACAAATTACGTTCTTCAACCAGGGAACCCTGCCTGCCTTT
GATTTAACCGGGAAGCATTTCCTAATGAAAGACCCAGTAAACCTATCTCAATCACCAGATGGAAATGGTGGACTC
TACCGTGCCATCAAGGAAAACAAGTTGAACGAAGACTTTGATAGGAGAGGAATCAAGCATGTTTACATGTACTGT
GTCGATAATGTCCTATCTAAAATCGCAGACCCTGTATTTATTGGTTTTGCCATCAAGCATGGCTTCGAACTGGCC
ACCAAAGCCGTTAGAAAGAGAGATGCGCATGAATCAGTTGGGTTAATTGCTACTAAAAACGAGAAACCATGTGTC
ATAGAATATTCTGAAATTTCCAATGAATTGGCTGAAGCAAAGGATAAAGATGGCTTATTAAAACTACGCGCAGGC
AACATTGTAAATCATTATTACCTAGTGGATTTACTAAAACGTGATTTGGATCAGTGGTGTGAGAATATGCCATAT
CACATTGCGAAGAAGAAAATTCCAGCTTATGATAGTGTTACCGGCAAGTACACTAAGCCTACCGAACCAAACGGT
ATAAAATTAGAGCAATTCATATTTGATGTCTTTGACACTGTACCACTGAACAAGTTTGGGTGCTTAGAAGTAGAT
AGATGCAAAGAATTTTCACCTTTAAAAAACGGTCCTGGTTCTAAGAACGATAATCCTGAGACCAGCAGACTAGCA
TATTTGAAACTAGGAACCTCGTGGTTGGAAGATGCAGGCGCTATTGTAAAAGATGGGGTACTAGTCGAAGTTTCC
AGCAAATTGAGTTATGCAGGTGAAAATCTATCCCAGTTCAAAGGTAAAGTCTTTGACAGAAGTGGTATAGTATTA
GAAAAATAA

SEQ ID NO: 22 is an amino acid sequence of UDP-N-acetylglucosamine
pyrophosphorylase (QRI1) originating from Saccharomyces cerevisiae
MTDTKQLFIEAGQSQLFHNWESLSRKDQEELLSNLEQISSKRSPAKLLEDCQNAIKFSLANSSKDTGVEISPLPP
TSYESLIGNSKKENEYWRLGLEAIGKGEVAVILMAGGQGTRLGSSQPKGCYDIGLPSKKSLFQIQAEKLIRLQDM

VKDKKVEIPWYIMTSGPTRAATEAYFQEHNYFGLNKEQITFFNQGTLPAFDLTGKHFLMKDPVNLSQSPDGNGGL
YRAIKENKLNEDFDRRGIKHVYMYCVDNVLSKIADPVFIGFAIKHGFELATKAVRKRDAHESVGLIATKNEKPCV
IEYSEISNELAEAKDKDGLLKLRAGNIVNHYYLVDLLKRDLDQWCENMPYHIAKKKIPAYDSVTGKYTKPTEPNG
IKLEQFIFDVFDTVPLNKFGCLEVDRCKEFSPLKNGPGSKNDNPETSRLAYLKLGTSWLEDAGAIVKDGVLVEVS
SKLSYAGENLSQFKGKVFDRSGIVLEK

SEQ ID NO: 23 is a nucleic acid sequence of Phosphoglucomutase-1 (PGM1)
originating from Saccharomyces cerevisiae
ATGTCACTTCTAATAGATTCTGTACCAACAGTTGCTTATAAGGACCAAAAACCGGGTACTTCAGGTTTACGTAAG
AAGACCAAGGTTTTCATGGATGAGCCTCATTATACTGAGAACTTCATTCAAGCAACAATGCAATCTATCCCTAAT
GGCTCAGAGGGAACCACTTTAGTTGTTGGAGGAGATGGTCGTTTCTACAACGATGTTATCATGAACAAGATTGCC
GCAGTAGGTGCTGCAAACGGTGTCAGAAAGTTAGTCATTGGTCAAGGCGGTTTACTTTCAACACCAGCTGCTTCT
CATATAATTAGAACATACGAGGAAAAGTGTACCGGTGGTGGTATCATATTAACTGCCTCACACAACCCAGGCGGT
CCAGAGAATGATTTAGGTATCAAGTATAATTTACCTAATGGTGGGCCAGCTCCAGAGAGTGTCACTAACGCTATC
TGGGAAGCGTCTAAAAAATTAACTCACTATAAAATTATAAAGAACTTCCCCAAGTTGAATTTGAACAAGCTTGGT
AAAAACCAAAAATATGGCCCATTGTTAGTGGACATAATTGATCCTGCCAAAGCATACGTTCAATTTCTGAAGGAA
ATTTTTGATTTTGACTTAATTAAAAGCTTCTTAGCGAAACAGCGCAAAGACAAAGGGTGGAAGTTGTTGTTTGAC
TCCTTAAATGGTATTACAGGACCATATGGTAAGGCTATATTTGTTGATGAATTTGGTTTACCGGCAGAGGAAGTT
CTTCAAAATTGGCACCCTTTACCTGATTTCGGCGGTTTACATCCCGATCCGAATCTAACCTATGCACGAACTCTT
GTTGACAGGGTTGACCGCGAAAAAATTGCCTTTGGAGCAGCCTCCGATGGTGATGGTGATAGGAATATGATTTAC
GGTTATGGCCCTGCTTTCGTTTCGCCAGGTGATTCTGTTGCCATTATTGCCGAATATGCACCCGAAATTCCATAC
TTCGCCAAACAAGGTATTTATGGCTTGGCACGTTCATTTCCTACATCCTCAGCCATTGATCGTGTTGCAGCAAAA
AAGGGATTAAGATGTTACGAAGTTCCAACCGGCTGGAAATTCTTCTGTGCCTTATTTGATGCTAAAAAGCTATCA
ATCTGTGGTGAAGAATCCTTCGGTACAGGTTCCAATCATATCAGAGAAAAGGACGGTCTATGGGCCATTATTGCT
TGGTTAAATATCTTGGCTATCTACCATAGGCGTAACCCTGAAAAGGAAGCTTCGATCAAAACTATTCAGGACGAA
TTTTGGAACGAGTATGGCCGTACTTTCTTCACAAGATACGATTACGAACATATCGAATGCGAGCAGGCCGAAAAA
GTTGTAGCTCTTTTGAGTGAATTTGTATCAAGGCCAAACGTTTGTGGCTCCCACTTCCCAGCTGATGAGTCTTTA
ACCGTTATCGATTGTGGTGATTTTTCGTATAGAGATCTAGATGGCTCCATCTCTGAAAATCAAGGCCTTTTCGTA
AAGTTTTCGAATGGGACTAAATTTGTTTTGAGGTTATCCGGCACAGGCAGTTCTGGTGCAACAATAAGATTATAC
GTAGAAAAGTATACTGATAAAAAGGAGAACTATGGCCAAACAGCTGACGTCTTCTTGAAACCCGTCATCAACTCC
ATTGTAAAATTCTTAGATTTAAAGAAATTTTAGGAACAGACGAACCAACAGTCCGCACATAG

SEQ ID NO: 24 is an amino acid sequence Phosphoglucomutase-1 (PGM1)
originating from Saccharomyces cerevisiae
MSLLIDSVPTVAYKDQKPGTSGLRKKTKVFMDEPHYTENFIQATMQSIPNGSEGTTLVVGGDGRFYNDVIMNKIA
AVGAANGVRKLVIGQGGLLSTPAASHIIRTYEEKCTGGGIILTASHNPGGPENDLGIKYNLPNGGPAPESVTNAI
WEASKKLTHYKIIKNFPKLNLNKLGKNQKYGPLLVDIIDPAKAYVQFLKEIFDFDLIKSFLAKQRKDKGWKLLFD
SLNGITGPYGKAIFVDEFGLPAEEVLQNWHPLPDFGGLHPDPNLTYARTLVDRVDREKIAFGAASDGDGDRNMIY

GYGPAFVSPGDSVAIIAEYAPEIPYFAKQGIYGLARSFPTSSAIDRVAAKKGLRCYEVPTGWKFFCALFDAKKLS
ICGEESFGTGSNHIREKDGLWAIIAWLNILAIYHRRNPEKEASIKTIQDEFWNEYGRTFFTRYDYEHIECEQAEK
VVALLSEFVSRPNVCGSHFPADESLTVIDCGDFSYRDLDGSISENQGLFVKFSNGTKFVLRLSGTGSSGATIRLY
VEKYTDKKENYGQTADVFLKPVINSIVKFLRFKEILGTDEPTVRT

SEQ ID NO: 25 is a nucleic acid sequence of UTP-glucose-1-phosphate
uridylyltransferase (UGP1) originating from Saccharomyces cerevisiae
ATGTCCACTAAGAAGCACACCAAAACACATTCCACTTATGCATTCGAGAGCAACACAAACAGCGTTGCTGCCTCA
CAAATGAGAAACGCCTTAAACAAGTTGGCGGACTCTAGTAAACTTGACGATGCTGCTCGCGCTAAGTTTGAGAAC
GAACTGGATTCGTTTTTCACGCTTTTCAGGAGATATTTGGTAGAGAAGTCTTCTAGAACCACCTTGGAATGGGAC
AAGATCAAGTCTCCCAACCCGGATGAAGTGGTTAAGTATGAAATTATTTCTCAGCAGCCCGAGAATGTCTCAAAC
CTTTCCAAATTGGCTGTTTTGAAGTTGAACGGTGGGCTGGGTACCTCCATGGGCTGCGTTGGCCCTAAATCTGTT
ATTGAAGTGAGAGAGGGAAACACCTTTTTGGATTTGTCTGTTCGTCAAATTGAATACTTGAACAGACAGTACGAT
AGCGACGTGCCATTGTTATTGATGAATTCTTTCAACACTGACAAGGATACGGAACACTTGATTAAGAAGTATTCC
GCTAACAGAATCAGAATCAGATCTTTCAATCAATCCAGGTTCCCAAGAGTCTACAAGGATTCTTTATTGCCTGTC
CCCACCGAATACGATTCTCCACTGGATGCTTGGTATCCACCAGGTCACGGTGATTTGTTTGAATCTTTACACGTA
TCTGGTGAACTGGATGCCTTAATTGCCCAAGGAAGAGAAATATTATTTGTTTCTAACGGTGACAACTTGGGTGCT
ACCGTCGACTTAAAAATTTTAAACCACATGATCGAGACTGGTGCCGAATATATAATGGAATTGACTGATAAGACC
AGAGCCGATGTTAAAGGTGGTACTTTGATTTCTTACGATGGTCAAGTCCGTTTATTGGAAGTCGCCCAAGTTCCA
AAAGAACACATTGACGAATTCAAAAATATCAGAAAGTTTACCAACTTCAACACGAATAACTTATGGATCAATCTG
AAAGCAGTAAAGAGGTTGATCGAATCGAGCAATTTGGAGATGGAAATCATTCCAAACCAAAAAACTATAACAAGA
GACGGTCATGAAATTAATGTCTTACAATTAGAAACCGCTTGTGGTGCTGCTATCAGGCATTTTGATGGTGCTCAC
GGTGTTGTCGTTCCAAGATCAAGATTCTTGCCTGTCAAGACCTGTTCCGATTTGTTGCTGGTTAAATCAGATCTA
TTCCGTCTGGAACACGGTTCTTTGAAGTTAGACCCATCCCGTTTTGGTCCAAACCCATTAATCAAGTTGGGCTCG
CATTTCAAAAAGGTTTCTGGTTTTAACGCAAGAATCCCTCACATCCCAAAAATCGTCGAGCTAGATCATTTGACC
ATCACTGGTAACGTCTTTTTAGGTAAAGATGTCACTTTGAGGGGTACTGTCATCATCGTTTGCTCCGACGGTCAT
AAAATCGATATTCCAAACGGCTCCATATTGGAAAATGTTGTCGTTACTGGTAATTTGCAAATCTTGGAACATTGA

SEQ ID NO: 26 is an amino acid sequence of UTP--glucose-1-phosphate uridylyltransferase (UGP1) originating from Saccharomyces cerevisiae
MSTKKHTKTHSTYAFESNTNSVAASQMRNALNKLADSSKLDDAARAKFENELDSFFTLFRRYLVEKSSRTTLEWD
KIKSPNPDEVVKYEIISQQPENVSNLSKLAVLKLNGGLGTSMGCVGPKSVIEVREGNTFLDLSVRQIEYLNRQYD
SDVPLLLMNSFNTDKDTEHLIKKYSANRIRIRSFNQSRFPRVYKDSLLPVPTEYDSPLDAWYPPGHGDLFESLHV
SGELDALIAQGREILFVSNGDNLGATVDLKILNHMIETGAEYIMELTDKTRADVKGGTLISYDGQVRLLEVAQVP
KEHIDEFKNIRKFTNFNTNNLWINLKAVKRLIESSNLEMEIIPNQKTITRDGHEINVLQLETACGAAIRHFDGAH
GVVVPRSRFLPVKTCSDLLLVKSDLFRLEHGSLKLDPSRFGPNPLIKLGSHFKKVSGFNARIPHIPKIVELDHLT
ITGNVFLGKDVTLRGTVIIVCSDGHKIDIPNGSILENVVVTGNLQILEH

SEQ ID NO: 27 is a nucleic acid sequence of Glucosamine 6-phosphate N-
acetyltransferase (GNA1) originating from Saccharomyces cerevisiae
ATGAGCTTACCCGATGGATTTTATATAAGGCGAATGGAAGAGGGGGATTTGGAACAGGTCACTGAGACGCTAAAG
GTTTTGACCACCGTGGGCACTATTACCCCCGAATCCTTCAGCAAACTCATAAAATACTGGAATGAAGCCACAGTA
TGGAATGATAACGAAGATAAAAAAATAATGCAATATAACCCCATGGTGATTGTGGACAAGCGCACCGAGACGGTT
GCCGCTACGGGGAATATCATCATCGAAAGAAAGATCATTCATGAACTGGGGCTATGTGGCCACATCGAGGACATT
GCAGTAAACTCCAAGTATCAGGGCCAAGGTTTGGGCAAGCTCTTGATTGATCAATTGGTAACTATCGGCTTTGAC
TACGGTTGTTATAAGATTATTTTAGATTGCGATGAGAAAAATGTCAAATTCTATGAAAAATGTGGGTTTAGCAAC
GCAGGCGTGGAAATGCAAATTAGAAAATAG

SEQ ID NO: 28 is an amino acid sequence of Glucosamine 6-phosphate N-
acetyltransferase (GNA1) originating from Saccharomyces cerevisiae
MSLPDGFYIRRMEEGDLEQVTETLKVLTTVGTITPESFSKLIKYWNEATVWNDNEDKKIMQYNPMVIVDKRTETV
AATGNIIIERKIIHELGLCGHIEDIAVNSKYQGQGLGKLLIDQLVTIGFDYGCYKIILDCDEKNVKFYEKCGFSN
AGVEMQIRK

SEQ ID NO: 29 is a nucleic acid sequence of phosphoacetylglucosamine mutase (PCM1) originating from Saccharomyces cerevisiae
ATGAAGGTTGATTACGAGCAATTGTGCAAACTCTACGATGACACGTGCCGCACAAAGAATGTGCAGTTCAGTTAC
GGTACGGCCGGATTCAGAACGCTGGCCAAGAATTTGGATACGGTGATGTTCAGTACTGGTATACTGGCGGTTCTC
AGGTCGCTGAAGCTTCAGGGTCAGTATGTGGGGGTGATGATCACGGCGTCGCACAACCCATACCAGGACAACGGG
GTCAAGATCGTGGAACCAGACGGATCGATGCTTTTGGCCACATGGGAGCCATATGCCATGCAGTTGGCCAATGCG
GCCTCTTTTGCCACTAATTTTGAAGAATTTCGTGTTGAGTTGGCCAAGCTGATTGAACACGAAAAGATTGATTTG
AATACAACCGTCGTGCCTCACATCGTGGTTGGGAGAGACTCTAGGGAAAGTAGTCCATACTTGCTGCGCTGCTTG
ACTTCCTCCATGGCCAGCGTCTTCCACGCGCAAGTTTTGGACCTAGGCTGTGTCACTACGCCTCAATTGCATTAC
ATTACTGATTTGTCCAACAGGCGGAAACTGGAAGGAGACACAGCGCCAGTTGCCACAGAACAGGACTACTATTCG
TTCTTTATAGGAGCCTTCAACGAGCTCTTCGCCACGTATCAGCTGGAGAAGAGGCTGTCTGTCCCAAAATTGTTC
ATAGACACAGCCAATGGTATCGGTGGTCCACAGTTGAAAAAACTACTGGCCTCCGAAGATTGGGACGTGCCAGCG
GAGCAAGTTGAGGTAATCAACGACAGGTCCGATGTTCCAGAACTGTTGAATTTTGAATGCGGTGCGGATTATGTG
AAGACTAACCAGAGATTACCCAAGGGTCTTTCTCCATCCTCGTTTGATTCGCTATATTGCTCCTTTGATGGTGAC
GCAGACAGGGTTGTGTTCTACTATGTCGACTCAGGATCAAAATTTCATTTGTTGGATGGTGACAAAATTTCCACT
TTGTTTGCAAAGTTCTTGTCTAAACAACTAGAATTGGCACACCTAGAACATTCTTTGAAGATTGGTGTTGTGCAA
ACTGCCTATGCAAACGGCAGTTCCACCGCTTACATAAAAAATACGTTGCACTGTCCCGTGTCTTGCACTAAGACA
GGTGTTAAACACTTGCATCATGAAGCTGCCACTCAGTACGATATTGGCATTTATTTCGAAGCAAATGGACATGGT
ACGATTATATTCAGCGAAAAATTTCATCGAACTATCAAATCTGAATTATCCAAGTCCAAGTTAAATGGTGATACG
TTAGCTTTGAGAACTTTGAAGTGTTTCTCTGAATTGATTAATCAGACCGTGGGAGATGCTATTTCAGACATGCTT
GCTGTCCTTGCTACTTTGGCGATTTTGAAAATGTCGCCAATGGATTGGGATGAAGAGTATACTGATTTGCCCAAC
AAGCTGGTTAAGTGCATCGTTCCTGATAGGTCAATTTTCCAAACCACGGACCAGGAAAGAAAATTGCTCAATCCA

GTGGGGTTGCAAGACAAGATAGATCTTGTGGTAGCCAAGTATCCCATGGAAGAAGCTTTGTCAGAGCCAGTGGT
ACGGAGGATGCGGTGAGGGTTTATGCGGAATGTAAGGACTCCTCTAAGTTAGGTCAATTTTGTGACGAAGTGGTG
GAGCACGTTAAGGCATCTGCTTGA

SEQ ID NO: 30 is an amino acid sequence of phosphoacetylglucosamine
mutase (PCM1) originating from Saccharomyces cerevisiae
MKVDYEQLCKLYDDTCRTKNVQFSYGTAGFRTLAKNLDTVMFSTGILAVLRSLKLQGQYVGVMITASHNPYQDNG
VKIVEPDGSMLLATWEPYAMQLANAASFATNFEEFRVELAKLIEHEKIDLNTTVVPHIVVGRDSRESSPYLLRCL
TSSMASVFHAQVLDLGCVTTPQLHYITDLSNRRKLEGDTAPVATEQDYYSFFIGAFNELFATYQLEKRLSVPKLF
IDTANGIGGPQLKKLLASEDWDVPAEQVEVINDRSDVPELLNFECGADYVKTNQRLPKGLSPSSFDSLYCSFDGD
ADRVVFYYVDSGSKFHLLDGDKISTLFAKFLSKQLELAHLEHSLKIGVVQTAYANGSSTAYIKNTLHCPVSCTKT
GVKHLHHEAATQYDIGIYFEANGHGTIIFSEKFHRTIKSELSKSKLNGDTLALRTLKCFSELINQTVGDAISDML
AVLATLAILKMSPMDWDEEYTDLPNKLVKCIVPDRSIFQTTDQERKLLNPVGLQDKIDLVVAKYPMGRSFVRASG
TEDAVRVYAECKDSSKLGQFCDEVVEHVKASA

SEQ ID NO: 31 is a nucleic acid sequence of promotor pTDH3
CTGCTGTAACCCGTACATGCCCAAAATAGGGGGCGGGTTACACAGAATATATAACATCGTAGGTGTCTGGGTGAA
CAGTTTATTCCTGGCATCCACTAAATATAATGGAGCCCGCTTTTTAAGCTGGCATCCAGAAAAAAAAAGAATCCC
AGCACCAAAATATTGTTTTCTTCACCAACCATCAGTTCATAGGTCCATTCTCTTAGCGCAACTACAGAGAACAGG
GGCACAAACAGGCAAAAAACGGGCACAACCTCAATGGAGTGATGCAACCTGCCTGGAGTAAATGATGACACAAGG
CAATTGACCCACGCATGTATCTATCTCATTTTCTTACACCTTCTATTACCTTCTGCTCTCTCTGATTTGGAAAAA
GCTGAAAAAAAAGGTTGAAACCAGTTCCCTGAAATTATTCCCCTACTTGACTAATAAGTATATAAAGACGGTAGG
TATTGATTGTAATTCTGTAAATCTATTTCTTAAACTTCTTAAATTCTACTTTTATAGTTAGTCTTTTTTTTAGTT
TTAAAACACCAAGAACTTAGTTTCGAATAAACACACATAAACAAACAAA

SEQ ID NO: 32 is a nucleic acid sequence of promotor pTDH3.Sk
CAATTCATCGGCCCTTTTAGCGGCTACCCGCGCCATCTAAATGATAGGGCGGGTGACACTATGGTAAATCCCATA
ATTAGGTGTCTGGGTGAGTGGTTCTGATGCCGGCATCCACTAAATATATTGGAGCCCATTTTTTACGCGGGCTTC
CAGAAAAAAAGAGAATCCCAGCACCAAAAGGTGGTTCTCTTCACCAACCATCAGATCATAGGTCCACAACCACAC
ATAACAGGGGCACAAAAAGGCAAAAAACGGACATAACCTCAATGGAGTGATGCAAATTGACTGGAGCAAAAGCTG
ACACAAGGCATTGATTGACCTACGCATGTATCTGTATTCTTTTCTTACACCTTCTATTACCTTCTAACTCTTTGG
GTTGGAAAAAACTGAAAAAAAAGGTTGGGACCTGGTTCCCCCAAGTTGTCCCCCTACTTGGTTATTAAATATATA
AAGACAGCAAGTGTTGATTATAATCTTGTAAATCTATAGTTCTTAATCTATACTTCTATTTATATTTTAAATTAG
TCTTTTTATTTCCAAGTCCCCAAGAACTTAGTTTCGAATAAACACACACAAATAAACACA

SEQ ID NO: 33 is a nucleic acid sequence of promotor pTDH3-1.sba
GCAGCGCTTCTTCCGCTCTAGTTTTATAGTTATTATTACTACCACCTTAAAAATACGTAAATACTCAAAATAGT
AGTGATATTCCCAACCTTATTCATCCAAGGCACATCATCATCATCAGCCATTCATCTTTCACCTGCCATTAGTAA

CCCGTCTTCTCATTGAGCGGGTTACGGCAGCCACAGGCCACATTCCGAATGTCTGGGTGAGCGGTCCCTTTTCCA
GCATCCACTAAATATCTCCGATCCCGCTTTTTAATCTGGCTTCCTGAAAAAAGAGAATCCCAGCACCAAAAAT
GGCTCTCTTCACCAACCATCAGATCATAGGTCCCATTCTCTTACCGCAACCGTACAGAACAGGGGAAAACGGGTA
CAACCTCAATGGAGTGATGCAAACTGACTGGAGCAAAAAGCTGACACAAGGCAATCGACCTACGTGTCTGTCTAT
TTTCTCACACCTTCTATTACCTTCTAACTCTCTGGGTTGGAAAAAACTGAAAAAAAGGTTGAGACCAGTTTCCAC
AAATCATCCCCCTGTTTGATTAATAAATATATAAAGACGACAACTATCGATCATAAACTCATAAAACTATAACTC
CTTTACACTTCTTATTTTATAGTTATTCTATTTTAATTCTTATTGATTTTAAAACCCCAAGAACTTAGTTTCGAA
AACACACACACACACAAACAATTAAAA

SEQ ID NO: 34 is a nucleic acid sequence of promotor pTDH3.Sar
GAGCTAAATATCAGCCCTTCGGGTCCTGCCTGCTACCCGGTCCTGTTCGAATAAAAACGCGGGTAACACGACCCA
GTAACACCTGTCGTTGGGTGTCTGGGTCAGAAGTTCTGATACCGGCTTCCACTAAATAGATTGGGTTCCGCTCTT
TACGCTGGCTTCCTGAAAAAAGAGATTCCGGGCACCAAAAAATTGGTCTCTTTGCCAACCATCAGATCATAGGTC
CATTCTCTTACCATAACCACACAGGATAGGGGCACCACAGGCGAAAATGGGCACAAAATCTCAATGGAGTGATGC
AAATTAGCTGGAACAAAAGCTGACACAAGGCAATTAACCTGCGCATGTATCCATCTCCTTTTCTTACACCTTCTC
TTACGTTCTAACTGTTTGGGTTGGAAAAATTAAAAAAAAAAGGTTGAGACCAGTTTCCCCAAATCGTCCCCCTAC
TTGATTCATAAATATATAAAGACGACAACTATTGATTATAATCTTGTAAATCTATAACTCTTTACTTTCTCCTAT
TTATAATTTAACTTAATCTTTTTAGATTTAAAACCCCAAGAACTTAGTTTCGAACAAACACACACAAATAAACAA
AA

SEQ ID NO: 35 is a nucleic acid sequence of promotor pENO2
CGCTCAGCATCTGCTTCTTCCCAAAGATGAACGCGGCGTTATGTCACTAACGACGTGCACCAACTTGCGGAAAGT
GGAATCCCGTTCCAAAACTGGCATCCACTAATTGATACATCTACACACCGCACGCCTTTTTTCTGAAGCCCACTT
TCGTGGACTTTGCCATATGCAAAATTCATGAAGTGTGATACCAAGTCAGCATACACCTCACTAGGGTAGTTTCTT
TGGTTGTATTGATCATTTGGTTCATCGTGGTTCATTAATTTTTTTTCTCCATTGCTTTCTGGCTTTGATCTTACT
ATCATTTGGATTTTTGTCGAAGGTTGTAGAATTGTATGTGACAAGTGGCACCAAGCATATATAAAAAAAAAAAGC
ATTATCTTCCTACCAGAGTTGATTGTTAAAAACGTATTTATAGCAAACGCAATTGTAATTAATTCTTATTTTGTA
TCTTTTCTTCCCTTGTCTCAATCTTTTATTTTTATTTTATTTTTCTTTTCTTAGTTTCTTTCATAACACCAAGCA
ACTAATACTATAACATACAATAATA

SEQ ID NO: 36 is a nucleic acid sequence of promotor pTEF3
GGCTGATAATAGCGTATAAACAATGCATACTTTGTACGTTCAAAATACAATGCAGTAGATATATTTATGCATATT
ACATATAATACATATCACATAGGAAGCAACAGGCGCGTTGGACTTTTAATTTTCGAGGACCGCGAATCCTTACAT
CACACCCAATCCCCCACAAGTGATCCCCCACACACCATAGCTTCAAAATGTTTCTACTCCTTTTTTACTCTTCCA
GATTTTCTCGGACTCCGCGCATCGCCGTACCACTTCAAAACACCCAAGCACAGCATACTAAATTTCCCCTCTTTC
TTCCTCTAGGGTGTCGTTAATTACCCGTACTAAAGGTTTGGAAAAGAAAAAAGAGACCGCCTCGTTTCTTTTTCT
TCGTCGAAAAAGGCAATAAAAATTTTTATCACGTTTCTTTTTCTTGAAAATTTTTTTTTTTGATTTTTTTCTCTT

TCGATGACCTCCCATTGATATTTAAGTTAATAAACGGTCTTCAATTTCTCAAGTTTCAGTTTCATTTTTCTTGTT
CTATTACAACTTTTTTTACTTCTTGCTCATTAGAAAGAAAGCATAGCAATCTAATCTAAGTTTTAATTACAAA

SEQ ID NO: 37 is a nucleic acid sequence of promotor pTEF1
GTTTAGCTTGCCTCGTCCCCGCCGGGTCACCCGGCCAGCGACATGGAGGCCCAGAATACCCTCCTTGACAGTCTT
GACGTGCGCAGCTCAGGGGCATGATGTGACTGTCGCCCGTACATTTAGCCCATACATCCCCATGTATAATCATTT
GCATCCATACATTTTGATGGCCGCACGGCGCGAAGCAAAAATTACGGCTCCTCGCTGCAGACCTGCGAGCAGGGA
AACGCTCCCCTCACAGACGCGTTGAATTGTCCCCACGCCGCGCCCCTGTAGAGAAATATAAAAGGTTAGGATTTG
CCACTGAGGTTCTTCTTTCATATACTTCCTTTTAAAATCTTGCTACGATACAGTTCTCACATCACATCCGAACAT
AAACAACC

SEQ ID NO: 38 is a nucleic acid sequence of promotor pTEF1.ago
GTTTAGCTTGCCTCGTCCCCGCCGGGTCACCCGGCCAGCGACATGGAGGCCCAGAATACCCTCCTTGACAGTCTT
GACGTGCGCAGCTCAGGGGCATGATGTGACTGTCGCCCGTACATTTAGCCCATACATCCCCATGTATAATCATTT
GCATCCATACATTTTGATGGCCGCACGGCGCGAAGCAAAAATTACGGCTCCTCGCTGCAGACCTGCGAGCAGGGA
AACGCTCCCCTCACAGACGCGTTGAATTGTCCCCACGCCGCGCCCCTGTAGAGAAATATAAAAGGTTAGGATTTG
CCACTGAGGTTCTTCTTTCATATACTTCCTTTTAAAATCTTGCTACGATACAGTTCTCACATCACATCCGAACAT
AAACAACC

SEQ ID NO: 39 is a nucleic acid sequence of promotor pTEF1.Sba
CGCCAACAAACCTTCGAACACTTTAATTTTCGAGGACCGCAGATCCTCACATCACACCCACACCCAAGACTGCTT
CCCCCACACACCCTGCATCTGTACACTTTCTTCTGCTCTGTTTTTCTCTCCGGCGTTCTCTCGGGTCGCCCGCAT
CGCCGCGCCGGCTGGAACCGCCCACGCACCGCATATTGCAAATCGCCTGCCCCCTCTTGCTCCTTTTTGAGGGCG
CGCCGTTACCCGCGCCCAGGGTCCGGAAAAAGAAACAAGGCTCTACCGCGTTTCTTTTTCCTTGTCGAAAAAGGC
AAAAATGAAAATTTTTATCACGTTTCTTTTTTTTTGAAAAATTTTTTTTTTGGTTTTTTTTCTTTCGATGGCCTC
CCATTGATATTTAAGTTAATAAATGGTTTTCAGTTTTCAAGTTTCAGTTTGTGTTCTTCTTTGCTAACTTTCACT
TACACCTCGAAAGAAAGTATAGCAATCTAATCTTAGTTTTAATTACAA

SEQ ID NO: 40 is a nucleic acid sequence of promotor pPDC1
TTATTTACCTATCTCTAAACTTCAACACCTTATATCATAACTAATATTTCTTGAGATAAGCACACTGCACCCATA
CCTTCCTTAAAAACGTAGCTTCCAGTTTTTGGTGGTTCCGGCTTCCTTCCCGATTCCGCCCGCTAAACGCATATT
TTTGTTGCCTGGTGGCATTTGCAAAATGCATAACCTATGCATTTAAAAGATTATGTATGCTCTTCTGACTTTTCG
TGTGATGAGGCTCGTGGAAAAAATGAATAATTTATGAATTTGAGAACAATTTTGTGTTGTTACGGTATTTTACTA
TGGAATAATCAATCAATTGAGGATTTTATGCAAATATCGTTTGAATATTTTTCCGACCCTTTGAGTACTTTTCTT
CATAATTGCATAATATTGTCCGCTGCCCCTTTTTCTGTTAGACGGTGTCTTGATCTACTTGCTATCGTTCAACAC
CACCTTATTTTCTAACTATTTTTTTTTTAGCTCATTTGAATCAGCTTATGGTGATGGCACATTTTTGCATAAACC
TAGCTGTCCTCGTTGAACATAGGAAAAAAAAATATATAAACAAGGCTCTTTCACTCTCCTTGCAATCAGATTTGG

GTTTGTTCCCTTTATTTTCATATTTCTTGTCATATTCCTTTCTCAATTATTATTTTCTACTCATAACCTCACGCA
AAATAACACAGTCAAATCAATCAAA

SEQ ID NO: 41 is a nucleic acid sequence of promotor pCCW12
AACCAGGGCAAAGCAAAATAAAAGAAACTTAATACGTTATGCCGTAATGAAGGGCTACCAAAAACGATAATCTCA
ACTGTAAACAGGTACAATGCGGACCCTTTTGCCACAAAACATACATCATTCATTGCCGGAAAAAGAAAGAAGTGA
AGACAGCAGTGCAGCCAGCCATGTTGCGCCAATCTAATTATAGATGCTGGTGCCCTGAGGATGTATCTGGAGCCA
GCCATGGCATCATGCGCTACCGCCGGATGTAAAATCCGACACGCAAAAGAAAACCTTCGAGGTTGCGCACTTCGC
CCACCCATGAACCACACGGTTAGTCCAAAAGGGGCAGTTCAGATTCCAGATGCGGGAATTAGCTTGCTGCCACCC
TCACCTCACTAACGCTGCGGTGTGCGGATACTTCATGCTATTTATAGACGCGCGTGTCGGAATCAGCACGCGCAA
GAACCAAATGGGAAAATCGGAATGGGTCCAGAACTGCTTTGAGTGCTGGCTATTGGCGTCTGATTTCCGTTTTGG
GAATCCTTTGCCGCGCGCCCCTCTCAAAACTCCGCACAAGTCCCAGAAAGCGGGAAAGAAATAAAACGCCACCAA
AAAAAAAAATAAAAGCCAATCCTCGAAGCGTGGGTGGTAGGCCCTGGATTATCCCGTACAAGTATTTCTCAGGAG
TAAAAAAACCGTTTGTTTTGGAATTCCCCATTTCGCGGCCACCTACGCCGCTATCTTTGCAACAACTATCTGCGA
TAACTCAGCAAATTTTGCATATTCGTGTTGCAGTATTGCGATAATGGGAGTCTTACTTCCAACATAACGGCAGAA
AGAAATGTGAGAAAATTTTGCATCCTTTGCCTCCGTTCAAGTATATAAAGTCGGCATGCTTGATAATCTTTCTTT
CCATCCTACATTGTTCTAATTATTCTTATTCTCCTTTATTCTTTCCTAACATACCAAGAAATTAATCTTCTGTCA
TTCGCTTAAACACTATATCAATA

SEQ ID NO: 42 is a nucleic acid sequence of promotor pCCW12.Sm
CGTTGTGCTGTAGTGAAGGAAGACTAAAAAGGATAATCACAGTTGTAAAGAGGTATAATGCGGACCCTTTTGCCA
CAAAACACACATCTTTCGTTTCCAAAATAAGAAAGAAAGCAAAAAGATTAGCAGCCACGTTGCTGCGATCTA
ATTATAGACGGTGGCGTCATCATTCTCACCCAAGATTGTGTCTTGAACCTGCCACGGGTCCTGCGTTATCGCCGG
ATGTAAAACTAGACATGCAAAAAAAGGACCTTCCAGGTAGCGTGCTCCACACCACCCATGACCACCACAGTTAGT
CCAAAAGAGGCAGCACCACTTCCCGATGGGGGAATTAGATTGCTGCCACCCTCACCTCACTAATGCTGCGGTGTG
CGGATATGCCCTGCTATATATAGCTCCGCGTTTTTGAACCAGCACAGCGCGAGCACCAAAAAGGAAAATCGCATA
GGCCCAGAACTGATTTCAGCACGGGCTATTGGCGTTGGGTTTCCGTTCTGGGAAACCTTCGCCGCGTCCCCCTCA
CAAACCTCCGCACAAGTTCGAGCAAGCGGGAAAAAACGAAAAACGCCATTAATACTAAATAAAGCAAATCCTCGA
AGCGTGGGTGGCAAGCCCCTGGATTTTTCCGCACAAGTACTCTTCTCAGGAGTAAAAAAACCCGTTTGTTTTGGA
ATTCCCCATTTCGCGGCCATCTACGCCGGTATCTTCGCAATATCTATCAGCGATAACTCAGCAATTTTAATATTC
GTGTTGCAGTGCTGCGATAGCGGGAGTCTTGTTTGTAACATAACGGCAGAAAGGAATGAGAGAAAATTTTCCATT
CTTTGGCCTCCGTTCAAGTATATAAAGCCGGCATGCTTGGTAATCTTTCTCTCTTCTGTATTGTTTCTATAAT
ACTTTTATCTTCTAATTATTTTCTGAAAAACCAAGAAATTAATCTTCTGTCATTCGCTTAAACACTATATCAAT
A

SEQ ID NO: 43 is a nucleic acid sequence of promotor pCCW12.Sk
TGTAAAATCCTACACGCAAAAAACCTCTTGGGTTGCGCGTTTTCCACCACCCACGACCCGCACAATCAATCCAAA
AGGGGCAACGCCAGTTCCCAATGCGGGAATTAGCTCCTCACCCCCCTCACCCGCTAACGCTGCGGTGTGCGGACA

CGCAGCAGTATTTATAGATCCTCGTGTTGGAACCAGCCCGCGTGAGCACCAAATTGGAAAGTCGCAATGGGCCCA
GAACCGCTTTCAGTACTGGGCCATTGGCGTCTAGTTTCCGTTTTGAGCGTCCTTCGCCGCGTCTCTCTGTGAAAT
CCCCGCACAAGTCTGAGCAGGCAAAAAAAAAAAACGCCACCAAAAATAAATAAAGCCAATCCTCGAAGGATGAGT
AGGAAAGGAAGCCCCTGGTTTTTCCCGCACGAATATTTTTCAGGAGTGAAAAAATCCGTTTGTTTTGGAATTCC
CCATTTCGCGCTCACCTACGCCGGTATCTTTGGAACAACTATCAGCGATAACTCAGCAAAATTTGCATATCCGTG
CTGCAATAGTGCGATAGTGGGATTGGGAGTCTTGTTGCATCATAACGGCAGAAAGGAATGAATAAAAATTTTCCG
TTCTTTGTCTCCGTTCAAGTATATAAAGTCGGCATGCTTGATTATCTTTTCTTCTCTTCTACTACATTTCTATTT
CACTTTCTACTCTATTCTTCCCTGAAAAACCCAAGAAAATAATCTTCTGTCATTCGCTTAAACAATATATCAAAA

SEQ ID NO: 44 is a nucleic acid sequence of promotor pCCW12.Sba
ACCATGCCACGGTGCTGGCCCCACTTCCACCCACGACCCGCACGGTTGGCCCGAAAGGGGCAACACCAGTTCCCA
ATACGGAAATTAGTCCGCCACCACCCTCACCTCGCTAGAGCTGCGGTGTGCGGGCGTGCATCGCTATTTATAGAC
ATGCCTGCTGGCGTCACCGCGCGCGAGCACCAAACAGGAAAATCGCACTGGGCCCAGAACCACGCTATGCGCTGG
GCCGATGGCGTCCGGTTTCCCTTTGGGAGCCCCCTGCCACGTTCGCCTAACAAATCCCCGCACCGGCTTGAGAAA
AAAGCGAAAAGCGAAAAAAAAAAATCAACGCCACCAAAATTAAAAAAAAAGAGCCATCCTCGAAGGGTGAATAGT
AGCCCCTGACTTTTCCCGCACAGACAGACACCTTTCAGGAGTGAACAAAAAAGCAGTTTGTTTTGGAATTCCCCC
ATTTCGCGGTGGCCTGCGCAGGTATCTCTGCGTCAACTATCAGCGATAACTCAGCAAATTTTGCATATTCGTGTT
GCGATACTACGATAATGGGAGTCTGTCGCCTAATAACGGCAACAAGGAATGAGAGAGAAAAATTTTCTTCATTCT
CCAGCTCCCGTTCAAGTATATAAGGTCGGCATGGTCGATTGTCTTTCCTTCTCTTCAGTTACGTCTCTCTATTTA
CATTATTCTTATTTTTATTTAATAAAAACCCCAAGAAATTAATCTTCTGTCATTCGCTTAAACACTATATCAAAA

SEQ ID NO: 45 is a nucleic acid sequence of promotor pCCW12.Sar
AAAAAACAACCTTCTGCCAACCTGCGTGCTTCTCACCACCCATGACCCACACAATTGACCCGAATGGGGCAACTC
CAGTTCCCAATACGGGAATTAACTCGCCACCATATTTACCGCGTTGAAGCTGTGGTGTGCGGACACTCCGTACTA
TTTATAGACCCACGCGGTGGAACCAGCACGCGCGCACTAAACAGGAAAATCGCATTGAGTCCAGAACCGCCAC
CAGCACTTGGCCATTGGCGTCTAATTTCCGTTTTCGGCGCCCCTCACCGCGTCCTTCTAACAAAGCGCGCACAAG
CTTGAGCAAGTGAAAAGAAAATTAAAAATAAAAAACCGCCACCAAAACAAATAAAGCAATCTTCGAAGTGTGGGT
TGGTGGGAAGCCCCTGGCTTTTCCCGCACCAGTCGTTTTCAGGAGTAAAAAAATACCCGTTTGTTTTGGAATTCC
CCATTTCGCGGCGACCTGCGCCGGTATCTTTGCAACAACTATTTGCGATAACTCAGCAAAATTTGCATATTCGTG
TTGGGATATTGCGATAGTGGGAGTCTTGTTGCATAATAACGGTAAAAAGAAGTGAAGGAAAAAAATTTGCATCCT
TTAGTCTCAGTTCAAGTATATAAAGTCGGGATATTCAATTATCTTTCTTTCTCTTGCTCAAAGGTTTCTATATTT
TTTTTATAGTATTTCTTTTGTTATAAAATACCAAGAAATTAATCTTCTGTCATTCGCTTAAATACTACATCAATA

SEQ ID NO: 46 is a nucleic acid sequence of promotor pNUP57
TCATCTGCGCAATGACTATCAAGACCTTCTGCAAGAATTTCAAATCTCACTGAAAATCTTGACCGAAAAGTGTCT
TGAAAACCCATCAAGCCTGCAAAACCTATCTTTGACATTAGTCTCCATTATAAAAACGGCATAGTTGGGAGAAAA
CTTTCCATACTTCAATTGTGGACTGATATAAGTATTTTAGTTTTGCCCGCATGATCATCCCACATGGCTACAGCA
GTTCTCTCATAGGAAAATAGTACAATAGCTACGTGATATAATCTAAATAATTGTTGCCAATGTGTAATTATATCA

TTTTGAACGTACGCGAAATGGATTATTTTCAAAAATTTTGTTTCTTGAAATGAGTAAAAGCAAAAGTCCAACTCT
CCAAGTCGATGTAAACAACTTTTTGCCAAAGGGACTGAAAGACTAAATCGAGGATTATCCCGTTCAAACTATTCC
AGAAACGCTCGTTAGTAACAAAAGACATACCTTGTTGACCAATTGATCAC

SEQ ID NO: 47 is a nucleic acid sequence of promotor pCCW10.ago
GGTACCACGGCAACCTCGTTCGCTGTTCATCCCCTTCGTCACACAGGACGTTGGATGCCGTAAGCAGCGTTGCTT
TTGATCCTCAGGATCGGCCGGGTAACCCGCGGCTGCTTCTATTTTAGTATTCATATCTCAAGCACATCCATTCCG
GCCGTTTGGGGGCGCCGCCGCACTCGTGTCCATTCCTACCGTGGCACTTAGGGCTATCCTGTCGGAGCGCCCCGC
CGACCGCCTTATCGGCACCAAAAGTAGAAGCCCCGGCCCCGCGTGGCTCAGACTCACCATCGGTGCTATTTACTT
TTCGATCAGATCGCGGCGCGCGGTGGCCGGCATTTCCGGAAGCGGCCACGGAGCAGAGGTGGCGCATTCGAATCG
CATACGTCTTCGCCACGCCGGAAAAAAATTTTCGGCTATATAAGGAGAGGCGGCCGTCTTGCTGCAGGCAGTTT
CACTTTCTCTAAAACCAAAGAACATCGATTTCTTTAGTCACTCGCTTCCTTACACCGAACTCGAGGCGGCCGC

SEQ ID NO: 48 is a nucleic acid sequence of promotor pCWP2
CTAGCCTCCCCTTTTTATTTTGTGCGGTCACCGCAAGGGACAAAGCTTTTCTTAGAAAACCGTCTGAGAAGCATA
ACGTACGCCATCCCCTAGACATATTAATAATGCTACAGATACTATGCTGCTCGTCTTTTTTTGACGACCCTTTTA
TTGCAATGTGCAACTAATGGCAAACAACCACATAGTATCACAGTATTACATTGCCTCCACCGATGCGGATGTTAG
GGCGCCAAGTCTGTCATGAAGCATGTTCCTGTCATAATCTTGTATGCAAAATACCGCGTTCTGCGCCACTGATAT
GCTAGGCAGCAGCAACCTATGCAGAAGATTGCTTTTCCCACGCCTGTTTACGTCTCCAGGGCACTTGAAACAAT
GCAGCGATCGCCGCCACAACACGCCAAAGAGAAGCGAAAGTGGGCCTGGGCGGCCTCAGTTTCGGCAGAGGTAAA
CAACACGAACTGAACTGCCTTAGCTCCGAAGGGCAATTCCACAGGCACTCCGCGGGGCCCGGCCAAGGCCCAAAA
GGCGTGGAATATGCGCGTTTTGGGGCCATAACACCCAGTACCACGGCCGGAACGGGCCATATAATAAGTTTTTCA
CTCTCAAGAATGGTAAACGTAAATAGGAACATCCCACTACCCTAGAAATTGCGGAAATTTCGCGCTTATCATTAG
AAAATCTGGAACCGTCCTTTTTCCTCTTTCTTGCATTTCCCTTTCCGTATTATTGCCATTCTTTAACTGCATTTG
GGGAACCGTAGACCAAAAGCCAAACAGAGAAATGTAACGTTCTAAAAAAAAACAACGAAAAAATTGAAAAATAA
GATACAATAATCGTATATAAATCAGGCTTCTTGTTCATCATTTTCAATTCTCTTCTTGCCATCCCTTTTCCTATC
TTTGTTCTTTTCTTCTCATAATCAAGAATAAATAACTTCATCA

SEQ ID NO: 49 is a nucleic acid sequence of promotor pCCW120.Sca
TATTTTTTTTAAATTAAAGATGCTGTGCAATTAATTGTCCGGAGGGACCCTTGCATACTGGCCAGTTTGTACGGG
CGTTACATAGCGCCGATCGACGAACAGCTTATTAATAGCAAGATTTGTTTCTCCCATGAGCAATTAACCAGTAAT
TTTCACAGAAACTAGCACAAAACCAACCAAACAGTTAAAGGCAGTTTCGGAGAATAAACAACGCGCTCACTGAGC
TTGCTGTTGCCAAAAATAACAGTCGCGTGCGATGGCGGGCTTGGCATTTTCCTCGAAGGGGAAACGCCGCCAGGG
TTCGCTGCGGTGTGTGGGTGGGGCAGCCCCCGGGCGCCCAGACGGTGCCGCAGGGCCTTGAGACAGCCCAAAATG
CCGCGAAAACGCAAGGGCGCGGTGACCTCTTTCCTAATGCGGAAAGCCCCACCGCCAGCGACGTGACGCGTTGTG
CGGGCTCTTCTCGGGACCCGGAAAAAGAAATACGTACACAAACACCAGCAAATTTTTCGCTTGAGAGGCTGCCC
GTGTTTCTGCGCAACGTGAAACACGGTGGACAAGACAAGAGAGATTGACAAGCGTCGTTATCGTCCCAGTGAGGT

TTCCCAATTATATAAATACATCCCTGTTGGTGGATTCTTAATTACAACTTCCATCTCTCTCTCTCTCACTTCTTT
TGTTCCCTTACATATCCCCAAGAAATCAATCTTCTGCCATTCGCTTTAACAACAAACAAACTTTTAACTTTTAAA

SEQ ID NO: 50 is a nucleic acid sequence of promotor pRPLA1
TCAAGTTGGATACTGATCTGATCTCTCCGCCCTACTACCAGGGACCCTCATGATTACCGCTCGAATGCGACGTTT
CCTGCCTCATAAAACTGGCTTGAAAATATTTATTCGCTGAACAGTAGCCTAGCTTATAAAAATTTCATTTAATTA
ATGTAATATGAAAACTCACATGCCTTCTGTTTCTAAAATTGTCACAGCAAGAAATAACATTACCATACGTGATCT
TATTAAACTCTAGTATCTTGTCTAATACTTCATTTAAAAGAAGCCTTAACCCTGTAGCCTCATCTATGTCTGCTA
CATATCGTGAGGTACGAATATCGTAAGATGATACCACGCAACTTTGTAATGATTTTTTTTTTTCATTTTTTAAA
GAATGCCTTTACATGGTATTTGAAAAAAATATCTTTATAAAGTTTGCGATCTCTTCTGTTCTGAATAATTTTTAG
TAAAAGAAATCAAAAGAATAAAGAAATAGTCCGCTTTGTCCAATACAACAGCTTAAACCGATTATCTCTAAAATA
ACAAGAAGAA

SEQ ID NO: 51 is a nucleic acid sequence of promotor pCUP1
CGGCAAACTTCAACGATTTCTATGATGCATTTTATAATTAGTAAGCCGATCCCATTACCGACATTTGGGCGCTAT
ACGTGCATATGTTCATGTATGTATCTGTATTTAAAACACTTTTGTATTATTTTTCCTCATATATGTGTATAGGTT
TATACGGATGATTTAATTATTACTTCACCACCCTTTATTTCAGGCTGATATCTTAGCCTTGTTACTAGTTAGAAA
AAGACATTTTTGCTGTCAGTCACTGTCAAGAGATTCTTTTGCTGGCATTTCTTCTAGAAGCAAAAAGAGCGATGC
GTCTTTTCCGCTGAACCGTTCCAGCAAAAAAGACTACCAACGCAATATGGATTGTCAGAATCATATAAAAGAGAA
GCAAATAACTCCTTGTCTTGTATCAATTGCATTATAATATCTTCTTGTTAGTGCAATATCATATAGAAGTCATCG
AAATAGATATTAAGAAAAACAAACTGTACAATCAATCAATCAATCATCACATAAA

SEQ ID NO: 52 is a nucleic acid sequence of promotor pMET6
CCACAGGAAATATTTCACGTGACTTACAAACAGAGTCGTACGTCAGGACCGGAGTCAGGTGAAAAAATGTGGGCC
GGTAAAGGGAAAAAACCAGAAACGGGACTACTATCGAACTCGTTTAGTCGCGAACGTGCAAAAGGCCAATATTTT
TCGCTAGAGTCATCGCAGTCATGGCAGCTCTTTCGCTCTATCTCCCGGTCGCAAAACTGTGGTAGTCATAGCTCG
TTCTGCTCAATTGAGAACTGTGAATGTGAATATGGAACAAATGCGATAGATGCACTAATTTAAGGGAAGCTAGCT
AGTTTTCCCAACTGCGAAAGAAAAAAAGGAAAGAAAAAAAAATTCTATATAAGTGATAGATATTTCCATCTTTAC
TAGCATTAGTTTCTCTTTTACGTATTCAATATTTTTGTTAAACTCTTCCTTTATCATAAAAAAGCAAGCATCTAA
GAGCATTGACAACACTCTAAGAAACAAAATACCAATATAATTTCAAAGTACATATCAAAA

SEQ ID NO: 53 is a nucleic acid sequence of promotor pMET25
TTACATTATCAATCCTTGCGTTTCAGCTTCCACTAATTTAGATGACTATTTCTCATCATTTGCGTCATCTTCTAA
CACCGTATATGATAATATACTAGTAACGTAAATACTAGTTAGTAGATGATAGTTGATTTTTATTCCAACACTAAG
AAATAATTTCGCCATTTCTTGAATGTATTTAAAGATATTTAATGCTATAATAGACATTTAAATCCAATTCTTCCA
ACATACAATGGGAGTTTGGCCGAGTGGTTTAAGGCGTCAGATTTAGGTGGATTTAACCTCTAAAATCTCTGATAT
CTTCGGATGCAAGGGTTCGAATCCCTTAGCTCTCATTATTTTTTGCTTTTTCTCTTGAGGTCACATGATCGCAAA
ATGGCAAATGGCACGTGAAGCTGTCGATATTGGGGAACTGTGGTGGTTGGCAAATGACTAATTAAGTTAGTCAAG

GCGCCATCCTCATGAAAACTGTGTAACATAATAACCGAAGTGTCGAAAAGGTGGCACCTTGTCCAATTGAACACG
CTCGATGAAAAAAATAAGATATATATAAGGTTAAGTAAAGCGTCTGTTAGAAAGGAAGTTTTTCCTTTTTCTTGC
TCTCTTGTCTTTTCATCTACTATTTCCTTCGTGTAATACAGGGTCGTCAGATACATAGATACAATTCTATTACCC
CCATCCATACA

SEQ ID NO: 54 is a nucleic acid sequence of promotor pSAM1
GAAACGGACGTAAGACGGAAATAGAATTTGAAGATAAAGTTATATATCACTACACACGAATACTTTCTTTTTTTT
TTTTCACAGGAAAACTGTGGTGGCGCCCTTGCCTACTAGTGCATTTCTTTTTTCGGGTTCTTGTCTCGACGAAAT
TTTAGCCTCATCGTAGTTTTTCACTCTGGTATCGATGAAAAAGGGAAGAGTAAAAAGTTTTCCGTTTAGTACTTA
ATGGGATTGGTTTGGGACGTATATATCGACTGGTGTTGTCTGTTATTCATCGTTGTTTTTCGGTTAGCTTCGAAA
AAAAAATAGAGTAAAAACCAGGAATTTACCCTAAAAACAAGAAAAAATAAGATAAACGAAAAT

SEQ ID NO: 55 is a nucleic acid sequence of terminator tTPI1
GATTAATATAATTATATAAAAATATTATCTTCTTTTCTTTATATCTAGTGTTATGTAAAATAAATTGATGACTAC
GGAAAGCTTTTTTATATTGTTTCTTTTTCATTCTGAGCCACTTAAATTTCGTGAATGTTCTTGTAAGGGACGGTA
GATTTACAAGTGATACAACAAAAAGCAAGGCGCTTTTTCTAATAAAAAGAAGAAAAGCATTTAACAATTGAACAC
CTCTATATCAACGAAGAATATTACTTTGTCTCTAAATCCTTGTAAAATGTGTACGATCTCTATATGGGTTACTCA

SEQ ID NO: 56 is a nucleic acid sequence of terminator tMET25
GTGTGCGTAATGAGTTGTAAAATTATGTATAAACCTACTTTCTCTCACAAGTACTATACTTTTATAAAACGAACT
TTATTGAAATGAATATCCTTTTTTTCCCTTGTTACATGTCGTGACTCGTACTTTGAACCTAAATTGTTCTAACAT
CAAAGAACAGTGTTAATTCGCAGTCGAGAAGAAAAATATGGTGAACAAGACTCATCTACTTCATGAGACTACTTT
ACGCCTCCTATAAAGCTGTCACACTGGATAAATTTATTGTAGGACCAAGTTACAAAAGAGGATGATGGAGGTTT

SEQ ID NO: 57 is a nucleic acid sequence of terminator tDIT1
TAAAGTAAGAGCGCTACATTGGTCTACCTTTTTGTTCTTTTACTTAAACATTAGTTAGTTCGTTTTCTTTTTCTC
ATTTTTTTATGTTTCCCCCCCAAAGTTCTGATTTTATAATATTTTATTTCACACAATTCCATTTAACAGAGGGGG
AATAGATTCTTTAGCTTAGAAAATTAGTGATCAATATATATTTGCCTTTCTTTTCATCTTTTCAGTGATATTAAT
GGTTTCGAGACACTGCAATGGCCCTAGTTGTCTAAGAGGATAGATGTTACTGTCAAAGATGATATTTTGAATTTC

SEQ ID NO: 58 is a nucleic acid sequence of terminator tRPL3
GAAGTTTTGTTAGAAAATAAATCATTTTTTAATTGAGCATTCTTATTCCTATTTTATTTAAATAGTTTTATGTAT
TGTTAGCTACATACAACAGTTTAAATCAAATTTTCTTTTTCCCAAGTCCAAAATGGAGGTTTATTTTGATGACCC
GCATGCGATTATGTTTTGAAAGTATAAGACTACATACATGTACATATATTTAAACATGTAAACCCGTCCATTATA
TTGCTTACTTTCTTCTTTTTTGCCGTTTTGACTTGGACCTCTGGTTTGCTATTTCCTTACAATCTTTGCTACAAT

SEQ ID NO: 59 is a nucleic acid sequence of terminator tRPL3.sm
GAAGTTTTTAAAGCATTTTTTAGACACTTCTCATTTTTCTAAGTTTTTTTAAAATAGTTTTATGTATTTACTACG
TATCACAATTTGAAATAATTCATCTTCCCAAAAAACTAAGATTTTATCCTTGTCACGATCCGTAACCAGTTTAT
AATATTTTAGAGCTTATACACGTACGTATACACACGTGTCGGTACATGAGAATTACGTTCAAAATTATTCACTTT
TTTTTTCTCTGCCGTTTTACTTTTGAACTCTGTCTCGCTATTTCCTTACAATCTTCGCTACAATACCACTTGCCC
TTGG

SEQ ID NO: 60 is a nucleic acid sequence of terminator tRPL3.sba
GAAGTTTTCTGACAAAAACATAACGTTTTTTCCAATCATTTCTTATTTTTCCGGTTTATTTAAATAGTTTTTATG
TACTATTATACGTATGACTATTTAACTTAAATTCTTCCTCCCAAGAAATCTCCCAAGTTTTTCATTATCATGGCA
TACACCACTATCAGTTACAAAATGGTAGCTCAACCATATATATATCTCTATATACACATATAAATGCAAACAGGT
CCAAGTCACCGCTCACTGCAGTTTCTTTTGCCGTTTTGACTTCGATCTCTGCTTGGCTATTTTCTCACAATCCT

SEQ ID NO: 61 is a nucleic acid sequence of terminator tRPL41B
GCGGATTGAGAGCAAATCGTTAAGTTCAGGTCAAGTAAAAATTGATTTCGAAAACTAATTTCTCTTATACAATCC
TTTGATTGGACCGTCATCCTTTCGAATATAAGATTTTGTTAAGAATATTTAGACAGAGATCTACTTTATATTTA
ATATCTAGATATTACATAATTTCCTCTCTAATAAAATATCATTAATAAAATAAAAATGAAGCGATTTGATTTTGT
GTTGTCAACTTAGTTTGCCGCTATGCCTCTTGGGTAATGCTATTATTGAATCGAAGGGCTTTATTATATTACCCT

SEQ ID NO: 62 is a nucleic acid sequence of terminator tRPL15A
GCTGGTTGATGGAAAATATAATTTTATTGGGCAAACTTTTGTTTATCTGATGTGTTTTATACTATTATCTTTTTA
ATTAATGATTCTATATACAAACCTGTATATTTTTCTTTAACCAATTTTTTTTTTATAGACCTAGAGCTGTACT
TTTATTCTGCTATCAAGCAAACCCCTACCCCCTCTTCTCAATCCTCCCCTCAGGCAGAACTTATCTACCTGTATC
AAGGAGCGGACGAGGGAGTCCTAATTGTTCTACGTATACCAATGCTAGCAGCTTACATAGGTGGTGGCACTACCA

SEQ ID NO: 63 is a nucleic acid sequence of terminator tRPL15A.sba
GCTGGCTGATGAAAAATATAGTTCTGTTGGGCAAGCATTTGTTTACCTAGCATCTCTTTTATACTATTATTATCT
TTATATTTGATGATTTTATATACAAGTTGTATACCTTTTCTTTAACCAATTTTTTTTTTCTAATGGTGCACCTA
GAAGTACATTTTTTCTCACCAATAGATAGTCAAGATACTCCCAGCCTCTATGGCGTTACCACGGAGCCGACAAGG
GAAAGCTGCTTATCTTACATATGCAGATGCCAAGGCCCGTTACAGGCCGCCTTATTGATGTTTAGAAATAGCTTC

SEQ ID NO: 64 is a nucleic acid sequence of terminator tIDP1
TCGAATTTACGTAGCCCAATCTACCACTTTTTTTTTTCATTTTTTAAAGTGTTATACTTAGTTATGCTCTAGGAT
AATGAACTACTTTTTTTTTTTTTTTTTTACTGTTATCATAAATATATATACCTTATTGTTGTTTGCAACCGTCGG
TTAATTCCTTATCAAGGTTCCCCAAGTTCGGATCATTACCATCAATTTCCAACATTTTCATGAGTTCTTCTTCTT
CATTACCGTGTTTTAGGGGGCTGTTCGCACTTCTAATAGGGCTATCACCAAGCTGTTCTAATTCGTCCAAAAGTT

SEQ ID NO: 65 is a nucleic acid sequence of terminator tTEF1.sba
GGAGATTGATAGGACTTTTCTAGTTGCATATCTTTTATTTTTAAATCTTATCTATTAGTTAATTTTTTGTAATTT
ATCCTTATATATATAGTTTGGTTATTCTAAAACATCATTTCAGTATCTAAAACCTCTCTTATTCATTACCTTTTT
ATTTAATGGTTTTTGCTACAGGCAAAAATTTAATGGTTTTTGCTACAGGCAAAAATCCCGCCGTGGACTTATTCC
ACGTTAACTCGGTTACAGGGTCATGAACCATTTTGTCAATTATCGAAATAACTTCTTCAAAAGTCCCTCTTACTT

**SEQ ID NO: 66** is a nucleic acid sequence of the secretion sequence added in 5' ATGCAATTTAGCACAGTCGCATCAGTAGCCTTCGTTGCCTTGGCCAACTTCGTGGCAGCA
**SEQ ID NO: 67** is an amino acid sequence of the secretion sequence added in N-ter MQFSTVASVAFVALANFVAA 
SEQ ID NO: 68 is a nucleic acid sequence of the anchoring sequence added in
3'
GGATCCGCCATTTCTCAAATCACTGACGGTCAAATCCAAGCTACTACCACTGCTACCACCGAAGCTACCACCACT
GCTGCCCCATCTTCCACCGTTGAAACTGTTTCTCCATCCAGCACCGAAACTATCTCTCAACAAACTGAAAATGGT
GCTGCTAAGGCCGCTGTCGGTATGGGTGCCGGTGCTCTAGCTGCTGCTGCTATGTTGTTATAA
**SEQ ID NO: 69** is an amino acid sequence of the anchoring sequence added in C-ter AISQITDGQIQATTTATTEATTTAAPSSTVETVSPSSTETISQQTENGAAKAAVGMGAGALAAAAMLL

## Claims

1. A recombinant yeast cell producing heparosan wherein the recombinant cell comprises:
(a) one or more recombinant nucleic acids encoding a polypeptide having a heparosan synthase (HSS) activity; and
(b) one or more recombinant nucleic acids encoding a polypeptide having a UDP-Glucose dehydrogenase (UDP-GlcDH or HASB) activity.

2. The recombinant cell according to claim 1 wherein the recombinant cell comprises a nucleic acid encoding a polypeptide having a glucuronosyl-disulfoglucosamine glucuronidase activity, wherein the polypeptide having glucuronosyl-disulfoglucosamine glucuronidase activity comprises a secretion signal and optionaly an anchoring signal.

3. A recombinant host cell producing heparosan wherein the recombinant cell comprises:
(a) one or more recombinant nucleic acids encoding a polypeptide having a heparosan synthase (HSS) activity;
(b) one or more recombinant nucleic acids encoding a polypeptide having a UDP-Glucose dehydrogenase (UDP-GlcDH or HASB) activity; and
(c) one or more recombinant nucleic acids encoding a polypeptide having a glucuronosyl-disulfoglucosamine glucuronidase activity,
wherein the polypeptide having a glucuronosyl-disulfoglucosamine glucuronidase activity comprises a secretion signal, and optionaly an anchoring signal, so that heparosan, in particular of a desired molecular weight is produced by the host cell.

4. The recombinant cell according to claim 2 or 3, wherein the nucleic acid encoding a polypeptide having glucuronosyl-disulfoglucosamine glucuronidase activity is obtained or derived from *Pedobacter heparinus.*

5. The recombinant cell according to any one of claims 2 to 4, wherein the molecular weight of the heparosan is in the range of less than 50kDa, preferably is in the range of about 20kDa to about 50kDa.

6. The recombinant cell according to any one of claims 2 to 4, wherein the molecular weight of the heparosan is in the range of greater than 50kDa, preferably in the range of about 50kDa to about 250kD.

7. The recombinant cell according to any one of claims 2 to 4 wherein the molecular weight of the heparosan is in the range of greater than 100kDa, preferably is in the range of about 100kDa to about 1500kDa.

8. The recombinant cell according to any one of claims 1 to 7, wherein the nucleic acid encoding a polypeptide having a UDP-Glucose dehydrogenase (UDP-GlcDH or HASB) activity is obtained or derived from at least one of *Arabidopsis thaliana, Chlorella virus PBCV1* or *Streptococcus zooepidemicus,* and is in particular from *Arabidopsis thaliana* or *Chlorella virus PBCV1.*

9. The recombinant cell according to any one of claims 1 to 8, wherein the nucleic acid encoding a polypeptide having a heparosan synthase (HSS) activity is obtained or derived from *Pasteurella multocida.*

10. The recombinant cell according to any one of claims 1 to 9, wherein the recombinant cell further comprises at least one recombinant nucleic acid encoding one or more of:
(i) a polypeptide having a glutamine-fructose-6-phosphate amidotransferase (GFA1) activity; and/or
(ii) a polypeptide having a UDP-N-acetylglucosamine pyrophosphorylase (QRI1) activity.

11. The recombinant cell according to any one of claims 1 to 10, wherein the recombinant cell further comprises at least one recombinant nucleic acid encoding one or more of:
(i) a polypeptide having a Phosphoglucomutase-1 (PGM1) activity; and/or
(ii) a polypeptide having a UTP-glucose-1-phosphate uridylyltransferase (UGP1) activity; and/or
(iii) a polypeptide having a Glucosamine-6-phosphate N-acetyltransferase (GNA1) activity; and/or
(iv) a polypeptide having a phosphoacetylglucosamine mutase (PCM1) activity.

12. The recombinant cell according to claim 10 or 11, wherein:
- the nucleic acid encoding the polypeptide having glutamine-fructose-6-phosphate amidotransferase (GFA1) activity; and/or
- the nucleic acid encoding the polypeptide having UDP-N-acetylglucosamine pyrophosphorylase (QRI1) activity; and/or
- the nucleic acid encoding the polypeptide having Phosphoglucomutase-1 (PGM1) activity; and/or
- the nucleic acid encoding the polypeptide having UTP-glucose-1-phosphate uridylyltransferase (UGP1) activity; and/or
- the nucleic acid encoding the polypeptide having Glucosamine-6-phosphate N-acetyltransferase (GNA1) activity; and/or
- the nucleic acid encoding the polypeptide having phosphoacetylglucosamine mutase (PCM1);
are obtained or derived from *Saccharomyces cerevisiae.*

13. The recombinant host cell according to any one of claims 3 to 12, wherein the recombinant host cell is a yeast.

14. The recombinant cell according to any one of claims 1 to 13, wherein the recombinant cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus, and in particular belongs to the *Saccharomyces* genus.

15. The recombinant cell according to claim 14, wherein the recombinant cell is selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

16. A method of producing heparosan comprising:
(a) cultivating a recombinant cell as defined in any one of claims 1 to 15 in a cultivation medium for a time sufficient to produce heparosan; and
(b) optionally isolating or recovering the heparosan from the recombinant cell and/or from the cultivation medium.

17. The method according to claim 16, wherein the heparosan has a molecular weight of from about 20kDa to about 50kDa.

18. The method according to claim 16, wherein the heparosan has a molecular weight of from about 50kDa to about 150kDa.

19. The method according to claim 14, wherein the heparosan has a molecular weight of from about 150kDa to about 1500kDa.

20. The method according to any one of claims 16 to 19, wherein the recombinant cell is a yeast belonging to the *Saccharomyces* genus, and in particular is *Saccharomyces cerevisiae.*

21. The method according to any one of claims 16 to 20, wherein the time sufficient to produce heparosan is a period of from about 35 to about 50 hours, preferably from about 40 to about 50 hours, preferably of about 48 hours.

22. The method according to any one of claims 16 to 21, wherein the molecular weight of the produced heparosan is controlled by regulating the pH of the cultivation medium during step (a).

23. The method according to any one of claims 16 to 22, wherein the method is carried out on an industrial scale, preferably where the cultivation medium is at least about 100L, more preferably is in the range of about 1000L to about 3000L, even more preferably is about 10,000L or even more preferably is about 100,000L, or even is about 250,000L.

24. Heparosan obtainable from a recombinant cell according to any one of claims 1 to 15 or from the method according to any one of claims 16 to 23.

25. A cultivation medium comprising the heparosan according to claim 24.

26. A composition comprising the heparosan according to claim 24.

27. An industrial product or a consumer product or a consumable comprising (i) the heparosan according to claim 24 having a molecular weight as defined in any one of claims 5 to 7, (ii) the cultivation medium of claim 25 or (iii) the composition of claim 26.

28. The industrial product or the consumer product or the consumable of claim 27, wherein the industrial product or the consumer product or the consumable is a cosmetic product, a flavour product, a fragrance product, a food product, a food, a beverage, a texturant, a pharmaceutical composition, a dietary supplement, a nutraceutical, a cleaning product, a dental and/or an oral hygiene composition.

29. Use of a recombinant cell as defined in any one of claims 1 to 15 for the production of heparosan having a molecular weight in the range of from about 20kDa to about 50kDa or from about 50kDa to about 1000kDa.
